# EUROPEAN PATENT SPECIFICATION

(11) **EP 2 401 378 B1**
(45) Date of publication and mention of the grant of the patent: **14.08.2013**
(21) Application number: 10706438.8
(22) Date of filing: 24.02.2010
(51) Int. Cl.: C12N 15/81, C12N 15/54, C12N 15/61, C12N 9/12, C12N 9/90, C12N 1/16, C12P 21/00, C07K 16/18

(54) **METABOLIC ENGINEERING OF A GALACTOSE ASSIMILATION PATHWAY IN THE GLYCOENGINEERED YEAST PICHIA PASTORIS**
STOFFWECHSELMANIPULATION EINES GALAKTOSEASSIMILATIONSWEGS BEI DER GLYCOENGINEERTEN HEFE PICHIA PASTORIS
INGÉNIERIE MÉTABOLIQUE D'UNE VOIE D'ASSIMILATION DU GALACTOSE DANS LA LEVURE GLYCOSYNTHÉTISÉE PICHIA PASTORIS

(30) Priority: 25.02.2009 US 208582 P
(43) Date of publication of application: 04.01.2012
(60) Divisional application: 12188235.1
(73) Proprietor: Merck Sharp & Dohme Corp., Rahway, NJ 07065-0907 (US)
(72) Inventor: DAVIDSON, Robert, C., Rahway New Jersey 07065-0907 (US); BOBROWICZ, Piotr, Rahway New Jersey 07065-0907 (US); ZHA, Dongxing, Rahway New Jersey 07065-0907 (US)
(74) Representative: Horgan, James Michael Frederic
(86) International application number: PCT/US2010/025163
(87) International publication number: WO 2010/099153

(56) References cited:
- WO-A1-2006/071280
- WO-A2-2004/074499
- WO-A2-2006/014683
- US-A1- 2005 106 664
- US-A1- 2006 040 353
- ROUTIER F H ET AL: "The glycosylation pattern of a humanized IgGI antibody (D1.3) expressed in CHO cells" GLYCOCONJUGATE JOURNAL, CHAPMAN & HALL, GB LNKD- DOI:10.1023/A:1018589704981, vol. 14, no. 2, 1 January 1997 (1997-01-01), pages 201-207, XP002375244 ISSN: 0282-0080
- BOBROWICZ P ET AL: "Engineering of an artificial glycosylation pathway blocked in core oligosaccharide assembly in the yeast Pichia pastoris: production of complex humanized glycoproteins with terminal galactose" GLYCOBIOLOGY, OXFORD UNIVERSITY PRESS, US LNKD- DOI:10.1093/GLYCOB/CWH104, vol. 14, no. 9, 9 June 2004 (2004-06-09), pages 757-766, XP002354412 ISSN: 0959-6658
- LI HUIJUAN ET AL: "Optimization of humanized IgGs in glycoengineered Pichia pastoris" NATURE BIOTECHNOLOGY, NATURE PUBLISHING GROUP, NEW YORK, NY, US LNKD- DOI:10.1038/NBT1178, vol. 24, no. 2, 1 February 2006 (2006-02-01), pages 210-215, XP002493048 ISSN: 1087-0156 cited in the application
- HAMILTON ET AL: "Glycosylation engineering in yeast: the advent of fully humanized yeast" CURRENT OPINION IN BIOTECHNOLOGY, LONDON, GB LNKD- DOI:10.1016/J.COPBIO.2007.09.001, vol. 18, no. 5, 24 October 2007 (2007-10-24), pages 387-392, XP022350908 ISSN: 0958-1669

### SEQUENCE LISTING

<110> DAVIDSON, Robert C. BOBROWICZ, Piotr
<120> METABOLIC ENGINEERING OF A GALACTOSE
   ASSIMILATION PATHWAY IN THE GLYCOENGINEERED YEAST PICHIA
PASTORIS
<130> GFI-MIS-00006
<150> 61/208,582
   <151> 2009-02-25
<160> 70
<170> FastSEQ for Windows Version 4.0
<210> 1
   <211> 27
   <212> DNA
   <213> Artificial Sequence
<220>
   <223> PCR primer RCD192
<400> 1
   gccgcgacct gagccgcctg ccccaac 27
<210> 2
   <211> 27
   <212> DNA
   <213> Artificial Sequence
<220>
   <223> PCR primer RCD186
<400> 2
   ctagctcggt gtcccgatgt ccactgt 27
<210> 3
   <211> 36
   <212> DNA
   <213> Artificial Sequence
<220>
   <223> PCR primer RCD198
<400> 3
   cttaggcgcg ccggccgcga cctgagccgc ctgccc 36
<210> 4
   <211> 21
   <212> DNA
   <213> Artificial Sequence
<220>
   <223> PCR primer RCD201
<400> 4
   ggggcatatc tgccgcccat c 21
<210> 5
   <211> 21
   <212> DNA
   <213> Artificial Sequence
<220>
   <223> PCR primer RCD200
<400> 5
   gatgggcggc agatatgccc c 21
<210> 6
   <211> 36
   <212> DNA
   <213> Artificial Sequence
<220>
   <223> PCR primer RCD199
<400> 6
   cttcttaatt aactagctcg gtgtcccgat gtccac 36
<210> 7
   <211> 49
   <212> DNA
   <213> Artificial Sequence
<220>
   <223> PCR primer DmUGT-5'
<400> 7
   ggctcgagcg gccgccacca tgaatagcat acacatgaac gccaatacg 49
<210> 8
   <211> 47
   <212> DNA
   <213> Artificial Sequence
<220>
   <223> PCR primer DmUGT-3'
<400> 8
   ccctcgagtt aattaactag acgcgcggca gcagcttctc ctcatcg 47
<210> 9
   <211> 21
   <212> DNA
   <213> Artificial Sequence
<220>
   <223> PCR primer GALE2-L
<400> 9
   atgactggtg ttcatgaagg g 21
<210> 10
   <211> 21
   <212> DNA
   <213> Artificial Sequence
<220>
   <223> PCR primer GALE2-R
<400> 10
   ttacttatat gtcttggtat g 21
<210> 11
   <211> 94
   <212> DNA
   <213> Artificial Sequence
<220>
   <223> PCR primer GD1
<400> 11
   gcggccgcat gactggtgtt catgaaggga ctgtgttggt tactggcggc gctggttata 60
   taggttctca tacgtgcgtt gttttgttag aaaa 94
<210> 12
   <211> 29
   <212> DNA
   <213> Artificial Sequence
<220>
   <223> PCR primer GD2
<400> 12
   ttaattaatt acttatatgt cttggtatg 29
<210> 13
   <211> 33
   <212> DNA
   <213> Artificial Sequence
<220>
   <223> PCR primer PB158
<400> 13
   ttagcggccg caggaatgac taaatctcat tca 33
<210> 14
   <211> 33
   <212> DNA
   <213> artficial
<220>
   <223> PCR primer PB159
<400> 14
   aacttaatta agcttataat tcatatagac agc 33
<210> 15
   <211> 11
   <212> DNA
   <213> Artificial Sequence
<220>
   <223> PCR primer PB156
<400> 15
   ttagcggccg c 11
<210> 16
   <211> 11
   <212> DNA
   <213> Artificial Sequence
<220>
   <223> PCR primer PB157
<400> 16
   aacttaatta a 11
<210> 17
   <211> 32
   <212> DNA
   <213> Artificial Sequence
<220>
   <223> PCR primer PB160
<400> 17
   ttagcggccg caggaatgac tgctgaagaa tt 32
<210> 18
   <211> 34
   <212> DNA
   <213> Artificial Sequence
<220>
   <223> PCR primer PB161
<400> 18
   aacttaatta agcttacagt ctttgtagat aatc 34
<210> 19
   <211> 108
   <212> DNA
   <213> Artificial Sequence
<220>
   <223> DNA encodes Mnn2 leader (53)
<400> 19
   atgctgctta ccaaaaggtt ttcaaagctg ttcaagctga cgttcatagt tttgatattg 60
   tgcgggctgt tcgtcattac aaacaaatac atggatgaga acacgtcg             108
<210> 20
   <211> 36
   <212> PRT
   <213> Artificial Sequence
<220>
   <223> Mnn2 leader (53)
<400> 20
   Met Leu Leu Thr Lys Arg Phe Ser Lys Leu Phe Lys Leu Thr Phe Ile
    1               5                  10                  15
   Val Leu Ile Leu Cys Gly Leu Phe Val Ile Thr Asn Lys Tyr Met Asp
               20                  25                  30
   Glu Asn Thr Ser
                                   35
<210> 21
   <211> 300
   <212> DNA
   <213> Artificial Sequence
<220>
   <223> DNA encodes Mnn2 leader (54)
<400> 21
   atgctgctta ccaaaaggtt ttcaaagctg ttcaagctga cgttcatagt tttgatattg 60
   tgcgggctgt tcgtcattac aaacaaatac atggatgaga acacgtcggt caaggagtac 120
   aaggagtact tagacagata tgtccagagt tactccaata agtattcatc ttcctcagac 180
   gccgccagcg ctgacgattc aaccccattg agggacaatg atgaggcagg caatgaaaag 240
   ttgaaaagct tctacaacaa cgttttcaac tttctaatgg ttgattcgcc cgggcgcgcc 300
<210> 22
   <211> 100
   <212> PRT
   <213> Artificial Sequence
<220>
   <223> Mnn2 leader (54)
<400> 22
   Met Leu Leu Thr Lys Arg Phe Ser Lys Leu Phe Lys Leu Thr Phe Ile
    1               5                  10                  15
   Val Leu Ile Leu Cys Gly Leu Phe Val Ile Thr Asn Lys Tyr Met Asp
               20                  25                  30
   Glu Asn Thr Ser Val Lys Glu Tyr Lys Glu Tyr Leu Asp Arg Tyr Val
           35                  40                  45
   Gln Ser Tyr Ser Asn Lys Tyr Ser Ser Ser Ser Asp Ala Ala Ser Ala
       50                  55                  60
   Asp Asp Ser Thr Pro Leu Arg Asp Asn Asp Glu Ala Gly Asn Glu Lys
   65                  70                  75                  80
   Leu Lys Ser Phe Tyr Asn Asn Val Phe Asn Phe Leu Met Val Asp Ser
                   85                  90                  95
   Pro Gly Arg Ala
               100
<210> 23
   <211> 57
   <212> DNA
   <213> Artificial Sequence
<220>
   <223> DNA encodes S. cerevisiae Mating Factor pre signal sequence
<400> 23
   atgagattcc catccatctt cactgctgtt ttgttcgctg cttcttctgc tttggct 57
<210> 24
   <211> 19
   <212> PRT
   <213> Artificial Sequence
<220>
   <223> S. cerevisiae Mating Factor pre signal sequence
<400> 24
   Met Arg Phe Pro Ser Ile Phe Thr Ala Val Leu Phe Ala Ala Ser Ser
    1               5                  10                  15
   Ala Leu Ala
<210> 25
   <211> 60
   <212> DNA
   <213> Artificial Sequence
<220>
   <223> DNA encodes alpha amylase signal sequence (from Aspergillus niger -amylase)
<400> 25
   atggttgctt ggtggtcctt gttcttgtac ggattgcaag ttgctgctcc agctttggct 60
<210> 26
   <211> 20
   <212> PRT
   <213> Artificial Sequence
<220>
   <223> alpha amylase signal sequence (from Aspergillus niger -amylase)
<400> 26
   Met Val Ala Trp Trp Ser Leu Phe Leu Tyr Gly Leu Gln Val Ala Ala
    1               5                  10                  15
   Pro Ala Leu Ala
               20
<210> 27
   <211> 99
   <212> DNA
   <213> Artificial Sequence
<220>
   <223> DNA encodes Pp SEC12 (10) leader
<400> 27
   atgcccagaa aaatatttaa ctacttcatt ttgactgtat tcatggcaat tcttgctatt 60
   gttttacaat ggtctataga gaatggacat gggcgcgcc                        99
<210> 28
   <211> 33
   <212> PRT
   <213> Artificial Sequence
<220>
   <223> Pp SEC12 (10) leader
<400> 28
   Met Pro Arg Lys Ile Phe Asn Tyr Phe Ile Leu Thr Val Phe Met Ala
    1               5                  10                  15
   Ile Leu Ala Ile Val Leu Gln Trp Ser Ile Glu Asn Gly His Gly Arg
               20                  25                  30
   Ala
<210> 29
   <211> 183
   <212> DNA
   <213> Artificial Sequence
<220>
   <223> DNA encodes ScMnt1 (Kre2) (33) leader
<400> 29
   atggccctct ttctcagtaa gagactgttg agatttaccg tcattgcagg tgcggttatt 60
   gttctcctcc taacattgaa ttccaacagt agaactcagc aatatattcc gagttccatc 120
   tccgctgcat ttgattttac ctcaggatct atatcccctg aacaacaagt catcgggcgc 180
   gcc                                                               183
<210> 30
   <211> 61
   <212> PRT
   <213> Artificial Sequence
<220>
   <223> ScMnt1 (Kre2) (33) leader
<400> 30
   Met Ala Leu Phe Leu Ser Lys Arg Leu Leu Arg Phe Thr Val Ile Ala
   1               5                   10                  15
   Gly Ala Val Ile Val Leu Leu Leu Thr Leu Asn Ser Asn Ser Arg Thr
               20                  25                  30
   Gln Gln Tyr Ile Pro Ser Ser Ile Ser Ala Ala Phe Asp Phe Thr Ser
           35                  40                  45
   Gly Ser Ile Ser Pro Glu Gln Gln Val Ile Gly Arg Ala
       50                  55                  60
<210> 31
   <211> 318
   <212> DNA
   <213> Artificial Sequence
<220>
   <223> DNA encodes ScSEC12 (8) leader
<400> 31
   atgaacacta tccacataat aaaattaccg cttaactacg ccaactacac ctcaatgaaa 60
   caaaaaatct ctaaattttt caccaacttc atccttattg tgctgctttc ttacatttta 120
   cagttctcct ataagcacaa tttgcattcc atgcttttca attacgcgaa ggacaatttt 180
   ctaacgaaaa gagacaccat ctcttcgccc tacgtagttg atgaagactt acatcaaaca 240
   actttgtttg gcaaccacgg tacaaaaaca tctgtaccta gcgtagattc cataaaagtg 300
   catggcgtgg ggcgcgcc                                               318
<210> 32
   <211> 106
   <212> PRT
   <213> Artificial Sequence
<220>
   <223> ScSEC12 (8) leader
<400> 32
   Met Asn Thr Ile His Ile Ile Lys Leu Pro Leu Asn Tyr Ala Asn Tyr
   1               5                   10                  15
   Thr Ser Met Lys Gln Lys Ile Ser Lys Phe Phe Thr Asn Phe Ile Leu
               20                  25                  30
   Ile Val Leu Leu Ser Tyr Ile Leu Gln Phe Ser Tyr Lys His Asn Leu
           35                  40                  45
   His Ser Met Leu Phe Asn Tyr Ala Lys Asp Asn Phe Leu Thr Lys Arg
       50                  55                  60
   Asp Thr Ile Ser Ser Pro Tyr Val Val Asp Glu Asp Leu His Gln Thr
   65                  70                  75                  80
   Thr Leu Phe Gly Asn His Gly Thr Lys Thr Ser Val Pro Ser Val Asp
                   85                  90                  95
   Ser Ile Lys Val His Gly Val Gly Arg Ala
               100                 105
<210> 33
   <211> 981
   <212> DNA
   <213> Mus musculus
<220>
   <221> CDS
   <222> (1)...(981)
   <223> MmSLC35A3 UDP-GlcNAc transporter
<400> 33
   atg tct gcc aac cta aaa tat ctt tcc ttg gga att ttg gtg ttt cag      48
   Met Ser Ala Asn Leu Lys Tyr Leu Ser Leu Gly Ile Leu Val Phe Gln
   1               5                   10                  15
   act acc agt ctg gtt cta acg atg cgg tat tct agg act tta aaa gag      96
   Thr Thr Ser Leu Val Leu Thr Met Arg Tyr Ser Arg Thr Leu Lys Glu
               20                  25                  30
   gag ggg cct cgt tat ctg tct tct aca gca gtg gtt gtg gct gaa ttt     144
   Glu Gly Pro Arg Tyr Leu Ser Ser Thr Ala Val Val Val Ala Glu Phe
           35                  40                  45
   ttg aag ata atg gcc tgc atc ttt tta gtc tac aaa gac agt aag tgt     192
   Leu Lys Ile Met Ala Cys Ile Phe Leu Val Tyr Lys Asp Ser Lys Cys
       50                  55                  60
   agt gtg aga gca ctg aat aga gta ctg cat gat gaa att ctt aat aag     240
   Ser Val Arg Ala Leu Asn Arg Val Leu His Asp Glu Ile Leu Asn Lys
   65                  70                  75                  80
   ccc atg gaa acc ctg aag ctc gct atc ccg tca ggg ata tat act ctt     288
   Pro Met Glu Thr Leu Lys Leu Ala Ile Pro Ser Gly Ile Tyr Thr Leu
                   85                  90                  95
   cag aac aac tta ctc tat gtg gca ctg tca aac cta gat gca gcc act     336
   Gln Asn Asn Leu Leu Tyr Val Ala Leu Ser Asn Leu Asp Ala Ala Thr
               100                 105                 110
   tac cag gtt aca tat cag ttg aaa ata ctt aca aca gca tta ttt tct     384
   Tyr Gln Val Thr Tyr Gln Leu Lys Ile Leu Thr Thr Ala Leu Phe Ser
           115                 120                 125
   gtg tct atg ctt ggt aaa aaa tta ggt gtg tac cag tgg ctc tcc cta      432
   Val Ser Met Leu Gly Lys Lys Leu Gly Val Tyr Gln Trp Leu Ser Leu
       130                 135                 140
   gta att ctg atg gca gga gtt gct ttt gta cag tgg cct tca gat tct      480
   Val Ile Leu Met Ala Gly Val Ala Phe Val Gln Trp Pro Ser Asp Ser
   145                 150                 155                 160
   caa gag ctg aac tct aag gac ctt tca aca ggc tca cag ttt gta ggc      528
   Gln Glu Leu Asn Ser Lys Asp Leu Ser Thr Gly Ser Gln Phe Val Gly
                   165                 170                 175
   ctc atg gca gtt ctc aca gcc tgt ttt tca agt ggc ttt gct gga gtt      576
   Leu Met Ala Val Leu Thr Ala Cys Phe Ser Ser Gly Phe Ala Gly Val
               180                 185                 190
   tat ttt gag aaa atc tta aaa gaa aca aaa cag tca gta tgg ata agg      624
   Tyr Phe Glu Lys Ile Leu Lys Glu Thr Lys Gln Ser Val Trp Ile Arg
           195                 200                 205
   aac att caa ctt ggt ttc ttt gga agt ata ttt gga tta atg ggt gta      672
   Asn Ile Gln Leu Gly Phe Phe Gly Ser Ile Phe Gly Leu Met Gly Val
       210                 215                 220
   tac gtt tat gat gga gaa ttg gtc tca aag aat gga ttt ttt cag gga      720
   Tyr Val Tyr Asp Gly Glu Leu Val Ser Lys Asn Gly Phe Phe Gln Gly
   225                 230                 235                 240
   tat aat caa ctg acg tgg ata gtt gtt gct ctg cag gca ctt gga ggc      768
   Tyr Asn Gln Leu Thr Trp Ile Val Val Ala Leu Gln Ala Leu Gly Gly
                   245                 250                 255
   ctt gta ata gct gct gtc atc aaa tat gca gat aac att tta aaa gga      816
   Leu Val Ile Ala Ala Val Ile Lys Tyr Ala Asp Asn Ile Leu Lys Gly
               260                 265                 270
   ttt gcg acc tcc tta tcc ata ata ttg tca aca ata ata tct tat ttt      864
   Phe Ala Thr Ser Leu Ser Ile Ile Leu Ser Thr Ile Ile Ser Tyr Phe
           275                 280                 285
   tgg ttg caa gat ttt gtg cca acc agt gtc ttt ttc ctt gga gcc atc      912
   Trp Leu Gln Asp Phe Val Pro Thr Ser Val Phe Phe Leu Gly Ala Ile
       290                 295                 300
   ctt gta ata gca gct act ttc ttg tat ggt tac gat ccc aaa cct gca      960
   Leu Val Ile Ala Ala Thr Phe Leu Tyr Gly Tyr Asp Pro Lys Pro Ala
   305                 310                 315                 320
   gga aat ccc act aaa gca tag                                          981
   Gly Asn Pro Thr Lys Ala  *
                   325
<210> 34
   <211> 326
   <212> PRT
   <213> Mus musculus
<400> 34
   Met Ser Ala Asn Leu Lys Tyr Leu Ser Leu Gly Ile Leu Val Phe Gln
    1               5                  10                  15
   Thr Thr Ser Leu Val Leu Thr Met Arg Tyr Ser Arg Thr Leu Lys Glu
               20                  25                  30
   Glu Gly Pro Arg Tyr Leu Ser Ser Thr Ala Val Val Val Ala Glu Phe
           35                  40                  45
   Leu Lys Ile Met Ala Cys Ile Phe Leu Val Tyr Lys Asp Ser Lys Cys
       50                  55                  60
   Ser Val Arg Ala Leu Asn Arg Val Leu His Asp Glu Ile Leu Asn Lys
   65                  70                  75                  80
   Pro Met Glu Thr Leu Lys Leu Ala Ile Pro Ser Gly Ile Tyr Thr Leu
                   85                  90                  95
   Gln Asn Asn Leu Leu Tyr Val Ala Leu Ser Asn Leu Asp Ala Ala Thr
               100                 105                 110
   Tyr Gln Val Thr Tyr Gln Leu Lys Ile Leu Thr Thr Ala Leu Phe Ser
           115                 120                 125
   Val Ser Met Leu Gly Lys Lys Leu Gly Val Tyr Gln Trp Leu Ser Leu
       130                 135                 140
   Val Ile Leu Met Ala Gly Val Ala Phe Val Gln Trp Pro Ser Asp Ser
   145                 150                 155                 160
   Gln Glu Leu Asn Ser Lys Asp Leu Ser Thr Gly Ser Gln Phe Val Gly
                   165                 170                 175
   Leu Met Ala Val Leu Thr Ala Cys Phe Ser Ser Gly Phe Ala Gly Val
               180                 185                 190
   Tyr Phe Glu Lys Ile Leu Lys Glu Thr Lys Gln Ser Val Trp Ile Arg
           195                 200                 205
   Asn Ile Gln Leu Gly Phe Phe Gly Ser Ile Phe Gly Leu Met Gly Val
       210                 215                 220
   Tyr Val Tyr Asp Gly Glu Leu Val Ser Lys Asn Gly Phe Phe Gln Gly
   225                 230                 235                 240
   Tyr Asn Gln Leu Thr Trp Ile Val Val Ala Leu Gln Ala Leu Gly Gly
                   245                 250                 255
   Leu Val Ile Ala Ala Val Ile Lys Tyr Ala Asp Asn Ile Leu Lys Gly
               260                 265                 270
   Phe Ala Thr Ser Leu Ser Ile Ile Leu Ser Thr Ile Ile Ser Tyr Phe
           275                 280                 285
   Trp Leu Gln Asp Phe Val Pro Thr Ser Val Phe Phe Leu Gly Ala Ile
       290                 295                 300
   Leu Val Ile Ala Ala Thr Phe Leu Tyr Gly Tyr Asp Pro Lys Pro Ala
   305                 310                 315                 320
   Gly Asn Pro Thr Lys Ala
                   325
<210> 35
   <211> 1068
   <212> DNA
   <213> Saccharomyces pombe
<220>
   <221> CDS
   <222> (1)...(1068)
   <223> DNA encodes SpGALE
<400> 35
   atg act ggt gtt cat gaa ggg act gtg ttg gtt act ggc ggc gct ggt   48
   Met Thr Gly Val His Glu Gly Thr Val Leu Val Thr Gly Gly Ala Gly
    1               5                  10                  15
   tat ata ggt tct cat acg tgc gtt gtt ttg tta gaa aaa gga tat gat   96
   Tyr Ile Gly Ser His Thr Cys Val Val Leu Leu Glu Lys Gly Tyr Asp
                    20                       25                       30
   gtt gta att gtc gat aat tta tgc aat tct cgc gtt gaa gcc gtg cac      144
   Val Val Ile Val Asp Asn Leu Cys Asn Ser Arg Val Glu Ala Val His
               35                      40                      45
   cgc att gaa aaa ctc act ggg aaa aaa gtc ata ttc cac cag gtg gat      192
   Arg Ile Glu Lys Leu Thr Gly Lys Lys Val Ile Phe His Gln Val Asp
           50                      55                      60
   ttg ctt gat gag cca gct ttg gac aag gtc ttc gca aat caa aac ata      240
   Leu Leu Asp Glu Pro Ala Leu Asp Lys Val Phe Ala Asn Gln Asn Ile
   65                      70                      75                  80
   tct gct gtc att cat ttt gct ggt ctc aaa gca gtt ggt gaa tct gta      288
   Ser Ala Val Ile His Phe Ala Gly Leu Lys Ala Val Gly Glu Ser Val
                   85                      90                      95
   cag gtt cct ttg agt tat tac aaa aat aac att tcc ggt acc att aat      336
   Gln Val Pro Leu Ser Tyr Tyr Lys Asn Asn Ile Ser Gly Thr Ile Asn
               100                     105                     110
   tta ata gag tgc atg aag aag tat aat gta cgt gac ttc gtc ttt tct      384
   Leu Ile Glu Cys Met Lys Lys Tyr Asn Val Arg Asp Phe Val Phe Ser
           115                     120                     125
   tca tct gct acc gtg tat ggc gat cct act aga cct ggt ggt acc att      432
   Ser Ser Ala Thr Val Tyr Gly Asp Pro Thr Arg Pro Gly Gly Thr Ile
       130                     135                     140
   cct att cca gag tca tgc cct cgt gaa ggt aca agc cca tat ggt cgc      480
   Pro Ile Pro Glu Ser Cys Pro Arg Glu Gly Thr Ser Pro Tyr Gly Arg
   145                     150                     155                 160
   aca aag ctt ttc att gaa aat atc att gag gat gag acc aag gtg aac      528
   Thr Lys Leu Phe Ile Glu Asn Ile Ile Glu Asp Glu Thr Lys Val Asn
                   165                     170                     175
   aaa tcg ctt aat gca gct tta tta cgc tat ttt aat ccc gga ggt gct      576
   Lys Ser Leu Asn Ala Ala Leu Leu Arg Tyr Phe Asn Pro Gly Gly Ala
               180                     185                     190
   cat ccc tct ggt gaa ctc ggt gaa gat cct ctt ggc atc cct aat aac      624
   His Pro Ser Gly Glu Leu Gly Glu Asp Pro Leu Gly Ile Pro Asn Asn
           195                     200                     205
   ttg ctt cct tat atc gcg caa gtt gct gta gga aga ttg gat cat ttg      672
   Leu Leu Pro Tyr Ile Ala Gln Val Ala Val Gly Arg Leu Asp His Leu
       210                     215                     220
   aat gta ttt ggc gac gat tat ccc aca tct gac ggt act cca att cgt      720
   Asn Val Phe Gly Asp Asp Tyr Pro Thr Ser Asp Gly Thr Pro Ile Arg
   225                     230                     235                 240
   gac tac att cac gta tgc gat ttg gca gag gct cat gtt gct gct ctc      768
   Asp Tyr Ile His Val Cys Asp Leu Ala Glu Ala His Val Ala Ala Leu
                   245                     250                     255
   gat tac ctg cgc caa cat ttt gtt agt tgc cgc cct tgg aat ttg gga      816
   Asp Tyr Leu Arg Gln His Phe Val Ser Cys Arg Pro Trp Asn Leu Gly
               260                     265                     270
   tca gga act ggt agt act gtt ttt cag gtg ctc aat gcg ttt tcg aaa    864
   Ser Gly Thr Gly Ser Thr Val Phe Gln Val Leu Asn Ala Phe Ser Lys
           275                 280                 285
   gct gtt gga aga gat ctt cct tat aag gtc acc cct aga aga gca ggg    912
   Ala Val Gly Arg Asp Leu Pro Tyr Lys Val Thr Pro Arg Arg Ala Gly
       290                 295                 300
   gac gtt gtt aac cta acc gcc aac ccc act cgc gct aac gag gag tta    960
   Asp Val Val Asn Leu Thr Ala Asn Pro Thr Arg Ala Asn Glu Glu Leu
   305                 310                 315                 320
   aaa tgg aaa acc agt cgt agc att tat gaa att tgc gtt gac act tgg    1008
   Lys Trp Lys Thr Ser Arg Ser Ile Tyr Glu Ile Cys Val Asp Thr Trp
                   325                 330                 335
   aga tgg caa cag aag tat ccc tat ggc ttt gac ctg acc cat acc aag    1056
   Arg Trp Gln Gln Lys Tyr Pro Tyr Gly Phe Asp Leu Thr His Thr Lys
               340                 345                 350
   aca tat aag taa                                                    1068
   Thr Tyr Lys  *
           355
<210> 36
   <211> 355
   <212> PRT
   <213> Saccharomyces pombe
<400> 36
   Met Thr Gly Val His Glu Gly Thr Val Leu Val Thr Gly Gly Ala Gly
   1               5                   10                  15
   Tyr Ile Gly Ser His Thr Cys Val Val Leu Leu Glu Lys Gly Tyr Asp
               20                  25                  30
   Val Val Ile Val Asp Asn Leu Cys Asn Ser Arg Val Glu Ala Val His
           35                  40                  45
   Arg Ile Glu Lys Leu Thr Gly Lys Lys Val Ile Phe His Gln Val Asp
       50                  55                  60
   Leu Leu Asp Glu Pro Ala Leu Asp Lys Val Phe Ala Asn Gln Asn Ile
   65                  70                  75                  80
   Ser Ala Val Ile His Phe Ala Gly Leu Lys Ala Val Gly Glu Ser Val
                   85                  90                  95
   Gln Val Pro Leu Ser Tyr Tyr Lys Asn Asn Ile Ser Gly Thr Ile Asn
               100                 105                 110
   Leu Ile Glu Cys Met Lys Lys Tyr Asn Val Arg Asp Phe Val Phe Ser
           115                 120                 125
   Ser Ser Ala Thr Val Tyr Gly Asp Pro Thr Arg Pro Gly Gly Thr Ile
       130                 135                 140
   Pro Ile Pro Glu Ser Cys Pro Arg Glu Gly Thr Ser Pro Tyr Gly Arg
   145                 150                 155                 160
   Thr Lys Leu Phe Ile Glu Asn Ile Ile Glu Asp Glu Thr Lys Val Asn
                   165                 170                 175
   Lys Ser Leu Asn Ala Ala Leu Leu Arg Tyr Phe Asn Pro Gly Gly Ala
               180                 185                 190
   His Pro Ser Gly Glu Leu Gly Glu Asp Pro Leu Gly Ile Pro Asn Asn
           195                 200                 205
   Leu Leu Pro Tyr Ile Ala Gln Val Ala Val Gly Arg Leu Asp His Leu
       210                 215                 220
   Asn Val Phe Gly Asp Asp Tyr Pro Thr Ser Asp Gly Thr Pro Ile Arg
   225                 230                 235                 240
   Asp Tyr Ile His Val Cys Asp Leu Ala Glu Ala His Val Ala Ala Leu
                   245                 250                 255
   Asp Tyr Leu Arg Gln His Phe Val Ser Cys Arg Pro Trp Asn Leu Gly
               260                 265                 270
   Ser Gly Thr Gly Ser Thr Val Phe Gln Val Leu Asn Ala Phe Ser Lys
           275                 280                 285
   Ala Val Gly Arg Asp Leu Pro Tyr Lys Val Thr Pro Arg Arg Ala Gly
       290                 295                 300
   Asp Val Val Asn Leu Thr Ala Asn Pro Thr Arg Ala Asn Glu Glu Leu
   305                 310                 315                 320
   Lys Trp Lys Thr Ser Arg Ser Ile Tyr Glu Ile Cys Val Asp Thr Trp
                   325                 330                 335
   Arg Trp Gln Gln Lys Tyr Pro Tyr Gly Phe Asp Leu Thr His Thr Lys
               340                 345                 350
   Thr Tyr Lys
           355
<210> 37
   <211> 1074
   <212> DNA
   <213> Drosophila melangaster
<220>
   <221> CDS
   <222> (1)...(1079)
   <223> DNA encodes DmUGT
<400> 37
   atg aat agc ata cac atg aac gcc aat acg ctg aag tac atc agc ctg      48
   Met Asn Ser Ile His Met Asn Ala Asn Thr Leu Lys Tyr Ile Ser Leu
    1               5                  10                  15
   ctg acg ctg acc ctg cag aat gcc atc ctg ggc ctc agc atg cgc tac      96
   Leu Thr Leu Thr Leu Gln Asn Ala Ile Leu Gly Leu Ser Met Arg Tyr
               20                  25                  30
   gcc cgc acc cgg cca ggc gac atc ttc ctc agc tcc acg gcc gta ctc      144
   Ala Arg Thr Arg Pro Gly Asp Ile Phe Leu Ser Ser Thr Ala Val Leu
           35                  40                  45
   atg gca gag ttc gcc aaa ctg atc acg tgc ctg ttc ctg gtc ttc aac      192
   Met Ala Glu Phe Ala Lys Leu Ile Thr Cys Leu Phe Leu Val Phe Asn
       50                  55                  60
   gag gag ggc aag gat gcc cag aag ttt gta cgc tcg ctg cac aag acc      240
   Glu Glu Gly Lys Asp Ala Gln Lys Phe Val Arg Ser Leu His Lys Thr
    65                  70                  75                  80
   atc att gcg aat ccc atg gac acg ctg aag gtg tgc gtc ccc tcg ctg      288
   Ile Ile Ala Asn Pro Met Asp Thr Leu Lys Val Cys Val Pro Ser Leu
                   85                  90                  95
   gtc tat atc gtt caa aac aat ctg ctg tac gtc tct gcc tcc cat ttg      336
   Val Tyr Ile Val Gln Asn Asn Leu Leu Tyr Val Ser Ala Ser His Leu
               100                 105                 110
   gat gcg gcc acc tac cag gtg acg tac cag ctg aag att ctc acc acg      384
   Asp Ala Ala Thr Tyr Gln Val Thr Tyr Gln Leu Lys Ile Leu Thr Thr
           115                 120                 125
   gcc atg ttc gcg gtt gtc att ctg cgc cgc aag ctg ctg aac acg cag      432
   Ala Met Phe Ala Val Val Ile Leu Arg Arg Lys Leu Leu Asn Thr Gln
       130                 135                 140
   tgg ggt gcg ctg ctg ctc ctg gtg atg ggc atc gtc ctg gtg cag ttg      480
   Trp Gly Ala Leu Leu Leu Leu Val Met Gly Ile Val Leu Val Gln Leu
   145                 150                 155                 160
   gcc caa acg gag ggt ccg acg agt ggc tca gcc ggt ggt gcc gca gct      528
   Ala Gln Thr Glu Gly Pro Thr Ser Gly Ser Ala Gly Gly Ala Ala Ala
                   165                 170                 175
   gca gcc acg gcc gcc tcc tct ggc ggt gct ccc gag cag aac agg atg      576
   Ala Ala Thr Ala Ala Ser Ser Gly Gly Ala Pro Glu Gln Asn Arg Met
               180                 185                 190
   ctc gga ctg tgg gcc gca ctg ggc gcc tgc ttc ctc tcc gga ttc gcg      624
   Leu Gly Leu Trp Ala Ala Leu Gly Ala Cys Phe Leu Ser Gly Phe Ala
           195                 200                 205
   ggc atc tac ttt gag aag atc ctc aag ggt gcc gag atc tcc gtg tgg      672
   Gly Ile Tyr Phe Glu Lys Ile Leu Lys Gly Ala Glu Ile Ser Val Trp
       210                 215                 220
   atg cgg aat gtg cag ttg agt ctg ctc agc att ccc ttc ggc ctg ctc      720
   Met Arg Asn Val Gln Leu Ser Leu Leu Ser Ile Pro Phe Gly Leu Leu
   225                 230                 235                 240
   acc tgt ttc gtt aac gac ggc agt agg atc ttc gac cag gga ttc ttc      768
   Thr Cys Phe Val Asn Asp Gly Ser Arg Ile Phe Asp Gln Gly Phe Phe
                   245                 250                 255
   aag ggc tac gat ctg ttt gtc tgg tac ctg gtc ctg ctg cag gcc ggc      816
   Lys Gly Tyr Asp Leu Phe Val Trp Tyr Leu Val Leu Leu Gln Ala Gly
               260                 265                 270
   ggt gga ttg atc gtt gcc gtg gtg gtc aag tac gcg gat aac att ctc      864
   Gly Gly Leu Ile Val Ala Val Val Val Lys Tyr Ala Asp Asn Ile Leu
           275                 280                 285
   aag ggc ttc gcc acc tcg ctg gcc atc atc atc tcg tgc gtg gcc tcc      912
   Lys Gly Phe Ala Thr Ser Leu Ala Ile Ile Ile Ser Cys Val Ala Ser
       290                 295                 300
   ata tac atc ttc gac ttc aat ctc acg ctg cag ttc agc ttc gga gct      960
   Ile Tyr Ile Phe Asp Phe Asn Leu Thr Leu Gln Phe Ser Phe Gly Ala
   305                 310                 315                 320
   ggc ctg gtc atc gcc tcc ata ttt ctc tac ggc tac gat ccg gcc agg     1008
   Gly Leu Val Ile Ala Ser Ile Phe Leu Tyr Gly Tyr Asp Pro Ala Arg
                   325                 330                 335
   tcg gcg ccg aag cca act atg cat ggt cct ggc ggc gat gag gag aag     1056
   Ser Ala Pro Lys Pro Thr Met His Gly Pro Gly Gly Asp Glu Glu Lys
               340                 345                 350
   ctg ctg ccg cgc gtc tag                                             1074
   Leu Leu Pro Arg Val  *
           355
<210> 38
   <211> 357
   <212> PRT
   <213> Drosophila melangaster
<400> 38
   Met Asn Ser Ile His Met Asn Ala Asn Thr Leu Lys Tyr Ile Ser Leu
    1               5                  10                  15
   Leu Thr Leu Thr Leu Gln Asn Ala Ile Leu Gly Leu Ser Met Arg Tyr
               20                  25                  30
   Ala Arg Thr Arg Pro Gly Asp Ile Phe Leu Ser Ser Thr Ala Val Leu
           35                  40                  45
   Met Ala Glu Phe Ala Lys Leu Ile Thr Cys Leu Phe Leu Val Phe Asn
       50                  55                  60
   Glu Glu Gly Lys Asp Ala Gln Lys Phe Val Arg Ser Leu His Lys Thr
   65                  70                  75                  80
   Ile Ile Ala Asn Pro Met Asp Thr Leu Lys Val Cys Val Pro Ser Leu
                   85                  90                  95
   Val Tyr Ile Val Gln Asn Asn Leu Leu Tyr Val Ser Ala Ser His Leu
               100                 105                 110
   Asp Ala Ala Thr Tyr Gln Val Thr Tyr Gln Leu Lys Ile Leu Thr Thr
           115                 120                 125
   Ala Met Phe Ala Val Val Ile Leu Arg Arg Lys Leu Leu Asn Thr Gln
       130                 135                 140
   Trp Gly Ala Leu Leu Leu Leu Val Met Gly Ile Val Leu Val Gln Leu
   145                 150                 155                 160
   Ala Gln Thr Glu Gly Pro Thr Ser Gly Ser Ala Gly Gly Ala Ala Ala
                   165                 170                 175
   Ala Ala Thr Ala Ala Ser Ser Gly Gly Ala Pro Glu Gln Asn Arg Met
               180                 185                 190
   Leu Gly Leu Trp Ala Ala Leu Gly Ala Cys Phe Leu Ser Gly Phe Ala
           195                 200                 205
   Gly Ile Tyr Phe Glu Lys Ile Leu Lys Gly Ala Glu Ile Ser Val Trp
       210                 215                 220
   Met Arg Asn Val Gln Leu Ser Leu Leu Ser Ile Pro Phe Gly Leu Leu
   225                 230                 235                 240
   Thr Cys Phe Val Asn Asp Gly Ser Arg Ile Phe Asp Gln Gly Phe Phe
                   245                 250                 255
   Lys Gly Tyr Asp Leu Phe Val Trp Tyr Leu Val Leu Leu Gln Ala Gly
               260                 265                 270
   Gly Gly Leu Ile Val Ala Val Val Val Lys Tyr Ala Asp Asn Ile Leu
           275                 280                 285
   Lys Gly Phe Ala Thr Ser Leu Ala Ile Ile Ile Ser Cys Val Ala Ser
       290                 295                 300
   Ile Tyr Ile Phe Asp Phe Asn Leu Thr Leu Gln Phe Ser Phe Gly Ala
   305                 310                 315                 320
   Gly Leu Val Ile Ala Ser Ile Phe Leu Tyr Gly Tyr Asp Pro Ala Arg
                   325                 330                 335
   Ser Ala Pro Lys Pro Thr Met His Gly Pro Gly Gly Asp Glu Glu Lys
               340                 345                 350
   Leu Leu Pro Arg Val
           355
<210> 39
   <211> 1587
   <212> DNA
   <213> Saccharomyces cerevisiae
<220>
   <221> CDS
   <222> (1)...(1587)
   <223> DNA encodes ScGAL1
<400> 39
   atg act aaa tct cat tca gaa gaa gtg att gta cct gag ttc aat tct      48
   Met Thr Lys Ser His Ser Glu Glu Val Ile Val Pro Glu Phe Asn Ser
    1               5                  10                  15
   agc gca aag gaa tta cca aga cca ttg gcc gaa aag tgc ccg agc ata      96
   Ser Ala Lys Glu Leu Pro Arg Pro Leu Ala Glu Lys Cys Pro Ser Ile
               20                  25                  30
   att aag aaa ttt ata agc gct tat gat gct aaa ccg gat ttt gtt gct     144
   Ile Lys Lys Phe Ile Ser Ala Tyr Asp Ala Lys Pro Asp Phe Val Ala
           35                  40                  45
   aga tcg cct ggt aga gtc aat cta att ggt gaa cat att gat tat tgt     192
   Arg Ser Pro Gly Arg Val Asn Leu Ile Gly Glu His Ile Asp Tyr Cys
       50                  55                  60
   gac ttc tcg gtt tta cct tta gct att gat ttt gat atg ctt tgc gcc     240
   Asp Phe Ser Val Leu Pro Leu Ala Ile Asp Phe Asp Met Leu Cys Ala
   65                  70                  75                  80
   gtc aaa gtt ttg aac gag aaa aat cca tcc att acc tta ata aat gct     288
   Val Lys Val Leu Asn Glu Lys Asn Pro Ser Ile Thr Leu Ile Asn Ala
                   85                  90                  95
   gat ccc aaa ttt gct caa agg aag ttc gat ttg ccg ttg gac ggt tct     336
   Asp Pro Lys Phe Ala Gln Arg Lys Phe Asp Leu Pro Leu Asp Gly Ser
               100                 105                 110
   tat gtc aca att gat cct tct gtg tcg gac tgg tct aat tac ttt aaa     384
   Tyr Val Thr Ile Asp Pro Ser Val Ser Asp Trp Ser Asn Tyr Phe Lys
           115                 120                 125
   tgt ggt ctc cat gtt gct cac tct ttt cta aag aaa ctt gca ccg gaa     432
   Cys Gly Leu His Val Ala His Ser Phe Leu Lys Lys Leu Ala Pro Glu
       130                 135                 140
   agg ttt gcc agt gct cct ctg gcc ggg ctg caa gtc ttc tgt gag ggt     480
   Arg Phe Ala Ser Ala Pro Leu Ala Gly Leu Gln Val Phe Cys Glu Gly
   145                 150                 155                 160
   gat gta cca act ggc agt gga ttg tct tct tcg gcc gca ttc att tgt     528
   Asp Val Pro Thr Gly Ser Gly Leu Ser Ser Ser Ala Ala Phe Ile Cys
                   165                 170                 175
   gcc gtt gct tta gct gtt gtt aaa gcg aat atg ggc cct ggt tat cat     576
   Ala Val Ala Leu Ala Val Val Lys Ala Asn Met Gly Pro Gly Tyr His
               180                 185                 190
   atg tcc aag caa aat tta atg cgt att acg gtc gtt gca gaa cat tat     624
   Met Ser Lys Gln Asn Leu Met Arg Ile Thr Val Val Ala Glu His Tyr
           195                 200                 205
   gtt ggt gtt aac aat ggc ggt atg gat cag gct gcc tct gtt tgc ggt     672
   Val Gly Val Asn Asn Gly Gly Met Asp Gln Ala Ala Ser Val Cys Gly
       210                 215                 220
   gag gaa gat cat gct cta tac gtt gag ttc aaa ccg cag ttg aag gct    720
   Glu Glu Asp His Ala Leu Tyr Val Glu Phe Lys Pro Gln Leu Lys Ala
   225                 230                 235                 240
   act ccg ttt aaa ttt ccg caa tta aaa aac cat gaa att agc ttt gtt    768
   Thr Pro Phe Lys Phe Pro Gln Leu Lys Asn His Glu Ile Ser Phe Val
                   245                 250                 255
   att gcg aac acc ctt gtt gta tct aac aag ttt gaa acc gcc cca acc    816
   Ile Ala Asn Thr Leu Val Val Ser Asn Lys Phe Glu Thr Ala Pro Thr
               260                 265                 270
   aac tat aat tta aga gtg gta gaa gtc act aca gct gca aat gtt tta    864
   Asn Tyr Asn Leu Arg Val Val Glu Val Thr Thr Ala Ala Asn Val Leu
           275                 280                 285
   gct gcc acg tac ggt gtt gtt tta ctt tct gga aaa gaa gga tcg agc    912
   Ala Ala Thr Tyr Gly Val Val Leu Leu Ser Gly Lys Glu Gly Ser Ser
       290                 295                 300
   acg aat aaa ggt aat cta aga gat ttc atg aac gtt tat tat gcc aga    960
   Thr Asn Lys Gly Asn Leu Arg Asp Phe Met Asn Val Tyr Tyr Ala Arg
   305                 310                 315                 320
   tat cac aac att tcc aca ccc tgg aac ggc gat att gaa tcc ggc atc    1008
   Tyr His Asn Ile Ser Thr Pro Trp Asn Gly Asp Ile Glu Ser Gly Ile
                   325                 330                 335
   gaa cgg tta aca aag atg cta gta cta gtt gaa gag tct ctc gcc aat    1056
   Glu Arg Leu Thr Lys Met Leu Val Leu Val Glu Glu Ser Leu Ala Asn
               340                 345                 350
   aag aaa cag ggc ttt agt gtt gac gat gtc gca caa tcc ttg aat tgt    1104
   Lys Lys Gln Gly Phe Ser Val Asp Asp Val Ala Gln Ser Leu Asn Cys
           355                 360                 365
   tct cgc gaa gaa ttc aca aga gac tac tta aca aca tct cca gtg aga    1152
   Ser Arg Glu Glu Phe Thr Arg Asp Tyr Leu Thr Thr Ser Pro Val Arg
       370                 375                 380
   ttt caa gtc tta aag cta tat cag agg gct aag cat gtg tat tct gaa    1200
   Phe Gln Val Leu Lys Leu Tyr Gln Arg Ala Lys His Val Tyr Ser Glu
   385                 390                 395                 400
   tct tta aga gtc ttg aag gct gtg aaa tta atg act aca gcg agc ttt    1248
   Ser Leu Arg Val Leu Lys Ala Val Lys Leu Met Thr Thr Ala Ser Phe
                   405                 410                 415
   act gcc gac gaa gac ttt ttc aag caa ttt ggt gcc ttg atg aac gag    1296
   Thr Ala Asp Glu Asp Phe Phe Lys Gln Phe Gly Ala Leu Met Asn Glu
               420                 425                 430
   tct caa gct tct tgc gat aaa ctt tac gaa tgt tct tgt cca gag att    1344
   Ser Gln Ala Ser Cys Asp Lys Leu Tyr Glu Cys Ser Cys Pro Glu Ile
           435                 440                 445
   gac aaa att tgt tcc att gct ttg tca aat gga tca tat ggt tcc cgt    1392
   Asp Lys Ile Cys Ser Ile Ala Leu Ser Asn Gly Ser Tyr Gly Ser Arg
       450                 455                 460
   ttg acc gga gct ggc tgg ggt ggt tgt act gtt cac ttg gtt cca ggg    1440
   Leu Thr Gly Ala Gly Trp Gly Gly Cys Thr Val His Leu Val Pro Gly
   465                 470                 475                 480
   ggc cca aat ggc aac ata gaa aag gta aaa gaa gcc ctt gcc aat gag     1488
   Gly Pro Asn Gly Asn Ile Glu Lys Val Lys Glu Ala Leu Ala Asn Glu
                   485                 490                 495
   ttc tac aag gtc aag tac cct aag atc act gat gct gag cta gaa aat     1536
   Phe Tyr Lys Val Lys Tyr Pro Lys Ile Thr Asp Ala Glu Leu Glu Asn
               500                 505                 510
   gct atc atc gtc tct aaa cca gca ttg ggc agc tgt cta tat gaa tta     1584
   Ala Ile Ile Val Ser Lys Pro Ala Leu Gly Ser Cys Leu Tyr Glu Leu
           515                 520                 525
   taa                                                                 1587
    *
<210> 40
   <211> 528
   <212> PRT
   <213> Saccharomyces cerevisiea
<400> 40
   Met Thr Lys Ser His Ser Glu Glu Val Ile Val Pro Glu Phe Asn Ser
   1               5                   10                  15
   Ser Ala Lys Glu Leu Pro Arg Pro Leu Ala Glu Lys Cys Pro Ser Ile
               20                  25                  30
   Ile Lys Lys Phe Ile Ser Ala Tyr Asp Ala Lys Pro Asp Phe Val Ala
           35                  40                  45
   Arg Ser Pro Gly Arg Val Asn Leu Ile Gly Glu His Ile Asp Tyr Cys
       50                  55                  60
   Asp Phe Ser Val Leu Pro Leu Ala Ile Asp Phe Asp Met Leu Cys Ala
   65                  70                  75                  80
   Val Lys Val Leu Asn Glu Lys Asn Pro Ser Ile Thr Leu Ile Asn Ala
                   85                  90                  95
   Asp Pro Lys Phe Ala Gln Arg Lys Phe Asp Leu Pro Leu Asp Gly Ser
               100                 105                 110
   Tyr Val Thr Ile Asp Pro Ser Val Ser Asp Trp Ser Asn Tyr Phe Lys
           115                 120                 125
   Cys Gly Leu His Val Ala His Ser Phe Leu Lys Lys Leu Ala Pro Glu
       130                 135                 140
   Arg Phe Ala Ser Ala Pro Leu Ala Gly Leu Gln Val Phe Cys Glu Gly
   145                 150                 155                 160
   Asp Val Pro Thr Gly Ser Gly Leu Ser Ser Ser Ala Ala Phe Ile Cys
                   165                 170                 175
   Ala Val Ala Leu Ala Val Val Lys Ala Asn Met Gly Pro Gly Tyr His
               180                 185                 190
   Met Ser Lys Gln Asn Leu Met Arg Ile Thr Val Val Ala Glu His Tyr
           195                 200                 205
   Val Gly Val Asn Asn Gly Gly Met Asp Gln Ala Ala Ser Val Cys Gly
       210                 215                 220
   Glu Glu Asp His Ala Leu Tyr Val Glu Phe Lys Pro Gln Leu Lys Ala
   225                 230                 235                 240
   Thr Pro Phe Lys Phe Pro Gln Leu Lys Asn His Glu Ile Ser Phe Val
                   245                 250                 255
   Ile Ala Asn Thr Leu Val Val Ser Asn Lys Phe Glu Thr Ala Pro Thr
               260                 265                 270
   Asn Tyr Asn Leu Arg Val Val Glu Val Thr Thr Ala Ala Asn Val Leu
           275                 280                 285
   Ala Ala Thr Tyr Gly Val Val Leu Leu Ser Gly Lys Glu Gly Ser Ser
       290                 295                 300
   Thr Asn Lys Gly Asn Leu Arg Asp Phe Met Asn Val Tyr Tyr Ala Arg
   305                 310                 315                 320
   Tyr His Asn Ile Ser Thr Pro Trp Asn Gly Asp Ile Glu Ser Gly Ile
                   325                 330                 335
   Glu Arg Leu Thr Lys Met Leu Val Leu Val Glu Glu Ser Leu Ala Asn
               340                 345                 350
   Lys Lys Gln Gly Phe Ser Val Asp Asp Val Ala Gln Ser Leu Asn Cys
           355                 360                 365
   Ser Arg Glu Glu Phe Thr Arg Asp Tyr Leu Thr Thr Ser Pro Val Arg
       370                 375                 380
   Phe Gln Val Leu Lys Leu Tyr Gln Arg Ala Lys His Val Tyr Ser Glu
   385                 390                 395                 400
   Ser Leu Arg Val Leu Lys Ala Val Lys Leu Met Thr Thr Ala Ser Phe
                   405                 410                 415
   Thr Ala Asp Glu Asp Phe Phe Lys Gln Phe Gly Ala Leu Met Asn Glu
               420                 425                 430
   Ser Gln Ala Ser Cys Asp Lys Leu Tyr Glu Cys Ser Cys Pro Glu Ile
           435                 440                 445
   Asp Lys Ile Cys Ser Ile Ala Leu Ser Asn Gly Ser Tyr Gly Ser Arg
       450                 455                 460
   Leu Thr Gly Ala Gly Trp Gly Gly Cys Thr Val His Leu Val Pro Gly
   465                 470                 475                 480
   Gly Pro Asn Gly Asn Ile Glu Lys Val Lys Glu Ala Leu Ala Asn Glu
                   485                 490                 495
   Phe Tyr Lys Val Lys Tyr Pro Lys Ile Thr Asp Ala Glu Leu Glu Asn
               500                 505                 510
   Ala Ile Ile Val Ser Lys Pro Ala Leu Gly Ser Cys Leu Tyr Glu Leu
           515                 520                 525
<210> 41
   <211> 1098
   <212> DNA
   <213> Saccharomyces cerevisiae
<220>
   <221> CDS
   <222> (1)...(1098)
   <223> DNA encodes ScGAL7
<400> 41
   atg act gct gaa gaa ttt gat ttt tct agc cat tcc cat aga cgt tac   48
   Met Thr Ala Glu Glu Phe Asp Phe Ser Ser His Ser His Arg Arg Tyr
    1               5                  10                  15
   aat cca cta acc gat tca tgg atc tta gtt tct cca cac aga gct aaa   96
   Asn Pro Leu Thr Asp Ser Trp Ile Leu Val Ser Pro His Arg Ala Lys
               20                  25                  30
   aga cct tgg tta ggt caa cag gag gct gct tac aag ccc aca gct cct   144
   Arg Pro Trp Leu Gly Gln Gln Glu Ala Ala Tyr Lys Pro Thr Ala Pro
           35                  40                  45
   ttg tat gat cca aaa tgc tat cta tgt cct ggt aac aaa aga gct act   192
   Leu Tyr Asp Pro Lys Cys Tyr Leu Cys Pro Gly Asn Lys Arg Ala Thr
       50                  55                  60
   ggt aac cta aac cca aga tat gaa tca acg tat att ttc ccc aat gat   240
   Gly Asn Leu Asn Pro Arg Tyr Glu Ser Thr Tyr Ile Phe Pro Asn Asp
    65                 70                  75                  80
   tat gct gcc gtt agc gat caa cct att tta cca cag aat gat tcc aat      288
   Tyr Ala Ala Val Ser Asp Gln Pro Ile Leu Pro Gln Asn Asp Ser Asn
                   85                  90                  95
   gag gat aat ctt aaa aat agg ctg ctt aaa gtg caa tct gtg aga ggc      336
   Glu Asp Asn Leu Lys Asn Arg Leu Leu Lys Val Gln Ser Val Arg Gly
               100                 105                 110
   aat tgt ttc gtc ata tgt ttt agc ccc aat cat aat cta acc att cca      384
   Asn Cys Phe Val Ile Cys Phe Ser Pro Asn His Asn Leu Thr Ile Pro
           115                 120                 125
   caa atg aaa caa tca gat ctg gtt cat att gtt aat tct tgg caa gca      432
   Gln Met Lys Gln Ser Asp Leu Val His Ile Val Asn Ser Trp Gln Ala
       130                 135                 140
   ttg act gac gat ctc tcc aga gaa gca aga gaa aat cat aag cct ttc      480
   Leu Thr Asp Asp Leu Ser Arg Glu Ala Arg Glu Asn His Lys Pro Phe
   145                 150                 155                 160
   aaa tat gtc caa ata ttt gaa aac aaa ggt aca gcc atg ggt tgt tcc      528
   Lys Tyr Val Gln Ile Phe Glu Asn Lys Gly Thr Ala Met Gly Cys Ser
                   165                 170                 175
   aac tta cat cca cat ggc caa gct tgg tgc tta gaa tcc atc cct agt      576
   Asn Leu His Pro His Gly Gln Ala Trp Cys Leu Glu Ser Ile Pro Ser
               180                 185                 190
   gaa gtt tcg caa gaa ttg aaa tct ttt gat aaa tat aaa cgt gaa cac      624
   Glu Val Ser Gln Glu Leu Lys Ser Phe Asp Lys Tyr Lys Arg Glu His
           195                 200                 205
   aat act gat ttg ttt gcc gat tac gtc aaa tta gaa tca aga gag aag      672
   Asn Thr Asp Leu Phe Ala Asp Tyr Val Lys Leu Glu Ser Arg Glu Lys
       210                 215                 220
   tca aga gtc gta gtg gag aat gaa tcc ttt att gtt gtt gtt cca tac      720
   Ser Arg Val Val Val Glu Asn Glu Ser Phe Ile Val Val Val Pro Tyr
   225                 230                 235                 240
   tgg gcc atc tgg cca ttt gag acc ttg gtc att tca aag aag aag ctt      768
   Trp Ala Ile Trp Pro Phe Glu Thr Leu Val Ile Ser Lys Lys Lys Leu
                   245                 250                 255
   gcc tca att agc caa ttt aac caa atg gcg aag gag gac ctc gcc tcg      816
   Ala Ser Ile Ser Gln Phe Asn Gln Met Ala Lys Glu Asp Leu Ala Ser
               260                 265                 270
   att tta aag caa cta act att aag tat gat aat tta ttt gaa acg agt      864
   Ile Leu Lys Gln Leu Thr Ile Lys Tyr Asp Asn Leu Phe Glu Thr Ser
           275                 280                 285
   ttc cca tac tca atg ggt atc cat cag gct cct ttg aat gcg act ggt      912
   Phe Pro Tyr Ser Met Gly Ile His Gln Ala Pro Leu Asn Ala Thr Gly
       290                 295                 300
   gat gaa ttg agt aat agt tgg ttt cac atg cat ttc tac cca cct tta      960
   Asp Glu Leu Ser Asn Ser Trp Phe His Met His Phe Tyr Pro Pro Leu
   305                 310                 315                 320
   ctg aga tca gct act gtt cgg aaa ttc ttg gtt ggt ttt gaa ttg tta      1008
   Leu Arg Ser Ala Thr Val Arg Lys Phe Leu Val Gly Phe Glu Leu Leu
                   325                 330                 335
   ggt gag cct caa aga gat tta att tcg gaa caa gct gct gaa aaa cta      1056
   Gly Glu Pro Gln Arg Asp Leu Ile Ser Glu Gln Ala Ala Glu Lys Leu
               340                 345                 350
   aga aat tta gat ggt cag att cat tat cta caa aga cta taa              1098
   Arg Asn Leu Asp Gly Gln Ile His Tyr Leu Gln Arg Leu  *
           355                 360                 365
<210> 42
   <211> 365
   <212> PRT
   <213> Saccharomyces cerevisiae
<400> 42
   Met Thr Ala Glu Glu Phe Asp Phe Ser Ser His Ser His Arg Arg Tyr
   1               5                   10                  15
   Asn Pro Leu Thr Asp Ser Trp Ile Leu Val Ser Pro His Arg Ala Lys
               20                  25                  30
   Arg Pro Trp Leu Gly Gln Gln Glu Ala Ala Tyr Lys Pro Thr Ala Pro
           35                  40                  45
   Leu Tyr Asp Pro Lys Cys Tyr Leu Cys Pro Gly Asn Lys Arg Ala Thr
       50                  55                  60
   Gly Asn Leu Asn Pro Arg Tyr Glu Ser Thr Tyr Ile Phe Pro Asn Asp
   65                  70                  75                  80
   Tyr Ala Ala Val Ser Asp Gln Pro Ile Leu Pro Gln Asn Asp Ser Asn
                   85                  90                  95
   Glu Asp Asn Leu Lys Asn Arg Leu Leu Lys Val Gln Ser Val Arg Gly
               100                 105                 110
   Asn Cys Phe Val Ile Cys Phe Ser Pro Asn His Asn Leu Thr Ile Pro
           115                 120                 125
   Gln Met Lys Gln Ser Asp Leu Val His Ile Val Asn Ser Trp Gln Ala
       130                 135                 140
   Leu Thr Asp Asp Leu Ser Arg Glu Ala Arg Glu Asn His Lys Pro Phe
   145                 150                 155                 160
   Lys Tyr Val Gln Ile Phe Glu Asn Lys Gly Thr Ala Met Gly Cys Ser
                   165                 170                 175
   Asn Leu His Pro His Gly Gln Ala Trp Cys Leu Glu Ser Ile Pro Ser
               180                 185                 190
   Glu Val Ser Gln Glu Leu Lys Ser Phe Asp Lys Tyr Lys Arg Glu His
           195                 200                 205
   Asn Thr Asp Leu Phe Ala Asp Tyr Val Lys Leu Glu Ser Arg Glu Lys
       210                 215                 220
   Ser Arg Val Val Val Glu Asn Glu Ser Phe Ile Val Val Val Pro Tyr
   225                 230                 235                 240
   Trp Ala Ile Trp Pro Phe Glu Thr Leu Val Ile Ser Lys Lys Lys Leu
                   245                 250                 255
   Ala Ser Ile Ser Gln Phe Asn Gln Met Ala Lys Glu Asp Leu Ala Ser
               260                 265                 270
   Ile Leu Lys Gln Leu Thr Ile Lys Tyr Asp Asn Leu Phe Glu Thr Ser
           275                 280                 285
   Phe Pro Tyr Ser Met Gly Ile His Gln Ala Pro Leu Asn Ala Thr Gly
       290                 295                 300
   Asp Glu Leu Ser Asn Ser Trp Phe His Met His Phe Tyr Pro Pro Leu
   305                 310                 315                 320
   Leu Arg Ser Ala Thr Val Arg Lys Phe Leu Val Gly Phe Glu Leu Leu
                   325                 330                 335
   Gly Glu Pro Gln Arg Asp Leu Ile Ser Glu Gln Ala Ala Glu Lys Leu
               340                 345                 350
   Arg Asn Leu Asp Gly Gln Ile His Tyr Leu Gln Arg Leu
           355                 360                 365
<210> 43
   <211> 1725
   <212> DNA
   <213> Saccharomyces cerevisiae
<220>
   <221> CDS
   <222> (1)...(1725)
   <223> DNA encodes ScGal permease
<400> 43
   atg gca gtt gag gag aac aat gtg cct gtt gtt tca cag caa ccc caa     48
   Met Ala Val Glu Glu Asn Asn Val Pro Val Val Ser Gln Gln Pro Gln
   1               5                   10                  15
   gct ggt gaa gac gtg atc tct tca ctc agt aaa gat tcc cat tta agc     96
   Ala Gly Glu Asp Val Ile Ser Ser Leu Ser Lys Asp Ser His Leu Ser
               20                  25                  30
   gca caa tct caa aag tat tcc aat gat gaa ttg aaa gcc ggt gag tca     144
   Ala Gln Ser Gln Lys Tyr Ser Asn Asp Glu Leu Lys Ala Gly Glu Ser
           35                  40                  45
   ggg cct gaa ggc tcc caa agt gtt cct ata gag ata ccc aag aag ccc     192
   Gly Pro Glu Gly Ser Gln Ser Val Pro Ile Glu Ile Pro Lys Lys Pro
       50                  55                  60
   atg tct gaa tat gtt acc gtt tcc ttg ctt tgt ttg tgt gtt gcc ttc     240
   Met Ser Glu Tyr Val Thr Val Ser Leu Leu Cys Leu Cys Val Ala Phe
   65                  70                  75                  80
   ggc ggc ttc atg ttt ggc tgg gat acc agt act att tct ggg ttt gtt     288
   Gly Gly Phe Met Phe Gly Trp Asp Thr Ser Thr Ile Ser Gly Phe Val
                   85                  90                  95
   gtc caa aca gac ttt ttg aga agg ttt ggt atg aaa cat aag gat ggt     336
   Val Gln Thr Asp Phe Leu Arg Arg Phe Gly Met Lys His Lys Asp Gly
               100                 105                 110
   acc cac tat ttg tca aac gtc aga aca ggt tta atc gtc gcc att ttc     384
   Thr His Tyr Leu Ser Asn Val Arg Thr Gly Leu Ile Val Ala Ile Phe
           115                 120                 125
   aat att ggc tgt gcc ttt ggt ggt att ata ctt tcc aaa ggt gga gat     432
   Asn Ile Gly Cys Ala Phe Gly Gly Ile Ile Leu Ser Lys Gly Gly Asp
       130                 135                 140
   atg tat ggc cgt aaa aag ggt ctt tcg att gtc gtc tcg gtt tat ata     480
   Met Tyr Gly Arg Lys Lys Gly Leu Ser Ile Val Val Ser Val Tyr Ile
   145                 150                 155                 160
   gtt ggt att atc att caa att gcc tct atc aac aag tgg tac caa tat     528
   Val Gly Ile Ile Ile Gln Ile Ala Ser Ile Asn Lys Trp Tyr Gln Tyr
                   165                 170                 175
   ttc att ggt aga atc ata tct ggt ttg ggt gtc ggc ggc atc gct gtc      576
   Phe Ile Gly Arg Ile Ile Ser Gly Leu Gly Val Gly Gly Ile Ala Val
               180                 185                 190
   tta tgt cct atg ttg atc tct gaa att gct cca aag cac ttg aga ggc      624
   Leu Cys Pro Met Leu Ile Ser Glu Ile Ala Pro Lys His Leu Arg Gly
           195                 200                 205
   aca cta gtt tct tgt tat cag ctg atg att act gca ggt atc ttt ttg      672
   Thr Leu Val Ser Cys Tyr Gln Leu Met Ile Thr Ala Gly Ile Phe Leu
       210                 215                 220
   ggc tac tgt act aat tac ggt aca aag agc tat tcg aac tca gtt caa      720
   Gly Tyr Cys Thr Asn Tyr Gly Thr Lys Ser Tyr Ser Asn Ser Val Gln
   225                 230                 235                 240
   tgg aga gtt cca tta ggg cta tgt ttc gct tgg tca tta ttt atg att      768
   Trp Arg Val Pro Leu Gly Leu Cys Phe Ala Trp Ser Leu Phe Met Ile
                   245                 250                 255
   ggc gct ttg acg tta gtt cct gaa tcc cca cgt tat tta tgt gag gtg      816
   Gly Ala Leu Thr Leu Val Pro Glu Ser Pro Arg Tyr Leu Cys Glu Val
               260                 265                 270
   aat aag gta gaa gac gcc aag cgt tcc att gct aag tct aac aag gtg      864
   Asn Lys Val Glu Asp Ala Lys Arg Ser Ile Ala Lys Ser Asn Lys Val
           275                 280                 285
   tca cca gag gat cct gcc gtc cag gcc gag tta gat ctg atc atg gcc      912
   Ser Pro Glu Asp Pro Ala Val Gln Ala Glu Leu Asp Leu Ile Met Ala
       290                 295                 300
   ggt ata gaa gct gaa aaa ctg gct ggc aat gcg tcc tgg ggg gaa tta      960
   Gly Ile Glu Ala Glu Lys Leu Ala Gly Asn Ala Ser Trp Gly Glu Leu
   305                 310                 315                 320
   ttt tcc acc aag acc aaa gta ttt caa cgt ttg ttg atg ggt gtg ttt     1008
   Phe Ser Thr Lys Thr Lys Val Phe Gln Arg Leu Leu Met Gly Val Phe
                   325                 330                 335
   gtt caa atg ttc caa caa tta acc ggt aac aat tat ttt ttc tac tac     1056
   Val Gln Met Phe Gln Gln Leu Thr Gly Asn Asn Tyr Phe Phe Tyr Tyr
               340                 345                 350
   ggt acc gtt att ttc aag tca gtt ggc ctg gat gat tcc ttt gaa aca     1104
   Gly Thr Val Ile Phe Lys Ser Val Gly Leu Asp Asp Ser Phe Glu Thr
           355                 360                 365
   tcc att gtc att ggt gta gtc aac ttt gcc tcc act ttc ttt agt ttg     1152
   Ser Ile Val Ile Gly Val Val Asn Phe Ala Ser Thr Phe Phe Ser Leu
       370                 375                 380
   tgg act gtc gaa aac ttg ggg cgt cgt aaa tgt tta ctt ttg ggc gct     1200
   Trp Thr Val Glu Asn Leu Gly Arg Arg Lys Cys Leu Leu Leu Gly Ala
   385                 390                 395                 400
   gcc act atg atg gct tgt atg gtc atc tac gcc tct gtt ggt gtt act     1248
   Ala Thr Met Met Ala Cys Met Val Ile Tyr Ala Ser Val Gly Val Thr
                   405                 410                 415
   aga tta tat cct cac ggt aaa agc cag cca tct tct aaa ggt gcc ggt    1296
   Arg Leu Tyr Pro His Gly Lys Ser Gln Pro Ser Ser Lys Gly Ala Gly
               420                 425                 430
   aac tgt atg att gtc ttt acc tgt ttt tat att ttc tgt tat gcc aca    1344
   Asn Cys Met Ile Val Phe Thr Cys Phe Tyr Ile Phe Cys Tyr Ala Thr
           435                 440                 445
   acc tgg gcg cca gtt gcc tgg gtc atc aca gca gaa tca ttc cca ctg    1392
   Thr Trp Ala Pro Val Ala Trp Val Ile Thr Ala Glu Ser Phe Pro Leu
       450                 455                 460
   aga gtc aag tcg aaa tgt atg gcg ttg gcc tct gct tcc aat tgg gta    1440
   Arg Val Lys Ser Lys Cys Met Ala Leu Ala Ser Ala Ser Asn Trp Val
   465                 470                 475                 480
   tgg ggg ttc ttg att gca ttt ttc acc cca ttc atc aca tct gcc att    1488
   Trp Gly Phe Leu Ile Ala Phe Phe Thr Pro Phe Ile Thr Ser Ala Ile
                   485                 490                 495
   aac ttc tac tac ggt tat gtc ttc atg ggc tgt ttg gtt gcc atg ttt    1536
   Asn Phe Tyr Tyr Gly Tyr Val Phe Met Gly Cys Leu Val Ala Met Phe
               500                 505                 510
   ttt tat gtc ttt ttc ttt gtt cca gaa act aaa ggc cta tcg tta gaa    1584
   Phe Tyr Val Phe Phe Phe Val Pro Glu Thr Lys Gly Leu Ser Leu Glu
           515                 520                 525
   gaa att caa gaa tta tgg gaa gaa ggt gtt tta cct tgg aaa tct gaa    1632
   Glu Ile Gln Glu Leu Trp Glu Glu Gly Val Leu Pro Trp Lys Ser Glu
       530                 535                 540
   ggc tgg att cct tca tcc aga aga ggt aat aat tac gat tta gag gat    1680
   Gly Trp Ile Pro Ser Ser Arg Arg Gly Asn Asn Tyr Asp Leu Glu Asp
   545                 550                 555                 560
   tta caa cat gac gac aaa ccg tgg tac aag gcc atg cta gaa taa        1725
   Leu Gln His Asp Asp Lys Pro Trp Tyr Lys Ala Met Leu Glu  *
                   565                 570
<210> 44
   <211> 574
   <212> PRT
   <213> Saccharomyces cerevisiae
<400> 44
   Met Ala Val Glu Glu Asn Asn Val Pro Val Val Ser Gln Gln Pro Gln
   1               5                   10                  15
   Ala Gly Glu Asp Val Ile Ser Ser Leu Ser Lys Asp Ser His Leu Ser
               20                  25                  30
   Ala Gln Ser Gln Lys Tyr Ser Asn Asp Glu Leu Lys Ala Gly Glu Ser
           35                  40                  45
   Gly Pro Glu Gly Ser Gln Ser Val Pro Ile Glu Ile Pro Lys Lys Pro
       50                  55                  60
   Met Ser Glu Tyr Val Thr Val Ser Leu Leu Cys Leu Cys Val Ala Phe
   65                  70                  75                  80
   Gly Gly Phe Met Phe Gly Trp Asp Thr Ser Thr Ile Ser Gly Phe Val
                       85                  90                  95
   Val Gln Thr Asp Phe Leu Arg Arg Phe Gly Met Lys His Lys Asp Gly
                   100                 105                 110
   Thr His Tyr Leu Ser Asn Val Arg Thr Gly Leu Ile Val Ala Ile Phe
           115                 120                 125
   Asn Ile Gly Cys Ala Phe Gly Gly Ile Ile Leu Ser Lys Gly Gly Asp
       130                 135                 140
   Met Tyr Gly Arg Lys Lys Gly Leu Ser Ile Val Val Ser Val Tyr Ile
   145                 150                 155                 160
   Val Gly Ile Ile Ile Gln Ile Ala Ser Ile Asn Lys Trp Tyr Gln Tyr
                   165                 170                 175
   Phe Ile Gly Arg Ile Ile Ser Gly Leu Gly Val Gly Gly Ile Ala Val
               180                 185                 190
   Leu Cys Pro Met Leu Ile Ser Glu Ile Ala Pro Lys His Leu Arg Gly
           195                 200                 205
   Thr Leu Val Ser Cys Tyr Gln Leu Met Ile Thr Ala Gly Ile Phe Leu
       210                 215                 220
   Gly Tyr Cys Thr Asn Tyr Gly Thr Lys Ser Tyr Ser Asn Ser Val Gln
   225                 230                 235                 240
   Trp Arg Val Pro Leu Gly Leu Cys Phe Ala Trp Ser Leu Phe Met Ile
                   245                 250                 255
   Gly Ala Leu Thr Leu Val Pro Glu Ser Pro Arg Tyr Leu Cys Glu Val
               260                 265                 270
   Asn Lys Val Glu Asp Ala Lys Arg Ser Ile Ala Lys Ser Asn Lys Val
           275                 280                 285
   Ser Pro Glu Asp Pro Ala Val Gln Ala Glu Leu Asp Leu Ile Met Ala
       290                 295                 300
   Gly Ile Glu Ala Glu Lys Leu Ala Gly Asn Ala Ser Trp Gly Glu Leu
   305                 310                 315                 320
   Phe Ser Thr Lys Thr Lys Val Phe Gln Arg Leu Leu Met Gly Val Phe
                   325                 330                 335
   Val Gln Met Phe Gln Gln Leu Thr Gly Asn Asn Tyr Phe Phe Tyr Tyr
               340                 345                 350
   Gly Thr Val Ile Phe Lys Ser Val Gly Leu Asp Asp Ser Phe Glu Thr
           355                 360                 365
   Ser Ile Val Ile Gly Val Val Asn Phe Ala Ser Thr Phe Phe Ser Leu
       370                 375                 380
   Trp Thr Val Glu Asn Leu Gly Arg Arg Lys Cys Leu Leu Leu Gly Ala
   385                 390                 395                 400
   Ala Thr Met Met Ala Cys Met Val Ile Tyr Ala Ser Val Gly Val Thr
                   405                 410                 415
   Arg Leu Tyr Pro His Gly Lys Ser Gln Pro Ser Ser Lys Gly Ala Gly
               420                 425                 430
   Asn Cys Met Ile Val Phe Thr Cys Phe Tyr Ile Phe Cys Tyr Ala Thr
           435                 440                 445
   Thr Trp Ala Pro Val Ala Trp Val Ile Thr Ala Glu Ser Phe Pro Leu
       450                 455                 460
   Arg Val Lys Ser Lys Cys Met Ala Leu Ala Ser Ala Ser Asn Trp Val
   465                 470                 475                 480
   Trp Gly Phe Leu Ile Ala Phe Phe Thr Pro Phe Ile Thr Ser Ala Ile
                   485                 490                 495
   Asn Phe Tyr Tyr Gly Tyr Val Phe Met Gly Cys Leu Val Ala Met Phe
               500                 505                 510
   Phe Tyr Val Phe Phe Phe Val Pro Glu Thr Lys Gly Leu Ser Leu Glu
           515                 520                 525
   Glu Ile Gln Glu Leu Trp Glu Glu Gly Val Leu Pro Trp Lys Ser Glu
       530                 535                 540
   Gly Trp Ile Pro Ser Ser Arg Arg Gly Asn Asn Tyr Asp Leu Glu Asp
   545                 550                 555                 560
   Leu Gln His Asp Asp Lys Pro Trp Tyr Lys Ala Met Leu Glu
                   565                 570
<210> 45
   <211> 2100
   <212> DNA
   <213> Saccharomyces cerevisiae
<220>
   <221> CDS
   <222> (1)...(2100)
   <223> DNA encodes ScGAL10
<400> 45
   atg aca gct cag tta caa agt gaa agt act tct aaa att gtt ttg gtt      48
   Met Thr Ala Gln Leu Gln Ser Glu Ser Thr Ser Lys Ile Val Leu Val
    1               5                  10                  15
   aca ggt ggt gct gga tac att ggt tca cac act gtg gta gag cta att      96
   Thr Gly Gly Ala Gly Tyr Ile Gly Ser His Thr Val Val Glu Leu Ile
               20                  25                  30
   gag aat gga tat gac tgt gtt gtt gct gat aac ctg tcg aat tca act     144
   Glu Asn Gly Tyr Asp Cys Val Val Ala Asp Asn Leu Ser Asn Ser Thr
           35                  40                  45
   tat gat tct gta gcc agg tta gag gtc ttg acc aag cat cac att ccc     192
   Tyr Asp Ser Val Ala Arg Leu Glu Val Leu Thr Lys His His Ile Pro
       50                  55                  60
   ttc tat gag gtt gat ttg tgt gac cga aaa ggt ctg gaa aag gtt ttc     240
   Phe Tyr Glu Val Asp Leu Cys Asp Arg Lys Gly Leu Glu Lys Val Phe
   65                  70                  75                  80
   aaa gaa tat aaa att gat tcg gta att cac ttt gct ggt tta aag gct     288
   Lys Glu Tyr Lys Ile Asp Ser Val Ile His Phe Ala Gly Leu Lys Ala
                   85                  90                  95
   gta ggt gaa tct aca caa atc ccg ctg aga tac tat cac aat aac att     336
   Val Gly Glu Ser Thr Gln Ile Pro Leu Arg Tyr Tyr His Asn Asn Ile
               100                 105                 110
   ttg gga act gtc gtt tta tta gag tta atg caa caa tac aac gtt tcc     384
   Leu Gly Thr Val Val Leu Leu Glu Leu Met Gln Gln Tyr Asn Val Ser
           115                 120                 125
   aaa ttt gtt ttt tca tct tct gct act gtc tat ggt gat gct acg aga     432
   Lys Phe Val Phe Ser Ser Ser Ala Thr Val Tyr Gly Asp Ala Thr Arg
       130                 135                 140
   ttc cca aat atg att cct atc cca gaa gaa tgt ccc tta ggg cct act     480
   Phe Pro Asn Met Ile Pro Ile Pro Glu Glu Cys Pro Leu Gly Pro Thr
   145                 150                 155                 160
   aat ccg tat ggt cat acg aaa tac gcc att gag aat atc ttg aat gat     528
   Asn Pro Tyr Gly His Thr Lys Tyr Ala Ile Glu Asn Ile Leu Asn Asp
                   165                 170                 175
   ctt tac aat agc gac aaa aaa agt tgg aag ttt gct atc ttg cgt tat     576
   Leu Tyr Asn Ser Asp Lys Lys Ser Trp Lys Phe Ala Ile Leu Arg Tyr
               180                 185                 190
   ttt aac cca att ggc gca cat ccc tct gga tta atc gga gaa gat ccg    624
   Phe Asn Pro Ile Gly Ala His Pro Ser Gly Leu Ile Gly Glu Asp Pro
           195                 200                 205
   cta ggt ata cca aac aat ttg ttg cca tat atg gct caa gta gct gtt    672
   Leu Gly Ile Pro Asn Asn Leu Leu Pro Tyr Met Ala Gln Val Ala Val
       210                 215                 220
   ggt agg cgc gag aag ctt tac atc ttc gga gac gat tat gat tcc aga    720
   Gly Arg Arg Glu Lys Leu Tyr Ile Phe Gly Asp Asp Tyr Asp Ser Arg
   225                 230                 235                 240
   gat ggt acc ccg atc agg gat tat atc cac gta gtt gat cta gca aaa    768
   Asp Gly Thr Pro Ile Arg Asp Tyr Ile His Val Val Asp Leu Ala Lys
                   245                 250                 255
   ggt cat att gca gcc ctg caa tac cta gag gcc tac aat gaa aat gaa    816
   Gly His Ile Ala Ala Leu Gln Tyr Leu Glu Ala Tyr Asn Glu Asn Glu
               260                 265                 270
   ggt ttg tgt cgt gag tgg aac ttg ggt tcc ggt aaa ggt tct aca gtt    864
   Gly Leu Cys Arg Glu Trp Asn Leu Gly Ser Gly Lys Gly Ser Thr Val
           275                 280                 285
   ttt gaa gtt tat cat gca ttc tgc aaa gct tct ggt att gat ctt cca    912
   Phe Glu Val Tyr His Ala Phe Cys Lys Ala Ser Gly Ile Asp Leu Pro
       290                 295                 300
   tac aaa gtt acg ggc aga aga gca ggt gat gtt ttg aac ttg acg gct    960
   Tyr Lys Val Thr Gly Arg Arg Ala Gly Asp Val Leu Asn Leu Thr Ala
   305                 310                 315                 320
   aaa cca gat agg gcc aaa cgc gaa ctg aaa tgg cag acc gag ttg cag    1008
   Lys Pro Asp Arg Ala Lys Arg Glu Leu Lys Trp Gln Thr Glu Leu Gln
                   325                 330                 335
   gtt gaa gac tcc tgc aag gat tta tgg aaa tgg act act gag aat cct    1056
   Val Glu Asp Ser Cys Lys Asp Leu Trp Lys Trp Thr Thr Glu Asn Pro
               340                 345                 350
   ttt ggt tac cag tta agg ggt gtc gag gcc aga ttt tcc gct gaa gat    1104
   Phe Gly Tyr Gln Leu Arg Gly Val Glu Ala Arg Phe Ser Ala Glu Asp
           355                 360                 365
   atg cgt tat gac gca aga ttt gtg act att ggt gcc ggc acc aga ttt    1152
   Met Arg Tyr Asp Ala Arg Phe Val Thr Ile Gly Ala Gly Thr Arg Phe
       370                 375                 380
   caa gcc acg ttt gcc aat ttg ggc gcc agc att gtt gac ctg aaa gtg    1200
   Gln Ala Thr Phe Ala Asn Leu Gly Ala Ser Ile Val Asp Leu Lys Val
   385                 390                 395                 400
   aac gga caa tca gtt gtt ctt ggc tat gaa aat gag gaa ggg tat ttg    1248
   Asn Gly Gln Ser Val Val Leu Gly Tyr Glu Asn Glu Glu Gly Tyr Leu
                   405                 410                 415
   aat cct gat agt gct tat ata ggc gcc acg atc ggc agg tat gct aat    1296
   Asn Pro Asp Ser Ala Tyr Ile Gly Ala Thr Ile Gly Arg Tyr Ala Asn
               420                 425                 430
   cgt att tcg aag ggt aag ttt agt tta tgc aac aaa gac tat cag tta    1344
   Arg Ile Ser Lys Gly Lys Phe Ser Leu Cys Asn Lys Asp Tyr Gln Leu
           435                 440                 445
   acc gtt aat aac ggc gtt aat gcg aat cat agt agt atc ggt tct ttc      1392
   Thr Val Asn Asn Gly Val Asn Ala Asn His Ser Ser Ile Gly Ser Phe
       450                 455                 460
   cac aga aaa aga ttt ttg gga ccc atc att caa aat cct tca aag gat      1440
   His Arg Lys Arg Phe Leu Gly Pro Ile Ile Gln Asn Pro Ser Lys Asp
   465                 470                 475                 480
   gtt ttt acc gcc gag tac atg ctg ata gat aat gag aag gac acc gaa      1488
   Val Phe Thr Ala Glu Tyr Met Leu Ile Asp Asn Glu Lys Asp Thr Glu
                   485                 490                 495
   ttt cca ggt gat cta ttg gta acc ata cag tat act gtg aac gtt gcc      1536
   Phe Pro Gly Asp Leu Leu Val Thr Ile Gln Tyr Thr Val Asn Val Ala
               500                 505                 510
   caa aaa agt ttg gaa atg gta tat aaa ggt aaa ttg act gct ggt gaa      1584
   Gln Lys Ser Leu Glu Met Val Tyr Lys Gly Lys Leu Thr Ala Gly Glu
           515                 520                 525
   gcg acg cca ata aat tta aca aat cat agt tat ttc aat ctg aac aag      1632
   Ala Thr Pro Ile Asn Leu Thr Asn His Ser Tyr Phe Asn Leu Asn Lys
       530                 535                 540
   cca tat gga gac act att gag ggt acg gag att atg gtg cgt tca aaa      1680
   Pro Tyr Gly Asp Thr Ile Glu Gly Thr Glu Ile Met Val Arg Ser Lys
   545                 550                 555                 560
   aaa tct gtt gat gtc gac aaa aac atg att cct acg ggt aat atc gtc      1728
   Lys Ser Val Asp Val Asp Lys Asn Met Ile Pro Thr Gly Asn Ile Val
                   565                 570                 575
   gat aga gaa att gct acc ttt aac tct aca aag cca acg gtc tta ggc      1776
   Asp Arg Glu Ile Ala Thr Phe Asn Ser Thr Lys Pro Thr Val Leu Gly
               580                 585                 590
   ccc aaa aat ccc cag ttt gat tgt tgt ttt gtg gtg gat gaa aat gct      1824
   Pro Lys Asn Pro Gln Phe Asp Cys Cys Phe Val Val Asp Glu Asn Ala
           595                 600                 605
   aag cca agt caa atc aat act cta aac aat gaa ttg acg ctt att gtc      1872
   Lys Pro Ser Gln Ile Asn Thr Leu Asn Asn Glu Leu Thr Leu Ile Val
       610                 615                 620
   aag gct ttt cat ccc gat tcc aat att aca tta gaa gtt tta agt aca      1920
   Lys Ala Phe His Pro Asp Ser Asn Ile Thr Leu Glu Val Leu Ser Thr
   625                 630                 635                 640
   gag cca act tat caa ttt tat acc ggt gat ttc ttg tct gct ggt tac      1968
   Glu Pro Thr Tyr Gln Phe Tyr Thr Gly Asp Phe Leu Ser Ala Gly Tyr
                   645                 650                 655
   gaa gca aga caa ggt ttt gca att gag cct ggt aga tac att gat gct      2016
   Glu Ala Arg Gln Gly Phe Ala Ile Glu Pro Gly Arg Tyr Ile Asp Ala
               660                 665                 670
   atc aat caa gag aac tgg aaa gat tgt gta acc ttg aaa aac ggt gaa      2064
   Ile Asn Gln Glu Asn Trp Lys Asp Cys Val Thr Leu Lys Asn Gly Glu
               675                 680                 685
   act tac ggg tcc aag att gtc tac aga ttt tcc tga                      2100
   Thr Tyr Gly Ser Lys Ile Val Tyr Arg Phe Ser  *
       690                 695
<210> 46
   <211> 699
   <212> PRT
   <213> Saccharomyces cerevisiae
<400> 46
   Met Thr Ala Gln Leu Gln Ser Glu Ser Thr Ser Lys Ile Val Leu Val
   1               5                   10                  15
   Thr Gly Gly Ala Gly Tyr Ile Gly Ser His Thr Val Val Glu Leu Ile
               20                  25                  30
   Glu Asn Gly Tyr Asp Cys Val Val Ala Asp Asn Leu Ser Asn Ser Thr
           35                  40                  45
   Tyr Asp Ser Val Ala Arg Leu Glu Val Leu Thr Lys His His Ile Pro
       50                  55                  60
   Phe Tyr Glu Val Asp Leu Cys Asp Arg Lys Gly Leu Glu Lys Val Phe
   65                  70                  75                  80
   Lys Glu Tyr Lys Ile Asp Ser Val Ile His Phe Ala Gly Leu Lys Ala
                   85                  90                  95
   Val Gly Glu Ser Thr Gln Ile Pro Leu Arg Tyr Tyr His Asn Asn Ile
               100                 105                 110
   Leu Gly Thr Val Val Leu Leu Glu Leu Met Gln Gln Tyr Asn Val Ser
           115                 120                 125
   Lys Phe Val Phe Ser Ser Ser Ala Thr Val Tyr Gly Asp Ala Thr Arg
       130                 135                 140
   Phe Pro Asn Met Ile Pro Ile Pro Glu Glu Cys Pro Leu Gly Pro Thr
   145                 150                 155                 160
   Asn Pro Tyr Gly His Thr Lys Tyr Ala Ile Glu Asn Ile Leu Asn Asp
                   165                 170                 175
   Leu Tyr Asn Ser Asp Lys Lys Ser Trp Lys Phe Ala Ile Leu Arg Tyr
               180                 185                 190
   Phe Asn Pro Ile Gly Ala His Pro Ser Gly Leu Ile Gly Glu Asp Pro
           195                 200                 205
   Leu Gly Ile Pro Asn Asn Leu Leu Pro Tyr Met Ala Gln Val Ala Val
       210                 215                 220
   Gly Arg Arg Glu Lys Leu Tyr Ile Phe Gly Asp Asp Tyr Asp Ser Arg
   225                 230                 235                 240
   Asp Gly Thr Pro Ile Arg Asp Tyr Ile His Val Val Asp Leu Ala Lys
                   245                 250                 255
   Gly His Ile Ala Ala Leu Gln Tyr Leu Glu Ala Tyr Asn Glu Asn Glu
               260                 265                 270
   Gly Leu Cys Arg Glu Trp Asn Leu Gly Ser Gly Lys Gly Ser Thr Val
           275                 280                 285
   Phe Glu Val Tyr His Ala Phe Cys Lys Ala Ser Gly Ile Asp Leu Pro
       290                 295                 300
   Tyr Lys Val Thr Gly Arg Arg Ala Gly Asp Val Leu Asn Leu Thr Ala
   305                 310                 315                 320
   Lys Pro Asp Arg Ala Lys Arg Glu Leu Lys Trp Gln Thr Glu Leu Gln
                   325                 330                 335
   Val Glu Asp Ser Cys Lys Asp Leu Trp Lys Trp Thr Thr Glu Asn Pro
               340                 345                 350
   Phe Gly Tyr Gln Leu Arg Gly Val Glu Ala Arg Phe Ser Ala Glu Asp
           355                 360                 365
   Met Arg Tyr Asp Ala Arg Phe Val Thr Ile Gly Ala Gly Thr Arg Phe
       370                 375                 380
   Gln Ala Thr Phe Ala Asn Leu Gly Ala Ser Ile Val Asp Leu Lys Val
   385                 390                 395                 400
   Asn Gly Gln Ser Val Val Leu Gly Tyr Glu Asn Glu Glu Gly Tyr Leu
                   405                 410                 415
   Asn Pro Asp Ser Ala Tyr Ile Gly Ala Thr Ile Gly Arg Tyr Ala Asn
               420                 425                 430
   Arg Ile Ser Lys Gly Lys Phe Ser Leu Cys Asn Lys Asp Tyr Gln Leu
           435                 440                 445
   Thr Val Asn Asn Gly Val Asn Ala Asn His Ser Ser Ile Gly Ser Phe
       450                 455                 460
   His Arg Lys Arg Phe Leu Gly Pro Ile Ile Gln Asn Pro Ser Lys Asp
   465                 470                 475                 480
   Val Phe Thr Ala Glu Tyr Met Leu Ile Asp Asn Glu Lys Asp Thr Glu
                   485                 490                 495
   Phe Pro Gly Asp Leu Leu Val Thr Ile Gln Tyr Thr Val Asn Val Ala
               500                 505                 510
   Gln Lys Ser Leu Glu Met Val Tyr Lys Gly Lys Leu Thr Ala Gly Glu
           515                 520                 525
   Ala Thr Pro Ile Asn Leu Thr Asn His Ser Tyr Phe Asn Leu Asn Lys
       530                 535                 540
   Pro Tyr Gly Asp Thr Ile Glu Gly Thr Glu Ile Met Val Arg Ser Lys
   545                 550                 555                 560
   Lys Ser Val Asp Val Asp Lys Asn Met Ile Pro Thr Gly Asn Ile Val
                   565                 570                 575
   Asp Arg Glu Ile Ala Thr Phe Asn Ser Thr Lys Pro Thr Val Leu Gly
               580                 585                 590
   Pro Lys Asn Pro Gln Phe Asp Cys Cys Phe Val Val Asp Glu Asn Ala
           595                 600                 605
   Lys Pro Ser Gln Ile Asn Thr Leu Asn Asn Glu Leu Thr Leu Ile Val
       610                 615                 620
   Lys Ala Phe His Pro Asp Ser Asn Ile Thr Leu Glu Val Leu Ser Thr
   625                 630                 635                 640
   Glu Pro Thr Tyr Gln Phe Tyr Thr Gly Asp Phe Leu Ser Ala Gly Tyr
                   645                 650                 655
   Glu Ala Arg Gln Gly Phe Ala Ile Glu Pro Gly Arg Tyr Ile Asp Ala
               660                 665                 670
   Ile Asn Gln Glu Asn Trp Lys Asp Cys Val Thr Leu Lys Asn Gly Glu
           675                 680                 685
   Thr Tyr Gly Ser Lys Ile Val Tyr Arg Phe Ser
       690                 695
<210> 47
   <211> 1047
   <212> DNA
   <213> Homo sapiens
<220>
   <221> CDS
   <222> (1)...(1097)
   <223> DNA encodes human GalE
<400> 47
   atg gca gag aag gtg ctg gta aca ggt ggg gct ggc tac att ggc agc    48
   Met Ala Glu Lys Val Leu Val Thr Gly Gly Ala Gly Tyr Ile Gly Ser
    1               5                  10                  15
   cac acg gtg ctg gag ctg ctg gag gct ggc tac ttg cct gtg gtc atc    96
   His Thr Val Leu Glu Leu Leu Glu Ala Gly Tyr Leu Pro Val Val Ile
               20                  25                  30
   gat aac ttc cat aat gcc ttc cgt gga ggg ggc tcc ctg cct gag agc     144
   Asp Asn Phe His Asn Ala Phe Arg Gly Gly Gly Ser Leu Pro Glu Ser
           35                  40                  45
   ctg cgg cgg gtc cag gag ctg aca ggc cgc tct gtg gag ttt gag gag     192
   Leu Arg Arg Val Gln Glu Leu Thr Gly Arg Ser Val Glu Phe Glu Glu
       50                  55                  60
   atg gac att ttg gac cag gga gcc cta cag cgt ctc ttc aaa aag tac     240
   Met Asp Ile Leu Asp Gln Gly Ala Leu Gln Arg Leu Phe Lys Lys Tyr
   65                  70                  75                  80
   agc ttt atg gcg gtc atc cac ttt gcg ggg ctc aag gcc gtg ggc gag     288
   Ser Phe Met Ala Val Ile His Phe Ala Gly Leu Lys Ala Val Gly Glu
                   85                  90                  95
   tcg gtg cag aag cct ctg gat tat tac aga gtt aac ctg acc ggg acc     336
   Ser Val Gln Lys Pro Leu Asp Tyr Tyr Arg Val Asn Leu Thr Gly Thr
               100                 105                 110
   atc cag ctt ctg gag atc atg aag gcc cac ggg gtg aag aac ctg gtg     384
   Ile Gln Leu Leu Glu Ile Met Lys Ala His Gly Val Lys Asn Leu Val
           115                 120                 125
   ttc agc agc tca gcc act gtg tac ggg aac ccc cag tac ctg ccc ctt     432
   Phe Ser Ser Ser Ala Thr Val Tyr Gly Asn Pro Gln Tyr Leu Pro Leu
       130                 135                 140
   gat gag gcc cac ccc acg ggt ggt tgt acc aac cct tac ggc aag tcc     480
   Asp Glu Ala His Pro Thr Gly Gly Cys Thr Asn Pro Tyr Gly Lys Ser
   145                 150                 155                 160
   aag ttc ttc atc gag gaa atg atc cgg gac ctg tgc cag gca gac aag     528
   Lys Phe Phe Ile Glu Glu Met Ile Arg Asp Leu Cys Gln Ala Asp Lys
                   165                 170                 175
   act tgg aac gca gtg ctg ctg cgc tat ttc aac ccc aca ggt gcc cat     576
   Thr Trp Asn Ala Val Leu Leu Arg Tyr Phe Asn Pro Thr Gly Ala His
               180                 185                 190
   gcc tct ggc tgc att ggt gag gat ccc cag ggc ata ccc aac aac ctc     624
   Ala Ser Gly Cys Ile Gly Glu Asp Pro Gln Gly Ile Pro Asn Asn Leu
           195                 200                 205
   atg cct tat gtc tcc cag gtg gcg atc ggg cga cgg gag gcc ctg aat     672
   Met Pro Tyr Val Ser Gln Val Ala Ile Gly Arg Arg Glu Ala Leu Asn
       210                 215                 220
   gtc ttt ggc aat gac tat gac aca gag gat ggc aca ggt gtc cgg gat     720
   Val Phe Gly Asn Asp Tyr Asp Thr Glu Asp Gly Thr Gly Val Arg Asp
   225                 230                 235                 240
   tac atc cat gtc gtg gat ctg gcc aag ggc cac att gca gcc tta agg     768
   Tyr Ile His Val Val Asp Leu Ala Lys Gly His Ile Ala Ala Leu Arg
                   245                 250                 255
   aag ctg aaa gaa cag tgt ggc tgc cgg atc tac aac ctg ggc acg ggc     816
   Lys Leu Lys Glu Gln Cys Gly Cys Arg Ile Tyr Asn Leu Gly Thr Gly
               260                 265                 270
   aca ggc tat tca gtg ctg cag atg gtc cag gct atg gag aag gcc tct     864
   Thr Gly Tyr Ser Val Leu Gln Met Val Gln Ala Met Glu Lys Ala Ser
           275                 280                 285
   ggg aag aag atc ccg tac aag gtg gtg gca cgg cgg gaa ggt gat gtg      912
   Gly Lys Lys Ile Pro Tyr Lys Val Val Ala Arg Arg Glu Gly Asp Val
       290                 295                 300
   gca gcc tgt tac gcc aac ccc agc ctg gcc caa gag gag ctg ggg tgg      960
   Ala Ala Cys Tyr Ala Asn Pro Ser Leu Ala Gln Glu Glu Leu Gly Trp
   305                 310                 315                 320
   aca gca gcc tta ggg ctg gac agg atg tgt gag gat ctc tgg cgc tgg     1008
   Thr Ala Ala Leu Gly Leu Asp Arg Met Cys Glu Asp Leu Trp Arg Trp
                   325                 330                 335
   cag aag cag aat cct tca ggc ttt ggc acg caa gcc tga                 1047
   Gln Lys Gln Asn Pro Ser Gly Phe Gly Thr Gln Ala  *
               340                 345
<210> 48
   <211> 348
   <212> PRT
   <213> Homo sapiens
<400> 48
   Met Ala Glu Lys Val Leu Val Thr Gly Gly Ala Gly Tyr Ile Gly Ser
    1               5                  10                  15
   His Thr Val Leu Glu Leu Leu Glu Ala Gly Tyr Leu Pro Val Val Ile
               20                  25                  30
   Asp Asn Phe His Asn Ala Phe Arg Gly Gly Gly Ser Leu Pro Glu Ser
           35                  40                  45
   Leu Arg Arg Val Gln Glu Leu Thr Gly Arg Ser Val Glu Phe Glu Glu
       50                  55                  60
   Met Asp Ile Leu Asp Gln Gly Ala Leu Gln Arg Leu Phe Lys Lys Tyr
   65                  70                  75                  80
   Ser Phe Met Ala Val Ile His Phe Ala Gly Leu Lys Ala Val Gly Glu
                   85                  90                  95
   Ser Val Gln Lys Pro Leu Asp Tyr Tyr Arg Val Asn Leu Thr Gly Thr
               100                 105                 110
   Ile Gln Leu Leu Glu Ile Met Lys Ala His Gly Val Lys Asn Leu Val
           115                 120                 125
   Phe Ser Ser Ser Ala Thr Val Tyr Gly Asn Pro Gln Tyr Leu Pro Leu
       130                 135                 140
   Asp Glu Ala His Pro Thr Gly Gly Cys Thr Asn Pro Tyr Gly Lys Ser
   145                 150                 155                 160
   Lys Phe Phe Ile Glu Glu Met Ile Arg Asp Leu Cys Gln Ala Asp Lys
                   165                 170                 175
   Thr Trp Asn Ala Val Leu Leu Arg Tyr Phe Asn Pro Thr Gly Ala His
               180                 185                 190
   Ala Ser Gly Cys Ile Gly Glu Asp Pro Gln Gly Ile Pro Asn Asn Leu
           195                 200                 205
   Met Pro Tyr Val Ser Gln Val Ala Ile Gly Arg Arg Glu Ala Leu Asn
       210                 215                 220
   Val Phe Gly Asn Asp Tyr Asp Thr Glu Asp Gly Thr Gly Val Arg Asp
   225                 230                 235                 240
   Tyr Ile His Val Val Asp Leu Ala Lys Gly His Ile Ala Ala Leu Arg
                   245                 250                 255
   Lys Leu Lys Glu Gln Cys Gly Cys Arg Ile Tyr Asn Leu Gly Thr Gly
               260                 265                 270
   Thr Gly Tyr Ser Val Leu Gln Met Val Gln Ala Met Glu Lys Ala Ser
           275                 280                 285
   Gly Lys Lys Ile Pro Tyr Lys Val Val Ala Arg Arg Glu Gly Asp Val
       290                 295                 300
   Ala Ala Cys Tyr Ala Asn Pro Ser Leu Ala Gln Glu Glu Leu Gly Trp
   305                 310                 315                 320
   Thr Ala Ala Leu Gly Leu Asp Arg Met Cys Glu Asp Leu Trp Arg Trp
                   325                 330                 335
   Gln Lys Gln Asn Pro Ser Gly Phe Gly Thr Gln Ala
               340                 345
<210> 49
   <211> 1068
   <212> DNA
   <213> Artificial Sequence
<220>
   <223> DNA encodes hGalT I catalytic domain
<220>
   <221> CDS
   <222> (1)...(1068)
   <223> hGalT catalytic domain
<400> 49
   ggc cgc gac ctg agc cgc ctg ccc caa ctg gtc gga gtc tcc aca ccg      48
   Gly Arg Asp Leu Ser Arg Leu Pro Gln Leu Val Gly Val Ser Thr Pro
    1               5                  10                  15
   ctg cag ggc ggc tcg aac agt gcc gcc gcc atc ggg cag tcc tcc ggg      96
   Leu Gln Gly Gly Ser Asn Ser Ala Ala Ala Ile Gly Gln Ser Ser Gly
               20                  25                  30
   gag ctc cgg acc gga ggg gcc cgg ccg ccg cct cct cta ggc gcc tcc      144
   Glu Leu Arg Thr Gly Gly Ala Arg Pro Pro Pro Pro Leu Gly Ala Ser
           35                  40                  45
   tcc cag ccg cgc ccg ggt ggc gac tcc agc cca gtc gtg gat tct ggc      192
   Ser Gln Pro Arg Pro Gly Gly Asp Ser Ser Pro Val Val Asp Ser Gly
       50                  55                  60
   cct ggc ccc gct agc aac ttg acc tcg gtc cca gtg ccc cac acc acc      240
   Pro Gly Pro Ala Ser Asn Leu Thr Ser Val Pro Val Pro His Thr Thr
   65                  70                  75                  80
   gca ctg tcg ctg ccc gcc tgc cct gag gag tcc ccg ctg ctt gtg ggc      288
   Ala Leu Ser Leu Pro Ala Cys Pro Glu Glu Ser Pro Leu Leu Val Gly
                   85                  90                  95
   ccc atg ctg att gag ttt aac atg cct gtg gac ctg gag ctc gtg gca      336
   Pro Met Leu Ile Glu Phe Asn Met Pro Val Asp Leu Glu Leu Val Ala
               100                 105                 110
   aag cag aac cca aat gtg aag atg ggc ggc cgc tat gcc ccc agg gac      384
   Lys Gln Asn Pro Asn Val Lys Met Gly Gly Arg Tyr Ala Pro Arg Asp
           115                 120                 125
   tgc gtc tct cct cac aag gtg gcc atc atc att cca ttc cgc aac cgg      432
   Cys Val Ser Pro His Lys Val Ala Ile Ile Ile Pro Phe Arg Asn Arg
       130                 135                 140
   cag gag cac ctc aag tac tgg cta tat tat ttg cac cca gtc ctg cag     480
   Gln Glu His Leu Lys Tyr Trp Leu Tyr Tyr Leu His Pro Val Leu Gln
   145                 150                 155                 160
   cgc cag cag ctg gac tat ggc atc tat gtt atc aac cag gcg gga gac     528
   Arg Gln Gln Leu Asp Tyr Gly Ile Tyr Val Ile Asn Gln Ala Gly Asp
                   165                 170                 175
   act ata ttc aat cgt gct aag ctc ctc aat gtt ggc ttt caa gaa gcc     576
   Thr Ile Phe Asn Arg Ala Lys Leu Leu Asn Val Gly Phe Gln Glu Ala
               180                 185                 190
   ttg aag gac tat gac tac acc tgc ttt gtg ttt agt gac gtg gac ctc     624
   Leu Lys Asp Tyr Asp Tyr Thr Cys Phe Val Phe Ser Asp Val Asp Leu
           195                 200                 205
   att cca atg aat gac cat aat gcg tac agg tgt ttt tca cag cca cgg     672
   Ile Pro Met Asn Asp His Asn Ala Tyr Arg Cys Phe Ser Gln Pro Arg
       210                 215                 220
   cac att tcc gtt gca atg gat aag ttt gga ttc agc cta cct tat gtt     720
   His Ile Ser Val Ala Met Asp Lys Phe Gly Phe Ser Leu Pro Tyr Val
   225                 230                 235                 240
   cag tat ttt gga ggt gtc tct gct cta agt aaa caa cag ttt cta acc     768
   Gln Tyr Phe Gly Gly Val Ser Ala Leu Ser Lys Gln Gln Phe Leu Thr
                   245                 250                 255
   atc aat gga ttt cct aat aat tat tgg ggc tgg gga gga gaa gat gat     816
   Ile Asn Gly Phe Pro Asn Asn Tyr Trp Gly Trp Gly Gly Glu Asp Asp
               260                 265                 270
   gac att ttt aac aga tta gtt ttt aga ggc atg tct ata tct cgc cca     864
   Asp Ile Phe Asn Arg Leu Val Phe Arg Gly Met Ser Ile Ser Arg Pro
           275                 280                 285
   aat gct gtg gtc ggg agg tgt cgc atg atc cgc cac tca aga gac aag     912
   Asn Ala Val Val Gly Arg Cys Arg Met Ile Arg His Ser Arg Asp Lys
       290                 295                 300
   aaa aat gaa ccc aat cct cag agg ttt gac cga att gca cac aca aag     960
   Lys Asn Glu Pro Asn Pro Gln Arg Phe Asp Arg Ile Ala His Thr Lys
   305                 310                 315                 320
   gag aca atg ctc tct gat ggt ttg aac tca ctc acc tac cag gtg ctg     1008
   Glu Thr Met Leu Ser Asp Gly Leu Asn Ser Leu Thr Tyr Gln Val Leu
                   325                 330                 335
   gat gta cag aga tac cca ttg tat acc caa atc aca gtg gac atc ggg     1056
   Asp Val Gln Arg Tyr Pro Leu Tyr Thr Gln Ile Thr Val Asp Ile Gly
               340                 345                 350
   aca ccg agc tag                                                     1068
   Thr Pro Ser  *
           355
<210> 50
   <211> 355
   <212> PRT
   <213> Artificial Sequence
<400> 50
   Gly Arg Asp Leu Ser Arg Leu Pro Gln Leu Val Gly Val Ser Thr Pro
   1               5                   10                  15
   Leu Gln Gly Gly Ser Asn Ser Ala Ala Ala Ile Gly Gln Ser Ser Gly
               20                  25                  30
   Glu Leu Arg Thr Gly Gly Ala Arg Pro Pro Pro Pro Leu Gly Ala Ser
           35                  40                  45
   Ser Gln Pro Arg Pro Gly Gly Asp Ser Ser Pro Val Val Asp Ser Gly
       50                  55                  60
   Pro Gly Pro Ala Ser Asn Leu Thr Ser Val Pro Val Pro His Thr Thr
   65                  70                  75                  80
   Ala Leu Ser Leu Pro Ala Cys Pro Glu Glu Ser Pro Leu Leu Val Gly
                   85                  90                  95
   Pro Met Leu Ile Glu Phe Asn Met Pro Val Asp Leu Glu Leu Val Ala
               100                 105                 110
   Lys Gln Asn Pro Asn Val Lys Met Gly Gly Arg Tyr Ala Pro Arg Asp
           115                 120                 125
   Cys Val Ser Pro His Lys Val Ala Ile Ile Ile Pro Phe Arg Asn Arg
       130                 135                 140
   Gln Glu His Leu Lys Tyr Trp Leu Tyr Tyr Leu His Pro Val Leu Gln
   145                 150                 155                 160
   Arg Gln Gln Leu Asp Tyr Gly Ile Tyr Val Ile Asn Gln Ala Gly Asp
                   165                 170                 175
   Thr Ile Phe Asn Arg Ala Lys Leu Leu Asn Val Gly Phe Gln Glu Ala
               180                 185                 190
   Leu Lys Asp Tyr Asp Tyr Thr Cys Phe Val Phe Ser Asp Val Asp Leu
           195                 200                 205
   Ile Pro Met Asn Asp His Asn Ala Tyr Arg Cys Phe Ser Gln Pro Arg
       210                 215                 220
   His Ile Ser Val Ala Met Asp Lys Phe Gly Phe Ser Leu Pro Tyr Val
   225                 230                 235                 240
   Gln Tyr Phe Gly Gly Val Ser Ala Leu Ser Lys Gln Gln Phe Leu Thr
                   245                 250                 255
   Ile Asn Gly Phe Pro Asn Asn Tyr Trp Gly Trp Gly Gly Glu Asp Asp
               260                 265                 270
   Asp Ile Phe Asn Arg Leu Val Phe Arg Gly Met Ser Ile Ser Arg Pro
           275                 280                 285
   Asn Ala Val Val Gly Arg Cys Arg Met Ile Arg His Ser Arg Asp Lys
       290                 295                 300
   Lys Asn Glu Pro Asn Pro Gln Arg Phe Asp Arg Ile Ala His Thr Lys
   305                 310                 315                 320
   Glu Thr Met Leu Ser Asp Gly Leu Asn Ser Leu Thr Tyr Gln Val Leu
                   325                 330                 335
   Asp Val Gln Arg Tyr Pro Leu Tyr Thr Gln Ile Thr Val Asp Ile Gly
               340                 345                 350
   Thr Pro Ser
           355
<210> 51
   <211> 1224
   <212> DNA
   <213> Artificial Sequence
<220>
   <223> DNA encodes hGnT I catalytic domain codon-optimized
<220>
   <221> CDS
   <222> (1) ... (1224)
   <223> hGnT I catalytic domain
<400> 51
   tca gtc agt gct ctt gat ggt gac cca gca agt ttg acc aga gaa gtg      48
   Ser Val Ser Ala Leu Asp Gly Asp Pro Ala Ser Leu Thr Arg Glu Val
   1               5                   10                  15
   att aga ttg gcc caa gac gca gag gtg gag ttg gag aga caa cgt gga      96
   Ile Arg Leu Ala Gln Asp Ala Glu Val Glu Leu Glu Arg Gln Arg Gly
               20                  25                  30
   ctg ctg cag caa atc gga gat gca ttg tct agt caa aga ggt agg gtg     144
   Leu Leu Gln Gln Ile Gly Asp Ala Leu Ser Ser Gln Arg Gly Arg Val
           35                  40                  45
   cct acc gca gct cct cca gca cag cct aga gtg cat gtg acc cct gca     192
   Pro Thr Ala Ala Pro Pro Ala Gln Pro Arg Val His Val Thr Pro Ala
       50                  55                  60
   cca gct gtg att cct atc ttg gtc atc gcc tgt gac aga tct act gtt     240
   Pro Ala Val Ile Pro Ile Leu Val Ile Ala Cys Asp Arg Ser Thr Val
   65                  70                  75                  80
   aga aga tgt ctg gac aag ctg ttg cat tac aga cca tct gct gag ttg     288
   Arg Arg Cys Leu Asp Lys Leu Leu His Tyr Arg Pro Ser Ala Glu Leu
                   85                  90                  95
   ttc cct atc atc gtt agt caa gac tgt ggt cac gag gag act gcc caa     336
   Phe Pro Ile Ile Val Ser Gln Asp Cys Gly His Glu Glu Thr Ala Gln
               100                 105                 110
   gcc atc gcc tcc tac gga tct gct gtc act cac atc aga cag cct gac     384
   Ala Ile Ala Ser Tyr Gly Ser Ala Val Thr His Ile Arg Gln Pro Asp
           115                 120                 125
   ctg tca tct att gct gtg cca cca gac cac aga aag ttc caa ggt tac     432
   Leu Ser Ser Ile Ala Val Pro Pro Asp His Arg Lys Phe Gln Gly Tyr
       130                 135                 140
   tac aag atc gct aga cac tac aga tgg gca ttg ggt caa gtc ttc aga     480
   Tyr Lys Ile Ala Arg His Tyr Arg Trp Ala Leu Gly Gln Val Phe Arg
   145                 150                 155                 160
   cag ttt aga ttc cct gct gct gtg gtg gtg gag gat gac ttg gag gtg     528
   Gln Phe Arg Phe Pro Ala Ala Val Val Val Glu Asp Asp Leu Glu Val
                   165                 170                 175
   gct cct gac ttc ttt gag tac ttt aga gca acc tat cca ttg ctg aag     576
   Ala Pro Asp Phe Phe Glu Tyr Phe Arg Ala Thr Tyr Pro Leu Leu Lys
               180                 185                 190
   gca gac cca tcc ctg tgg tgt gtc tct gcc tgg aat gac aac ggt aag     624
   Ala Asp Pro Ser Leu Trp Cys Val Ser Ala Trp Asn Asp Asn Gly Lys
           195                 200                 205
   gag caa atg gtg gac gct tct agg cct gag ctg ttg tac aga acc gac     672
   Glu Gln Met Val Asp Ala Ser Arg Pro Glu Leu Leu Tyr Arg Thr Asp
       210                 215                 220
   ttc ttt cct ggt ctg gga tgg ttg ctg ttg gct gag ttg tgg gct gag     720
   Phe Phe Pro Gly Leu Gly Trp Leu Leu Leu Ala Glu Leu Trp Ala Glu
   225                 230                 235                 240
   ttg gag cct aag tgg cca aag gca ttc tgg gac gac tgg atg aga aga      768
   Leu Glu Pro Lys Trp Pro Lys Ala Phe Trp Asp Asp Trp Met Arg Arg
                   245                 250                 255
   cct gag caa aga cag ggt aga gcc tgt atc aga cct gag atc tca aga      816
   Pro Glu Gln Arg Gln Gly Arg Ala Cys Ile Arg Pro Glu Ile Ser Arg
               260                 265                 270
   acc atg acc ttt ggt aga aag gga gtg tct cac ggt caa ttc ttt gac      864
   Thr Met Thr Phe Gly Arg Lys Gly Val Ser His Gly Gln Phe Phe Asp
           275                 280                 285
   caa cac ttg aag ttt atc aag ctg aac cag caa ttt gtg cac ttc acc      912
   Gln His Leu Lys Phe Ile Lys Leu Asn Gln Gln Phe Val His Phe Thr
       290                 295                 300
   caa ctg gac ctg tct tac ttg cag aga gag gcc tat gac aga gat ttc      960
   Gln Leu Asp Leu Ser Tyr Leu Gln Arg Glu Ala Tyr Asp Arg Asp Phe
   305                 310                 315                 320
   cta gct aga gtc tac gga gct cct caa ctg caa gtg gag aaa gtg agg     1008
   Leu Ala Arg Val Tyr Gly Ala Pro Gln Leu Gln Val Glu Lys Val Arg
                   325                 330                 335
   acc aat gac aga aag gag ttg gga gag gtg aga gtg cag tac act ggt     1056
   Thr Asn Asp Arg Lys Glu Leu Gly Glu Val Arg Val Gln Tyr Thr Gly
               340                 345                 350
   agg gac tcc ttt aag gct ttc gct aag gct ctg ggt gtc atg gat gac     1104
   Arg Asp Ser Phe Lys Ala Phe Ala Lys Ala Leu Gly Val Met Asp Asp
           355                 360                 365
   ctt aag tct gga gtt cct aga gct ggt tac aga ggt att gtc acc ttt     1152
   Leu Lys Ser Gly Val Pro Arg Ala Gly Tyr Arg Gly Ile Val Thr Phe
       370                 375                 380
   caa ttc aga ggt aga aga gtc cac ttg gct cct cca cct act tgg gag     1200
   Gln Phe Arg Gly Arg Arg Val His Leu Ala Pro Pro Pro Thr Trp Glu
   385                 390                 395                 400
   ggt tat gat cct tct tgg aat tag                                     1224
   Gly Tyr Asp Pro Ser Trp Asn  *
                   405
<210> 52
   <211> 407
   <212> PRT
   <213> Artificial Sequence
<400> 52
   Ser Val Ser Ala Leu Asp Gly Asp Pro Ala Ser Leu Thr Arg Glu Val
    1               5                  10                  15
   Ile Arg Leu Ala Gln Asp Ala Glu Val Glu Leu Glu Arg Gln Arg Gly
               20                  25                  30
   Leu Leu Gln Gln Ile Gly Asp Ala Leu Ser Ser Gln Arg Gly Arg Val
           35                  40                  45
   Pro Thr Ala Ala Pro Pro Ala Gln Pro Arg Val His Val Thr Pro Ala
       50                  55                  60
   Pro Ala Val Ile Pro Ile Leu Val Ile Ala Cys Asp Arg Ser Thr Val
   65                  70                  75                  80
   Arg Arg Cys Leu Asp Lys Leu Leu His Tyr Arg Pro Ser Ala Glu Leu
                   85                  90                  95
   Phe Pro Ile Ile Val Ser Gln Asp Cys Gly His Glu Glu Thr Ala Gln
               100                 105                 110
   Ala Ile Ala Ser Tyr Gly Ser Ala Val Thr His Ile Arg Gln Pro Asp
           115                 120                 125
   Leu Ser Ser Ile Ala Val Pro Pro Asp His Arg Lys Phe Gln Gly Tyr
       130                 135                 140
   Tyr Lys Ile Ala Arg His Tyr Arg Trp Ala Leu Gly Gln Val Phe Arg
   145                 150                 155                 160
   Gln Phe Arg Phe Pro Ala Ala Val Val Val Glu Asp Asp Leu Glu Val
                   165                 170                 175
   Ala Pro Asp Phe Phe Glu Tyr Phe Arg Ala Thr Tyr Pro Leu Leu Lys
               180                 185                 190
   Ala Asp Pro Ser Leu Trp Cys Val Ser Ala Trp Asn Asp Asn Gly Lys
           195                 200                 205
   Glu Gln Met Val Asp Ala Ser Arg Pro Glu Leu Leu Tyr Arg Thr Asp
       210                 215                 220
   Phe Phe Pro Gly Leu Gly Trp Leu Leu Leu Ala Glu Leu Trp Ala Glu
   225                 230                 235                 240
   Leu Glu Pro Lys Trp Pro Lys Ala Phe Trp Asp Asp Trp Met Arg Arg
                   245                 250                 255
   Pro Glu Gln Arg Gln Gly Arg Ala Cys Ile Arg Pro Glu Ile Ser Arg
               260                 265                 270
   Thr Met Thr Phe Gly Arg Lys Gly Val Ser His Gly Gln Phe Phe Asp
           275                 280                 285
   Gln His Leu Lys Phe Ile Lys Leu Asn Gln Gln Phe Val His Phe Thr
       290                 295                 300
   Gln Leu Asp Leu Ser Tyr Leu Gln Arg Glu Ala Tyr Asp Arg Asp Phe
   305                 310                 315                 320
   Leu Ala Arg Val Tyr Gly Ala Pro Gln Leu Gln Val Glu Lys Val Arg
                   325                 330                 335
   Thr Asn Asp Arg Lys Glu Leu Gly Glu Val Arg Val Gln Tyr Thr Gly
               340                 345                 350
   Arg Asp Ser Phe Lys Ala Phe Ala Lys Ala Leu Gly Val Met Asp Asp
           355                 360                 365
   Leu Lys Ser Gly Val Pro Arg Ala Gly Tyr Arg Gly Ile Val Thr Phe
       370                 375                 380
   Gln Phe Arg Gly Arg Arg Val His Leu Ala Pro Pro Pro Thr Trp Glu
   385                 390                 395                 400
   Gly Tyr Asp Pro Ser Trp Asn
                   405
<210> 53
   <211> 1407
   <212> DNA
   <213> Artificial Sequence
<220>
   <223> DNA encodes Mm ManI catalytic domain
<220>
   <221> CDS
   <222> (1)...(1407)
   <223> Mm ManI catalytic domain
<400> 53
   gag ccc gct gac gcc acc atc cgt gag aag agg gca aag atc aaa gag     48
   Glu Pro Ala Asp Ala Thr Ile Arg Glu Lys Arg Ala Lys Ile Lys Glu
    1               5                  10                  15
   atg atg acc cat gct tgg aat aat tat aaa cgc tat gcg tgg ggc ttg     96
   Met Met Thr His Ala Trp Asn Asn Tyr Lys Arg Tyr Ala Trp Gly Leu
               20                  25                  30
   aac gaa ctg aaa cct ata tca aaa gaa ggc cat tca agc agt ttg ttt    144
   Asn Glu Leu Lys Pro Ile Ser Lys Glu Gly His Ser Ser Ser Leu Phe
           35                  40                  45
   ggc aac atc aaa gga gct aca ata gta gat gcc ctg gat acc ctt ttc    192
   Gly Asn Ile Lys Gly Ala Thr Ile Val Asp Ala Leu Asp Thr Leu Phe
       50                  55                  60
   att atg ggc atg aag act gaa ttt caa gaa gct aaa tcg tgg att aaa    240
   Ile Met Gly Met Lys Thr Glu Phe Gln Glu Ala Lys Ser Trp Ile Lys
   65                  70                  75                  80
   aaa tat tta gat ttt aat gtg aat gct gaa gtt tct gtt ttt gaa gtc    288
   Lys Tyr Leu Asp Phe Asn Val Asn Ala Glu Val Ser Val Phe Glu Val
                   85                  90                  95
   aac ata cgc ttc gtc ggt gga ctg ctg tca gcc tac tat ttg tcc gga    336
   Asn Ile Arg Phe Val Gly Gly Leu Leu Ser Ala Tyr Tyr Leu Ser Gly
               100                 105                 110
   gag gag ata ttt cga aag aaa gca gtg gaa ctt ggg gta aaa ttg cta    384
   Glu Glu Ile Phe Arg Lys Lys Ala Val Glu Leu Gly Val Lys Leu Leu
           115                 120                 125
   cct gca ttt cat act ccc tct gga ata cct tgg gca ttg ctg aat atg    432
   Pro Ala Phe His Thr Pro Ser Gly Ile Pro Trp Ala Leu Leu Asn Met
       130                 135                 140
   aaa agt ggg atc ggg cgg aac tgg ccc tgg gcc tct gga ggc agc agt    480
   Lys Ser Gly Ile Gly Arg Asn Trp Pro Trp Ala Ser Gly Gly Ser Ser
   145                 150                 155                 160
   atc ctg gcc gaa ttt gga act ctg cat tta gag ttt atg cac ttg tcc    528
   Ile Leu Ala Glu Phe Gly Thr Leu His Leu Glu Phe Met His Leu Ser
                   165                 170                 175
   cac tta tca gga gac cca gtc ttt gcc gaa aag gtt atg aaa att cga    576
   His Leu Ser Gly Asp Pro Val Phe Ala Glu Lys Val Met Lys Ile Arg
               180                 185                 190
   aca gtg ttg aac aaa ctg gac aaa cca gaa ggc ctt tat cct aac tat    624
   Thr Val Leu Asn Lys Leu Asp Lys Pro Glu Gly Leu Tyr Pro Asn Tyr
           195                 200                 205
   ctg aac ccc agt agt gga cag tgg ggt caa cat cat gtg tcg gtt gga    672
   Leu Asn Pro Ser Ser Gly Gln Trp Gly Gln His His Val Ser Val Gly
       210                 215                 220
   gga ctt gga gac agc ttt tat gaa tat ttg ctt aag gcg tgg tta atg    720
   Gly Leu Gly Asp Ser Phe Tyr Glu Tyr Leu Leu Lys Ala Trp Leu Met
   225                 230                 235                 240
   tct gac aag aca gat ctc gaa gcc aag aag atg tat ttt gat gct gtt    768
   Ser Asp Lys Thr Asp Leu Glu Ala Lys Lys Met Tyr Phe Asp Ala Val
                   245                 250                 255
   cag gcc atc gag act cac ttg atc cgc aag tca agt ggg gga cta acg      816
   Gln Ala Ile Glu Thr His Leu Ile Arg Lys Ser Ser Gly Gly Leu Thr
               260                 265                 270
   tac atc gca gag tgg aag ggg ggc ctc ctg gaa cac aag atg ggc cac      864
   Tyr Ile Ala Glu Trp Lys Gly Gly Leu Leu Glu His Lys Met Gly His
           275                 280                 285
   ctg acg tgc ttt gca gga ggc atg ttt gca ctt ggg gca gat gga gct      912
   Leu Thr Cys Phe Ala Gly Gly Met Phe Ala Leu Gly Ala Asp Gly Ala
       290                 295                 300
   ccg gaa gcc cgg gcc caa cac tac ctt gaa ctc gga gct gaa att gcc      960
   Pro Glu Ala Arg Ala Gln His Tyr Leu Glu Leu Gly Ala Glu Ile Ala
   305                 310                 315                 320
   cgc act tgt cat gaa tct tat aat cgt aca tat gtg aag ttg gga ccg     1008
   Arg Thr Cys His Glu Ser Tyr Asn Arg Thr Tyr Val Lys Leu Gly Pro
                   325                 330                 335
   gaa gcg ttt cga ttt gat ggc ggt gtg gaa gct att gcc acg agg caa     1056
   Glu Ala Phe Arg Phe Asp Gly Gly Val Glu Ala Ile Ala Thr Arg Gln
               340                 345                 350
   aat gaa aag tat tac atc tta cgg ccc gag gtc atc gag aca tac atg     1104
   Asn Glu Lys Tyr Tyr Ile Leu Arg Pro Glu Val Ile Glu Thr Tyr Met
           355                 360                 365
   tac atg tgg cga ctg act cac gac ccc aag tac agg acc tgg gcc tgg     1152
   Tyr Met Trp Arg Leu Thr His Asp Pro Lys Tyr Arg Thr Trp Ala Trp
       370                 375                 380
   gaa gcc gtg gag gct cta gaa agt cac tgc aga gtg aac gga ggc tac     1200
   Glu Ala Val Glu Ala Leu Glu Ser His Cys Arg Val Asn Gly Gly Tyr
   385                 390                 395                 400
   tca ggc tta cgg gat gtt tac att gcc cgt gag agt tat gac gat gtc     1248
   Ser Gly Leu Arg Asp Val Tyr Ile Ala Arg Glu Ser Tyr Asp Asp Val
                   405                 410                 415
   cag caa agt ttc ttc ctg gca gag aca ctg aag tat ttg tac ttg ata     1296
   Gln Gln Ser Phe Phe Leu Ala Glu Thr Leu Lys Tyr Leu Tyr Leu Ile
               420                 425                 430
   ttt tcc gat gat gac ctt ctt cca cta gaa cac tgg atc ttc aac acc     1344
   Phe Ser Asp Asp Asp Leu Leu Pro Leu Glu His Trp Ile Phe Asn Thr
           435                 440                 445
   gag gct cat cct ttc cct ata ctc cgt gaa cag aag aag gaa att gat     1392
   Glu Ala His Pro Phe Pro Ile Leu Arg Glu Gln Lys Lys Glu Ile Asp
       450                 455                 460
   ggc aaa gag aaa tga                                                 1407
   Gly Lys Glu Lys  *
   465
<210> 54
   <211> 468
   <212> PRT
   <213> Artificial Sequence
<400> 54
   Glu Pro Ala Asp Ala Thr Ile Arg Glu Lys Arg Ala Lys Ile Lys Glu
   1               5                   10                  15
   Met Met Thr His Ala Trp Asn Asn Tyr Lys Arg Tyr Ala Trp Gly Leu
               20                  25                  30
   Asn Glu Leu Lys Pro Ile Ser Lys Glu Gly His Ser Ser Ser Leu Phe
           35                  40                  45
   Gly Asn Ile Lys Gly Ala Thr Ile Val Asp Ala Leu Asp Thr Leu Phe
       50                  55                  60
   Ile Met Gly Met Lys Thr Glu Phe Gln Glu Ala Lys Ser Trp Ile Lys
   65                  70                  75                  80
   Lys Tyr Leu Asp Phe Asn Val Asn Ala Glu Val Ser Val Phe Glu Val
                   85                  90                  95
   Asn Ile Arg Phe Val Gly Gly Leu Leu Ser Ala Tyr Tyr Leu Ser Gly
               100                 105                 110
   Glu Glu Ile Phe Arg Lys Lys Ala Val Glu Leu Gly Val Lys Leu Leu
           115                 120                 125
   Pro Ala Phe His Thr Pro Ser Gly Ile Pro Trp Ala Leu Leu Asn Met
       130                 135                 140
   Lys Ser Gly Ile Gly Arg Asn Trp Pro Trp Ala Ser Gly Gly Ser Ser
   145                 150                 155                 160
   Ile Leu Ala Glu Phe Gly Thr Leu His Leu Glu Phe Met His Leu Ser
                   165                 170                 175
   His Leu Ser Gly Asp Pro Val Phe Ala Glu Lys Val Met Lys Ile Arg
               180                 185                 190
   Thr Val Leu Asn Lys Leu Asp Lys Pro Glu Gly Leu Tyr Pro Asn Tyr
           195                 200                 205
   Leu Asn Pro Ser Ser Gly Gln Trp Gly Gln His His Val Ser Val Gly
       210                 215                 220
   Gly Leu Gly Asp Ser Phe Tyr Glu Tyr Leu Leu Lys Ala Trp Leu Met
   225                 230                 235                 240
   Ser Asp Lys Thr Asp Leu Glu Ala Lys Lys Met Tyr Phe Asp Ala Val
                   245                 250                 255
   Gln Ala Ile Glu Thr His Leu Ile Arg Lys Ser Ser Gly Gly Leu Thr
               260                 265                 270
   Tyr Ile Ala Glu Trp Lys Gly Gly Leu Leu Glu His Lys Met Gly His
           275                 280                 285
   Leu Thr Cys Phe Ala Gly Gly Met Phe Ala Leu Gly Ala Asp Gly Ala
       290                 295                 300
   Pro Glu Ala Arg Ala Gln His Tyr Leu Glu Leu Gly Ala Glu Ile Ala
   305                 310                 315                 320
   Arg Thr Cys His Glu Ser Tyr Asn Arg Thr Tyr Val Lys Leu Gly Pro
                   325                 330                 335
   Glu Ala Phe Arg Phe Asp Gly Gly Val Glu Ala Ile Ala Thr Arg Gln
               340                 345                 350
   Asn Glu Lys Tyr Tyr Ile Leu Arg Pro Glu Val Ile Glu Thr Tyr Met
           355                 360                 365
   Tyr Met Trp Arg Leu Thr His Asp Pro Lys Tyr Arg Thr Trp Ala Trp
       370                 375                 380
   Glu Ala Val Glu Ala Leu Glu Ser His Cys Arg Val Asn Gly Gly Tyr
   385                 390                 395                 400
   Ser Gly Leu Arg Asp Val Tyr Ile Ala Arg Glu Ser Tyr Asp Asp Val
                   405                 410                 415
   Gln Gln Ser Phe Phe Leu Ala Glu Thr Leu Lys Tyr Leu Tyr Leu Ile
               420                 425                 430
   Phe Ser Asp Asp Asp Leu Leu Pro Leu Glu His Trp Ile Phe Asn Thr
           435                 440                 445
   Glu Ala His Pro Phe Pro Ile Leu Arg Glu Gln Lys Lys Glu Ile Asp
   450 455 460
   Gly Lys Glu Lys
   465
<210> 55
   <211> 1494
   <212> DNA
   <213> Artificial Sequence
<220>
   <223> DNA encodes Tr ManI catalytic domain
<220>
   <221> CDS
   <222> (1)...(1999)
   <223> Tr ManI catalytic domain
<400> 55
   cgc gcc gga tct ccc aac cct acg agg gcg gca gca gtc aag gcc gca    48
   Arg Ala Gly Ser Pro Asn Pro Thr Arg Ala Ala Ala Val Lys Ala Ala
    1               5                  10                  15
   ttc cag acg tcg tgg aac gct tac cac cat ttt gcc ttt ccc cat gac    96
   Phe Gln Thr Ser Trp Asn Ala Tyr His His Phe Ala Phe Pro His Asp
               20                  25                  30
   gac ctc cac ccg gtc agc aac agc ttt gat gat gag aga aac ggc tgg   144
   Asp Leu His Pro Val Ser Asn Ser Phe Asp Asp Glu Arg Asn Gly Trp
           35                  40                  45
   ggc tcg tcg gca atc gat ggc ttg gac acg gct atc ctc atg ggg gat   192
   Gly Ser Ser Ala Ile Asp Gly Leu Asp Thr Ala Ile Leu Met Gly Asp
       50                  55                  60
   gcc gac att gtg aac acg atc ctt cag tat gta ccg cag atc aac ttc   240
   Ala Asp Ile Val Asn Thr Ile Leu Gln Tyr Val Pro Gln Ile Asn Phe
   65                  70                  75                  80
   acc acg act gcg gtt gcc aac caa ggc atc tcc gtg ttc gag acc aac   288
   Thr Thr Thr Ala Val Ala Asn Gln Gly Ile Ser Val Phe Glu Thr Asn
                   85                  90                  95
   att cgg tac ctc ggt ggc ctg ctt tct gcc tat gac ctg ttg cga ggt   336
   Ile Arg Tyr Leu Gly Gly Leu Leu Ser Ala Tyr Asp Leu Leu Arg Gly
               100                 105                 110
   cct ttc agc tcc ttg gcg aca aac cag acc ctg gta aac agc ctt ctg   384
   Pro Phe Ser Ser Leu Ala Thr Asn Gln Thr Leu Val Asn Ser Leu Leu
           115                 120                 125
   agg cag gct caa aca ctg gcc aac ggc ctc aag gtt gcg ttc acc act   432
   Arg Gln Ala Gln Thr Leu Ala Asn Gly Leu Lys Val Ala Phe Thr Thr
       130                 135                 140
   ccc agc ggt gtc ccg gac cct acc gtc ttc ttc aac cct act gtc cgg   480
   Pro Ser Gly Val Pro Asp Pro Thr Val Phe Phe Asn Pro Thr Val Arg
   145                 150                 155                 160
   aga agt ggt gca tct agc aac aac gtc gct gaa att gga agc ctg gtg   528
   Arg Ser Gly Ala Ser Ser Asn Asn Val Ala Glu Ile Gly Ser Leu Val
                   165                 170                 175
   ctc gag tgg aca cgg ttg agc gac ctg acg gga aac ccg cag tat gcc      576
   Leu Glu Trp Thr Arg Leu Ser Asp Leu Thr Gly Asn Pro Gln Tyr Ala
               180                 185                 190
   cag ctt gcg cag aag ggc gag tcg tat ctc ctg aat cca aag gga agc      624
   Gln Leu Ala Gln Lys Gly Glu Ser Tyr Leu Leu Asn Pro Lys Gly Ser
           195                 200                 205
   ccg gag gca tgg cct ggc ctg att gga acg ttt gtc agc acg agc aac      672
   Pro Glu Ala Trp Pro Gly Leu Ile Gly Thr Phe Val Ser Thr Ser Asn
       210                 215                 220
   ggt acc ttt cag gat agc agc ggc agc tgg tcc ggc ctc atg gac agc      720
   Gly Thr Phe Gln Asp Ser Ser Gly Ser Trp Ser Gly Leu Met Asp Ser
   225                 230                 235                 240
   ttc tac gag tac ctg atc aag atg tac ctg tac gac ccg gtt gcg ttt      768
   Phe Tyr Glu Tyr Leu Ile Lys Met Tyr Leu Tyr Asp Pro Val Ala Phe
                   245                 250                 255
   gca cac tac aag gat cgc tgg gtc ctt gct gcc gac tcg acc att gcg      816
   Ala His Tyr Lys Asp Arg Trp Val Leu Ala Ala Asp Ser Thr Ile Ala
               260                 265                 270
   cat ctc gcc tct cac ccg tcg acg cgc aag gac ttg acc ttt ttg tct      864
   His Leu Ala Ser His Pro Ser Thr Arg Lys Asp Leu Thr Phe Leu Ser
           275                 280                 285
   tcg tac aac gga cag tct acg tcg cca aac tca gga cat ttg gcc agt      912
   Ser Tyr Asn Gly Gln Ser Thr Ser Pro Asn Ser Gly His Leu Ala Ser
       290                 295                 300
   ttt gcc ggt ggc aac ttc atc ttg gga ggc att ctc ctg aac gag caa      960
   Phe Ala Gly Gly Asn Phe Ile Leu Gly Gly Ile Leu Leu Asn Glu Gln
   305                 310                 315                 320
   aag tac att gac ttt gga atc aag ctt gcc agc tcg tac ttt gcc acg      1008
   Lys Tyr Ile Asp Phe Gly Ile Lys Leu Ala Ser Ser Tyr Phe Ala Thr
                   325                 330                 335
   tac aac cag acg gct tct gga atc ggc ccc gaa ggc ttc gcg tgg gtg      1056
   Tyr Asn Gln Thr Ala Ser Gly Ile Gly Pro Glu Gly Phe Ala Trp Val
               340                 345                 350
   gac agc gtg acg ggc gcc ggc ggc tcg ccg ccc tcg tcc cag tcc ggg      1104
   Asp Ser Val Thr Gly Ala Gly Gly Ser Pro Pro Ser Ser Gln Ser Gly
           355                 360                 365
   ttc tac tcg tcg gca gga ttc tgg gtg acg gca ccg tat tac atc ctg      1152
   Phe Tyr Ser Ser Ala Gly Phe Trp Val Thr Ala Pro Tyr Tyr Ile Leu
       370                 375                 380
   cgg ccg gag acg ctg gag agc ttg tac tac gca tac cgc gtc acg ggc      1200
   Arg Pro Glu Thr Leu Glu Ser Leu Tyr Tyr Ala Tyr Arg Val Thr Gly
   385                 390                 395                 400
   gac tcc aag tgg cag gac ctg gcg tgg gaa gcg ttc agt gcc att gag      1248
   Asp Ser Lys Trp Gln Asp Leu Ala Trp Glu Ala Phe Ser Ala Ile Glu
                   405                 410                 415
   gac gca tgc cgc gcc ggc agc gcg tac tcg tcc atc aac gac gtg acg    1296
   Asp Ala Cys Arg Ala Gly Ser Ala Tyr Ser Ser Ile Asn Asp Val Thr
               420                 425                 430
   cag gcc aac ggc ggg ggt gcc tct gac gat atg gag agc ttc tgg ttt    1344
   Gln Ala Asn Gly Gly Gly Ala Ser Asp Asp Met Glu Ser Phe Trp Phe
           435                 440                 445
   gcc gag gcg ctc aag tat gcg tac ctg atc ttt gcg gag gag tcg gat    1392
   Ala Glu Ala Leu Lys Tyr Ala Tyr Leu Ile Phe Ala Glu Glu Ser Asp
       450                 455                 460
   gtg cag gtg cag gcc aac ggc ggg aac aaa ttt gtc ttt aac acg gag    1440
   Val Gln Val Gln Ala Asn Gly Gly Asn Lys Phe Val Phe Asn Thr Glu
   465                 470                 475                 480
   gcg cac ccc ttt agc atc cgt tca tca tca cga cgg ggc ggc cac ctt    1488
   Ala His Pro Phe Ser Ile Arg Ser Ser Ser Arg Arg Gly Gly His Leu
                   485                 490                 495
   gct taa                                                            1494
   Ala  *
<210> 56
   <211> 497
   <212> PRT
   <213> Artificial Sequence
<400> 56
   Arg Ala Gly Ser Pro Asn Pro Thr Arg Ala Ala Ala Val Lys Ala Ala
   1               5                   10                  15
   Phe Gln Thr Ser Trp Asn Ala Tyr His His Phe Ala Phe Pro His Asp
               20                  25                  30
   Asp Leu His Pro Val Ser Asn Ser Phe Asp Asp Glu Arg Asn Gly Trp
           35                  40                  45
   Gly Ser Ser Ala Ile Asp Gly Leu Asp Thr Ala Ile Leu Met Gly Asp
       50                  55                  60
   Ala Asp Ile Val Asn Thr Ile Leu Gln Tyr Val Pro Gln Ile Asn Phe
   65                  70                  75                  80
   Thr Thr Thr Ala Val Ala Asn Gln Gly Ile Ser Val Phe Glu Thr Asn
                   85                  90                  95
   Ile Arg Tyr Leu Gly Gly Leu Leu Ser Ala Tyr Asp Leu Leu Arg Gly
               100                 105                 110
   Pro Phe Ser Ser Leu Ala Thr Asn Gln Thr Leu Val Asn Ser Leu Leu
           115                 120                 125
   Arg Gln Ala Gln Thr Leu Ala Asn Gly Leu Lys Val Ala Phe Thr Thr
       130                 135                 140
   Pro Ser Gly Val Pro Asp Pro Thr Val Phe Phe Asn Pro Thr Val Arg
   145                 150                 155                 160
   Arg Ser Gly Ala Ser Ser Asn Asn Val Ala Glu Ile Gly Ser Leu Val
                   165                 170                 175
   Leu Glu Trp Thr Arg Leu Ser Asp Leu Thr Gly Asn Pro Gln Tyr Ala
               180                 185                 190
   Gln Leu Ala Gln Lys Gly Glu Ser Tyr Leu Leu Asn Pro Lys Gly Ser
           195                 200                 205
   Pro Glu Ala Trp Pro Gly Leu Ile Gly Thr Phe Val Ser Thr Ser Asn
       210                 215                 220
   Gly Thr Phe Gln Asp Ser Ser Gly Ser Trp Ser Gly Leu Met Asp Ser
   225                 230                 235                 240
   Phe Tyr Glu Tyr Leu Ile Lys Met Tyr Leu Tyr Asp Pro Val Ala Phe
                   245                 250                 255
   Ala His Tyr Lys Asp Arg Trp Val Leu Ala Ala Asp Ser Thr Ile Ala
               260                 265                 270
   His Leu Ala Ser His Pro Ser Thr Arg Lys Asp Leu Thr Phe Leu Ser
           275                 280                 285
   Ser Tyr Asn Gly Gln Ser Thr Ser Pro Asn Ser Gly His Leu Ala Ser
       290                 295                 300
   Phe Ala Gly Gly Asn Phe Ile Leu Gly Gly Ile Leu Leu Asn Glu Gln
   305                 310                 315                 320
   Lys Tyr Ile Asp Phe Gly Ile Lys Leu Ala Ser Ser Tyr Phe Ala Thr
                   325                 330                 335
   Tyr Asn Gln Thr Ala Ser Gly Ile Gly Pro Glu Gly Phe Ala Trp Val
               340                 345                 350
   Asp Ser Val Thr Gly Ala Gly Gly Ser Pro Pro Ser Ser Gln Ser Gly
           355                 360                 365
   Phe Tyr Ser Ser Ala Gly Phe Trp Val Thr Ala Pro Tyr Tyr Ile Leu
       370                 375                 380
   Arg Pro Glu Thr Leu Glu Ser Leu Tyr Tyr Ala Tyr Arg Val Thr Gly
   385                 390                 395                 400
   Asp Ser Lys Trp Gln Asp Leu Ala Trp Glu Ala Phe Ser Ala Ile Glu
                   405                 410                 415
   Asp Ala Cys Arg Ala Gly Ser Ala Tyr Ser Ser Ile Asn Asp Val Thr
               420                 425                 430
   Gln Ala Asn Gly Gly Gly Ala Ser Asp Asp Met Glu Ser Phe Trp Phe
           435                 440                 445
   Ala Glu Ala Leu Lys Tyr Ala Tyr Leu Ile Phe Ala Glu Glu Ser Asp
       450                 455                 460
   Val Gln Val Gln Ala Asn Gly Gly Asn Lys Phe Val Phe Asn Thr Glu
   465                 470                 475                 480
   Ala His Pro Phe Ser Ile Arg Ser Ser Ser Arg Arg Gly Gly His Leu
                   485                 490                 495
   Ala
<210> 57
   <211> 1068
   <212> DNA
   <213> Artificial Sequence
<220>
   <223> DNA encodes rat GnTII catalytic domain (TC)
<220>
   <221> CDS
   <222> (1)...(1068)
   <223> rat GnTII catalytic domain
<400> 57
   tcc cta gtg tac cag ttg aac ttt gat cag atg ctg agg aat gtc gat    48
   Ser Leu Val Tyr Gln Leu Asn Phe Asp Gln Met Leu Arg Asn Val Asp
    1               5                  10                  15
   aaa gac ggc acc tgg agt ccg ggg gag ctg gtg ctg gtg gtc caa gtg    96
   Lys Asp Gly Thr Trp Ser Pro Gly Glu Leu Val Leu Val Val Gln Val
               20                  25                  30
   cat aac agg ccg gaa tac ctc agg ctg ctg ata gac tcg ctt cga aaa   144
   His Asn Arg Pro Glu Tyr Leu Arg Leu Leu Ile Asp Ser Leu Arg Lys
           35                  40                  45
   gcc cag ggt att cgc gaa gtc cta gtc atc ttt agc cat gac ttc tgg    192
   Ala Gln Gly Ile Arg Glu Val Leu Val Ile Phe Ser His Asp Phe Trp
       50                  55                  60
   tcg gca gag atc aac agt ctg atc tct agt gtg gac ttc tgt ccg gtt    240
   Ser Ala Glu Ile Asn Ser Leu Ile Ser Ser Val Asp Phe Cys Pro Val
   65                  70                  75                  80
   ctg caa gtg ttc ttt ccg ttc agc att cag ctg tac ccg agt gag ttt    288
   Leu Gln Val Phe Phe Pro Phe Ser Ile Gln Leu Tyr Pro Ser Glu Phe
                   85                  90                  95
   ccg ggt agt gat ccc aga gat tgc ccc aga gac ctg aag aag aat gca    336
   Pro Gly Ser Asp Pro Arg Asp Cys Pro Arg Asp Leu Lys Lys Asn Ala
               100                 105                 110
   gct ctc aag ttg ggg tgc atc aat gcc gaa tac cca gac tcc ttc ggc    384
   Ala Leu Lys Leu Gly Cys Ile Asn Ala Glu Tyr Pro Asp Ser Phe Gly
           115                 120                 125
   cat tac aga gag gcc aaa ttc tcg caa acc aaa cat cac tgg tgg tgg    432
   His Tyr Arg Glu Ala Lys Phe Ser Gln Thr Lys His His Trp Trp Trp
       130                 135                 140
   aag ctg cat ttt gta tgg gaa aga gtc aaa gtt ctt caa gat tac act    480
   Lys Leu His Phe Val Trp Glu Arg Val Lys Val Leu Gln Asp Tyr Thr
   145                 150                 155                 160
   ggc ctt ata ctt ttc ctg gaa gag gac cac tac tta gcc cca gac ttt    528
   Gly Leu Ile Leu Phe Leu Glu Glu Asp His Tyr Leu Ala Pro Asp Phe
                   165                 170                 175
   tac cat gtc ttc aaa aag atg tgg aaa ttg aag cag cag gag tgt cct    576
   Tyr His Val Phe Lys Lys Met Trp Lys Leu Lys Gln Gln Glu Cys Pro
               180                 185                 190
   ggg tgt gac gtc ctc tct cta ggg acc tac acc acc att cgg agt ttc    624
   Gly Cys Asp Val Leu Ser Leu Gly Thr Tyr Thr Thr Ile Arg Ser Phe
           195                 200                 205
   tat ggt att gct gac aaa gta gat gtg aaa act tgg aaa tcg aca gag    672
   Tyr Gly Ile Ala Asp Lys Val Asp Val Lys Thr Trp Lys Ser Thr Glu
       210                 215                 220
   cac aat atg ggg cta gcc ttg acc cga gat gca tat cag aag ctt atc    720
   His Asn Met Gly Leu Ala Leu Thr Arg Asp Ala Tyr Gln Lys Leu Ile
   225                 230                 235                 240
   gag tgc acg gac act ttc tgt act tac gat gat tat aac tgg gac tgg    768
   Glu Cys Thr Asp Thr Phe Cys Thr Tyr Asp Asp Tyr Asn Trp Asp Trp
                   245                 250                 255
   act ctt caa tat ttg act cta gct tgt ctt cct aaa gtc tgg aaa gtc    816
   Thr Leu Gln Tyr Leu Thr Leu Ala Cys Leu Pro Lys Val Trp Lys Val
               260                 265                 270
   tta gtt cct caa gct cct agg att ttt cat gct gga gac tgt ggt atg    864
   Leu Val Pro Gln Ala Pro Arg Ile Phe His Ala Gly Asp Cys Gly Met
           275                 280                 285
   cat cac aag aaa aca tgt agg cca tcc acc cag agt gcc caa att gag      912
   His His Lys Lys Thr Cys Arg Pro Ser Thr Gln Ser Ala Gln Ile Glu
       290                 295                 300
   tca tta tta aat aat aat aaa cag tac ctg ttt cca gaa act cta gtt      960
   Ser Leu Leu Asn Asn Asn Lys Gln Tyr Leu Phe Pro Glu Thr Leu Val
   305                 310                 315                 320
   atc ggt gag aag ttt cct atg gca gcc att tcc cca cct agg aaa aat     1008
   Ile Gly Glu Lys Phe Pro Met Ala Ala Ile Ser Pro Pro Arg Lys Asn
                   325                 330                 335
   gga ggg tgg gga gat att agg gac cat gaa ctc tgt aaa agt tat aga     1056
   Gly Gly Trp Gly Asp Ile Arg Asp His Glu Leu Cys Lys Ser Tyr Arg
               340                 345                 350
   aga ctg cag tga                                                     1068
   Arg Leu Gln  *
           355
<210> 58
   <211> 355
   <212> PRT
   <213> Artificial Sequence
<400> 58
   Ser Leu Val Tyr Gln Leu Asn Phe Asp Gln Met Leu Arg Asn Val Asp
   1               5                   10                  15
   Lys Asp Gly Thr Trp Ser Pro Gly Glu Leu Val Leu Val Val Gln Val
               20                  25                  30
   His Asn Arg Pro Glu Tyr Leu Arg Leu Leu Ile Asp Ser Leu Arg Lys
           35                  40                  45
   Ala Gln Gly Ile Arg Glu Val Leu Val Ile Phe Ser His Asp Phe Trp
       50                  55                  60
   Ser Ala Glu Ile Asn Ser Leu Ile Ser Ser Val Asp Phe Cys Pro Val
   65                  70                  75                  80
   Leu Gln Val Phe Phe Pro Phe Ser Ile Gln Leu Tyr Pro Ser Glu Phe
                   85                  90                  95
   Pro Gly Ser Asp Pro Arg Asp Cys Pro Arg Asp Leu Lys Lys Asn Ala
               100                 105                 110
   Ala Leu Lys Leu Gly Cys Ile Asn Ala Glu Tyr Pro Asp Ser Phe Gly
           115                 120                 125
   His Tyr Arg Glu Ala Lys Phe Ser Gln Thr Lys His His Trp Trp Trp
       130                 135                 140
   Lys Leu His Phe Val Trp Glu Arg Val Lys Val Leu Gln Asp Tyr Thr
   145                 150                 155                 160
   Gly Leu Ile Leu Phe Leu Glu Glu Asp His Tyr Leu Ala Pro Asp Phe
                   165                 170                 175
   Tyr His Val Phe Lys Lys Met Trp Lys Leu Lys Gln Gln Glu Cys Pro
               180                 185                 190
   Gly Cys Asp Val Leu Ser Leu Gly Thr Tyr Thr Thr Ile Arg Ser Phe
           195                 200                 205
   Tyr Gly Ile Ala Asp Lys Val Asp Val Lys Thr Trp Lys Ser Thr Glu
       210                 215                 220
   His Asn Met Gly Leu Ala Leu Thr Arg Asp Ala Tyr Gln Lys Leu Ile
   225                 230                 235                 240
   Glu Cys Thr Asp Thr Phe Cys Thr Tyr Asp Asp Tyr Asn Trp Asp Trp
                   245                 250                 255
   Thr Leu Gln Tyr Leu Thr Leu Ala Cys Leu Pro Lys Val Trp Lys Val
               260                 265                 270
   Leu Val Pro Gln Ala Pro Arg Ile Phe His Ala Gly Asp Cys Gly Met
           275                 280                 285
   His His Lys Lys Thr Cys Arg Pro Ser Thr Gln Ser Ala Gln Ile Glu
       290                 295                 300
   Ser Leu Leu Asn Asn Asn Lys Gln Tyr Leu Phe Pro Glu Thr Leu Val
   305                 310                 315                 320
   Ile Gly Glu Lys Phe Pro Met Ala Ala Ile Ser Pro Pro Arg Lys Asn
                   325                 330                 335
   Gly Gly Trp Gly Asp Ile Arg Asp His Glu Leu Cys Lys Ser Tyr Arg
               340                 345                 350
   Arg Leu Gln
           355
<210> 59
   <211> 1068
   <212> DNA
   <213> Artificial Sequence
<220>
   <223> DNA encodes rat GnTII catalytic domain (TC) codon-optimized
<400> 59
   tccttggttt accaattgaa cttcgaccag atgttgagaa acgttgacaa ggacggtact 60
   tggtctcctg gtgagttggt tttggttgtt caggttcaca acagaccaga gtacttgaga 120
   ttgttgatcg actccttgag aaaggctcaa ggtatcagag aggttttggt tatcttctcc 180
   cacgatttct ggtctgctga gatcaactcc ttgatctcct ccgttgactt ctgtccagtt 240
   ttgcaggttt tcttcccatt ctccatccaa ttgtacccat ctgagttccc aggttctgat 300
   ccaagagact gtccaagaga cttgaagaag aacgctgctt tgaagttggg ttgtatcaac 360
   gctgaatacc cagattcttt cggtcactac agagaggcta agttctccca aactaagcat 420
   cattggtggt ggaagttgca ctttgtttgg gagagagtta aggttttgca ggactacact 480
   ggattgatct tgttcttgga ggaggatcat tacttggctc cagacttcta ccacgttttc 540
   aagaagatgt ggaagttgaa gcaacaagag tgtccaggtt gtgacgtttt gtccttggga 600
   acttacacta ctatcagatc cttctacggt atcgctgaca aggttgacgt taagacttgg 660
   aagtccactg aacacaacat gggattggct ttgactagag atgcttacca gaagttgatc 720
   gagtgtactg acactttctg tacttacgac gactacaact gggactggac tttgcagtac 780
   ttgactttgg cttgtttgcc aaaagtttgg aaggttttgg ttccacaggc tccaagaatt 840
   ttccacgctg gtgactgtgg aatgcaccac aagaaaactt gtagaccatc cactcagtcc 900
   gctcaaattg agtccttgtt gaacaacaac aagcagtact tgttcccaga gactttggtt 960
   atcggagaga agtttccaat ggctgctatt tccccaccaa gaaagaatgg tggatggggt 1020
   gatattagag accacgagtt gtgtaaatcc tacagaagat tgcagtag             1068
<210> 60
   <211> 1240
   <212> DNA
   <213> Artificial Sequence
<220>
   <223> DNA encodes rat GnTII catalytic domain (TA)
<220>
   <221> CDS
   <222> (1)...(1240)
   <223> rat GnTII catalytic domain (TA)
<400> 60
   agg aag aac gac gcc ctt gcc ccg ccg ctg ctg gac tcg gag ccc cta   48
   Arg Lys Asn Asp Ala Leu Ala Pro Pro Leu Leu Asp Ser Glu Pro Leu
   1               5                   10                  15
   cgg ggt gcg ggc cat ttc gcc gcg tcc gta ggc atc cgc agg gtt tct     96
   Arg Gly Ala Gly His Phe Ala Ala Ser Val Gly Ile Arg Arg Val Ser
               20                  25                  30
   aac gac tcg gcc gct cct ctg gtt ccc gcg gtc ccg cgg ccg gag gtg    144
   Asn Asp Ser Ala Ala Pro Leu Val Pro Ala Val Pro Arg Pro Glu Val
           35                  40                  45
   gac aac cta acg ctg cgg tac cgg tcc cta gtg tac cag ttg aac ttt    192
   Asp Asn Leu Thr Leu Arg Tyr Arg Ser Leu Val Tyr Gln Leu Asn Phe
       50                  55                  60
   gat cag atg ctg agg aat gtc gat aaa gac ggc acc tgg agt ccg ggg    240
   Asp Gln Met Leu Arg Asn Val Asp Lys Asp Gly Thr Trp Ser Pro Gly
   65                  70                  75                  80
   gag ctg gtg ctg gtg gtc caa gtg cat aac agg ccg gaa tac ctc agg    288
   Glu Leu Val Leu Val Val Gln Val His Asn Arg Pro Glu Tyr Leu Arg
                   85                  90                  95
   ctg ctg ata gac tcg ctt cga aaa gcc cag ggt att cgc gaa gtc cta    336
   Leu Leu Ile Asp Ser Leu Arg Lys Ala Gln Gly Ile Arg Glu Val Leu
               100                 105                 110
   gtc atc ttt agc cat gac ttc tgg tcg gca gag atc aac agt ctg atc    384
   Val Ile Phe Ser His Asp Phe Trp Ser Ala Glu Ile Asn Ser Leu Ile
           115                 120                 125
   tct agt gtg gac ttc tgt ccg gtt ctg caa gtg ttc ttt ccg ttc agc    432
   Ser Ser Val Asp Phe Cys Pro Val Leu Gln Val Phe Phe Pro Phe Ser
       130                 135                 140
   att cag ctg tac ccg agt gag ttt ccg ggt agt gat ccc aga gat tgc    480
   Ile Gln Leu Tyr Pro Ser Glu Phe Pro Gly Ser Asp Pro Arg Asp Cys
   145                 150                 155                 160
   ccc aga gac ctg aag aag aat gca gct ctc aag ttg ggg tgc atc aat    528
   Pro Arg Asp Leu Lys Lys Asn Ala Ala Leu Lys Leu Gly Cys Ile Asn
                   165                 170                 175
   gcc gaa tac cca gac tcc ttc ggc cat tac aga gag gcc aaa ttc tcg    576
   Ala Glu Tyr Pro Asp Ser Phe Gly His Tyr Arg Glu Ala Lys Phe Ser
               180                 185                 190
   caa acc aaa cat cac tgg tgg tgg aag ctg cat ttt gta tgg gaa aga    624
   Gln Thr Lys His His Trp Trp Trp Lys Leu His Phe Val Trp Glu Arg
           195                 200                 205
   gtc aaa gtt ctt caa gat tac act ggc ctt ata ctt ttc ctg gaa gag    672
   Val Lys Val Leu Gln Asp Tyr Thr Gly Leu Ile Leu Phe Leu Glu Glu
       210                 215                 220
   gac cac tac tta gcc cca gac ttt tac cat gtc ttc aaa aag atg tgg    720
   Asp His Tyr Leu Ala Pro Asp Phe Tyr His Val Phe Lys Lys Met Trp
   225                 230                 235                 240
   aaa ttg aag cag cag gag tgt cct ggg tgt gac gtc ctc tct cta ggg    768
   Lys Leu Lys Gln Gln Glu Cys Pro Gly Cys Asp Val Leu Ser Leu Gly
                   245                 250                 255
   acc tac acc acc att cgg agt ttc tat ggt att gct gac aaa gta gat      816
   Thr Tyr Thr Thr Ile Arg Ser Phe Tyr Gly Ile Ala Asp Lys Val Asp
               260                 265                 270
   gtg aaa act tgg aaa tcg aca gag cac aat atg ggg cta gcc ttg acc      864
   Val Lys Thr Trp Lys Ser Thr Glu His Asn Met Gly Leu Ala Leu Thr
           275                 280                 285
   cga gat gca tat cag aag ctt atc gag tgc acg gac act ttc tgt act      912
   Arg Asp Ala Tyr Gln Lys Leu Ile Glu Cys Thr Asp Thr Phe Cys Thr
       290                 295                 300
   tac gat gat tat aac tgg gac tgg act ctt caa tat ttg act cta gct      960
   Tyr Asp Asp Tyr Asn Trp Asp Trp Thr Leu Gln Tyr Leu Thr Leu Ala
   305                 310                 315                 320
   tgt ctt cct aaa gtc tgg aaa gtc tta gtt cct caa gct cct agg att      1008
   Cys Leu Pro Lys Val Trp Lys Val Leu Val Pro Gln Ala Pro Arg Ile
                   325                 330                 335
   ttt cat gct gga gac tgt ggt atg cat cac aag aaa aca tgt agg cca      1056
   Phe His Ala Gly Asp Cys Gly Met His His Lys Lys Thr Cys Arg Pro
               340                 345                 350
   tcc acc cag agt gcc caa att gag tca tta tta aat aat aat aaa cag      1104
   Ser Thr Gln Ser Ala Gln Ile Glu Ser Leu Leu Asn Asn Asn Lys Gln
           355                 360                 365
   tac ctg ttt cca gaa act cta gtt atc ggt gag aag ttt cct atg gca      1152
   Tyr Leu Phe Pro Glu Thr Leu Val Ile Gly Glu Lys Phe Pro Met Ala
       370                 375                 380
   gcc att tcc cca cct agg aaa aat gga ggg tgg gga gat att agg gac      1200
   Ala Ile Ser Pro Pro Arg Lys Asn Gly Gly Trp Gly Asp Ile Arg Asp
   385                 390                 395                 400
   cat gaa ctc tgt aaa agt tat aga aga ctg cag tga gtt a                1240
   His Glu Leu Cys Lys Ser Tyr Arg Arg Leu Gln  *  Val
                   405                 410
<210> 61
   <211> 412
   <212> PRT
   <213> Artificial Sequence
<400> 61
   Arg Lys Asn Asp Ala Leu Ala Pro Pro Leu Leu Asp Ser Glu Pro Leu
   1               5                   10                  15
   Arg Gly Ala Gly His Phe Ala Ala Ser Val Gly Ile Arg Arg Val Ser
               20                  25                  30
   Asn Asp Ser Ala Ala Pro Leu Val Pro Ala Val Pro Arg Pro Glu Val
           35                  40                  45
   Asp Asn Leu Thr Leu Arg Tyr Arg Ser Leu Val Tyr Gln Leu Asn Phe
       50                  55                  60
   Asp Gln Met Leu Arg Asn Val Asp Lys Asp Gly Thr Trp Ser Pro Gly
   65                  70                  75                  80
   Glu Leu Val Leu Val Val Gln Val His Asn Arg Pro Glu Tyr Leu Arg
                   85                  90                  95
   Leu Leu Ile Asp Ser Leu Arg Lys Ala Gln Gly Ile Arg Glu Val Leu
               100                 105                 110
   Val Ile Phe Ser His Asp Phe Trp Ser Ala Glu Ile Asn Ser Leu Ile
           115                 120                 125
   Ser Ser Val Asp Phe Cys Pro Val Leu Gln Val Phe Phe Pro Phe Ser
       130                 135                 140
   Ile Gln Leu Tyr Pro Ser Glu Phe Pro Gly Ser Asp Pro Arg Asp Cys
   145                 150                 155                 160
   Pro Arg Asp Leu Lys Lys Asn Ala Ala Leu Lys Leu Gly Cys Ile Asn
                   165                 170                 175
   Ala Glu Tyr Pro Asp Ser Phe Gly His Tyr Arg Glu Ala Lys Phe Ser
               180                 185                 190
   Gln Thr Lys His His Trp Trp Trp Lys Leu His Phe Val Trp Glu Arg
           195                 200                 205
   Val Lys Val Leu Gln Asp Tyr Thr Gly Leu Ile Leu Phe Leu Glu Glu
       210                 215                 220
   Asp His Tyr Leu Ala Pro Asp Phe Tyr His Val Phe Lys Lys Met Trp
   225                 230                 235                 240
   Lys Leu Lys Gln Gln Glu Cys Pro Gly Cys Asp Val Leu Ser Leu Gly
                   245                 250                 255
   Thr Tyr Thr Thr Ile Arg Ser Phe Tyr Gly Ile Ala Asp Lys Val Asp
               260                 265                 270
   Val Lys Thr Trp Lys Ser Thr Glu His Asn Met Gly Leu Ala Leu Thr
           275                 280                 285
   Arg Asp Ala Tyr Gln Lys Leu Ile Glu Cys Thr Asp Thr Phe Cys Thr
       290                 295                 300
   Tyr Asp Asp Tyr Asn Trp Asp Trp Thr Leu Gln Tyr Leu Thr Leu Ala
   305                 310                 315                 320
   Cys Leu Pro Lys Val Trp Lys Val Leu Val Pro Gln Ala Pro Arg Ile
                   325                 330                 335
   Phe His Ala Gly Asp Cys Gly Met His His Lys Lys Thr Cys Arg Pro
               340                 345                 350
   Ser Thr Gln Ser Ala Gln Ile Glu Ser Leu Leu Asn Asn Asn Lys Gln
           355                 360                 365
   Tyr Leu Phe Pro Glu Thr Leu Val Ile Gly Glu Lys Phe Pro Met Ala
       370                 375                 380
   Ala Ile Ser Pro Pro Arg Lys Asn Gly Gly Trp Gly Asp Ile Arg Asp
   385                 390                 395                 400
   His Glu Leu Cys Lys Ser Tyr Arg Arg Leu Gln Val
                   405                 410
<210> 62
   <211> 3105
   <212> DNA
   <213> Artificial Sequence
<220>
   <223> DNA encodes Dm ManII catalytic domain (KD)
<220>
   <221> CDS
   <222> (1)...(3105)
   <223> Dm ManII catalytic domain
<400> 62
   cgc gac gat cca ata aga cct cca ctt aaa gtg gct cgt tcc ccg agg   48
   Arg Asp Asp Pro Ile Arg Pro Pro Leu Lys Val Ala Arg Ser Pro Arg
    1               5                  10                  15
   cca ggg caa tgc caa gat gtg gtc caa gac gtg ccc aat gtg gat gta   96
   Pro Gly Gln Cys Gln Asp Val Val Gln Asp Val Pro Asn Val Asp Val
               20                  25                  30
   cag atg ctg gag cta tac gat cgc atg tcc ttc aag gac ata gat gga     144
   Gln Met Leu Glu Leu Tyr Asp Arg Met Ser Phe Lys Asp Ile Asp Gly
           35                  40                  45
   ggc gtg tgg aaa cag ggc tgg aac att aag tac gat cca ctg aag tac     192
   Gly Val Trp Lys Gln Gly Trp Asn Ile Lys Tyr Asp Pro Leu Lys Tyr
       50                  55                  60
   aac gcc cat cac aaa cta aaa gtc ttc gtt gtg ccg cac tcg cac aac     240
   Asn Ala His His Lys Leu Lys Val Phe Val Val Pro His Ser His Asn
   65                  70                  75                  80
   gat cct gga tgg att cag acg ttt gag gaa tac tac cag cac gac acc     288
   Asp Pro Gly Trp Ile Gln Thr Phe Glu Glu Tyr Tyr Gln His Asp Thr
                   85                  90                  95
   aag cac atc ctg tcc aat gca cta cgg cat ctg cac gac aat ccc gag     336
   Lys His Ile Leu Ser Asn Ala Leu Arg His Leu His Asp Asn Pro Glu
               100                 105                 110
   atg aag ttc atc tgg gcg gaa atc tcc tac ttt gct cgg ttc tat cac     384
   Met Lys Phe Ile Trp Ala Glu Ile Ser Tyr Phe Ala Arg Phe Tyr His
           115                 120                 125
   gat ttg gga gag aac aaa aag ctg cag atg aag tcc att gta aag aat     432
   Asp Leu Gly Glu Asn Lys Lys Leu Gln Met Lys Ser Ile Val Lys Asn
       130                 135                 140
   gga cag ttg gaa ttt gtg act gga gga tgg gta atg ccg gac gag gcc     480
   Gly Gln Leu Glu Phe Val Thr Gly Gly Trp Val Met Pro Asp Glu Ala
   145                 150                 155                 160
   aac tcc cac tgg cga aac gta ctg ctg cag ctg acc gaa ggg caa aca     528
   Asn Ser His Trp Arg Asn Val Leu Leu Gln Leu Thr Glu Gly Gln Thr
                   165                 170                 175
   tgg ttg aag caa ttc atg aat gtc aca ccc act gct tcc tgg gcc atc     576
   Trp Leu Lys Gln Phe Met Asn Val Thr Pro Thr Ala Ser Trp Ala Ile
               180                 185                 190
   gat ccc ttc gga cac agt ccc act atg ccg tac att ttg cag aag agt     624
   Asp Pro Phe Gly His Ser Pro Thr Met Pro Tyr Ile Leu Gln Lys Ser
           195                 200                 205
   ggt ttc aag aat atg ctt atc caa agg acg cac tat tcg gtt aag aag     672
   Gly Phe Lys Asn Met Leu Ile Gln Arg Thr His Tyr Ser Val Lys Lys
       210                 215                 220
   gaa ctg gcc caa cag cga cag ctt gag ttc ctg tgg cgc cag atc tgg     720
   Glu Leu Ala Gln Gln Arg Gln Leu Glu Phe Leu Trp Arg Gln Ile Trp
   225                 230                 235                 240
   gac aac aaa ggg gac aca gct ctc ttc acc cac atg atg ccc ttc tac     768
   Asp Asn Lys Gly Asp Thr Ala Leu Phe Thr His Met Met Pro Phe Tyr
                   245                 250                 255
   tcg tac gac att cct cat acc tgt ggt cca gat ccc aag gtt tgc tgt     816
   Ser Tyr Asp Ile Pro His Thr Cys Gly Pro Asp Pro Lys Val Cys Cys
               260                 265                 270
   cag ttc gat ttc aaa cga atg ggc tcc ttc ggt ttg agt tgt cca tgg    864
   Gln Phe Asp Phe Lys Arg Met Gly Ser Phe Gly Leu Ser Cys Pro Trp
           275                 280                 285
   aag gtg ccg ccg cgt aca atc agt gat caa aat gtg gca gca cgc tca    912
   Lys Val Pro Pro Arg Thr Ile Ser Asp Gln Asn Val Ala Ala Arg Ser
       290                 295                 300
   gat ctg ctg gtt gat cag tgg aag aag aag gcc gag ctg tat cgc aca    960
   Asp Leu Leu Val Asp Gln Trp Lys Lys Lys Ala Glu Leu Tyr Arg Thr
   305                 310                 315                 320
   aac gtg ctg ctg att ccg ttg ggt gac gac ttc cgc ttc aag cag aac    1008
   Asn Val Leu Leu Ile Pro Leu Gly Asp Asp Phe Arg Phe Lys Gln Asn
                   325                 330                 335
   acc gag tgg gat gtg cag cgc gtg aac tac gaa agg ctg ttc gaa cac    1056
   Thr Glu Trp Asp Val Gln Arg Val Asn Tyr Glu Arg Leu Phe Glu His
               340                 345                 350
   atc aac agc cag gcc cac ttc aat gtc cag gcg cag ttc ggc aca ctg    1104
   Ile Asn Ser Gln Ala His Phe Asn Val Gln Ala Gln Phe Gly Thr Leu
           355                 360                 365
   cag gaa tac ttt gat gca gtg cac cag gcg gaa agg gcg gga caa gcc    1152
   Gln Glu Tyr Phe Asp Ala Val His Gln Ala Glu Arg Ala Gly Gln Ala
       370                 375                 380
   gag ttt ccc acg cta agc ggt gac ttt ttc aca tac gcc gat cga tcg    1200
   Glu Phe Pro Thr Leu Ser Gly Asp Phe Phe Thr Tyr Ala Asp Arg Ser
   385                 390                 395                 400
   gat aac tat tgg agt ggc tac tac aca tcc cgc ccg tat cat aag cgc    1248
   Asp Asn Tyr Trp Ser Gly Tyr Tyr Thr Ser Arg Pro Tyr His Lys Arg
                   405                 410                 415
   atg gac cgc gtc ctg atg cac tat gta cgt gca gca gaa atg ctt tcc    1296
   Met Asp Arg Val Leu Met His Tyr Val Arg Ala Ala Glu Met Leu Ser
               420                 425                 430
   gcc tgg cac tcc tgg gac ggt atg gcc cgc atc gag gaa cgt ctg gag    1344
   Ala Trp His Ser Trp Asp Gly Met Ala Arg Ile Glu Glu Arg Leu Glu
           435                 440                 445
   cag gcc cgc agg gag ctg tca ttg ttc cag cac cac gac ggt ata act    1392
   Gln Ala Arg Arg Glu Leu Ser Leu Phe Gln His His Asp Gly Ile Thr
       450                 455                 460
   ggc aca gca aaa acg cac gta gtc gtc gac tac gag caa cgc atg cag    1440
   Gly Thr Ala Lys Thr His Val Val Val Asp Tyr Glu Gln Arg Met Gln
   465                 470                 475                 480
   gaa gct tta aaa gcc tgt caa atg gta atg caa cag tcg gtc tac cga    1488
   Glu Ala Leu Lys Ala Cys Gln Met Val Met Gln Gln Ser Val Tyr Arg
                   485                 490                 495
   ttg ctg aca aag ccc tcc atc tac agt ccg gac ttc agt ttc tcg tac    1536
   Leu Leu Thr Lys Pro Ser Ile Tyr Ser Pro Asp Phe Ser Phe Ser Tyr
               500                 505                 510
   ttt acg ctc gac gac tcc cgc tgg cca gga tct ggt gtg gag gac agt   1584
   Phe Thr Leu Asp Asp Ser Arg Trp Pro Gly Ser Gly Val Glu Asp Ser
           515                 520                 525
   cga acc acc ata ata ctg ggc gag gat ata ctg ccc tcc aag cat gtg   1632
   Arg Thr Thr Ile Ile Leu Gly Glu Asp Ile Leu Pro Ser Lys His Val
       530                 535                 540
   gtg atg cac aac acc ctg ccc cac tgg cgg gag cag ctg gtg gac ttt   1680
   Val Met His Asn Thr Leu Pro His Trp Arg Glu Gln Leu Val Asp Phe
   545                 550                 555                 560
   tat gta tcc agt ccg ttt gta agc gtt acc gac ttg gca aac aat ccg   1728
   Tyr Val Ser Ser Pro Phe Val Ser Val Thr Asp Leu Ala Asn Asn Pro
                   565                 570                 575
   gtg gag gct cag gtg tcc ccg gtg tgg agc tgg cac cac gac aca ctc   1776
   Val Glu Ala Gln Val Ser Pro Val Trp Ser Trp His His Asp Thr Leu
               580                 585                 590
   aca aag act atc cac cca caa ggc tcc acc acc aag tac cgc atc atc   1824
   Thr Lys Thr Ile His Pro Gln Gly Ser Thr Thr Lys Tyr Arg Ile Ile
           595                 600                 605
   ttc aag gct cgg gtg ccg ccc atg ggc ttg gcc acc tac gtt tta acc   1872
   Phe Lys Ala Arg Val Pro Pro Met Gly Leu Ala Thr Tyr Val Leu Thr
       610                 615                 620
   atc tcc gat tcc aag cca gag cac acc tcg tat gca tcg aat ctc ttg   1920
   Ile Ser Asp Ser Lys Pro Glu His Thr Ser Tyr Ala Ser Asn Leu Leu
   625                 630                 635                 640
   ctc cgt aaa aac ccg act tcg tta cca ttg ggc caa tat ccg gag gat   1968
   Leu Arg Lys Asn Pro Thr Ser Leu Pro Leu Gly Gln Tyr Pro Glu Asp
                   645                 650                 655
   gtg aag ttt ggc gat cct cga gag atc tca ttg cgg gtt ggt aac gga   2016
   Val Lys Phe Gly Asp Pro Arg Glu Ile Ser Leu Arg Val Gly Asn Gly
               660                 665                 670
   ccc acc ttg gcc ttt tcg gag cag ggt ctc ctt aag tcc att cag ctt   2064
   Pro Thr Leu Ala Phe Ser Glu Gln Gly Leu Leu Lys Ser Ile Gln Leu
           675                 680                 685
   act cag gat agc cca cat gta ccg gtg cac ttc aag ttc ctc aag tat   2112
   Thr Gln Asp Ser Pro His Val Pro Val His Phe Lys Phe Leu Lys Tyr
       690                 695                 700
   ggc gtt cga tcg cat ggc gat aga tcc ggt gcc tat ctg ttc ctg ccc   2160
   Gly Val Arg Ser His Gly Asp Arg Ser Gly Ala Tyr Leu Phe Leu Pro
   705                 710                 715                 720
   aat gga cca gct tcg cca gtc gag ctt ggc cag cca gtg gtc ctg gtg   2208
   Asn Gly Pro Ala Ser Pro Val Glu Leu Gly Gln Pro Val Val Leu Val
                   725                 730                 735
   act aag ggc aaa ctg gag tcg tcc gtg agc gtg gga ctt ccg agc gtg   2256
   Thr Lys Gly Lys Leu Glu Ser Ser Val Ser Val Gly Leu Pro Ser Val
               740                 745                 750
   gtg cac cag acg ata atg cgc ggt ggt gca cct gag att cgc aat ctg   2304
   Val His Gln Thr Ile Met Arg Gly Gly Ala Pro Glu Ile Arg Asn Leu
           755                 760                 765
   gtg gat ata ggc tca ctg gac aac acg gag atc gtg atg cgc ttg gag    2352
   Val Asp Ile Gly Ser Leu Asp Asn Thr Glu Ile Val Met Arg Leu Glu
       770                 775                 780
   acg cat atc gac agc ggc gat atc ttc tac acg gat ctc aat gga ttg    2400
   Thr His Ile Asp Ser Gly Asp Ile Phe Tyr Thr Asp Leu Asn Gly Leu
   785                 790                 795                 800
   caa ttt atc aag agg cgg cgt ttg gac aaa tta cct ttg cag gcc aac    2448
   Gln Phe Ile Lys Arg Arg Arg Leu Asp Lys Leu Pro Leu Gln Ala Asn
                   805                 810                 815
   tat tat ccc ata cct tct ggt atg ttc att gag gat gcc aat acg cga    2496
   Tyr Tyr Pro Ile Pro Ser Gly Met Phe Ile Glu Asp Ala Asn Thr Arg
               820                 825                 830
   ctc act ctc ctc acg ggt caa ccg ctg ggt gga tct tct ctg gcc tcg    2544
   Leu Thr Leu Leu Thr Gly Gln Pro Leu Gly Gly Ser Ser Leu Ala Ser
           835                 840                 845
   ggc gag cta gag att atg caa gat cgt cgc ctg gcc agc gat gat gaa    2592
   Gly Glu Leu Glu Ile Met Gln Asp Arg Arg Leu Ala Ser Asp Asp Glu
       850                 855                 860
   cgc ggc ctg gga cag ggt gtt ttg gac aac aag ccg gtg ctg cat att    2640
   Arg Gly Leu Gly Gln Gly Val Leu Asp Asn Lys Pro Val Leu His Ile
   865                 870                 875                 880
   tat cgg ctg gtg ctg gag aag gtt aac aac tgt gtc cga ccg tca aag    2688
   Tyr Arg Leu Val Leu Glu Lys Val Asn Asn Cys Val Arg Pro Ser Lys
                   885                 890                 895
   ctt cat cct gcc ggc tat ttg aca agt gcc gca cac aaa gca tcg cag    2736
   Leu His Pro Ala Gly Tyr Leu Thr Ser Ala Ala His Lys Ala Ser Gln
               900                 905                 910
   tca ctg ctg gat cca ctg gac aag ttt ata ttc gct gaa aat gag tgg    2784
   Ser Leu Leu Asp Pro Leu Asp Lys Phe Ile Phe Ala Glu Asn Glu Trp
           915                 920                 925
   atc ggg gca cag ggg caa ttt ggt ggc gat cat cct tcg gct cgt gag    2832
   Ile Gly Ala Gln Gly Gln Phe Gly Gly Asp His Pro Ser Ala Arg Glu
       930                 935                 940
   gat ctc gat gtg tcg gtg atg aga cgc tta acc aag agc tcg gcc aaa    2880
   Asp Leu Asp Val Ser Val Met Arg Arg Leu Thr Lys Ser Ser Ala Lys
   945                 950                 955                 960
   acc cag cga gta ggc tac gtt ctg cac cgc acc aat ctg atg caa tgc    2928
   Thr Gln Arg Val Gly Tyr Val Leu His Arg Thr Asn Leu Met Gln Cys
                   965                 970                 975
   ggc act cca gag gag cat aca cag aag ctg gat gtg tgc cac cta ctg    2976
   Gly Thr Pro Glu Glu His Thr Gln Lys Leu Asp Val Cys His Leu Leu
               980                 985                 990
   ccg aat gtg gcg aga tgc gag cgc acg acg ctg act ttc ctg cag aat    3024
   Pro Asn Val Ala Arg Cys Glu Arg Thr Thr Leu Thr Phe Leu Gln Asn
           995                 1000                1005
   ttg gag cac ttg gat ggc atg gtg gcg ccg gaa gtg tgc ccc atg gaa     3072
   Leu Glu His Leu Asp Gly Met Val Ala Pro Glu Val Cys Pro Met Glu
       1010                1015                1020
   acc gcc gct tat gtg agc agt cac tca agc tga                         3105
   Thr Ala Ala Tyr Val Ser Ser His Ser Ser  *
   1025                1030
<210> 63
   <211> 1034
   <212> PRT
   <213> Artificial Sequence
<400> 63
   Arg Asp Asp Pro Ile Arg Pro Pro Leu Lys Val Ala Arg Ser Pro Arg
   1               5                   10                  15
   Pro Gly Gln Cys Gln Asp Val Val Gln Asp Val Pro Asn Val Asp Val
               20                  25                  30
   Gln Met Leu Glu Leu Tyr Asp Arg Met Ser Phe Lys Asp Ile Asp Gly
           35                  40                  45
   Gly Val Trp Lys Gln Gly Trp Asn Ile Lys Tyr Asp Pro Leu Lys Tyr
       50                  55                  60
   Asn Ala His His Lys Leu Lys Val Phe Val Val Pro His Ser His Asn
   65                  70                  75                  80
   Asp Pro Gly Trp Ile Gln Thr Phe Glu Glu Tyr Tyr Gln His Asp Thr
                   85                  90                  95
   Lys His Ile Leu Ser Asn Ala Leu Arg His Leu His Asp Asn Pro Glu
               100                 105                 110
   Met Lys Phe Ile Trp Ala Glu Ile Ser Tyr Phe Ala Arg Phe Tyr His
           115                 120                 125
   Asp Leu Gly Glu Asn Lys Lys Leu Gln Met Lys Ser Ile Val Lys Asn
       130                 135                 140
   Gly Gln Leu Glu Phe Val Thr Gly Gly Trp Val Met Pro Asp Glu Ala
   145                 150                 155                 160
   Asn Ser His Trp Arg Asn Val Leu Leu Gln Leu Thr Glu Gly Gln Thr
                   165                 170                 175
   Trp Leu Lys Gln Phe Met Asn Val Thr Pro Thr Ala Ser Trp Ala Ile
               180                 185                 190
   Asp Pro Phe Gly His Ser Pro Thr Met Pro Tyr Ile Leu Gln Lys Ser
           195                 200                 205
   Gly Phe Lys Asn Met Leu Ile Gln Arg Thr His Tyr Ser Val Lys Lys
       210                 215                 220
   Glu Leu Ala Gln Gln Arg Gln Leu Glu Phe Leu Trp Arg Gln Ile Trp
   225                 230                 235                 240
   Asp Asn Lys Gly Asp Thr Ala Leu Phe Thr His Met Met Pro Phe Tyr
                   245                 250                 255
   Ser Tyr Asp Ile Pro His Thr Cys Gly Pro Asp Pro Lys Val Cys Cys
               260                 265                 270
   Gln Phe Asp Phe Lys Arg Met Gly Ser Phe Gly Leu Ser Cys Pro Trp
           275                 280                 285
   Lys Val Pro Pro Arg Thr Ile Ser Asp Gln Asn Val Ala Ala Arg Ser
       290                 295                 300
   Asp Leu Leu Val Asp Gln Trp Lys Lys Lys Ala Glu Leu Tyr Arg Thr
   305                 310                 315                 320
   Asn Val Leu Leu Ile Pro Leu Gly Asp Asp Phe Arg Phe Lys Gln Asn
                   325                 330                 335
   Thr Glu Trp Asp Val Gln Arg Val Asn Tyr Glu Arg Leu Phe Glu His
               340                 345                 350
   Ile Asn Ser Gln Ala His Phe Asn Val Gln Ala Gln Phe Gly Thr Leu
           355                 360                 365
   Gln Glu Tyr Phe Asp Ala Val His Gln Ala Glu Arg Ala Gly Gln Ala
       370                 375                 380
   Glu Phe Pro Thr Leu Ser Gly Asp Phe Phe Thr Tyr Ala Asp Arg Ser
   385                 390                 395                 400
   Asp Asn Tyr Trp Ser Gly Tyr Tyr Thr Ser Arg Pro Tyr His Lys Arg
                   405                 410                 415
   Met Asp Arg Val Leu Met His Tyr Val Arg Ala Ala Glu Met Leu Ser
               420                 425                 430
   Ala Trp His Ser Trp Asp Gly Met Ala Arg Ile Glu Glu Arg Leu Glu
           435                 440                 445
   Gln Ala Arg Arg Glu Leu Ser Leu Phe Gln His His Asp Gly Ile Thr
       450                 455                 460
   Gly Thr Ala Lys Thr His Val Val Val Asp Tyr Glu Gln Arg Met Gln
   465                 470                 475                 480
   Glu Ala Leu Lys Ala Cys Gln Met Val Met Gln Gln Ser Val Tyr Arg
                   485                 490                 495
   Leu Leu Thr Lys Pro Ser Ile Tyr Ser Pro Asp Phe Ser Phe Ser Tyr
               500                 505                 510
   Phe Thr Leu Asp Asp Ser Arg Trp Pro Gly Ser Gly Val Glu Asp Ser
           515                 520                 525
   Arg Thr Thr Ile Ile Leu Gly Glu Asp Ile Leu Pro Ser Lys His Val
       530                 535                 540
   Val Met His Asn Thr Leu Pro His Trp Arg Glu Gln Leu Val Asp Phe
   545                 550                 555                 560
   Tyr Val Ser Ser Pro Phe Val Ser Val Thr Asp Leu Ala Asn Asn Pro
                   565                 570                 575
   Val Glu Ala Gln Val Ser Pro Val Trp Ser Trp His His Asp Thr Leu
               580                 585                 590
   Thr Lys Thr Ile His Pro Gln Gly Ser Thr Thr Lys Tyr Arg Ile Ile
           595                 600                 605
   Phe Lys Ala Arg Val Pro Pro Met Gly Leu Ala Thr Tyr Val Leu Thr
       610                 615                 620
   Ile Ser Asp Ser Lys Pro Glu His Thr Ser Tyr Ala Ser Asn Leu Leu
   625                 630                 635                 640
   Leu Arg Lys Asn Pro Thr Ser Leu Pro Leu Gly Gln Tyr Pro Glu Asp
                   645                 650                 655
   Val Lys Phe Gly Asp Pro Arg Glu Ile Ser Leu Arg Val Gly Asn Gly
               660                 665                 670
   Pro Thr Leu Ala Phe Ser Glu Gln Gly Leu Leu Lys Ser Ile Gln Leu
           675                 680                 685
   Thr Gln Asp Ser Pro His Val Pro Val His Phe Lys Phe Leu Lys Tyr
       690                 695                 700
   Gly Val Arg Ser His Gly Asp Arg Ser Gly Ala Tyr Leu Phe Leu Pro
   705                 710                 715                 720
   Asn Gly Pro Ala Ser Pro Val Glu Leu Gly Gln Pro Val Val Leu Val
                   725                 730                 735
   Thr Lys Gly Lys Leu Glu Ser Ser Val Ser Val Gly Leu Pro Ser Val
               740                 745                 750
   Val His Gln Thr Ile Met Arg Gly Gly Ala Pro Glu Ile Arg Asn Leu
           755                 760                 765
   Val Asp Ile Gly Ser Leu Asp Asn Thr Glu Ile Val Met Arg Leu Glu
       770                 775                 780
   Thr His Ile Asp Ser Gly Asp Ile Phe Tyr Thr Asp Leu Asn Gly Leu
   785                 790                 795                 800
   Gln Phe Ile Lys Arg Arg Arg Leu Asp Lys Leu Pro Leu Gln Ala Asn
                   805                 810                 815
   Tyr Tyr Pro Ile Pro Ser Gly Met Phe Ile Glu Asp Ala Asn Thr Arg
               820                 825                 830
   Leu Thr Leu Leu Thr Gly Gln Pro Leu Gly Gly Ser Ser Leu Ala Ser
           835                 840                 845
   Gly Glu Leu Glu Ile Met Gln Asp Arg Arg Leu Ala Ser Asp Asp Glu
       850                 855                 860
   Arg Gly Leu Gly Gln Gly Val Leu Asp Asn Lys Pro Val Leu His Ile
   865                 870                 875                 880
   Tyr Arg Leu Val Leu Glu Lys Val Asn Asn Cys Val Arg Pro Ser Lys
                   885                 890                 895
   Leu His Pro Ala Gly Tyr Leu Thr Ser Ala Ala His Lys Ala Ser Gln
               900                 905                 910
   Ser Leu Leu Asp Pro Leu Asp Lys Phe Ile Phe Ala Glu Asn Glu Trp
           915                 920                 925
   Ile Gly Ala Gln Gly Gln Phe Gly Gly Asp His Pro Ser Ala Arg Glu
       930                 935                 940
   Asp Leu Asp Val Ser Val Met Arg Arg Leu Thr Lys Ser Ser Ala Lys
   945                 950                 955                 960
   Thr Gln Arg Val Gly Tyr Val Leu His Arg Thr Asn Leu Met Gln Cys
                   965                 970                 975
   Gly Thr Pro Glu Glu His Thr Gln Lys Leu Asp Val Cys His Leu Leu
               980                 985                 990
   Pro Asn Val Ala Arg Cys Glu Arg Thr Thr Leu Thr Phe Leu Gln Asn
           995                 1000                1005
   Leu Glu His Leu Asp Gly Met Val Ala Pro Glu Val Cys Pro Met Glu
       1010                1015                1020
   Thr Ala Ala Tyr Val Ser Ser His Ser Ser
   1025                1030
<210> 64
   <211> 3105
   <212> DNA
   <213> Artificial Sequence
<220>
   <223> DNA encodes Dm ManII catalytic domain (KD) codon-optimized
<400> 64
   agagacgatc caattagacc tccattgaag gttgctagat ccccaagacc aggtcaatgt 60
   caagatgttg ttcaggacgt cccaaacgtt gatgtccaga tgttggagtt gtacgataga 120
   atgtccttca aggacattga tggtggtgtt tggaagcagg gttggaacat taagtacgat 180
   ccattgaagt acaacgctca tcacaagttg aaggtcttcg ttgtcccaca ctcccacaac 240
   gatcctggtt ggattcagac cttcgaggaa tactaccagc acgacaccaa gcacatcttg 300
   tccaacgctt tgagacattt gcacgacaac ccagagatga agttcatctg ggctgaaatc 360
   tcctacttcg ctagattcta ccacgatttg ggtgagaaca agaagttgca gatgaagtcc 420
   atcgtcaaga acggtcagtt ggaattcgtc actggtggat gggtcatgcc agacgaggct 480
   aactcccact ggagaaacgt tttgttgcag ttgaccgaag gtcaaacttg gttgaagcaa 540
   ttcatgaacg tcactccaac tgcttcctgg gctatcgatc cattcggaca ctctccaact 600
   atgccataca ttttgcagaa gtctggtttc aagaatatgt tgatccagag aacccactac 660
   tccgttaaga aggagttggc tcaacagaga cagttggagt tcttgtggag acagatctgg 720
   gacaacaaag gtgacactgc tttgttcacc cacatgatgc cattctactc ttacgacatt 780
   cctcatacct gtggtccaga tccaaaggtt tgttgtcagt tcgatttcaa aagaatgggt 840
   tccttcggtt tgtcttgtcc atggaaggtt ccacctagaa ctatctctga tcaaaatgtt 900
   gctgctagat ccgatttgtt ggttgatcag tggaagaaga aggctgagtt gtacagaacc 960
   aacgtcttgt tgattccatt gggtgacgac ttcagattca agcagaacac cgagtgggat 1020
   gttcagagag tcaactacga aagattgttc gaacacatca actctcaggc tcacttcaat 1080
   gtccaggctc agttcggtac tttgcaggaa tacttcgatg ctgttcacca ggctgaaaga 1140
   gctggacaag ctgagttccc aaccttgtct ggtgacttct tcacttacgc tgatagatct 1200
   gataactact ggtctggtta ctacacttcc agaccatacc ataagagaat ggacagagtc 1260
   ttgatgcact acgttagagc tgctgaaatg ttgtccgctt ggcactcctg ggacggtatg 1320
   gctagaatcg aggaaagatt ggagcaggct agaagagagt tgtccttgtt ccagcaccac 1380
   gacggtatta ctggtactgc taaaactcac gttgtcgtcg actacgagca aagaatgcag 1440
   gaagctttga aagcttgtca aatggtcatg caacagtctg tctacagatt gttgactaag 1500
   ccatccatct actctccaga cttctccttc tcctacttca ctttggacga ctccagatgg 1560
   ccaggttctg gtgttgagga ctctagaact accatcatct tgggtgagga tatcttgcca 1620
   tccaagcatg ttgtcatgca caacaccttg ccacactgga gagagcagtt ggttgacttc 1680
   tacgtctcct ctccattcgt ttctgttacc gacttggcta acaatccagt tgaggctcag 1740
   gtttctccag tttggtcttg gcaccacgac actttgacta agactatcca cccacaaggt 1800
   tccaccacca agtacagaat catcttcaag gctagagttc caccaatggg tttggctacc 1860
   tacgttttga ccatctccga ttccaagcca gagcacacct cctacgcttc caatttgttg 1920
   cttagaaaga acccaacttc cttgccattg ggtcaatacc cagaggatgt caagttcggt 1980
   gatccaagag agatctcctt gagagttggt aacggtccaa ccttggcttt ctctgagcag 2040
   ggtttgttga agtccattca gttgactcag gattctccac atgttccagt tcacttcaag 2100
   ttcttgaagt acggtgttag atctcatggt gatagatctg gtgcttactt gttcttgcca 2160
   aatggtccag cttctccagt cgagttgggt cagccagttg tcttggtcac taagggtaaa 2220
   ttggagtctt ccgtttctgt tggtttgcca tctgtcgttc accagaccat catgagaggt 2280
   ggtgctccag agattagaaa tttggtcgat attggttctt tggacaacac tgagatcgtc 2340
   atgagattgg agactcatat cgactctggt gatatcttct acactgattt gaatggattg 2400
   caattcatca agaggagaag attggacaag ttgccattgc aggctaacta ctacccaatt 2460
   ccatctggta tgttcattga ggatgctaat accagattga ctttgttgac cggtcaacca 2520
   ttgggtggat cttctttggc ttctggtgag ttggagatta tgcaagatag aagattggct 2580
   tctgatgatg aaagaggttt gggtcagggt gttttggaca acaagccagt tttgcatatt 2640
   tacagattgg tcttggagaa ggttaacaac tgtgtcagac catctaagtt gcatccagct 2700
   ggttacttga cttctgctgc tcacaaagct tctcagtctt tgttggatcc attggacaag 2760
   ttcatcttcg ctgaaaatga gtggatcggt gctcagggtc aattcggtgg tgatcatcca 2820
   tctgctagag aggatttgga tgtctctgtc atgagaagat tgaccaagtc ttctgctaaa 2880
   acccagagag ttggttacgt tttgcacaga accaatttga tgcaatgtgg tactccagag 2940
   gagcatactc agaagttgga tgtctgtcac ttgttgccaa atgttgctag atgtgagaga 3000
   actaccttga ctttcttgca gaatttggag cacttggatg gtatggttgc tccagaagtt 3060
   tgtccaatgg aaaccgctgc ttacgtctct tctcactctt cttga                3105
<210> 65
   <211> 702
   <212> DNA
   <213> Artificial Sequence
<220>
   <223> DNA encodes human Fc
<220>
   <221> CDS
   <222> (1)...(702)
   <223> human Fc
<400> 65
   gct gaa cca aaa tct tgt gat aaa act cat aca tgt cca cca tgt cca     48
   Ala Glu Pro Lys Ser Cys Asp Lys Thr His Thr Cys Pro Pro Cys Pro
    1               5                  10                  15
   gct cct gaa ctt ctg ggt gga cca tca gtt ttc ttg ttc cca cca aaa     96
   Ala Pro Glu Leu Leu Gly Gly Pro Ser Val Phe Leu Phe Pro Pro Lys
               20                  25                  30
   cca aag gat acc ctt atg att tct aga act cct gaa gtc aca tgt gtt    144
   Pro Lys Asp Thr Leu Met Ile Ser Arg Thr Pro Glu Val Thr Cys Val
           35                  40                  45
   gtt gtt gat gtt tct cat gaa gat cct gaa gtc aag ttc aac tgg tac    192
   Val Val Asp Val Ser His Glu Asp Pro Glu Val Lys Phe Asn Trp Tyr
       50                  55                  60
   gtt gat ggt gtt gaa gtt cat aat gct aag aca aag cca aga gaa gaa    240
   Val Asp Gly Val Glu Val His Asn Ala Lys Thr Lys Pro Arg Glu Glu
   65                  70                  75                  80
   caa tac aac tct act tac aga gtt gtc tct gtt ctt act gtt ctg cat    288
   Gln Tyr Asn Ser Thr Tyr Arg Val Val Ser Val Leu Thr Val Leu His
                   85                  90                  95
   caa gat tgg ctg aat ggt aag gaa tac aag tgt aag gtc tcc aac aaa    336
   Gln Asp Trp Leu Asn Gly Lys Glu Tyr Lys Cys Lys Val Ser Asn Lys
               100                 105                 110
   gct ctt cca gct cca att gag aaa acc att tcc aaa gct aaa ggt caa    384
   Ala Leu Pro Ala Pro Ile Glu Lys Thr Ile Ser Lys Ala Lys Gly Gln
           115                 120                 125
   cca aga gaa cca caa gtt tac acc ttg cca cca tcc aga gat gaa ctg    432
   Pro Arg Glu Pro Gln Val Tyr Thr Leu Pro Pro Ser Arg Asp Glu Leu
       130                 135                 140
   act aag aac caa gtc tct ctg act tgt ctg gtt aaa ggt ttc tat cca    480
   Thr Lys Asn Gln Val Ser Leu Thr Cys Leu Val Lys Gly Phe Tyr Pro
   145                 150                 155                 160
   tct gat att gct gtt gaa tgg gag tct aat ggt caa cca gaa aac aac    528
   Ser Asp Ile Ala Val Glu Trp Glu Ser Asn Gly Gln Pro Glu Asn Asn
                   165                 170                 175
   tac aag act act cct cct gtt ctg gat tct gat ggt tcc ttc ttc ctt    576
   Tyr Lys Thr Thr Pro Pro Val Leu Asp Ser Asp Gly Ser Phe Phe Leu
               180                 185                 190
   tac tct aag ctt act gtt gat aag tcc aga tgg caa caa ggt aac gtc    624
   Tyr Ser Lys Leu Thr Val Asp Lys Ser Arg Trp Gln Gln Gly Asn Val
           195                 200                 205
   ttc tca tgt tcc gtt atg cat gaa gct ttg cat aac cat tac act cag    672
   Phe Ser Cys Ser Val Met His Glu Ala Leu His Asn His Tyr Thr Gln
       210                 215                 220
   aag tct ctt tcc ctg tct cca ggt aaa taa                            702
   Lys Ser Leu Ser Leu Ser Pro Gly Lys  *
   225                 230
<210> 66
   <211> 233
   <212> PRT
   <213> Artificial Sequence
<400> 66
   Ala Glu Pro Lys Ser Cys Asp Lys Thr His Thr Cys Pro Pro Cys Pro
    1               5                  10                  15
   Ala Pro Glu Leu Leu Gly Gly Pro Ser Val Phe Leu Phe Pro Pro Lys
               20                  25                  30
   Pro Lys Asp Thr Leu Met Ile Ser Arg Thr Pro Glu Val Thr Cys Val
           35                  40                  45
   Val Val Asp Val Ser His Glu Asp Pro Glu Val Lys Phe Asn Trp Tyr
       50                  55                  60
   Val Asp Gly Val Glu Val His Asn Ala Lys Thr Lys Pro Arg Glu Glu
   65                  70                  75                  80
   Gln Tyr Asn Ser Thr Tyr Arg Val Val Ser Val Leu Thr Val Leu His
                   85                  90                  95
   Gln Asp Trp Leu Asn Gly Lys Glu Tyr Lys Cys Lys Val Ser Asn Lys
               100                 105                 110
   Ala Leu Pro Ala Pro Ile Glu Lys Thr Ile Ser Lys Ala Lys Gly Gln
           115                 120                 125
   Pro Arg Glu Pro Gln Val Tyr Thr Leu Pro Pro Ser Arg Asp Glu Leu
       130                 135                 140
   Thr Lys Asn Gln Val Ser Leu Thr Cys Leu Val Lys Gly Phe Tyr Pro
   145                 150                 155                 160
   Ser Asp Ile Ala Val Glu Trp Glu Ser Asn Gly Gln Pro Glu Asn Asn
                   165                 170                 175
   Tyr Lys Thr Thr Pro Pro Val Leu Asp Ser Asp Gly Ser Phe Phe Leu
               180                 185                 190
   Tyr Ser Lys Leu Thr Val Asp Lys Ser Arg Trp Gln Gln Gly Asn Val
           195                 200                 205
   Phe Ser Cys Ser Val Met His Glu Ala Leu His Asn His Tyr Thr Gln
       210                 215                 220
   Lys Ser Leu Ser Leu Ser Pro Gly Lys
   225                 230
<210> 67
   <211> 1353
   <212> DNA
   <213> Artificial Sequence
<220>
   <223> DNA encodes anti-Her2 HC
<220>
   <221> CDS
   <222> (11)...(1353)
   <223> anti-Her2 HC
<400> 67
   gag gtc caa ttg gtt gaa tct ggt gga ggt ttg gtc caa cca ggt gga    48
   Glu Val Gln Leu Val Glu Ser Gly Gly Gly Leu Val Gln Pro Gly Gly
    1               5                  10                  15
   tct ctg aga ctt tct tgt gct gcc tct ggt ttc aac att aag gat act    96
   Ser Leu Arg Leu Ser Cys Ala Ala Ser Gly Phe Asn Ile Lys Asp Thr
               20                  25                  30
   tac atc cac tgg gtt aga cag gct cca ggt aag ggt ttg gag tgg gtt    144
   Tyr Ile His Trp Val Arg Gln Ala Pro Gly Lys Gly Leu Glu Trp Val
           35                  40                  45
   gct aga atc tac cca acc aac ggt tac acc aga tac gct gat tcc gtt    192
   Ala Arg Ile Tyr Pro Thr Asn Gly Tyr Thr Arg Tyr Ala Asp Ser Val
       50                  55                  60
   aag ggt aga ttc acc att tcc gct gac act tcc aag aac act gct tac    240
   Lys Gly Arg Phe Thr Ile Ser Ala Asp Thr Ser Lys Asn Thr Ala Tyr
   65                  70                  75                  80
   ttg caa atg aac tct ttg aga gct gag gac act gcc gtc tac tac tgt    288
   Leu Gln Met Asn Ser Leu Arg Ala Glu Asp Thr Ala Val Tyr Tyr Cys
                   85                  90                  95
   tcc aga tgg ggt ggt gac ggt ttc tac gcc atg gac tac tgg ggt caa    336
   Ser Arg Trp Gly Gly Asp Gly Phe Tyr Ala Met Asp Tyr Trp Gly Gln
               100                 105                 110
   ggt acc ttg gtt act gtc tct tcc gct tct act aag gga cca tcc gtt      384
   Gly Thr Leu Val Thr Val Ser Ser Ala Ser Thr Lys Gly Pro Ser Val
           115                 120                 125
   ttt cca ttg gct cca tcc tct aag tct act tcc ggt ggt act gct gct      432
   Phe Pro Leu Ala Pro Ser Ser Lys Ser Thr Ser Gly Gly Thr Ala Ala
       130                 135                 140
   ttg gga tgt ttg gtt aag gac tac ttc cca gag cct gtt act gtt tct      480
   Leu Gly Cys Leu Val Lys Asp Tyr Phe Pro Glu Pro Val Thr Val Ser
   145                 150                 155                 160
   tgg aac tcc ggt gct ttg act tct ggt gtt cac act ttc cca gct gtt      528
   Trp Asn Ser Gly Ala Leu Thr Ser Gly Val His Thr Phe Pro Ala Val
                   165                 170                 175
   ttg caa tct tcc ggt ttg tac tcc ttg tcc tcc gtt gtt act gtt cca      576
   Leu Gln Ser Ser Gly Leu Tyr Ser Leu Ser Ser Val Val Thr Val Pro
               180                 185                 190
   tcc tct tcc ttg ggt act cag act tac atc tgt aac gtt aac cac aag      624
   Ser Ser Ser Leu Gly Thr Gln Thr Tyr Ile Cys Asn Val Asn His Lys
           195                 200                 205
   cca tcc aac act aag gtt gac aag aag gtt gag cca aag tcc tgt gac      672
   Pro Ser Asn Thr Lys Val Asp Lys Lys Val Glu Pro Lys Ser Cys Asp
       210                 215                 220
   aag aca cat act tgt cca cca tgt cca gct cca gaa ttg ttg ggt ggt      720
   Lys Thr His Thr Cys Pro Pro Cys Pro Ala Pro Glu Leu Leu Gly Gly
   225                 230                 235                 240
   cca tcc gtt ttc ttg ttc cca cca aag cca aag gac act ttg atg atc      768
   Pro Ser Val Phe Leu Phe Pro Pro Lys Pro Lys Asp Thr Leu Met Ile
                   245                 250                 255
   tcc aga act cca gag gtt aca tgt gtt gtt gtt gac gtt tct cac gag      816
   Ser Arg Thr Pro Glu Val Thr Cys Val Val Val Asp Val Ser His Glu
               260                 265                 270
   gac cca gag gtt aag ttc aac tgg tac gtt gac ggt gtt gaa gtt cac      864
   Asp Pro Glu Val Lys Phe Asn Trp Tyr Val Asp Gly Val Glu Val His
           275                 280                 285
   aac gct aag act aag cca aga gag gag cag tac aac tcc act tac aga      912
   Asn Ala Lys Thr Lys Pro Arg Glu Glu Gln Tyr Asn Ser Thr Tyr Arg
       290                 295                 300
   gtt gtt tcc gtt ttg act gtt ttg cac cag gat tgg ttg aac gga aag      960
   Val Val Ser Val Leu Thr Val Leu His Gln Asp Trp Leu Asn Gly Lys
   305                 310                 315                 320
   gag tac aag tgt aag gtt tcc aac aag gct ttg cca gct cca atc gaa      1008
   Glu Tyr Lys Cys Lys Val Ser Asn Lys Ala Leu Pro Ala Pro Ile Glu
                   325                 330                 335
   aag act atc tcc aag gct aag ggt caa cca aga gag cca cag gtt tac      1056
   Lys Thr Ile Ser Lys Ala Lys Gly Gln Pro Arg Glu Pro Gln Val Tyr
               340                 345                 350
   act ttg cca cca tcc aga gat gag ttg act aag aac cag gtt tcc ttg      1104
   Thr Leu Pro Pro Ser Arg Asp Glu Leu Thr Lys Asn Gln Val Ser Leu
           355                 360                 365
   act tgt ttg gtt aaa gga ttc tac cca tcc gac att gct gtt gag tgg      1152
   Thr Cys Leu Val Lys Gly Phe Tyr Pro Ser Asp Ile Ala Val Glu Trp
       370                 375                 380
   gaa tct aac ggt caa cca gag aac aac tac aag act act cca cca gtt      1200
   Glu Ser Asn Gly Gln Pro Glu Asn Asn Tyr Lys Thr Thr Pro Pro Val
   385                 390                 395                 400
   ttg gat tct gac ggt tcc ttc ttc ttg tac tcc aag ttg act gtt gac      1248
   Leu Asp Ser Asp Gly Ser Phe Phe Leu Tyr Ser Lys Leu Thr Val Asp
                   405                 410                 415
   aag tcc aga tgg caa cag ggt aac gtt ttc tcc tgt tcc gtt atg cat      1296
   Lys Ser Arg Trp Gln Gln Gly Asn Val Phe Ser Cys Ser Val Met His
               420                 425                 430
   gag gct ttg cac aac cac tac act caa aag tcc ttg tct ttg tcc cca      1344
   Glu Ala Leu His Asn His Tyr Thr Gln Lys Ser Leu Ser Leu Ser Pro
           435                 440                 445
   ggt aag taa                                                          1353
   Gly Lys  *
       450
<210> 68
   <211> 450
   <212> PRT
   <213> Artificial Sequence
<400> 68
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 69
   <211> 645
   <212> DNA
   <213> Artificial Sequence
<220>
   <223> DNA encodes anti-Her2 LC
<220>
   <221> CDS
   <222> (1)...(695)
   <223> anti-Her2 LC
<400> 69
   gac att cag atg aca cag tct cca tct tct ttg tcc gct tcc gtc ggt    48
   Asp Ile Gln Met Thr Gln Ser Pro Ser Ser Leu Ser Ala Ser Val Gly
    1               5                  10                  15
   gat aga gtt act atc acc tgt aga gct tcc caa gac gtc aac acc gct    96
   Asp Arg Val Thr Ile Thr Cys Arg Ala Ser Gln Asp Val Asn Thr Ala
               20                  25                  30
   gtc gcc tgg tac caa cag aag cca ggt aag gct cca aaa ctt ttg atc    144
   Val Ala Trp Tyr Gln Gln Lys Pro Gly Lys Ala Pro Lys Leu Leu Ile
           35                  40                  45
   tac tct gcc tct ttc ttg tac tcc ggt gtt cca tcc aga ttt tct ggt    192
   Tyr Ser Ala Ser Phe Leu Tyr Ser Gly Val Pro Ser Arg Phe Ser Gly
        50                  55                  60
   tct aga tcc ggt acc gac ttc acc ttg acc atc tct tcc ttg caa cca     240
   Ser Arg Ser Gly Thr Asp Phe Thr Leu Thr Ile Ser Ser Leu Gln Pro
    65                  70                  75                  80
   gaa gac ttc gct acc tac tac tgt caa caa cac tac act act cct cca     288
   Glu Asp Phe Ala Thr Tyr Tyr Cys Gln Gln His Tyr Thr Thr Pro Pro
                    85                  90                  95
   act ttc ggt caa gga act aag gtt gag att aag aga act gtt gct gct     336
   Thr Phe Gly Gln Gly Thr Lys Val Glu Ile Lys Arg Thr Val Ala Ala
               100                 105                 110
   cca tcc gtt ttc att ttc cca cca tcc gac gaa caa ttg aag tct ggt     384
   Pro Ser Val Phe Ile Phe Pro Pro Ser Asp Glu Gln Leu Lys Ser Gly
           115                 120                 125
   aca gct tcc gtt gtt tgt ttg ttg aac aac ttc tac cca aga gag gct     432
   Thr Ala Ser Val Val Cys Leu Leu Asn Asn Phe Tyr Pro Arg Glu Ala
       130                 135                 140
   aag gtt cag tgg aag gtt gac aac gct ttg caa tcc ggt aac tcc caa     480
   Lys Val Gln Trp Lys Val Asp Asn Ala Leu Gln Ser Gly Asn Ser Gln
   145                 150                 155                 160
   gaa tcc gtt act gag cag gat tct aag gat tcc act tac tcc ttg tcc     528
   Glu Ser Val Thr Glu Gln Asp Ser Lys Asp Ser Thr Tyr Ser Leu Ser
                   165                 170                 175
   tcc act ttg act ttg tcc aag gct gat tac gag aag cac aag gtt tac     576
   Ser Thr Leu Thr Leu Ser Lys Ala Asp Tyr Glu Lys His Lys Val Tyr
               180                 185                 190
   gct tgt gag gtt aca cat cag ggt ttg tcc tcc cca gtt act aag tcc     624
   Ala Cys Glu Val Thr His Gln Gly Leu Ser Ser Pro Val Thr Lys Ser
           195                 200                 205
   ttc aac aga gga gag tgt taa                                         645
   Phe Asn Arg Gly Glu Cys  *
       210
<210> 70
   <211> 214
   <212> PRT
   <213> Artificial Sequence
<400> 70
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Val Asn Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Arg Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210

## Description

### BACKGROUND OF THE INVENTION

### (1) Field of the Invention

The present invention relates to lower eukaryotic cells, such as *Pichia pastoris,* that normally are unable to use galactose as a carbon source but which are rendered capable of using galactose as a sole source of carbon by genetically engineering the cells to express several of the enzymes comprising the Leloir pathway. In particular, the cells are genetically engineered to express a galactokinase, a UDP-galactose-C4-epimerase, and a galactose-1-phosphate uridyltransferase, and optionally a galactose permease. In addition, the present invention further relates to a method for improving the yield of glycoproteins that have galactose-terminated or - containing *N*-glycans in lower eukaryotes that have been genetically engineered to produce glycoproteins with *N*-glycans having galactose residues but which normally lack the enzymes comprising the Leloir pathway comprising transforming the lower eukaryote with one or more nucleic acid molecules encoding a galactokinase, a UDP-galactose-C4-epimerase, and a galactose-1-phosphate uridyltransferase.

### (2) Description of Related Art

Protein-based therapeutics constitute one of the most active areas of drug discovery and are expected to be a major source of new therapeutic compounds in the next decade (Walsh, Nat. Biotechnol. 18(8): 831-3 (2000)). Therapeutic proteins, which are not glycosylated in their native state, can be expressed in hosts that lack a glycosylation machinery, such as *Escherichia coli.* However, most therapeutic proteins are glycoproteins, which require the post-translational addition of glycans to specific asparagine residues of the protein to ensure proper folding and subsequent stability in the human serum (Helenius and Aebi, Science 291: 2364-9 (2001)). In certain cases, the efficacy of therapeutic proteins has been improved by engineering in additional glycosylation sites. One example is the human erythropoietin, which upon the addition of two additional glycosylation sites has been demonstrated to exhibit a threefold longer half-life *in vivo* (Macdougall et al., J. Am. Soc. Nephrol. 10: 2392-2395 (1999)). Most glycoproteins intended for therapeutic use in humans require N-glycosylation and thus, mammalian cell lines, such as Chinese Hamster Ovary (CHO) cells, that approximate human glycoprotein processing are currently most often used for production of therapeutic glycoproteins. However, these cell lines have significant drawbacks including poor genetic tractability, long fermentation times, heterogeneous glycosylation, and ongoing viral containment issues (Birch and Racher, Adv. Drug Deliv. Rev. 58: 671-85 (2006); Kalyanpur, Mol. Biotechnol. 22: 87-98(2002)).

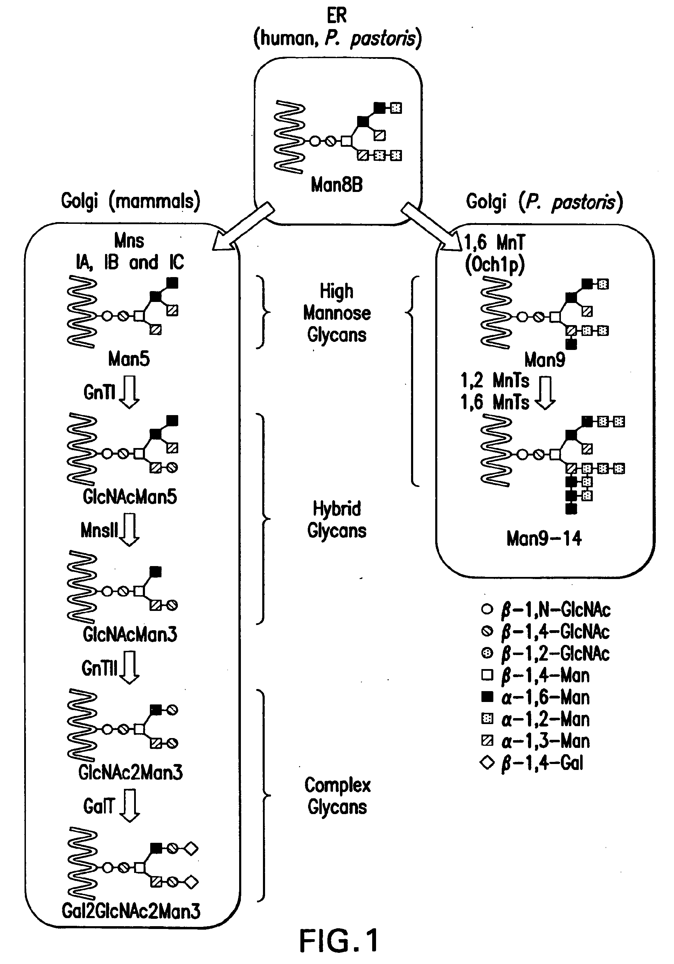

Many industrial protein expression systems are based on yeast strains that can be grown to high cell density in chemically defined medium and generally do not suffer from the abovementioned limitations (Cereghino and Cregg FEMS Microbiol. Rev. 24: 45-66 (2000); Hollenberg and Gellisen, Curr. Opin. Biotechnol. 8: 554-560 (1997); Muller, Yeast 14: 1267-1283 (1998). While yeasts have been used for the production of aglycosylated therapeutic proteins, such as insulin, they have not been used for glycoprotein production because yeast produce the glycoproteins with non-human, high mannose-type *N*-glycans (See Fig. 1), which result in glycoproteins with a shortened *in vivo* half-life and which have the potential to be immunogenic in higher mammals (Tanner et al., Biochim. Biophys. Acta. 906: 81-99 (1987)).

To address these issues, the present inventors and others have focused on the re-engineering of glycosylation pathways in a variety of different yeasts and filamentous fungi to obtain human-like glycoproteins from these protein expression hosts. For example, Gerngross et al., U.S. Published Application No. 2004/0018590 provides cells of the yeast *Pichia pastoris,* which have been genetically engineered to eliminate production of high mannose-type *N*-glycans typical of yeasts and filamentous fungi, and to provide a host cell with the glycosylation machinery to produce glycoproteins with hybrid or complex *N*-glycans more typical of glycoproteins produced from mammalian cells.

Gerngross *et al.* above discloses recombinant yeast strains that can produce recombinant glycoproteins in which a high percentage of the *N*-glycans thereon contain galactose residues: i.e., yeast strains that produce *N*-glycans having predominantly the oligosaccharide structures GalGlcNAc₂Man₃GlcNAc₂ (G1) or Gal₂GlcNAc₂Man₃GlcNAc₂ (G2) and lesser amounts of the oligosaccharide structure GlcNAc₂Man₃GlcNAc₂ (G0). Yields of 70-85% G2 have been obtained. However, it has been found some glycoproteins such as immunoglobulins and immunoadhesins are produced in these cells in which the ratio of G0:G1/G2 is reduced to about 2:1 (*See* for example, Li et al., Nature Biotechnol. 24: 210-215 (2006) wherein the yield of galactose-terminated *N*-glycans from these cells was improved by treating the glycoproteins in *vitro* with galactose and a soluble form of β-1,4-galactosyltransferase). In contrast, immunoglobulins produced in mammalian cells such as CHO cells have a G0:G1/G2 ratio of about 1:1. Thus, it would be desirable to provide a recombinant yeast host cell that is capable of producing recombinant glycoproteins *in vivo* in which the G0:G1/G2 ratio is less than 2:1.

*Pichia pastoris,* can use only a limited number of carbon sources for survival. Currently, these carbon sources are glycerol, glucose, methanol, and perhaps rhamnose and mannose but not galactose. It would be desirable to have *Pichia pastoris* strains that can use carbon source other than those listed above.

### BRIEF SUMMARY OF THE INVENTION

The present invention solves the above identified problems. The present invention provides methods and materials for generating from host cells that lack the ability to assimilate galactose as a carbon source, recombinant host cells that have the ability to use galactose as an energy source. When the recombinant host cells are further genetically engineered to produce glycoproteins that have galactose-terminated or-containing *N*-glycans, the host cells are capable of producing recombinant glycoproteins such as antibodies in which the G0:G1/G2 ratio is less than 2:1, or a G0:G1/G2 ratio that is about 1:1 or less, or a G0:G1/G2 ratio that is about 1:2 or less. In general, the method comprises introducing into the host cells nucleic acid molecules encoding the Leloir pathway enzymes: galactokinase, UDP-galactose-C4-epimerase, and galactose-1-phosphate uridyltransferase, and optionally a galactose permease. Thus, the methods and materials herein provide a selection system that can be used to identify host cells that have been transformed simply by growing the cells on medium containing galactose as the carbon source and provides a method for producing glycoproteins such as immunoglobulins that have a high level of galactose-terminated or -containing *N*-glycans.

The ability to utilize galactose as a carbon source provides flexibility and economy as to the choice of expression systems to use. For example, in systems designed for the expression of recombinant glycoproteins with terminal galactose or terminal sialylation, galactose can be added to the medium where it is taken up by the cells and used by the cells both as an energy source and to provide galactose residues for incorporation into *N*-glycans being synthesized on the recombinant glycoproteins. The advantage of the present invention is that by having galactose present in the medium or adding galactose during fermentation and/or induction of recombinant glycoprotein, production of the recombinant protein can result in higher levels of galactosylated or sialylated glycoprotein. Accordingly, as demonstrated with *Pichia pastoris,* a yeast species that normally lacks the Leloir pathway, genetically engineering *Pichia pastoris* in the manner disclosed herein results in recombinant *Pichia pastoris* cell lines that can use galactose as a sole carbon source. In addition, genetically engineering *Pichia pastoris* cell lines to include the Leloir pathway enzymes and the enzymes needed to render the cells capable of making glycoproteins that have galactose-terminated or -containing *N*-glycans results in a recombinant cell line in which the yield of galactose-terminated or -containing *N*-glycans is greater than when the cell line lacks the Leloir pathway enzymes. Thus, the present invention results in increased productivity in *Pichia pastoris* cell lines that have been genetically engineered to produce galactosylated or sialylated glycoproteins.

In particular, the present invention provides methods and materials which are useful for the production of antibodies with high levels of galactose or sialic acid *in vivo.* Using the methods and materials of the present invention, galactose is added to cell growth medium in order to accomplish multiple purposes including (a) selection of host cells which are able to use galactose as a sugar source; (b) providing a carbon source for the growth of the host cells; and (c) providing a source of galactose residues for incorporation into *N*-glycans, either as the terminal galactose residues in the *N*-glycans or to provide a substrate for subsequent addition of terminal sialic acid residues to the N-glycans. Thus, the present invention provides methods and materials by which levels of galactosylation can be increased through *in vivo* processes, rather than using less efficient and more expensive *in vitro* reactions in which charged galactose and a soluble galactosyl transferase enzyme are added to the medium or solution containing purified but partially galactosylated recombinant glycoproteins.

One embodiment of the present invention is the development of *Pichia pastoris* host cells that are capable of surviving on media in which galactose is present as the sole carbon source. Using the materials and methods of the present invention, one skilled in the art will be able to produce recombinant glycoproteins from the transformed host cells disclosed herein using galactose as the carbon source for selecting and maintaining transformed host cells. Further, by supplying the cell culture medium with galactose, the present invention can be used to increase the levels of galactosylated or sialylated glycoprotein which is produced from the cells when the host cell has been genetically engineered to produce galactosylated or sialylated *N*-glycans.

Therefore, a *Pichia pastoris* host cell is provided that has been genetically engineered to express a galactokinase activity, a UDP-galactose-4-epimerase activity, a galactose-1-phosphate uridyl transferase activity, and optionally a galactose permease activity, wherein the host cell is capable of using galactose as a sole carbon energy source.

In particular aspects, the *Pichia pastoris* host cell has been further genetically engineered to be capable of producing recombinant glycoproteins that have hybrid or complex *N-*glycans that comprise galactose residues. In particular embodiments, the UDP-galactose-4-epimerase activity is provided in a fusion protein comprising the catalytic domain of a galactosyltransferase and the catalytic domain of an UDP-galactose-4-epimerase.

In general, the host cell is capable of producing glycoproteins that have complex *N*-glycans in which the G0:G1/G2 ratio is less than 2:1.

In particular embodiments, the glycoproteins produced in the above cells have predominantly an *N*-glycan selected from the group consisting of GalGlcNAcMansGlcNAc₂; NANAGalGlcNAcMan₅GlcNAc₂; GalGlcNAcMan₃GlcNAc₂; NANAGalGlcNAcMan₃GlcNAc₂; GalGlcNAc₂Man₃GlcNAc₂; Gal₂GlcNAc₂Man₃GlcNAc₂; NANAGal₂GlcNAc₂Man₃GlcNAc₂; and NANA₂Gal₂GlcNAc₂Man₃GlcNAc₂.

In further embodiments, *N*-glycan is a galactose-terminated *N*-glycan selected from the group consisting of GalGlcNAcMan₅GlcNAc₂; Gal₂GlcNAc₂Man₃GlcNAc₂; and Gal₂GlcNAc₂Man₃GlcNAc₂. In other embodiments, the *N*-glycan is a galaclose-terminated hybrid *N*-glycan and in further embodiments, the *N*-glycan is a sialylated *N*-glycan selected from the group consisting of: NANAGalGlcNAcMan₅GlcNAc₂; NANAGal₂GlCNAc₂Man₃GlcNAc₂; and NANA₂Gal₂GlcNAc₂Man₃GlcNAc₂.

Further provided is a method of producing a recombinant glycoprotein in a *Pichia pastoris* host with *N*-glycans that have galactose residues, said method comprising; a) providing a recombinant host cell that has been genetically engineered to express (i) a glycosylation pathway that renders the host cell capable of producing recombinant glycoproteins that have hybrid or complex *N*-glycans that comprise galactose residues; (ii) a galactokinase activity, a UDP-galactose-4-epimerase activity, a galactose-1-phosphate uridyl transferase activity and optionally a galactose permease activity; and (iii) a recombinant glycoprotein; and b) culturing the host cells in a medium containing galactose to produce the recombinant glycoprotein that has one or more *N-*glycans that have galactose residues.

In further aspects of the method, the UDP-galactose-4-epimerase activity is provided in a fusion protein comprising the catalytic domain of a galactosyltransferase and the catalytic domain of an UDP-galactose-4-epimerase.

The present invention further provides a method for selecting a recombinant host cell that expresses a heterologous protein. Recombinant host cells that express one or two but not all of the Leloir pathway enzyme activities are transformed with one or more nucleic acid molecules encoding the heterologous protein and with one or more nucleic acid molecules encoding the Leloir pathway enzymes not present in the recombinant host cell. Since the transformed recombinant host cell contains a complete Leloir pathway, selection of the transformed recombinant host cell that expresses the heterologous protein from non-transformed cells can be achieved by culturing the transformed recombinant host cells in a medium in which galactose is the sole carbon source. Thus, further provided is a method for producing a recombinant host cell that expresses a heterologous protein, comprising: (a) providing a host cell that has been genetically engineered to express one or two enzymes selected from the group consisting of a galactokinase, a UDP-galactose-4-epimerase, and a galactose-1-phosphate uridyl transferase; (b) transforming the host cell with one or more nucleic acid molecules encoding the heterologous protein; and with one or more nucleic acid molecules encoding the enzyme or enzymes from the group in step (a) not expressed in the host cell of step (a) so that all the enzymes from the group in step (a) are present in the host cell; and (c) culturing the host cells in a medium containing galactose as the sole carbon source to provide the recombinant *Pichia pastoris* host cell that expresses a heterologous protein.

In further aspects of the method, the host cell is further genetically engineered to express a galactose permease. In further still aspects, the host cell is genetically modified to produce glycoproteins that have one or more *N*-glycans that comprise galactose.

Further provided is a method of producing and selecting *Pichia pastoris* host cells capable of using galactose as a sole carbon source, the method comprising;
a) providing a *Pichia pastoris* host cell; b) transforming the host cell with one or more nucleic acid molecules encoding a galactokinase, a UDP-galactose-4-epimerase, a galactose-1-phosphate uridyl transferase, and optionally a galactose permease; c) culturing the transformed host cells of on a medium containing galactose as the sole carbon source; and d) selecting the host cells that can grow on the medium containing galactose as the sole carbon source.

The host cells and methods herein enable the production of compositions comprising a recombinant glycoprotein wherein the ratio of G0:G1/G2 glycoforms thereon is less than 2:1 in a pharmaceutically acceptable carrier.

While *Pichia pastoris* is proved as an example of a host cell that can be modified as disclosed herein, the methods and host cells are not limited to *Pichia pastoris.* The methods herein can be used to produce recombinant host cells from other lower eukaryote species that normally do not express the Leloir pathway enzymes and as such are incapable of using galactose as a carbon source. Thus, in further embodiments, the host cell is any lower eukaryote species that normally do not express the Leloir pathway enzymes.

### Definitions

Unless otherwise defined, all technical and scientific terms used herein have the same meaning as commonly understood by one of ordinary skill in the art to which this invention pertains. Further, unless otherwise required by context, singular terms shall include the plural and plural terms shall include the singular. Generally, nomenclatures used in connection with, and techniques of biochemistry, enzymology, molecular and cellular biology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. *See, e.g.,* Sambrook et al. Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lane, Antibodies: A Laboratory Manual, Cold Spring Harbor Laboratory Press, Cold Spring Harbor, N.Y. (1990); Taylor and Drickamer, Introduction to Glycobiology, Oxford Univ. Press (2003); Worthington Enzyme Manual, Worthington Biochemical Corp., Freehold, NJ; Handbook of Biochemistry: Section A Proteins, Vol I, CRC Press (1976); Handbook of Biochemistry: Section A Proteins, Vol II, CRC Press (1976); Essentials of Glycobiology, Cold Spring Harbor Laboratory Press (1999). Exemplary methods and materials are described below, although methods and materials similar or equivalent to those described herein can also be used in the practice of the present invention and will be apparent to those of skill in the art.

The following terms, unless otherwise indicated, shall be understood to have the following meanings:
As used herein, the terms "humanized," "humanization" and "human-like" are used interchangeably, and refer to the process of engineering non-human cells, such as lower eukaryotic host cells, in a manner which results in the ability of the engineered cells to produce proteins, in particular, glycoproteins, which have glycosylation which more closely resembles mammalian glycosylation patterns than glycoproteins produced by non-engineered, wild-type non-human cell of the same species. Humanization may be perfomed with respect to either N-glycosylation, O-glycosylation, or both. For example, wild-type *Pichia pastoris* and other lower eukaryotic cells typically produce hypermannosylated proteins at N-glycosylation sites. In preferred embodiments of the present invention, "humanized" host cells of the present invention are capable of producing glycoproteins with hybrid and/or complex *N*-glycans; i.e., "human-like N-glycosylation." The specific "human-like" glycans predominantly present on glycoproteins produced from the humanized host cells will depend upon the specific humanization steps that are performed.

As used herein, the terms "*N*-glycan" and "glycoform" are used interchangeably and refer to an *N*-linked oligosaccharide, e.g., one that is attached by an asparagine-N-acetylglucosamine linkage to an asparagine residue of a polypeptide. *N*-linked glycoproteins contain an *N*-acetylglucosamine residue linked to the amide nitrogen of an asparagine residue in the protein. The predominant sugars found on glycoproteins are glucose, galactose, mannose, fucose, *N*-acetylgalactosamine (GalNAc), *N*-acetylglucosamine (GlcNAc) and sialic acid (e.g., *N-*acetyl-neuraminic acid (NANA)). The processing of the sugar groups occurs cotranslationally in the lumen of the ER and continues in the Golgi apparatus for *N*-linked glycoproteins.

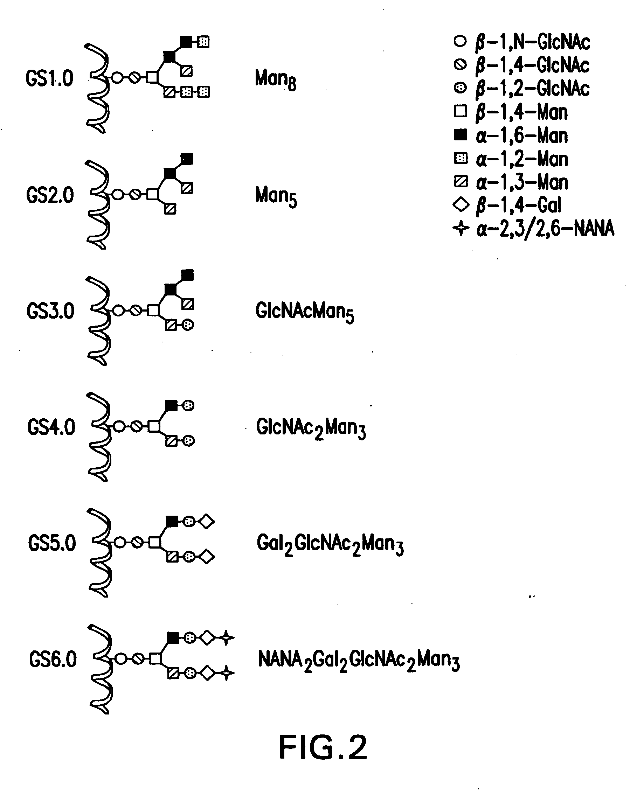

*N*-glycans have a common pentasaccharide core of Man₃GlcNAc₂ ("Man" refers to mannose; "Glc" refers to glucose; and "NAc" refers to *N*-acetyl; GlcNAc refers to *N-*acetylglucosamine). *N*-glycans differ with respect to the number of branches (antennae) comprising peripheral sugars (e.g., GlcNAc, galactose, fucose and sialic acid) that are added to the Man₃GlcNAc₂ ("Man3") core structure which is also referred to as the "trimannose core", the "pentasaccharide core" or the "paucimannose core". *N*-glycans are classified according to their branched constituents (e.g., high mannose, complex or hybrid). A "high mannose" type *N*-glycans has five or more mannose residues. A "complex" type *N*-glycan typically has at least one GlcNAc attached to the 1,3 mannose arm and at least one GlcNAc attached to the 1,6 mannose arm of a "trimannose" core. Complex *N*-glycans may also have galactose ("Gal") or *N-*acetylgalactosamine ("GalNAc") residues that are optionally modified with sialic acid or derivatives (e.g., "NANA" or "NeuAc", where "Neu" refers to neuraminic acid and "Ac" refers to acetyl). Complex *N*-glycans may also have intrachain substitutions comprising "bisecting" GlcNAc and core fucose ("Fuc"). Complex *N*-glycans may also have multiple antennae on the "trimannose core," often referred to as "multiple antennary glycans." A "hybrid" *N*-glycan has at least one GlcNAc on the terminal of the 1,3 mannose arm of the trimannose core and zero or more mannoses on the 1,6 mannose arm of the trimannose core. The various *N*-glycans are also referred to as "glycoforms." Figure 2 shows various high mannose, hybrid, and complex *N-*glycans that have been produced in Pichia pastoris genetically engineered to produce mammalian-like *N*-glycans.

As used herein, the terms "*O*-glycan"and "glycoform" are used interchangeably and refer to an *O*-linked oligosaccharide, e.g., a glycan that is attached to a peptide chain via the hydroxyl group of either a serine or threonine residue. In fungal cells, native *O*-glycosylation occurs through attachment of a first mannosyl residue transferred from a dolichol monophosphate mannose (Dol-P-Man) to the protein in the endoplasmic reticulum, and additional mannosyl residues may be attached via transfer from GPD-Man in the Golgi apparatus. Higher eukaryotic cells, such as human or mammalian cells, undergo O-glycosylation through covalent attachment of N-acetyl-galactosamine (GlcNac) to the serine or threonine residue.

As used herein, the term "human-like *O*-glycosylation" will be understood to mean that fungal-specific phosphorylated mannose structures are reduced or eliminated, resulting in reduction or elimination of charge and beta-mannose structures, or that the predominant O-glycan species present on a glycoprotein or in a composition of glycoprotein comprises a glycan capped with a terminal residue selected from GlcNac; Gal, or NANA (or Sia). In this manner, the recombinant glycoprotein bearing predominantly human-like *O*-glycosylation may be recognized by a human or mammalian cell as if it were a natively produced glycoprotein, which result in improved therapeutic properties of the recombinant glycoprotein.

Abbreviations used herein are of common usage in the art, see, e.g., abbreviations of sugars, above. Other common abbreviations include "PNGase", or "glycanase" or "glucosidase" which all refer to peptide *N*-glycosidase F (EC 3.2.2.18).

As used herein, the terms "antibody," "immunoglobulin," "immunoglobulins" and "immunoglobulin molecule" are used interchangeably. Each immunoglobulin molecule has a unique structure that allows it to bind its specific antigen, but all immunoglobulins have the same overall structure as described herein. The basic immunoglobulin structural unit is known to comprise a tetramer of subunits. Each tetramer has two identical pairs of polypeptide chains, each pair having one "light" chain (LC) (about 25 kDa) and one "heavy" chain (HC) (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Light chains (LCs) are classified as either kappa or lambda. Heavy chains (HCs) are classified as gamma, mu, alpha, delta, or epsilon, and define the antibody's isotype as IgG, IgM, IgA, IgD and IgE, respectively.

The light and heavy chains are subdivided into variable regions and constant regions (See generally, Fundamental Immunology (Paul, W., ed., 2nd ed. Raven Press, N.Y., 1989), Ch. 7. The variable regions of each light/heavy chain pair form the antibody binding site. Thus, an intact antibody has two binding sites. Except in bifunctional or bispecific antibodies, the two binding sites are the same. The chains all exhibit the same general structure of relatively conserved framework regions (FR) joined by three hypervariable regions, also called complementarity determining regions or CDRs. The CDRs from the two chains of each pair are aligned by the framework regions, enabling binding to a specific epitope. The terms include naturally occurring forms, as well as fragments and derivatives. Included within the scope of the term are classes of immunoglobulins (Igs), namely, IgG, IgA, IgE, IgM, and IgD. Also included within the scope of the terms are the subtypes of IgGs, namely, IgG1, IgG2, IgG3 and IgG4. The term is used in the broadest sense and includes single monoclonal antibodies (including agonist and antagonist antibodies) as well as antibody compositions which will bind to multiple epitopes or antigens. The terms specifically cover monoclonal antibodies (including full length monoclonal antibodies), polyclonal antibodies, multispecific antibodies (for example, bispecific antibodies), and antibody fragments so long as they contain or are modified to contain at least the portion of the C_{H}2 domain of the heavy chain immunoglobulin constant region which comprises an N-linked glycosylation site of the C_{H}2 domain, or a variant thereof. Included within the terms are molecules comprising only the Fc region, such as immunoadhesins (U.S. Published Patent Application No. 20040136986), Fc fusions, and antibody-like molecules. Alternatively, these terms can refer to an antibody fragment of at least the Fab region that at least contains an N-linked glycosylation site.

The term "Fc" fragment refers to the 'fragment crystallized' C-terminal region of the antibody containing the C_{H}2 and C_{H}3 domains. The term "Fab" fragment refers to the 'fragment antigen binding' region of the antibody containing the VH, C_{H}1, V_{L} and C_{L} domains.

The term "monoclonal antibody" (mAb) as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each mAb is directed against a single determinant on the antigen. In addition to their specificity, monoclonal antibodies are advantageous in that they can be synthesized by hybridoma culture, uncontaminated by other immunoglobulins. The term "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., (1975) Nature, 256: 495, or may be made by recombinant DNA methods *(See,* for example, U.S. Patent No. 4,816,567 to Cabilly et al.*).*

The term "fragments" within the scope of the terms "antibody" or "immunoglobulin" include those produced by digestion with various proteases, those produced by chemical cleavage and/or chemical dissociation and those produced recombinantly, so long as the fragment remains capable of specific binding to a target molecule. Among such fragments are Fc, Fab, Fab', Fv, F(ab')₂, and single chain Fv (scFv) fragments. Hereinafter, the term "immunoglobulin" also includes the term "fragments" as well.

Immunoglobulins further include immunoglobulins or fragments that have been modified in sequence but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (*See,* for example, Intracellular Antibodies: Research and Disease Applications, (Marasco, ed., Springer-Verlag New York, Inc., 1998).

The term "catalytic antibody" refers to immunoglobulin molecules that are capable of catalyzing a biochemical reaction. Catalytic antibodies are well known in the art and have been described in U.S. Patent Nos. 7,205,136; 4,888,281; and 5,037,750 to Schochetman et al.*,* U.S. Patent Nos. 5,733,757; 5,985,626; and 6,368,839 to Barbas, III *et al.*

The term "vector" as used herein is intended to refer to a nucleic acid molecule capable of transporting another nucleic acid to which it has been linked. One type of vector is a "plasmid", which refers to a circular double stranded DNA loop into which additional DNA segments may be ligated. Other vectors include cosmids, bacterial artificial chromosomes (BAC) and yeast artificial chromosomes (YAC). Another type of vector is a viral vector, wherein additional DNA segments may be ligated into the viral genome (discussed in more detail below). Certain vectors are capable of autonomous replication in a host cell into which they are introduced (*e.g.,* vectors having an origin of replication which functions in the host cell). Other vectors can be integrated into the genome of a host cell upon introduction into the host cell, and are thereby replicated along with the host genome. Moreover, certain preferred vectors are capable of directing the expression of genes to which they are operatively linked. Such vectors are referred to herein as "recombinant expression vectors" (or simply, "expression vectors").

As used herein, the term "sequence of interest" or "gene of interest" refers to a nucleic acid sequence, typically encoding a protein, that is not normally produced in the host cell. The methods disclosed herein allow efficient expression of one or more sequences of interest or genes of interest stably integrated into a host cell genome. Non-limiting examples of sequences of interest include sequences encoding one or more polypeptides having an enzymatic activity, e.g., an enzyme which affects *N*-glycan synthesis in a host such as mannosyltransferases, *N-*acetylglucosaminyltransferases, UDP-*N*-acetylglucosamine transporters, galactosyltransferases, UDP-*N*-acetylgalactosyltransferase, sialyltransferases and fucosyltransferases.

The term "marker sequence" or "marker gene" refers to a nucleic acid sequence capable of expressing an activity that allows either positive or negative selection for the presence or absence of the sequence within a host cell. For example, the *P. pastoris URA5* gene is a marker gene because its presence can be selected for by the ability of cells containing the gene to grow in the absence of uracil. Its presence can also be selected against by the inability of cells containing the gene to grow in the presence of 5-FOA. Marker sequences or genes do not necessarily need to display both positive and negative selectability. Non-limiting examples of marker sequences or genes from *P. pastoris* include *ADE1, ARG4, HIS4* and *URA3.* For antibiotic resistance marker genes, kanamycin, neomycin, geneticin (or G418), paromomycin and hygromycin resistance genes are commonly used to allow for growth in the presence of these antibiotics.

The term "operatively linked" expression control sequences refers to a linkage in which the expression control sequence is contiguous with the gene of interest to control the gene of interest, as well as expression control sequences that act in *trans* or at a distance to control the gene of interest.

The term "expression control sequence" or "regulatory sequences" are used interchangeably and as used herein refer to polynucleotide sequences which are necessary to affect the expression of coding sequences to which they are operatively linked. Expression control sequences are sequences which control the transcription, post-transcriptional events and translation of nucleic acid sequences. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (*e.g*., ribosome binding sites); sequences that enhance protein stability; and when desired, sequences that enhance protein secretion. The nature of such control sequences differs depending upon the host organism; in prokaryotes, such control sequences generally include promoter, ribosomal binding site, and transcription termination sequence. The term "control sequences" is intended to include, at a minimum, all components whose presence is essential for expression, and can also include additional components whose presence is advantageous, for example, leader sequences and fusion partner sequences.

The term "recombinant host cell" ("expression host cell", "expression host system", "expression system" or simply "host cell"), as used herein, is intended to refer to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism.

The term "eukaryotic" refers to a nucleated cell or organism, and includes insect cells, plant cells, mammalian cells, animal cells and lower eukaryotic cells.

The term "lower eukaryotic cells" includes yeast, fungi, collar-flagellates, microsporidia, alveolates (e.g., dinoflagellates), stramenopiles (e.g, brown algae, protozoa), rhodophyta (e.g., red algae), plants (e.g., green algae, plant cells, moss) and other protists. Yeast and filamentous fungi include, but are not limited to: *Pichia pastoris, Pichia finlandica, Pichia trehalophila, Pichia koclamae, Pichia membranaefaciens, Pichia mircuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia* sp., *Saccharomyces cerevisiae, Saccharomyces* sp., *Hansenula polymorpha, Kluyveromyces sp., Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens* and *Neurospora crassa.*

The term "peptide" as used herein refers to a short polypeptide, *e.g*., one that is typically less than about 50 amino acids long and more typically less than about 30 amino acids long. The term as used herein encompasses analogs and mimetics that mimic structural and thus biological function.

The term "polypeptide" encompasses both naturally-occurring and non-naturally-occurring proteins, and fragments, mutants, derivatives and analogs thereof. A polypeptide may be monomeric or polymeric. Further, a polypeptide may comprise a number of different domains each of which has one or more distinct activities.

The term "isolated protein" or "isolated polypeptide" is a protein or polypeptide that by virtue of its origin or source of derivation (1) is not associated with naturally associated components that accompany it in its native state, (2) exists in a purity not found in nature, where purity can be adjudged with respect to the presence of other cellular material (*e.g*., is free of other proteins from the same species) (3) is expressed by a cell from a different species, or (4) does not occur in nature (*e.g*., it is a fragment of a polypeptide found in nature or it includes amino acid analogs or derivatives not found in nature or linkages other than standard peptide bonds). Thus, a polypeptide that is chemically synthesized or synthesized in a cellular system different from the cell from which it naturally originates will be "isolated" from its naturally associated components. A polypeptide or protein may also be rendered substantially free of naturally associated components by isolation, using protein purification techniques well known in the art. As thus defined, "isolated" does not necessarily require that the protein, polypeptide, peptide or oligopeptide so described has been physically removed from its native environment.

The term "polypeptide fragment" as used herein refers to a polypeptide that has a deletion, *e.g*., an amino-terminal and/or carboxy-terminal deletion compared to a full-length polypeptide. In a preferred embodiment, the polypeptide fragment is a contiguous sequence in which the amino acid sequence of the fragment is identical to the corresponding positions in the naturally-occurring sequence. Fragments typically are at least 5, 6, 7, 8, 9 or 10 amino acids long, preferably at least 12, 14, 16 or 18 amino acids long, more preferably at least 20 amino acids long, more preferably at least 25, 30, 35, 40 or 45, amino acids, even more preferably at least 50 or 60 amino acids long, and even more preferably at least 70 amino acids long.

A "modified derivative" refers to polypeptides or fragments thereof that are substantially homologous in primary structural sequence but which include, *e.g., in vivo* or *in vitro* chemical and biochemical modifications or which incorporate amino acids that are not found in the native polypeptide. Such modifications include, for example, acetylation, carboxylation, phosphorylation, glycosylation, ubiquitination, labeling, *e.g*., with radionuclides, and various enzymatic modifications, as will be readily appreciated by those skilled in the art. A variety of methods for labeling polypeptides and of substituents or labels useful for such purposes are well known in the art, and include radioactive isotopes such as ¹²⁵I, ³²P, ³⁵S, and ³H, ligands which bind to labeled antiligands (*e.g.*, antibodies), fluorophores, chemiluminescent agents, enzymes, and antiligands which can serve as specific binding pair members for a labeled ligand. The choice of label depends on the sensitivity required, ease of conjugation with the primer, stability requirements, and available instrumentation. Methods for labeling polypeptides are well known in the art. *See, e.g.,* Ausubel et al., Current Protocols in Molecular Biology, Greene Publishing Associates (1992, and Supplements to 2002) .

The term "chimeric gene" or "chimeric nucleotide sequences" refers to a nucleotide sequence comprising a nucleotide sequence or fragment coupled to heterologous nucleotide sequences. Chimeric sequences are useful for the expression of fusion proteins. Chimeric genes or chimeric nucleotide sequences may also comprise one or more fragments or domains which are heterologous to the intended host cell, and which may have beneficial properties for the production of heterologous recombinant proteins. Generally, a chimeric nucleotide sequence comprises at least 30 contiguous nucleotides from a gene, more preferably at least 60 or 90 or more nucleotides. Chimeric nucleotide sequences which have at least one fragment or domain which is heterologous to the intended host cell, but which is homologous to the intended recombinant protein, have particular utility in the present invention. For example, a chimeric gene intended for use in an expression system using *P. pastoris* host cells to express recombinant human glycoproteins will preferably have at least one fragment or domain which is of human origin, such as a sequence which encodes a human protein with potential therapeutic value, while the remainder of the chimeric gene, such as regulatory sequences which will allow the host cell to process and express the chimeric gene, will preferably be of *P. pastoris* origin. If desired, the fragment of human origin may also be codon-optimized for expression in the host cell. *(See, e.g.,* US Patent 6,884,602).

The term "fusion protein" or "chimeric protein" refers to a polypeptide comprising a polypeptide or fragment coupled to heterologous amino acid sequences. Fusion proteins are useful because they can be constructed to contain two or more desired functional elements from two or more different proteins. A fusion protein comprises at least 10 contiguous amino acids from a polypeptide of interest, more preferably at least 20 or 30 amino acids, even more preferably at least 40, 50 or 60 amino acids, yet more preferably at least 75, 100 or 125 amino acids. Fusions that include the entirety of the proteins of the present invention have particular utility. The heterologous polypeptide included within the fusion protein of the present invention is at least 6 amino acids in length, often at least 8 amino acids in length, and usefully at least 15, 20, and 25 amino acids in length. Fusions also include larger polypeptides, or even entire proteins, such as the green fluorescent protein ("GFP") chromophore-containing proteins having particular utility. Fusion proteins can be produced recombinantly by constructing a nucleic acid sequence which encodes the polypeptide or a fragment thereof in frame with a nucleic acid sequence encoding a different protein or peptide and then expressing the fusion protein. Alternatively, a fusion protein can be produced chemically by crosslinking the polypeptide or a fragment thereof to another protein.

The term "non-peptide analog" refers to a compound with properties that are analogous to those of a reference polypeptide. A non-peptide compound may also be termed a "peptide mimetic" or a "peptidomimetic". *See, e.g.,* Jones, Amino Acid and Peptide Synthesis, Oxford University Press (1992); Jung, Combinatorial Peptide and Nonpeptide Libraries: A Handbook, John Wiley (1997); Bodanszky et al., Peptide Chemistry--A Practical Textbook, Springer Verlag (1993); Synthetic Peptides: A Users Guide, (Grant, ed., W. H. Freeman and Co., 1992); Evans et al., J. Med. Chem. 30:1229 (1987); Fauchere, J Adv. Drug Res. 15: 29 (1986); Veber and Freidinger, Trends Neurosci., 8: 392-396 (1985); and references sited in each of the above. Such compounds are often developed with the aid of computerized molecular modeling. Peptide mimetics that are structurally similar to useful peptides of the invention may be used to produce an equivalent effect and are therefore envisioned to be part of the invention.

The term "region" as used herein refers to a physically contiguous portion of the primary structure of a biomolecule. In the case of proteins, a region is defined by a contiguous portion of the amino acid sequence of that protein.

The term "domain" as used herein refers to a structure of a biomolecule that contributes to a known or suspected function of the biomolecule. Domains may be co-extensive with regions or portions thereof; domains may also include distinct, non-contiguous regions of a biomolecule.

As used herein, the term "molecule'" means any compound, including, but not limited to, a small molecule, peptide, protein, sugar, nucleotide, nucleic acid, lipid, etc., and such a compound can be natural or synthetic.

As used herein, the term "comprise" or variations such as "comprises" or comprising", will be understood to imply the inclusion of a stated integer or group of integers but not the exclusion of any other integer or group of integers.

As used herein, the term "predominantly" or variations such as "the predominant" or "which is predominant" will be understood to mean the glycan species that has the highest mole percent (%) of total O-glycans or *N*-glycans after the glycoprotein has been treated with enzymes and released glycans analyzed by mass spectroscopy, for example, MALDI-TOF MS. In other words, the phrase "predominantly" is defined as an individual entity, such that a specific "predominant" glycoform is present in greater mole percent than any other individual entity. For example, if a composition consists of species A in 40 mole percent, species B in 35 mole percent and species C in 25 mole percent, the composition comprises predominantly species A.

### BRIEF DESCRIPTION OF THE DRAWINGS

Figure 1 illustrates the *N*-glycosylation pathways in humans and *P. pastoris.* Early events in the ER are highly conserved, including removal of three glucose residues by glucosidases I and II and trimming of a single specific α-1,2-linked mannose residue by the ER mannosidase leading to the same core structure, Man₈GlcNAc₂ (Man8B). However, processing events diverge in the Golgi. Mns, α-1,2-mannosidase; MnsII, mannosidase II; GnT I, α-1,2-*N-*acetylglucosaminyltransferase I; GnT II, α-1,2-*N*-acetylglucosaminyltransferase II; MnT, mannosyltransferase. The two core GlcNAc residues, though present in all cases, were omitted in the nomenclature.
Figure 2 illustrates the key intermediate steps in *N*-glycosylation as well as a shorthand nomenclature referring to the genetically engineered *Pichia pastoris* strains producing the respective glycan structures (GS).

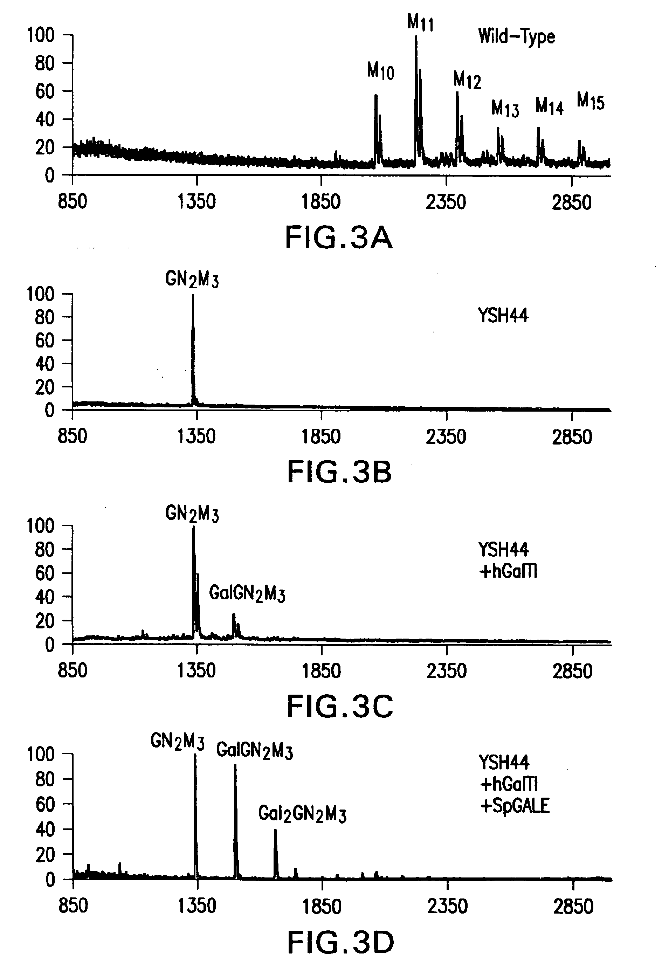

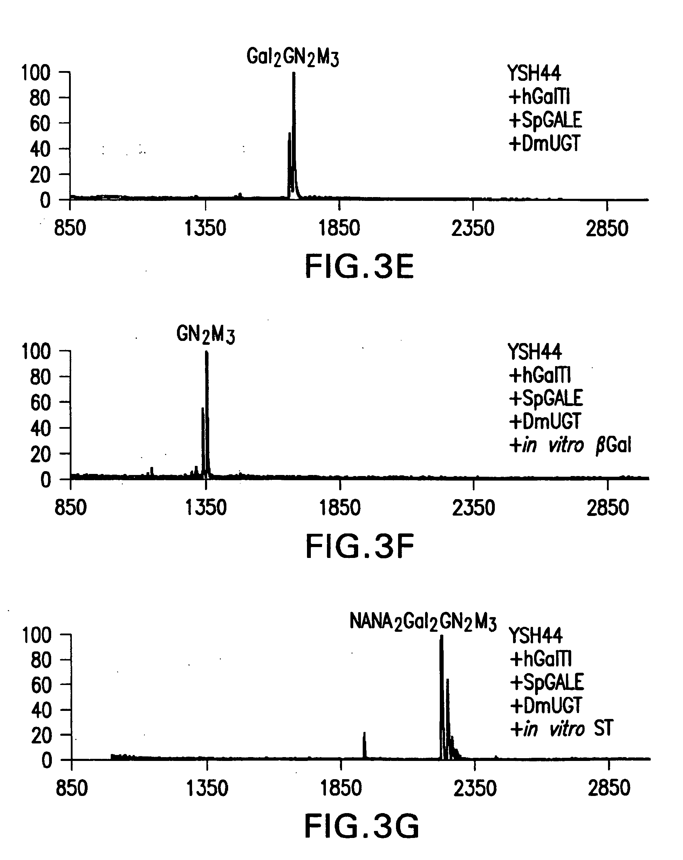

Figure 3 illustrates MALDI-TOF Mass Spectroscopy (MS) analysis of *N-*glycosidase F released *N*-glycans. K3 (the kringle 3 domain of human Plasminogen) was produced in *P. pastoris* strains GS115-derived wild-type control (Invitrogen, Carlsbad, CA), YSH44, YSH71, RDP52, and RDP80 and purified from culture supernatants by Ni-affinity chromatography. *N*-glycans were released by N-glycosidase F treatment and subjected to MALDI-TOF MS analysis (positive mode, except for Figure 3G which was negative mode) appearing as sodium or potassium adducts. The two core GlcNAc residues, though present, were omitted in the nomenclature. GN, GlcNAc; M, mannose.
Figure 3A: *N*-glycans produced in GS115-derived wild-type control strain;
Figure 3B: *N*-glycans produced on K3 in strain YSH44;
Figure 3C: *N*-glycans produced on K3 in strain YSH71 (YSH44 expressing hGalTI),
Figure (3D) *N*-glycans produced on K3 in strain RDP52 (YSH44 expressing hGalTI and SpGALE),
Figure (3E) *N*-glycans produced on K3 in strain RDP80 (YSH44 expressing hGalTI, SpGALE, and DmUGT);
Figure (3F) glycans from RDP80 after α-galactosidase treatment *in vitro;* and
Figure (3G) glycans from RDP80 in negative mode after treatment with α-2,6-(*N*)-sialyltransferase.

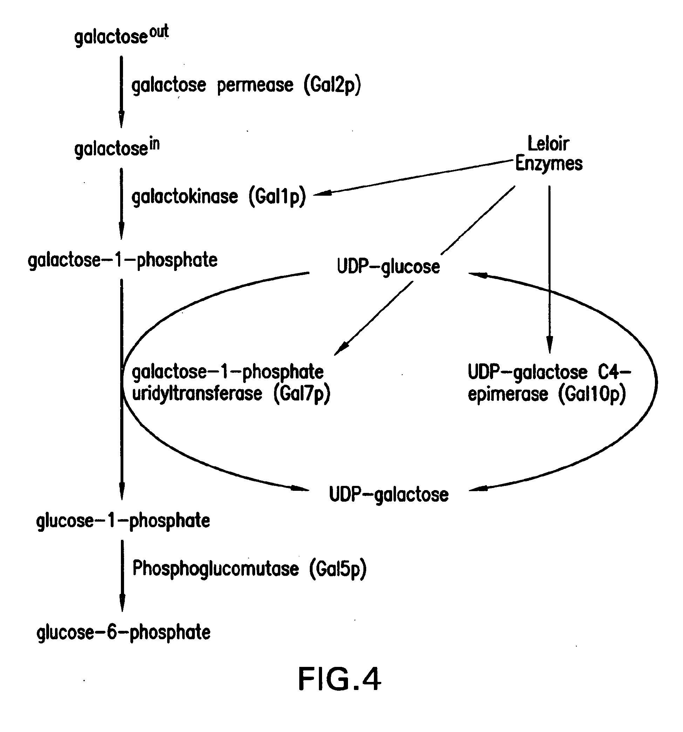

Figure 4 illustrates the Leloir galactose utilization pathway. Extracellular galactose is imported via a galactose permease. The galactose is converted into glucose-6-phosphate by the action of the enzymes galactokinase, galactose-1-phosphate uridyltransferase, and UDP-galactose C4-epimerase. Protein names from S. *cerevisiae* are in parentheses.

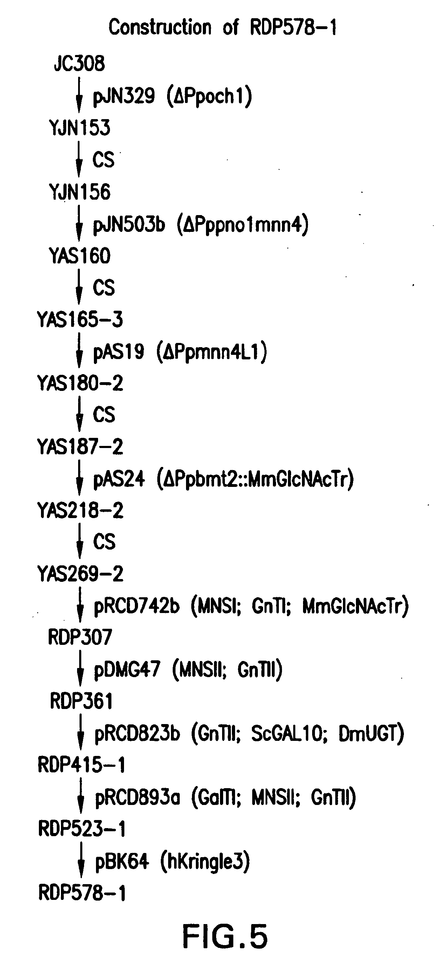

Figure 5 shows the construction of *P. pastoris* glycoengineered strain YGLY578-1. The *P. pastoris* genes *OCH1, MNN4, PNO1, MNN4L1,* and *BMT2* encoding Golgi glycosyltransferases were knocked out followed by knock-in of 12 heterologous genes, including the expression cassette for secreted hK3. YGLY578-1 is capable of producing glycoproteins that have Gal₂GlcNAc₂Man₃GlcNAc₂ *N*-glycans. CS, counterselect.

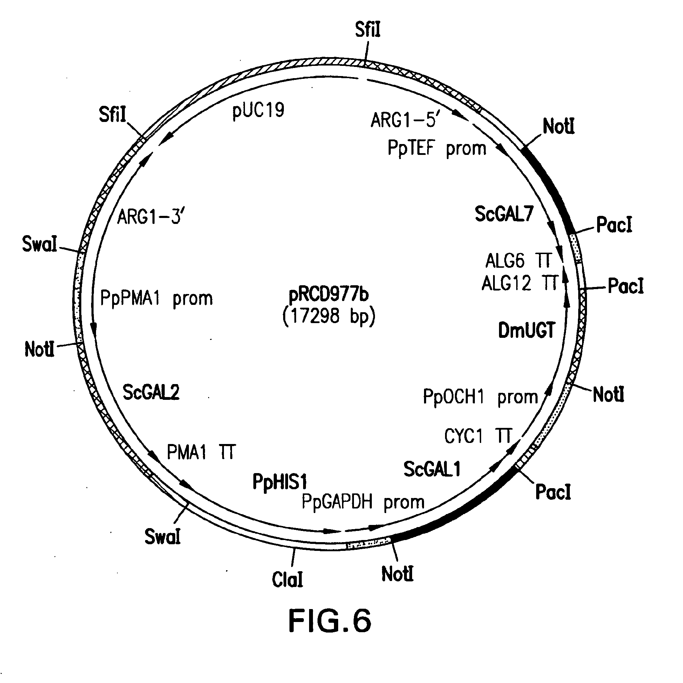

Figure 6 shows a feature diagram of plasmid pRCD977b. This plasmid is an *arg1::HIS1* knock out plasmid that integrates into and deletes the *P. pastoris ARG1* gene while using the *PpHIS1* gene as a selectable marker and contains expression cassettes encoding the full-length *D. melanogaster* Golgi UDP-galactose transporter (*DmUGT*), full-length S. *cerevisiae* galactokinase (*ScGAL1*)*,* full-length S. *cerevisiae* galactose-1-phosphate uridyl transferase *(ScGAL7),* and *S. cerevisiae* galactose permease *(ScGAL2)* under the control of the *PpOCH1, PpGAPDH, PpPMA1,* and *PpTEF* promoters, respectively. TT refers to transcription termination sequence.

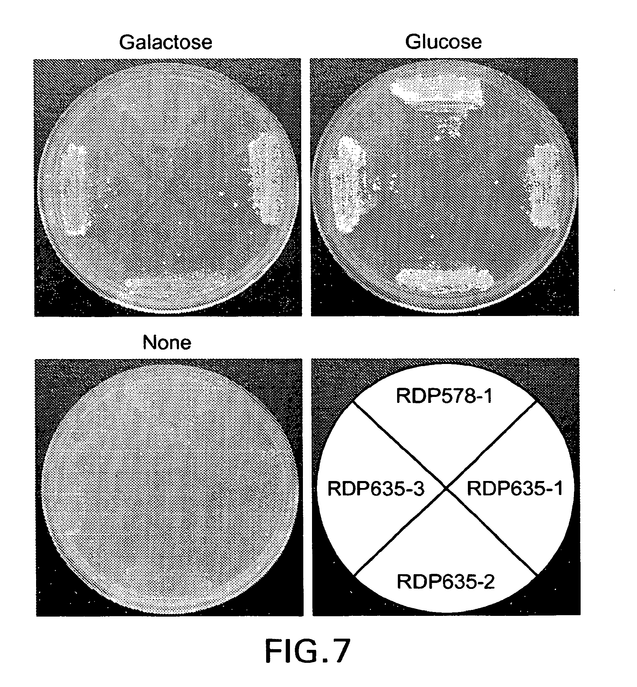

Figure 7 shows that glycoengineered *P. pastoris* strains expressing S. *cerevisiae GAL1, GAL2,* and *GAL7* genes can grow on galactose as a sole carbon source whereas the parent strain cannot. Glycoengineered strain YGLY578-1, which expresses the *ScGAL10* and hGalTIβ, was transformed with the plasmid pRCD977b, which contains expression cassettes encoding *ScGAL1, ScGAL7,* and *ScGAL2.* Strains were cultivated on defined medium containing yeast nitrogen base, biotin, and either 3% galactose or 2% glucose as a carbon source, or neither.

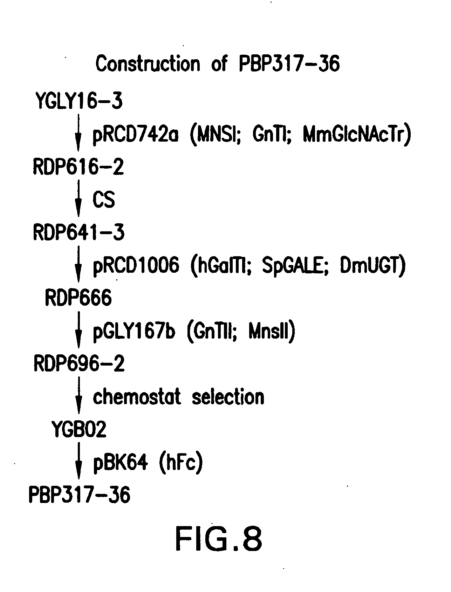

Figure 8 shows the construction of *P. pastoris* glycoengineered strain YGLY317-36. *P. pastoris* strain YGLY16-3 was generated by knock-out of five yeast glycosyltransferases. Subsequent knock-in of eight heterologous genes, yielded RDP696-2, a strain capable of transferring the human *N*-glycan Gal₂GlcNAc₂Man₃GlcNAc₂ to secreted proteins. Selection of robust clones via CSTR cultivation and introduction of a plasmid expressing secreted human Fc yielded strain YGLY317-36. CS, counterselect.

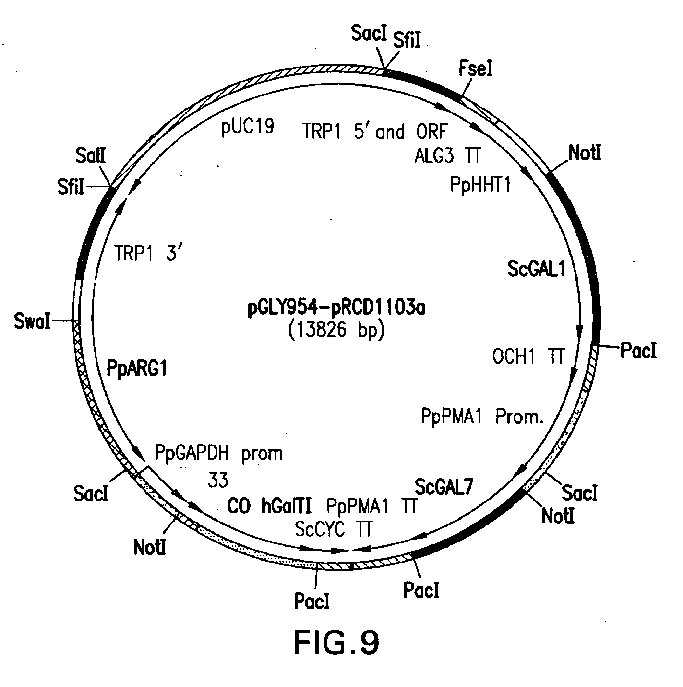

Figure 9 shows a feature diagram of plasmid pGLY954. This plasmid is a KINKO plasmid that integrates into the *P. pastoris TRP1* locus without deleting the gene. The plasmid contains expression cassettes encoding the full-length *S. cerevisiae* galactokinase *(ScGAL1)* and the full-length *S. cerevisiae* galactose-1-phosphate uridyl transferase *(ScGAL7)* under the control of the *PpHHT1* and *PpPMA1* promoters, respectively. The plasmid also contains an expression cassette encoding a secretory pathway targeted fusion protein (CO hGalTI) comprising the *ScMnt1* (*ScKre2*) leader peptide (33) fused to the *N*-terminus of the human Galactosyl Transferase I catalytic domain under the control of the *PpGAPDH* promoter. TT refers to transcription termination sequence.

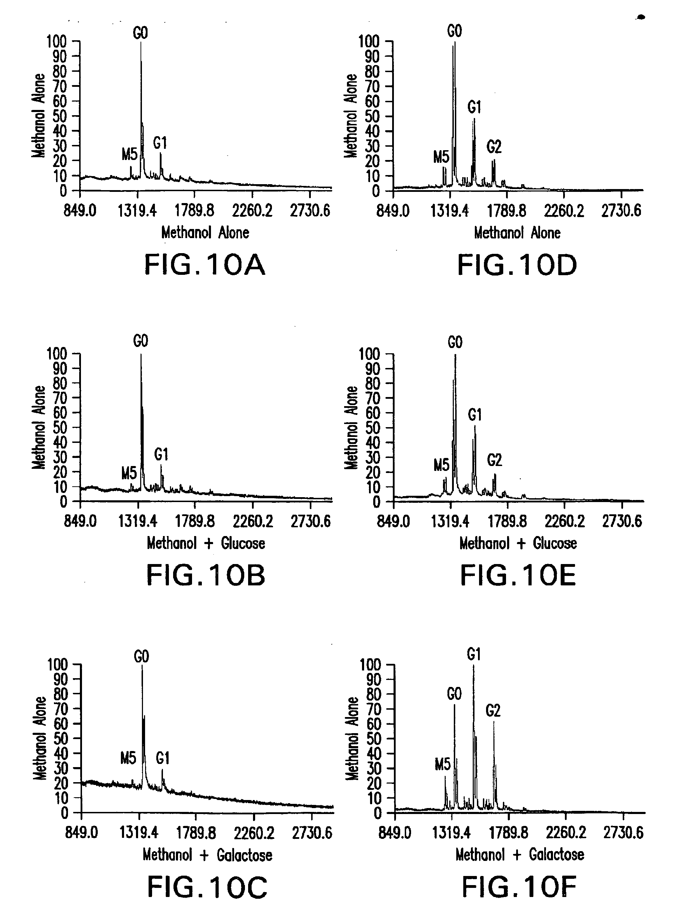

Figure 10 shows a MALDI-TOF MS analysis of the *N*-glycans on a human Fc fragment produced in strains PBP317-36 and RDP783 either induced in BMMY medium alone or in medium containing glucose or galactose. Strains were inoculated from a saturated seed culture to about one OD, cultivated in 800 mL of BMGY for 72 hours, then split and 100 mL aliquots of culture broths were centrifuged and induced for 24 hours in 25 mL of BMMY, 25 mL of BMMY + 0.5% glucose, or 25 mL of BMMY + 0.5% galactose. Protein A purified protein was subjected to Protein N-glycosidase F digestion and the released *N*-glycans analyzed by MALDI-TOF MS. Figures A-C, *N*-glycans on the human Fc produced in strain PBP317-36; Figures D-E, *N*-glycans on the human Fc produced in strain RDP783.

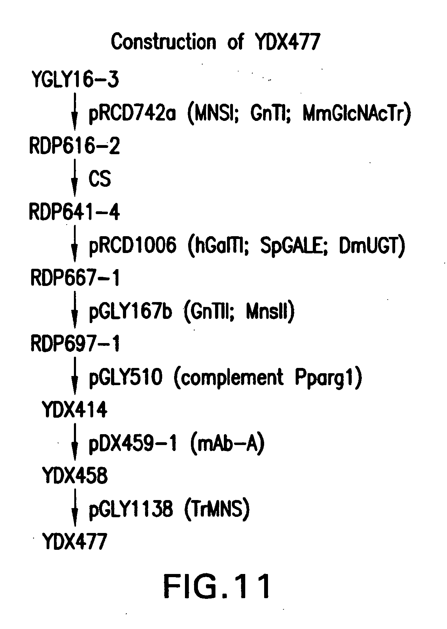

Figure 11 shows the construction of *P. pastoris* glycoengineered strain YDX477. *P. pastoris* strain YGLY16-3 *(Δoch1, Δpno1, Δbmt2, Δmnn4α, Δmnn4b)* was generated by knock-out of five yeast glycosyltransferases. Subsequent knock-in of eight heterologous genes, yielded RDP697-1, a strain capable of transferring the human *N*-glycan Gal₂GlcNAc₂Man₃GlcNAc₂ to secreted proteins. Introduction of a plasmid expressing a secreted antibody and a plasmid expressing a secreted form of *Trichoderma reesei* MNS1 yielded strain YDX477. CS, counterselect

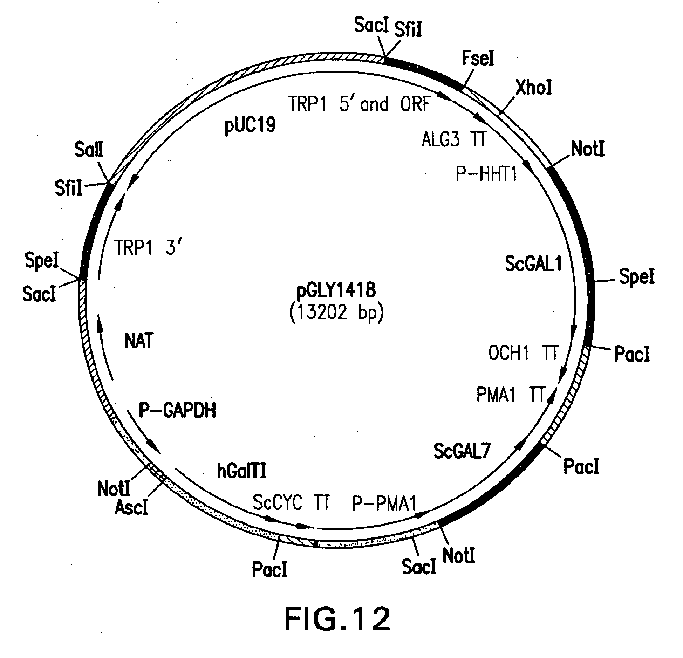

Figure 12 shows a feature diagram of plasmid pGLY1418. This plasmid is a KINKO plasmid that integrates into the *P. pastoris TRP1* locus without deleting the gene. The plasmid contains expression cassettes encoding the full-length *SeGAL1* and *ScGAL7* under the control of the *PpHHT1* and *PpPMA1* promoters, respectively. The plasmid also contains an expression cassette encoding a secretory pathway targeted fusion protein (hGalTI) comprising the *ScMnt1 (ScKre2)* leader peptide fused to the *N*-terminus of the human Galactosyl Transferase I catalytic domain under the control of the *PpGAPDH* promoter. TT refers to transcription termination sequence.

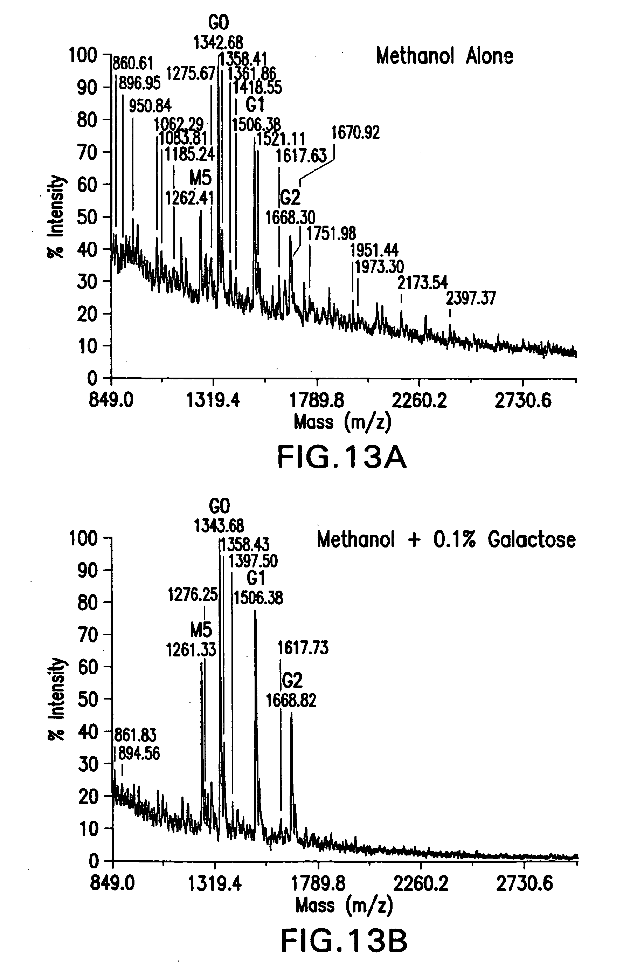

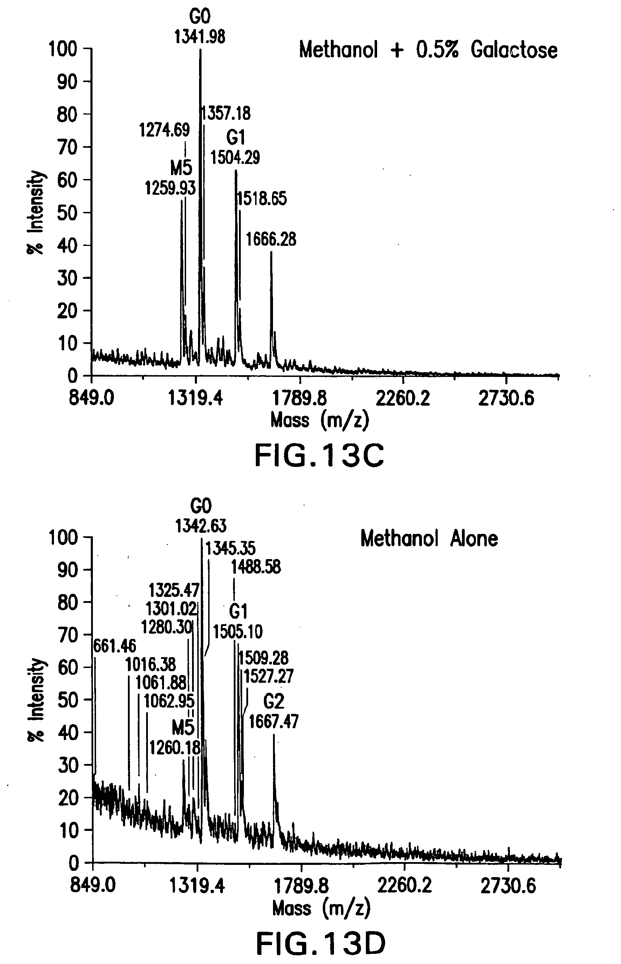

Figure 13A-F shows a MALDI-TOF MS analysis of *N*-glycans on an anti-Her2 antibody produced in strains YDX477 and RDP968-1 either induced in BMMY medium alone or in medium containing galactose. Strains were cultivated in 150 mL of BMGY for 72 hours, then split and 50 mL aliquots of culture broths were centrifuged and induced for 24 hours in 25 mL of BMMY, 25 mL of BMMY + 0.1% galactose, or 25 mL of BMMY + 0.5% galactose. Protein A purified protein was subjected to Protein *N*-glycosidase F digestion and the released *N*-glycan analyzed by MALDI-TOF MS. Figures 13A-C, *N*-glycans on the antibody produced in strain YDX477; Figures 13D-F, *N*-glycans on the antibody produced in strain RDP968-1.

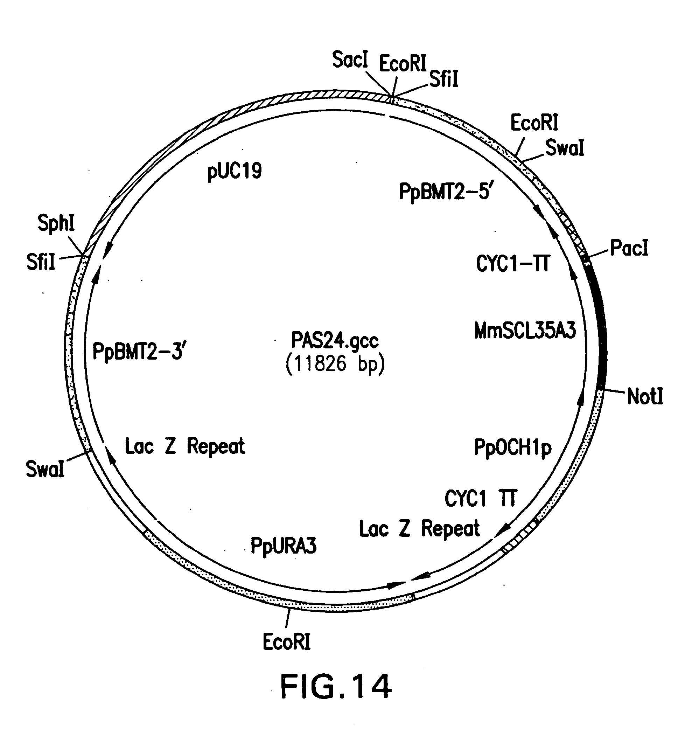

Figure 14 shows a feature diagram of plasmid pAS24. This plasmid is a *P*. *pastoris bmt2* knock-out plasmid that contains the *PpURA3* selectable marker and contains an expression cassette encoding the full length Mouse Golgi UDP-GlcNAc Transporter (MmSLC35A3) under control of the *PpOCH1* promoter. TT refers to transcription termination sequence.

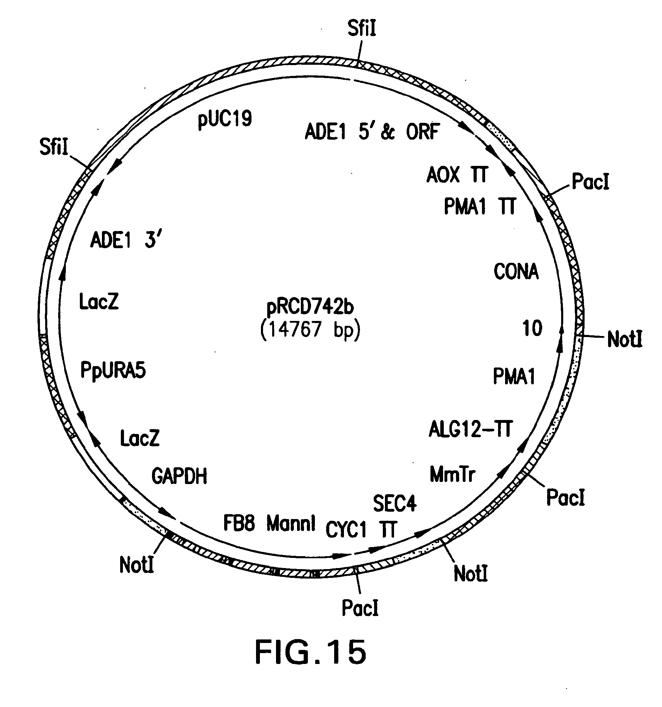

Figure 15 shows a feature diagram of plasmid pRCD742b. This plasmid is a KINKO plasmid that contains the *PpURA5* selectable marker as well as expression cassette encoding a secretory pathway targeted fusion protein (FB8 Mannl) comprising a *ScSec12* leader peptide fused to the *N*-terminus of a mouse Mannosidase I catalytic domain under control of the *PpGAPDH* promoter, an expression cassette encoding a secretory pathway targeted fusion protein (CONA10) comprising a *PpSec12* leader peptide fused to the *N*-terminus of a human GlcNAc Transferase I (GnT I) catalytic domain under control of the *PpPMA1* promoter, and a full length gene encoding the Mouse Golgi UDP-GlcNAc transporter (MmSLC35A3) under control of the *PpSEC4* promoter. TT refers to transcription termination sequence.

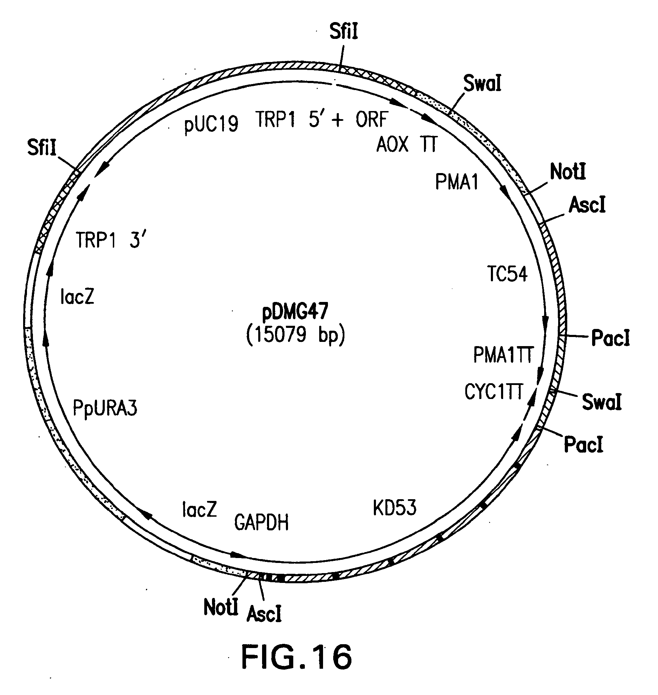

Figure 16 shows a feature diagram of Plasmid pDMG47. The plasmid comprises an expression cassette encoding a secretory pathway targeted fusion protein (KD53) comprising the *ScMnn2* leader peptide fused to the *N*-terminus of the catalytic domain of the *Drosophila melanogaster* Mannosidase II under control of the *PpGAPDH* promoter. The plasmid also contains an expression cassette encoding a secretory pathway targeted fusion protein (TC54) comprising the *ScMnn2* leader peptide fused to the *N*-terminus of the catalytic domain of the rat GlcNAc Transferase II (GnT II) under control of the *PpPMA1* promoter. TT refers to transcription termination sequence.

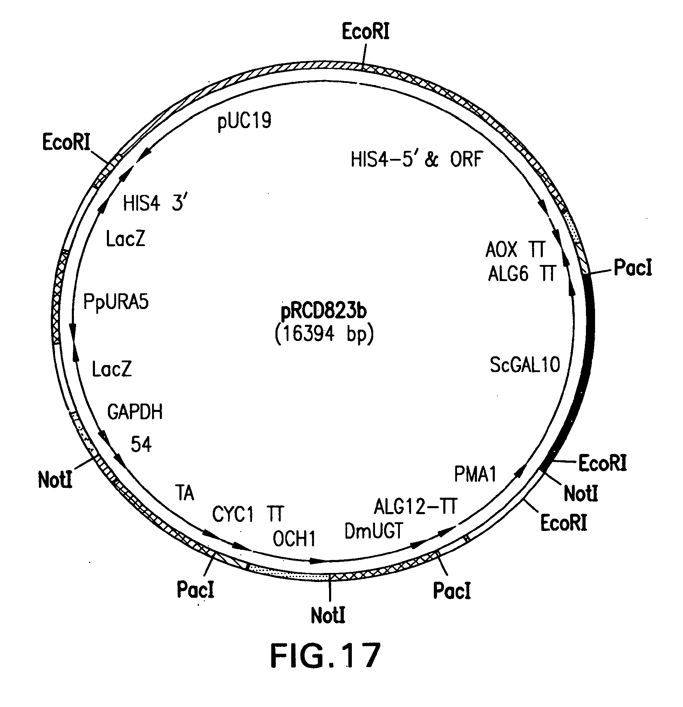

Figure 17 shows a feature diagram of plasmid pRCD823b. This plasmid is a KINKO plasmid that integrates into the *P. pastoris HIS4* locus without deleting the gene, and contains the *PpURA5* selectable marker. The plasmid comprises an expression cassette encoding a secretory pathway targeted fusion protein (TA54) comprising the *ScMnn2* leader peptide fused to the *N*-terminus of the rat GIcNAc Transferase II (GnT II) catalytic domain under the control of the *PpGAPDH* promoter and expression cassettes encoding the full-length *D. melanogaster* Golgi UDP-galactose transporter (*DmUGT*) and the *S. cerevisiae* UDP-galactose C4-epimerase (ScGAL10) under the control of the *PpOCH1* and *PpPMA1* promoters respectively. TT refers to transcription termination sequence.

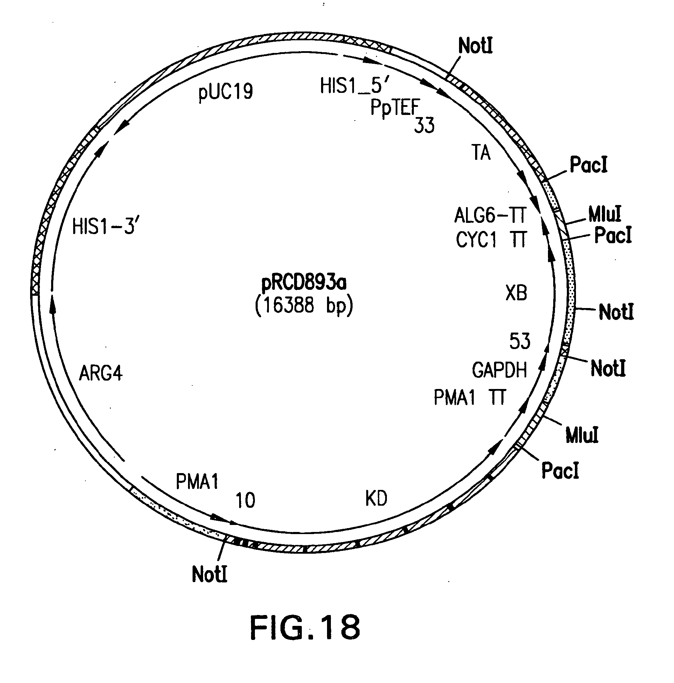

Figure 18 shows a feature diagram of plasmid pGLY893a. This plasmid is a *P*. *pastoris his1* knock-out plasmid that contains the *PpARG4* selectable marker. The plasmid contains an expression cassette encoding a secretory pathway targeted fusion protein (KD10) comprising the *PpSEC12* leader peptide fused to the *N*-terminus of the *Drosophila melanogaster* Mannosidase II catalytic domain under control of the *PpPAMA1* promoter, an expression cassette encoding a secretory pathway targeted fusion protein (TA33) comprising the *ScMnt1 (ScKre2)* leader peptide fused to the *N*-terminus of the rat GIcNAc Transferase II (GnT II) catalytic domain under the control of the *PpTEF* promoter, and an expression cassette encoding a secretory pathway targeted fusion protein comprising the *ScMnn2* leader peptide fused to the *N*-terminus of the human Galactosyl Transferase I catalytic domain under the control of the *PpGAPDH* promoter. TT refers to transcription termination sequence.

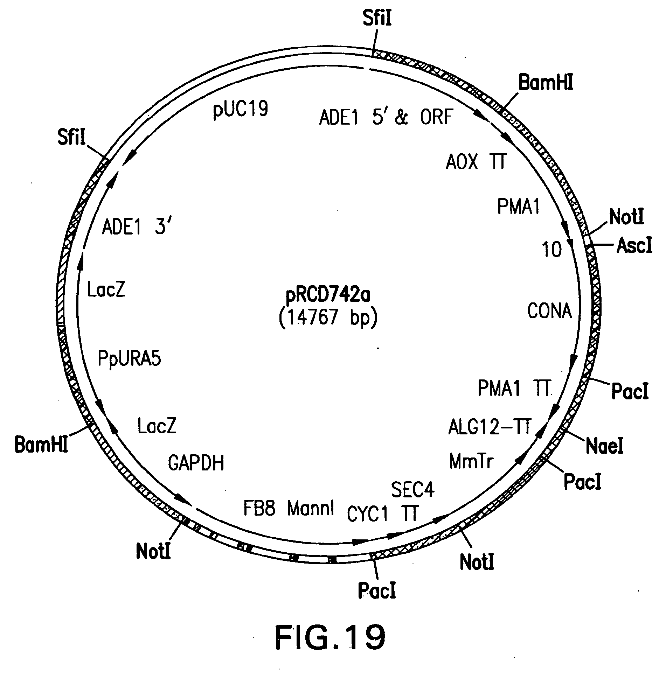

Figure 19 shows a feature diagram of plasmid pRCD742a. This plasmid is a KINKO plasmid that integrates into the *P. pastoris ADE1* locus without deleting the gene, and contains the *PpURA5* selectable marker. The plasmid contains an expression cassette encoding a secretory pathway targeted fusion protein (FB8 MannI) comprising the *ScSEC12* leader peptide fused to the N-terminus of the mouse Mannosidase I catalytic domain under the control of the *PpGAPDH* promoter, an expression cassette encoding a secretory pathway targeted fusion protein (CONA10) comprising the *PpSEC12* leader peptide fused to the N-terminus of the human GlcNAc Transferase I (GnT I) catalytic domain under the control of the *PpPMA1* promoter, and an expression cassette encoding the full length mouse Golgi UDP-GlcNAc transporter (MmSLC35A3) under the control of the *PpSEC4* promoter. TT refers to transcription termination sequence.

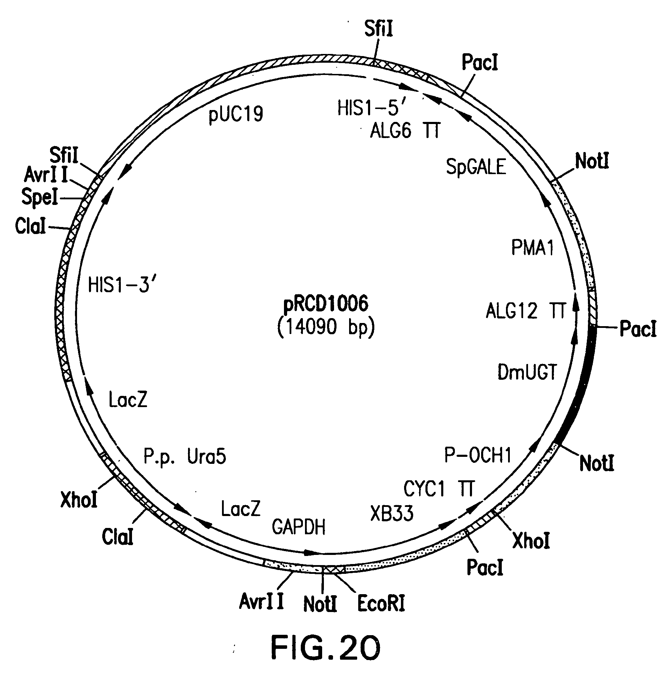

Figure 20 shows a feature diagram of plasmid pRCD1006. This plasmid is a *P*. *pastoris his1* knock-out plasmid that contains the *PpURA5* gene as a selectable marker. The plasmid contains an expression cassette encoding a secretory pathway targeted fusion protein (XB33) comprising the *ScMnt1 (ScKre2)* leader peptide fused to the *N*-terminus of the human Galactosyl Transferase I catalytic domain under the control of the *PpGAPDH* promoter and expression cassettes encoding the full-length *D. melanogaster* Golgi UDP-galactose transporter (*DmUGT*) and the *S. pombe* UDP-galactose C4-epimerase (*SpGALE*) under the control of the *PpOCH1* and *PpPMA1* promoters, respectively. TT refers to transcription termination sequence.

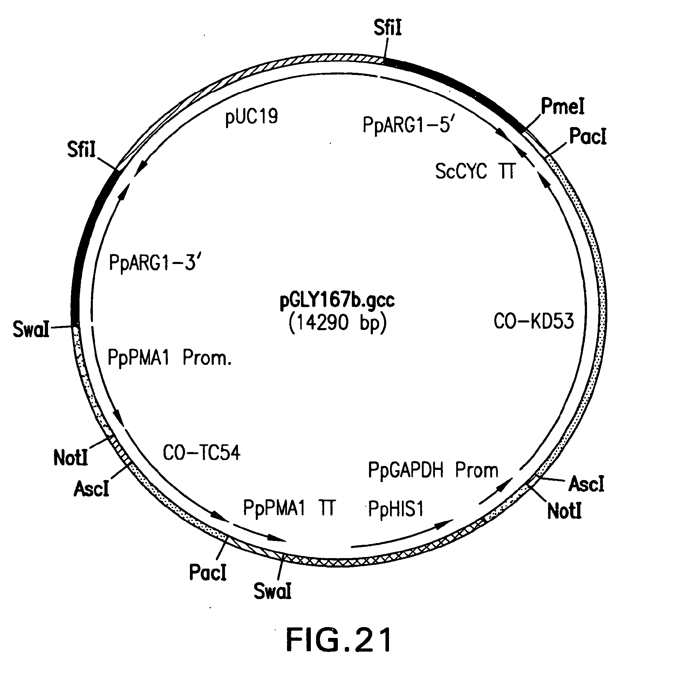

Figure 21 shows a feature diagram of plasmid pGLY167b. The plasmid is a *P*. *pastoris arg1* knock-out plasmid that contains the *PpURA3* selectable marker and contains an expression cassette encoding a secretory pathway targeted fusion protein (CO-KD53) comprising the ScMNN2 leader peptide fused to the *N*-terminus of the *Drosophila melanogaster* Mannosidase II catalytic domain under the control of the *PpGAPDH* promoter and an expression cassette encoding a secretory pathway targeted fusion protein (CO-TC54) comprising the *ScMnn2* leader peptide fused to the *N*-terminus of the rat GlcNAc Transferase II (GnT II) catalytic domain under the control of the *PpPM41* promoter. TT refers to transcription termination sequence.

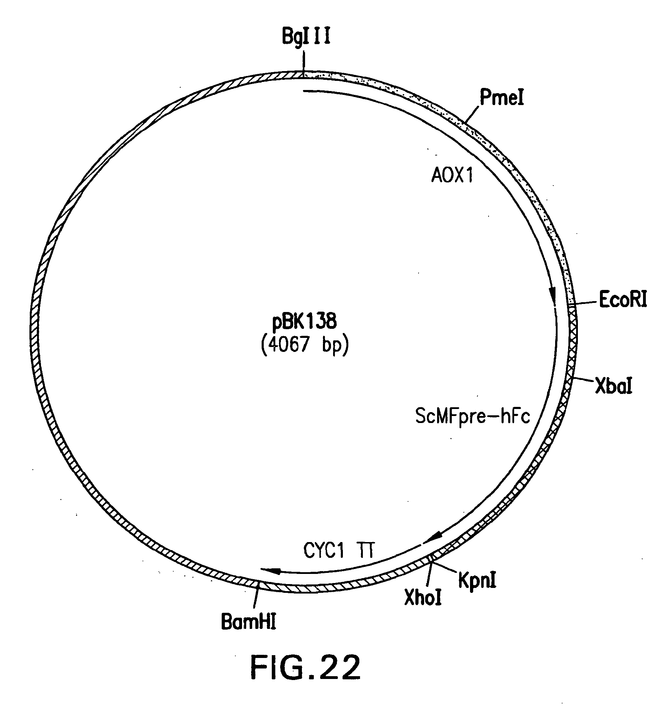

Figure 22 shows a feature diagram of plasmid pBK138. The plasmid is a roll-in plasmid that integrates into the *P. pastoris AOX1.* promoter while duplicating the promoter. The plasmid contains an expression cassette encoding a fusion protein comprising the S. *cerevisiae* Alpha Mating Factor pre-signal sequence fused to the *N*-terminus of the human Fc antibody fragment (C-terminal 233-aa of human IgG1 H chain). TT refers to transcription termination sequence.

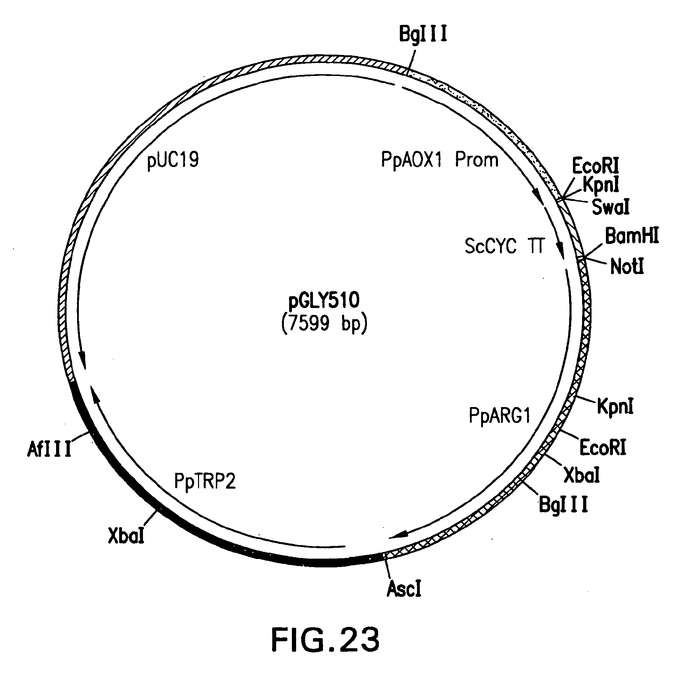

Figure 23 shows a feature diagram of plasmid pGLY510. The plasmid is a roll-in plasmid that integrates into the *P. pastoris TRP2* gene while duplicating the gene and contains an *AOX1* promoter-*ScCYC1* terminator expression cassette as well as the *PpARG1* selectable marker. TT refers to transcription termination sequence.

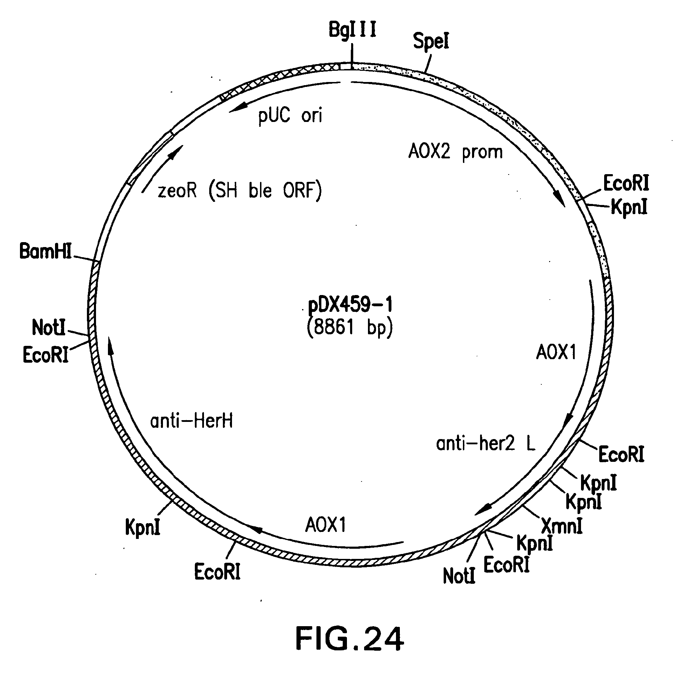

Figure 24 shows a feature diagram of plasmid pDX459-1. The plasmid is a roll-in plasmid that targets and integrates into the *P. pastoris AOX2* promoter and contains the Zeo^{R} while duplicating the promoter. The plasmid contains separate expression cassettes encoding an anti-HER2 antibody Heavy chain and an anti-HER2 antibody Light chain, each fused at the N-terminus to the *Aspergillus niger* alpha-amylase signal sequence and under the control of the P. *pastoris AOX1* promoter. TT refers to transcription termination sequence.

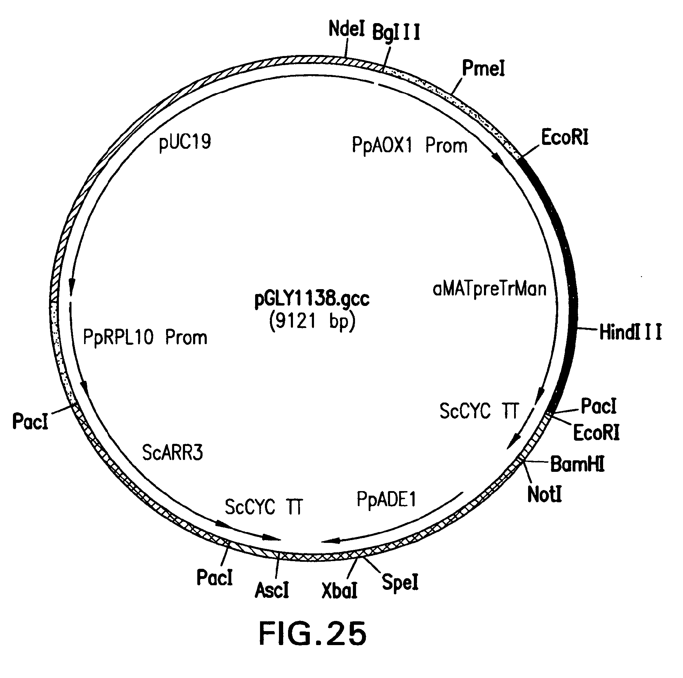

Figure 25 shows a feature diagram of plasmid pGLY1138. This plasmid is a roll-in plasmid that integrates into the *P. pastoris ADE1* locus while duplicating the gene and contains a *ScARR3* selectable marker gene cassette that confers arsenite resistance as well as an expression cassette encoding a secreted *Trichoderma reesei* MNS1 comprising the MNS1 catalytic domain fused at its *N*-terminus to the *S. cerevisiae* alpha factor pre signal sequence under the control of the *PpAOX1* promoter. TT refers to transcription termination sequence.

### DETAILED DESCRIPTION OF THE INVENTION

Yeast have been successfully used for the production of recombinant proteins, both intracellular and secreted *(See* for example, Cereghino Cregg FEMS Microbiology Reviews 24(1): 45-66 (2000); Harkki, et al. Bio-Technology 7(6): 596 (1989); Berka, et al. Abstr.Papers Amer. Chem.Soc.203: 121-BIOT (1992); Svetina, et al. J. Biotechnol. 76(2-3): 245-251 (2000)). Various yeasts, such as *K. lactis, Pichia pastoris, Pichia methanolica,* and *Hansenuta polymorpha,* have played particularly important roles as eukaryotic expression systems for producing recombinant proteins because they are able to grow to high cell densities and secrete large quantities of recombinant protein. However, glycoproteins expressed in any of these eukaryotic microorganisms differ substantially in N-glycan structure from those produced in mammals. This difference in glycosylation has prevented the use of yeast or filamentous fungi as hosts for the production of many therapeutic glycoproteins.

To enable the use of yeast to produce therapeutic glycoproteins, yeast have been genetically engineered to produce glycoproteins having hybrid or complex *N*-glycans. Recombinant yeast capable of producing compositions comprising particular hybrid or complex *N*-glycans have been disclosed in for example, U.S. Patent No. 7,029,872 and U.S. Patent No. 7,449,308. In addition, Hamilton et al., Science 313:1441-1443 (2006) and U.S. Published application No. 2006/0286637 reported the humanization of the glycosylation pathway in the yeast *Pichia pastoris* and the secretion of a recombinant human glycoprotein with complex *N-*glycosylation with terminal sialic acid. A precursor *N*-glycan for terminal sialic acid having the oligosaccharide structure Gal₂GlcNAc₂Man₃GlcNAc₂ (G2) is a structure that has also been found as the predominant *N*-glycan on several proteins isolated from human serum including, follicle stimulating hormone (FSH), asialotransferrin and, most notably, in differing amounts on human immunoglobulins (antibodies). Davidson et al. in U.S. Published Application No. 2006/0040353 teaches an efficient process for obtaining galactosylated glycoproteins using yeast cells that have been genetically engineered to produce galactose terminated *N*-glycans. These host cells are capable of producing glycoprotein compositions having various mixtures of G2 or G1 (GalGlcNAc₂Man₃GlcNAc₂) oligosaccharide structures with varying amount of G0 oligosaccharide structures. G0 is GlcNAc₂Man₃GlcNAc₂, which is a substrate for galactosyltransfierase. However, for certain glycoproteins, in particular antibodies and Fc fragments, it has been found that the efficiency of the galactose transfer process is less than optimal. In the case of antibodies and Fc fragments, it is believed that the location and accessibility of the glycosylation sites on the antibody or Fe fragment during intracellular processing inhibit efficient galactose transfer onto the *N*-glycan of the antibody or Fc fragment. Thus, the amount of oligosaccharide structures containing galactose is less that what has been observed for other glycoproteins such as the Kringle 3 protein. To overcome this problem, the present invention provides a means for increasing the amount of galactose transfer onto the *N-*glycan of the antibody or Fc fragment, thus increasing the amount of G1 and G2 containing antibodies or Fc fragments over G0 containing antibodies or Fc fragments.

*Pichia pastoris,* can use only a limited number of carbon sources for survival. Currently, these carbon sources are known to be glycerol, glucose, methanol, and perhaps rhamnose and mannose but not galactose. In many commercial production processes using *Pichia pastoris,* expression of recombinant proteins is under control of the AOX promoter, which is active in the presence ofmethanol but is repressed in the presence of glycerol. Thus, *Pichia pastoris* is usually grown in a medium containing glycerol or glycerol/methanol until the concentration of cells reaches a desired level at which time expression of the recombinant protein is by replaying the medium with medium containing only methanol as the carbon source. However, the cells are in a low energy state because methanol contains only one carbon which makes it a poor carbon source. Thus, it would be desirable to have a production process in which the *Pichia pastoris* could use a higher energy source such as galactose. The present invention solves this problem as well by providing genetically engineered *Pichia pastoris* that are able to use galactose as a sole carbon source.

Thus, the present invention has solved both of the above identified problems. The present invention provides recombinant Pichia pastoris cells, that have been glycoengineered to produce glycoproteins such as antibodies or Fc fragments in which the level of terminal galactose on the *N*-glycans thereon is increased compared to cells that have not been genetically engineered as taught herein. The genetically engineered host cells can be used in methods for making glycoproteins having *N*-glycans containing galactose wherein the amount of galactose in the *N*-glycan is higher than what would be obtainable in host cells that have not been genetically engineered as taught herein. The present invention also provides genetically engineered host cells wherein host cells that normally are incapable of using galactose as a sole carbon source have been genetically engineered as taught herein to be capable of using galactose as a sole carbon source. The methods herein for rendering host cells capable of using galactose as a sole carbon source used *Pichia pastoris* as a model. The methods herein can be used to render other yeast or fungal species that normally cannot use galactose as a carbon source capable of using galactose as a carbon source.

To solve both problems, genetically engineered host cells, which have been genetically engineered be capable of producing galactose-terminated *N*-glycans, are further genetically engineered to express the Leloir pathway enzymes: a galactokinase (EC 2.7.1.6), a UDP-galactose-4-epimerase (EC 5.1.3.2), and a galactose-1-phosphate uridyl transferase (EC 2.7.7.12). Optionally, the host cells can further express a galactose permease. This enables the host cells to use galactose as a carbon source and to produce a pool of UDP-galactose, which in turn serves as a substrate for galactose transferase involved in the synthesis of *N*-glycans that include galactose residues. Thus, the host cells and methods herein enable the production of glycoprotein compositions, in particular, antibody and Fc fragment compositions, wherein the proportion of galactose-terminated *N*-glycans is higher than which is obtainable in glycoengineered lower eukaryote cells. The recombinant host cells can produce recombinant glycoproteins such as antibodies and Fc fusion proteins in which the G0:G1/G2 ratio is less than 2:1, or a G0:G1/G2 ratio that is about 1:1 or less, or a G0:G1/G2 ratio that is about 1:2 or less.

The host cells and methods described herein are particularly useful for producing antibodies and Fc fragment containing fusion proteins that have *N*-glycans that are terminated with galactose residues. The *N*-glycan at Asn-297 of the heavy chain of antibodies or antibody fragments is important to the structure and function of an antibody. These functions include Fc gamma receptor binding, ability to activate complement, ability to activate cytotoxic T cells (ADCC), and serum stability. However, current antibody production in yeast or mammalian cells generally suffers from a lack of control over *N*-glycosylation, particularly that which occurs at Asn-297 of the constant or Fc region of the heavy chain, and particularly in the ability to control the level of terminal galactose on the *N*-glycans. In general, it has been found that while yeast cells that have been genetically engineered to produce glycoproteins that include galactose residues in the *N*-glycan can produce many glycoproteins with *N*-glycans that contain galactose efficiently, the ability of the cells to produce antibodies with *N*-glycans that contain galactose is not as efficient. As shown herein, the host cells and methods herein provide host cells that can produce antibodies in which a higher level of the antibodies have *N*-glycans containing galactose than in cells that are not genetically engineered as described herein.

While terminal galactose levels on *N*-glycans that do not have terminal galactose residues can be increased *in vitro* in a reaction that uses a soluble galactosyltransferase to add a charged galactose residue to the termini of the *N*-glycans, this *in vitro* process is expensive, cumbersome, and not easily scalable for production quantities of the glycoprotein. Thus, the host cells disclosed herein and which are capable of producing secreted glycoproteins, including antibodies or antibody fragments, with *N*-glycans having increased levels of terminal galactose in *vivo* provide a more desirable means for producing antibody compositions with increased levels of galactose-containing *N*-glycans. Thus, the present invention is a significant advancement in antibody production and provides for the first time, the ability to control particular antibody characteristics, e.g., level of galactose in the *N*-glycans, and in particular, the ability to produce recombinant glycoproteins with improved functional characteristics.

In addition, when the methods herein are used with host cells that have been genetically engineered to make glycoproteins that have sialylated *N*-glycans. Galactose terminated *N*-glycans are a substrate for sialyltransferase. Therefore, the amount of sialylated *N-*glycans is, in part, a function of the amount of galactose-terminated *N*-glycans available for sialylation. The host cells and methods herein provide a means for increasing the amount of galactose-terminated or -containing *N*-glycans, which when produced in a host cell that has been genetically engineered to make sialylated *N*-glycans, results in a host cell that makes an increased amount of sialylated *N*-glycans compared to the host cell not genetically engineered as taught herein.

While the present invention is useful for producing glycoproteins comprising galactose-terminated or -containing *N*-glycans, the present invention is also useful as a selection method for selecting a recombinant host cell that expresses a heterologous protein of any type, glycoprotein or not. Recombinant host cells that express one or two but not all of the Leloir pathway enzyme activities are transformed with one or more nucleic acid molecules encoding the heterologous protein and the Leloir pathway enzymes not present in the recombinant host cell. Since the transformed recombinant host cell contains a complete Leloir pathway, selection of the transformed recombinant host cell that expresses the heterologous protein from non-transforrned cells can be achieved by culturing the transformed recombinant host cells in a medium in which galactose is the sole carbon source. Thus, provided is a method for producing a recombinant host cell that expresses a heterologous protein, comprising the following steps. Providing a host cell that has been genetically engineered to express one or two Leloir pathway enzymes selected from the group consisting of galactokinase, UDP-galactose-4-epimerase, and galactose-1-phosphate uridyl transferase. In some embodiments, a host cell is capable of making glycoproteins that have human-like *N*-glycans, and in other embodiments, the host cell does not make glycoproteins that have human-like *N*-glycans because the heterologous protein that is to be expressed in the host cell does not have *N*-glycans. The host cell is transformed with one or more nucleic acid molecules encoding the heterologous protein and the Leloir pathway enzyme or enzymes not expressed in the provided recombinant host cell. The transformed host cell is cultured in a medium containing galactose as the sole carbon source to provide the recombinant host cell that expresses the heterologous protein. Optionally, the host cell can further include a nucleic acid molecule encoding a galactose permease. In particular embodiments, the host cells are genetically engineered to control *O*-glycosylation or grown under conditions that control *O-*glycosylation or both. In further embodiments, the host cells further have been modified to reduce phosphomannosyltransferase and/or beta-mannosyltransferase activity.

### Genetically Engineering Glycosylation Pathways in Lower Eukaryotes

*N*-glycosylation in most eukaryotes begins in the endoplasmic reticulum (ER) with the transfer of a lipid-linked Glc₃Man₉GlcNAc₂ oligosaccharide structure onto specific Asn residues of a nascent polypeptide (Lehle and Tanner, Biochim. Biophys. Acta 399: 364-74 (1975); Kornfeld and Kornfeld, Annu. Rev. Biochem 54: 631-64 (1985); Burda and Aebi, Biochim. Biophys. Acta-General Subjects 1426: 239-257 (1999)). Trimming of all three glucose moieties and a single specific mannose sugar from the *N*-linked oligosaccharide results in MangGlcNAc₂ *(See* Figure 1), which allows translocation of the glycoprotein to the Golgi apparatus where further oligosaccharide processing occurs (Herscovics, Biochim. Biophys. Acta 1426: 275-285 (1999); Moremen et al., Glycobiology 4: 113-125 (1994)). It is in the Golgi apparatus that mammalian *N*-glycan processing diverges from yeast and many other eukaryotes, including plants and insects. Mammals process *N*-glycans in a specific sequence of reactions involving the removal of three terminal α-1,2-mannose sugars from the oligosaccharide before adding GlcNAc to form the hybrid intermediate *N*-glycan GlcNAcMan₅GlcNAc₂ (Schachter, Glycoconj. J. 17: 465-483 (2000)) *(See* Figure 1). This hybrid structure is the substrate for mannosidase II, which removes the terminal α-1,3- and α-1,6-mannose sugars on the oligosaccharide to yield the *N*-glycan GlcNAcMan₃GlcNAc₂ (Moremen, Biochim. Biophys. Acta 1573(3): 225-235 (1994)). Finally, as shown in Figure 1, complex *N*-glycans are generated through the addition of at least one more GlcNAc residue followed by addition of galactose and sialic acid residues (Schachter, (2000), above), although sialic acid is often absent on certain human proteins, including IgGs (Keusch et al., Clin. Chim. Acta 252: 147-158 (1996); Creus et al., Clin. Endocrinol. (Oxf) 44: 181-189 (1996)).

In *Saccharomyces cerevisiae, N*-glycan processing involves the addition of mannose sugars to the oligosaccharide as it passes throughout the entire Golgi apparatus, sometimes leading to hypermannosylated glycans with over 100 mannose residues (Trimble and Verostek, Trends Glycosci. Glycotechnol. 7: 1-30 (1995); Dean, Biochim. Biophys. Acta-General Subjects 1426: 309-322 (1999)) *(See* Figure 1). Following the addition of the first α-1,6-mannose to MangGlcNAc₂ by α-1,6-mannosyltransferase (Och1p), additional mannosyltransferases extend the Man₉GlcNAc₂ glycan with α-1,2-, α-1,6-, and terminal α-1,3-linked mannose as well as marinosylphosphate. *Pichia pastoris* is a methylotrophic yeast frequently used for the expression of heterologous proteins, which has glycosylation machinery similar to that in *S. cerevisiae,* (Bretthauer and Castellino, Biotechnol. Appl. Biochem.30: 193-200 (1999); Cereghino and Cregg, Fems Microbiol. Rev.24: 45-66 (2000); Verostek and Trimble, Glycobiol. 5: 671-681 (1995)). However, consistent with the complexity of *N-*glycosylation, glycosylation in *P. pastoris* differs from that in *S. cerevisiae* in that it lacks the ability to add terminal α-1,3- linked mannose, but instead adds other mannose residues including phosphomannose and β-linked mannose (Miura et al., Gene 324: 129-137 (2004); Blanchard et al., Glycoconj. J. 24: 33-47 (2007); Mille et al., J. Biol. Chem. 283: 9724-9736 (2008)).

In previous work, we demonstrated that an *och1* mutant of *P. pastoris* lacked the ability to initiate yeast-type outer chain formation and, therefore, was not able to hypermannosylate *N*-glycans (Choi et al., Proc. Natl. Acad. Sci. USA 100: 5022-5027 (2003)). Furthermore, we identified a novel gene family encoding Golgi-residing enzymes responsible for β-mannose transfer and demonstrated that deletion of various members of this family reduces or eliminates immunogenic β-mannose transfer *(See* U.S. Published Application No. US2006/0211085). Subsequent introduction of five separate glycosylation enzymes yielded a strain that produced complex human *N*-glycans on a secreted model protein (*See* Figure 3A vs. Figure 3B)(Choi et al., Proc. Natl. Acad. Sci. USA 100: 5022-5027 (2003); Hamilton et al. Science 310: 1244-1246 (2003); U.S. Patent No. 7,029,872; U.S. Published Application No. 2004/0018590; U.S. Published Application No. 2004/023004; U.S. Published Application No. 2005/0208617; U.S. Published Application Number 2004/0171826; U.S. Published Application No. 2005/0208617; U.S. Published Application No. 2005/0170452; and U.S. Published Application No. 2006/0040353. More recently, the construction of recombinant yeast strains capable of producing fully sialylated *N*-glycans on a secreted protein produced by the yeast strain have been described in U.S. Published Application Nos. 2005/0260729 and 2006/0286637 and Hamilton et al., Science 313: 1441-1443 (2006).

The maturation of complex *N*-glycans involves the addition of galactose to terminal GlcNAc moieties, a reaction that can be catalyzed by several galactosyltransferases (GalTs). In humans, there are seven isoforms of GalTs (I-VII), at least four of which have been shown to transfer galactose to terminal GlcNAc in the presence of UDP-galactose *in vitro* (Guo, et al., Glycobiol. 11: 813-820 (2001)). The first enzyme identified, known as GalTI, is generally regarded as the primary enzyme acting on *N*-glycans, which is supported by *in vitro* experiments, mouse knock-out studies, and tissue distribution analysis (Berger and Rohrer, Biochimie 85: 261-74 (2003); Furukawa and Sato, Biochim. Biophys. Acta 1473: 54-66 (1999)). As shown herein, expression of human GalTI, when properly localized in the Golgi apparatus of the host cell, can transfer galactose onto complex *N*-glycans in a glycoengineered yeast strain capable of generating the terminal GlcNAc-containing precursor. Moreover, expression of a UDP-galactose 4-epimerase to generate a pool of UDP-galactose and a UDP-galactose transporter to move the substrate into the Golgi yields nearly quantitative transfer of β-1,4-galactose onto *N*-glycans in a strain capable of generating the terminal GlcNAc precursor. Iterative screening of a localization sub-library yielded improved generation of complex N-glycan structures over hybrid *N*-glycans structures presumably via separation of and reduced competition amongst GlcNAc and galactosyltransferases for intermediate *N*-glycan substrates.

Previously, human GalTI was shown to be active in transferring β-1,4-galactose to terminal GlcNAc in an elegant set of experiments that required first generating a mutant of the Alg1p enzyme that transfers the core or Pauci mannose to the growing *N*-glycan precursor molecule (Schwientek et al, J. of Biol. Chem 271: 3398-3405 (1996)). This mutation results in the partial transfer of a GlcNAc₂ truncated *N*-glycan to proteins, and yields a terminal GlcNAc. Following this, the authors show that human GalTI is capable of transferring galactose in a β-1,4-linkage to this artificial terminal GlcNAc structure. Importantly, it is shown that human GalTI can be expressed in active form in the Golgi of a yeast.

Based upon the above, the present invention was first tested with expression of human GalTI-leader peptide fusion proteins targeted to the Golgi apparatus of the host cell. After subsequent screening of human GalTII, GalTIII, GalTIV, GalTV, Bovine GalTI, and a pair of putative *C. elegans* GalTs, it was found that human GalTI appeared to be the most active enzyme in transferring galactose to complex biantennary *N*-glycans in this heterologous system. This may indicate that hGalTI is the most capable enzyme for transferring to this substrate (biantennary complex *N*-glycan) or it might simply be the most stable and active of the GalT enzymes tested or a combination of both. Interestingly, when GalT was localized to the Golgi apparatus using the same leader peptide used to localize the mannosidase II and GnT II catalytic domain-leader fusion proteins to the Golgi apparatus, a significant percentage of hybrid *N*-glycans structures (up to 20%) resulted in which a terminal galactose was on the α-1,3 arm. An increase in expression of the mannosidase II or GnT II activity did not significantly reduce this phenomenon. However, screening a library of yeast type II secretory pathway localization leader peptides (peptides that localize to a desired organelle of the secretory pathway such as the ER, Golgi or the trans Golgi network) yielded several active GalT-leader peptide fusion proteins that when transformed into the host cell resulted in significantly reduced levels of hybrid *N*-glycan structures and an increase in complex *N*-glycan structures. Previously, it has been reported that bisecting GlcNAc transfer is a stop signal for subsequent sugar transfer in the maturation of *N-*glycans in the Golgi, including preventing the transfer of fucose (Umana et al., Nature Biotechnol. 17: 176-180 (1999)). The data here suggests that transfer of galactose to the α-1,3 arm results in a similar stop signal preventing maturation of the α-1,6 arm by mannosidase II and GnT II.

The above results suggest that the ratio of galactose-terminated or -containing hybrid *N*-glycans to galactose-terminated or -containing complex *N*-glycans produced in a recombinant host cells is a product of where the GalTI is localized in the Golgi apparatus with respect to where the mannosidase II and GnT II are localized and that by manipulating where the three enzymes are localized, the ratio of hybrid *N*-glycans to complex *N*-glycans can be manipulated. To increase the yield of galactose-terminated or -containing hybrid *N*-glycans, all three enzyme activities should be targeted to the same region of the Golgi apparatus, for example, by using the same secretory pathway targeting leader peptide for targeting all three enzyme activities. Alternatively, a library of GalT-leader peptide fusion proteins is screened to identify a fusion protein that places the GalT activity in a position in the Golgi apparatus where it is more likely to act on the *N*-glycan substrate before the other two enzymes can act. This increases the yield of galactose-terminated or containing hybrid *N*-glycans compared to galactose-terminated or containing complex *N*-glycans. Conversely, to reduce the yield of galactose-terminated or -containing hybrid *N*-glycans, the GalT activity should be localized using a secretory pathway targeting leader peptide that is different from secretory pathway targeting leader peptide that is used with the other two enzyme activities. This can be achieved by screening GaIT-leader peptide fusion protein libraries to identify a GalT-leader peptide fusion protein combination that results in a host cell in which the yield of galactose-terminated or containing complex *N*-glycans is increased compared to the yield of galactose-terminated or containing hybrid *N*-glycans.

The results further underscore the value of screening libraries of both catalytic domains and secretory pathway localization leader peptides when expressing chimeric glycosylation enzymes in a heterologous host system, a concept previously illustrated by Choi et al., Proc. Natl. Acad. Sci. USA 100: 5022-5027 (2003) and described in U.S. Patent Nos. 7,029,872 and 7,449,308. With the availability of whole genome sequences, more sophisticated PCR and cloning techniques, and higher throughput analytical techniques like mass spectrometry, screening hundreds even thousands of combinations is possible. Importantly, this type of combinatorial screening has proven to be the difference between detecting enzyme activity and driving stepwise reactions, each dependent on the previous product to completion. Here, while in the case of UDP-galactose 4-epimerase several enzymes screened seemed to have relatively equal abilities to generate a pool of UDP-galactose, only one of the four UDP-galactose transporters tested proved active in this heterologous host including, surprisingly, one inactive transporter from a fellow yeast (*S. pombe*)*.* Furthermore, from a screen of dozens of secretory pathway localization leader peptides, many of which yielded active enzyme combinations, only three were found that yielded a high degree of uniform complex *N*-glycans (>85%) with biantennary terminal galactose (G2). Reduction of high mannose and hybrid intermediate structures has important consequences for the use of such a heterologous expression system in the production of therapeutic glycoproteins as high mannose structures have been shown to be potently immunogenic and recent evidence has suggested that liver toxicity can result from an overabundance of hybrid *N*-glycans.

Thus, U.S. Published application No. 2006/0286637 taught that to achieve galactose transfer in a host cell that does not normally produce glycoproteins that contain galactose, three conditions had to be overcome: (1) the absence of endogenous galactosyltransferase (GalT) in the Golgi, (2) the absence of endogenous UDP-Gal transport into the Golgi, and (3) a low endogenous cytosolic UDP-Gal pool. In the absence of a UDP-Gal transporter, the transfer of galactose to the terminal GlcNAc residue on the *N*-glycan was about 55-60%. In the absence of a UDP-glucose-4-epimerase, the transfer of galactose to the terminal GlcNAc residue on the *N*-glycan was about 10-15%.

All of the above modifications to produce host cells that can make galactosylated *N*-glycans had been made using a reporter protein (K3 domain of human plasminogen) with exposed *N*-glycans that are typically sialylated in humans and has been reported in U.S. Published Application No. 20060040353. However, in a manner that is comparable to mammalian cells, these glycoengineered yeast strain yielded only partial galactose transfer onto the Fc glycan of antibodies, resulting in a pool of *N*-glycans with less than 10% G2 and less than 25% G1 structures in favor of complex *N*-glycans with terminal GlcNAc. This is similar to mammalian cell lines, where antibody (IgG Fc Asn 297) *N*-glycans contain reduced amounts of terminal galactose.

The transfer of galactose residues onto *N*-glycans requires a pool of activated galactose (UDP-Gal). One way to generate such a pool above endogenous levels in a lower eukaryote is the expression of a UDP-galactose 4 epimerase as stated above and shown in U.S. Published Application No. 2006/0040353. Another way is the present invention, which includes the above cells, wherein the host cells are transformed with nucleic acid molecules encoding at least the following three Leloir pathway enzymes: galactokinase (EC 2.7.1.6), galactose-1-phosphate uridyl transferase EC 2.7.7.12), and UDP-galactose 4 epimerase (EC 5.1.3.2). Galactokinase is an enzyme that catalyzes the first step of galactose metabolism, namely the phosphorylation of galactose to galactose-1-phosphate. Galactose-1-phosphate uridyl transferase catalyzes the second step of galactose metabolism, which is the conversion of UDP-glucose and galactose-1-phosphate to UDP-galactose and glucose-1-phosphate. Optionally, further included can be a nucleic acid molecule encoding a plasma membrane galactose permease. Galactose permease is a plasma membrane hexose transporter, which imports galactose from an exogenous source. The Leloir pathway is shown in Figure 4.

As shown herein, when the genes encoding a galactokinase activity, a UDP-galactose-4-epimerase activity, a galactose-1-phosphate uridyl transferase activity and optionally a galactose permease activity into the above yeast along with feeding the yeast exogenous galactose are introduced into the cells and antibody expression is induced, the levels of terminal galactose on the Fc *N*-glycan of the antibody are substantially increased, thus increasing the amount of G2 N-glycans produced: *N*-glycans could be obtained on a human Fc wherein greater than 50% of the *N*-glycans were G2. The permease is optional because it was found that endogenous permeases in the cell were capable of importing galactose into the cell. Therefore, when used, the galactose permease can be any plasma membrane hexose transporter capable of transporting galactose across the cell membrane, for example, the *GAL2* galactose permease from *S. cerevisiae (See* GenBank: M81879). The galactokinase can be any enzyme that can catalyze the phosphorylation of galactose to galactose-1-phosphate, for example, the *GAL1* gene from *S. cerevisiae (See* GenBank: X76078). The galactose-1-phosphate uridyl transferase can be any enzyme that catalyzes the conversion of UDP-glucose and galactose-1-phosphate to UDP-galactose and glucose-1-phosphate, for example, the *GAL7* of *S. cerevisiae* (GenBank: *See* M12348). The UDP-galactose 4 epimerase can be any enzyme that catalyzes the conversion of UDP-glucose to UDP-galactose, for example the *GAL10* of S. *cerevisiae (See* GenBank NC_001134), *GALE (See* GenBank NC_003423) of *S*. *pombe,* and *hGALE* of human *(See* GenBank NM_000403). The epimerase can also be provided as a fusion protein in which the catalytic domain of the epimerase is fused to the catalytic domain of a galactosyltransferase *(See* U.S. Published Application No. US2006/0040353).

Introducing the above Leloir pathway enzymes into a *P. pastoris* strain produced a recombinant cell that was capable of assimilating environmental galactose. Further, when the above Leloir pathway enzymes are introduced into a cell capable of producing glycoproteins with *N*-glycans that contain galactose, the resulting recombinant cells were able to produce glycoproteins that had higher levels of β-1,4-galactose on complex *N*-glycans than glycoproteins produced in cells that had not been so engineered. Thus, the combination of these engineering steps has yielded host cells specifically glycoengineered and metabolically engineered for increased control over glycosylation of the *N*-glycans of glycoproteins such as antibodies and Fc-fusion proteins. Thus, these glycoengineered yeast cell lines enable efficient production of recombinant antibodies and Fc fusion proteins while allowing control over *N*-glycan processing.

### Expression vectors

In general, the galactokinase, UDP-galactose-4-epimerase, and galactoctose-1-phosphate uridyl transferase (and optionally galactose permease) are expressed as components of an expression cassette from an expression vector. In further aspects, further included in the host cell is an expression vector encoding a recombinant protein of interest, which in particular embodiments further includes a sequence that facilitates secretion of the recombinant protein from the host cell. For each Leloir pathway enzyme and recombinant protein of interest, the expression vector encoding it minimally contains a sequence, which affects expression of the nucleic acid sequence encoding the Leloir pathway enzyme or recombinant protein. This sequence is operably linked to a nucleic acid molecule encoding the Leloir pathway enzyme or recombinant protein. Such an expression vector can also contain additional elements like origins of replication, selectable markers, transcription or termination signals, centromeres, autonomous replication sequences, and the like.

Nucleic acid molecules encoding a recombinant protein of interest and the above Leloir pathway enzymes, respectively, can be placed within expression vectors to permit regulated expression of the overexpressed recombinant protein of interest and the above Leloir pathway enzymes. While the recombinant protein and the above Leloir pathway enzymes can be encoded in the same expression vector, the above Leloir pathway enzymes are preferably encoded in an expression vector which is separate from the vector encoding the recombinant protein. Placement of nucleic acid molecules encoding the above Leloir pathway enzymes and the recombinant protein in separate expression vectors can increase the amount of recombinant protein produced.

As used herein, an expression vector can be a replicable or a non-replicable expression vector. A replicable expression vector can replicate either independently of host cell chromosomal DNA or because such a vector has integrated into host cell chromosomal DNA. Upon integration into host cell chromosomal DNA such an expression vector can lose some structural elements but retains the nucleic acid molecule encoding the recombinant protein or the above Leloir pathway enzymes and a segment which can effect expression of the recombinant protein or the above Leloir pathway enzymes. Therefore, the expression vectors can be chromosomally integrating or chromosomally nonintegrating expression vectors.

Following introduction of nucleic acid molecules encoding the above Leloir pathway enzymes and the recombinant protein, the recombinant protein is then overexpressed by inducing expression of the nucleic acid encoding the recombinant protein. In another embodiment, cell lines are established which constitutively or inducibly express the above Leloir pathway enzymes. An expression vector encoding the recombinant protein to be overexpressed is introduced into such cell lines to achieve increased production of the recombinant protein. In particular embodiments, the nucleic acid molecules encoding the Leloir pathway enzymes are operably linked to constitutive promoters.

The present expression vectors can be replicable in one host cell type, e.g., *Escherichia coli,* and undergo little or no replication in another host cell type, e.g., a eukaryotic host cell, so long as an expression vector permits expression of the above Leloir pathway enzymes or overexpressed recombinant protein and thereby facilitates secretion of such recombinant proteins in a selected host cell type.

expression vectors as described herein include DNA or RNA molecules engineered for controlled expression of a desired gene, that is, genes encoding the above Leloir pathway enzymes or recombinant protein. Such vectors also encode nucleic acid molecule segments which are operably linked to nucleic acid molecules encoding the present above Leloir pathway enzymes or recombinant protein. Operably linked in this context means that such segments can effect expression of nucleic acid molecules encoding above Leloir pathway enzymes or recombinant protein. These nucleic acid sequences include promoters, enhancers, upstream control elements, transcription factors or repressor binding sites, termination signals and other elements which can control gene expression in the contemplated host cell. Preferably the vectors are vectors, bacteriophages, cosmids, or viruses.

Expression vectors function in yeast or mammalian cells. Yeast vectors can include the yeast 2µ circle and derivatives thereof, yeast vectors encoding yeast autonomous replication sequences, yeast minichromosomes, any yeast integrating vector and the like. A comprehensive listing of many types of yeast vectors is provided in Parent et al. (Yeast 1: 83-138 (1985)).

Elements or nucleic acid sequences capable of effecting expression of a gene product include promoters, enhancer elements, upstream activating sequences, transcription termination signals and polyadenylation sites. All such promoter and transcriptional regulatory elements, singly or in combination, are contemplated for use in the present expression vectors. Moreover, genetically-engineered and mutated regulatory sequences are also contemplated herein.

Promoters are DNA sequence elements for controlling gene expression. In particular, promoters specify transcription initiation sites and can include a TATA box and upstream promoter elements. The promoters selected are those which would be expected to be operable in the particular host system selected. For example, yeast promoters are used in the present expression vectors when a yeast host cell such as *Saccharomyces cerevisiae, Kluyveromyces lactis,* or *Pichia pastoris* is used whereas fungal promoters would be used in host cells such as *Aspergillus niger, Neurospora crassa,* or *Tricoderma reesei.* Examples of yeast promoters include but are not limited to the *GAPDH, AOX1, SEC4, HH1, PMA1, OCH1, GAL1, PGK, GAP, TP1, CYC1, ADH2, PHO5, CUP1, MFα1, FLDI, PMA1,* PDI, *TEF,* and *GUT1* promoters. Romanos et al. (Yeast 8: 423-488 (1992)) provide a review of yeast promoters and expression vectors. Hartner et al., Nucl. Acid Res. 36: e76 (pub on-line 6 June 2008) describes a library of promoters for fine-tuned expression of heterologous proteins in *Pichia pastoris.*

The promoters that are operably linked to the nucleic acid molecules disclosed herein can be constitutive promoters or inducible promoters. Inducible promoters, that is promoters which direct transcription at an increased or decreased rate upon binding of a transcription factor. Transcription factors as used herein include any factor that can bind to a regulatory or control region of a promoter and thereby affect transcription. The synthesis or the promoter binding ability of a transcription factor within the host cell can be controlled by exposing the host to an inducer or removing an inducer from the host cell medium. Accordingly, to regulate expression of an inducible promoter, an inducer is added or removed from the growth medium of the host cell. Such inducers can include sugars, phosphate, alcohol, metal ions, hormones, heat, cold and the like. For example, commonly used inducers in yeast are glucose, galactose, and the like.

Transcription termination sequences that are selected are those that are operable in the particular host cell selected. For example, yeast transcription termination sequences are used in the present expression vectors when a yeast host cell such as *Saccharomyces cerevisiae, Kluyveromyces lactis,* or *Pichia pastoris* is used whereas fungal transcription termination sequences would be used in host cells such as *Aspergillus niger, Neurospora crassa,* or *Tricoderma reesei.* Transcription termination sequences include but are not limited to the *Saccharomyces cerevisiae CYC* transcription termination sequence (ScCYC TT), the *Pichia pastoris ALG3* transcription termination sequence (ALG3 TT), the *Pichia pastoris ALG6* transcription termination sequence (ALG6 TT), the *Pichia pastoris ALG12* transcription termination sequence (ALG12 TT), the *Pichia pastoris AOX1* transcription termination sequence (AOX1 TT), the *Pichiapastoris OCH1* transcription termination sequence (OCH1 TT) and *Pichia pastoris PMA1* transcription termination sequence (PMA1 TT).

The expression vectors can also encode selectable markers. Selectable markers are genetic functions that confer an identifiable trait upon a host cell so that cells transformed with a vector carrying the selectable marker can be distinguished from non-transformed cells. Inclusion of a selectable marker into a vector can also be used to ensure that genetic functions linked to the marker are retained in the host cell population. Such selectable markers can confer any easily identified dominant trait, e.g. drug resistance, the ability to synthesize or metabolize cellular nutrients and the like.

Yeast selectable markers include drug resistance markers and genetic functions which allow the yeast host cell to synthesize essential cellular nutrients, e.g. amino acids. Drug resistance markers which are commonly used in yeast include chloramphenicol, kanamycin, methotrexate, G418 (geneticin), Zeocin, and the like. Genetic functions which allow the yeast host cell to synthesize essential cellular nutrients are used with available yeast strains having auxotrophic mutations in the corresponding genomic function. Common yeast selectable markers provide genetic functions for synthesizing leucine (*LEU2*)*,* tryptophan (*TRP1* and *TRP2),* uracil (*URA3, URA5, URA6*)*,* histidine (*HIS3*)*,* lysine (*LYS2*)*,* adenine (*ADE1* or *ADE2*)*,* and the like. Other yeast selectable markers include the *ARR3* gene from *S. cerevisiae,* which confers arsenite resistance to yeast cells that are grown in the presence of arsenite (Bobrowicz et al., Yeast, 13:819-828 (1997); Wysocki et al., J. Biol. Chem. 272:30061-30066 (1997)). A number of suitable integration sites include those enumerated in U.S. Published application No. 2007/0072262 and include homologs to loci known for *Saccharomyces cerevisiae* and other yeast or fungi: Methods for integrating vectors into yeast are well known, for example, see WO2007136865.

Therefore the present expression vectors can encode selectable markers which are useful for identifying and maintaining vector-containing host cells within a cell population present in culture. In some circumstances selectable markers can also be used to amplify the copy number of the expression vector. After inducing transcription from the present expression vectors to produce an RNA encoding an overexpressed recombinant protein or Leloir pathway enzymes, the RNA is translated by cellular factors to produce the recombinant protein or Leloir pathway enzymes.

In yeast and other eukaryotes, translation of a messenger RNA (mRNA) is initiated by ribosomal binding to the 5' cap of the mRNA and migration of the ribosome along the mRNA to the first AUG start codon where polypeptide synthesis can begin. Expression in yeast and mammalian cells generally does not require specific number of nucleotides between a ribosomal-binding site and an initiation codon, as is sometimes required in prokaryotic expression systems. However, for expression in a yeast or a mammalian host cell, the first AUG codon in an mRNA is preferably the desired translational start codon.

Moreover, when expression is performed in a yeast host cell the presence of long untranslated leader sequences, e.g. longer than 50-100 nucleotides, can diminish translation of an mRNA. Yeast mRNA leader sequences have an average length of about 50 nucleotides, are rich in adenine, have little secondary structure and almost always use the first AUG for initiation. Since leader sequences which do not have these characteristics can decrease the efficiency of protein translation, yeast leader sequences are preferably used for expression of an overexpressed gene product or a chaperone protein in a yeast host cell. The sequences of many yeast leader sequences are known and are available to the skilled artisan, for example, by reference to Cigan et al. (Gene 59: 1-18 (1987)).

In addition to the promoter, the ribosomal-binding site and the position of the start codon, factors which can affect the level of expression obtained include the copy number of a replicable expression vector. The copy number of a vector is generally determined by the vector's origin of replication and any cis-acting control elements associated therewith. For example, an increase in copy number of a yeast episomal vector encoding a regulated centromere can be achieved by inducing transcription from a promoter which is closely juxtaposed to the centromere. Moreover, encoding the yeast FLP function in a yeast vector can also increase the copy number of the vector.

One skilled in the art can also readily design and make expression vectors which include the above-described sequences by combining DNA fragments from available vectors, by synthesizing nucleic acid molecules encoding such regulatory elements or by cloning and placing new regulatory elements into the present vectors. Methods for making expression vectors are well-known. Overexpressed DNA methods are found in any of the myriad of standard laboratory manuals on genetic engineering.

The expression vectors can be made by ligating the coding regions for the above Leloir pathway enzymes and recombinant protein in the proper orientation to the promoter and other sequence elements being used to control gene expression. After construction of the present expression vectors, such vectors are transformed into host cells where the overexpressed recombinant protein and the Leloir pathway enzymes can be expressed. Methods for transforming yeast and other lower eukaryotic cells with expression vectors are well known and readily available to the skilled artisan. For example, expression vectors can be transformed into yeast cells by any of several procedures including lithium acetate, spheroplast, electroporation, and similar procedures.

### Host cells

Yeast such as *Pichia pastoris, Pichia methanolica,* and *Hansenula polymorpha* are useful for cell culture because they are able to grow to high cell densities and secrete large quantities of recombinant protein. Likewise, filamentous fungi, such as *Aspergillus niger, Fusarium sp, Neurospora crassa* and others can be used to produce glycoproteins of the invention at an industrial scale. In general, lower eukaryotes useful for practicing the methods herein include yeast and fungi that cannot normally use galactose as a carbon source. Examples of yeast that cannot use galactose as a carbon source include but are not limited to methylotrophic yeast of the *Pichia* genus, e.g., *Pichia pastoris,* and yeast such as S. *kudriavzevii, C. glabrata, K waltii,* and *E. gossypii.* Yeast are useful for expression of glycoproteins because they can be economically cultured, give high yields, and when appropriately modified are capable of suitable glycosylation. Yeast particularly offers established genetics allowing for rapid transformations, tested protein localization strategies and facile gene knock-out techniques. Suitable vectors have expression control sequences, such as promoters, including 3-phosphoglycerate kinase or other glycolytic enzymes, and an origin of replication, termination sequences and the like as desired. Thus, the above host cells, which cannot normally use galactose as a carbon source, are genetically engineered to express a galactokinase activity, a UDP-galactose-4-epimerase activity, a galactoctose-1-phosphate uridyl transferase activity and optionally a galactose permease activity, which renders the host cells capable of using galactose as a carbon source.

Lower eukaryotes, particularly yeast, can also be genetically modified so that they express glycoproteins in which the glycosylation pattern is human-like or humanized. Such can be achieved by eliminating selected endogenous glycosylation enzymes and/or supplying exogenous enzymes as described by Gerngross et al., U.S. Published Application No. 2004/0018590. For example, a host cell can be selected or engineered to be depleted in 1,6-mannosyl transferase activities, which would otherwise add mannose residues onto the *N*-glycan on a glycoprotein.

In one embodiment, the host cells genetically engineered to assimilate environmental galactose as a carbon source as described herein is also genetically engineered to make complex *N*-glycans as described below. Such host cells further includes an α1,2-mannosidase catalytic domain fused to a cellular targeting signal peptide not normally associated with the catalytic domain and selected to target the α1,2-mannosidase activity to the ER or Golgi apparatus of the host cell. Passage of a recombinant glycoprotein through the ER or Golgi apparatus of the host cell produces a recombinant glycoprotein comprising a Man₅GlcNAc₂ glycoform, for example, a recombinant glycoprotein composition comprising predominantly a Man₅GlcNAc₂ glycoform. For example, U.S. Patent No, 7,029,872 and U.S. Published Patent Application Nos. 2004/0018590 and 2005/0170452 disclose lower eukaryote host cells capable of producing a glycoprotein comprising a Man₅GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source.

The immediately preceding host cell further includes a GlcNAc transferase I (GnT I) catalytic domain fused to a cellular targeting signal peptide not normally associated with the catalytic domain and selected to target the GIcNAc transferase I activity to the ER or Golgi apparatus of the host cell. Passage of the recombinant glycoprotein through the ER or Golgi apparatus of the host cell produces a recombinant glycoprotein comprising a GlcNAcMan₅GleNAc₂ glycoform, for example a recombinant glycoprotein composition comprising predominantly a GlcNAcMan₅GlcNAc₂ glycoform. U.S. Patent No, 7,029,872 and U.S. Published Patent Application Nos. 2004/0018590 and 2005/0170452 disclose lower eukaryote host cells capable of producing a glycoprotein comprising a GlcNAcMan₅GlcNAc₂ glycoform. The glycoprotein produced in the above cells can be treated *in vitro* with a hexaminidase to produce a recombinant glycoprotein comprising a Man₅GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source.

Then, the immediately preceding host cell further includes a mannosidase II catalytic domain fused to a cellular targeting signal peptide not normally associated with the catalytic domain and selected to target mannosidase II activity to the ER or Golgi apparatus of the host cell. Passage of the recombinant glycoprotein through the ER or Golgi apparatus of the host cell produces a recombinant glycoprotein comprising a GlcNAcMan₃GlcNAc₂ glycoform, for example a recombinant glycoprotein composition comprising predominantly a GlcNAcMan₃GlcNAc₂ glycoform. U.S. Patent No, 7,029,872 and U.S. Published Patent Application No. 2004/0230042 discloses lower eukaryote host cells that express mannosidase II enzymes and are capable of producing glycoproteins having predominantly a GlcNAc₂Man₃GlcNAc₂ glycoform. The glycoprotein produced in the above cells can be treated *in vitro* with a hexaminidase to produce a recombinant glycoprotein comprising a Man₃GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source.

Then, the immediately preceding host cell further includes GlcNAc transferase II (GnT II) catalytic domain fused to a cellular targeting signal peptide not normally associated with the catalytic domain and selected to target the GlcNAc transferase II activity to the ER or Golgi apparatus of the host cell. Passage of the recombinant glycoprotein through the ER or Golgi apparatus of the host cell produces a recombinant glycoprotein comprising a GlcNAc₂Man₃GlcNAc₂ (G0) glycoform, for example a recombinant glycoprotein composition comprising predominantly a GlcNAc₂Man₃GlcNAc₂ glycofonn. U.S. Patent No, 7,029,872 and U.S. Published Patent Application Nos. 2004/0018590 and 2005/0170452 disclose lower eukaryote host cells capable of producing a glycoprotein comprising a GlcNAc₂Man₃GlcNAc₂ glycoform. The glycoprotein produced in the above cells can be treated *in vitro* with a hexaminidase to produce a recombinant glycoprotein comprising a Man₃GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source.

Finally, the immediately preceding host cell further includes a galactosyltransferase catalytic domain fused to a cellular targeting signal peptide not normally associated with the catalytic domain and selected to target galactosyltransferase activity to the ER or Golgi apparatus of the host cell: Passage of the recombinant glycoprotein through the ER or Golgi apparatus of the host cell produces a recombinant glycoprotein comprising a GalGlcNAc₂Man₃GlcNAc₂ (G1) or Gal₂GlcNAc₂Man₃GlcNAc₂ (G2) glycoform, or mixture thereof for example a recombinant glycoprotein composition comprising predominantly a GalGlcNAc₂Man₃GlcNAc₂ (G1) glycoform or Gal₂GlcNAc₂Man₃GlcNAc₂ (G2) glycoform or mixture thereof. U.S. Patent No, 7,029,872 and U.S. Published Patent Application No. 2006/0040353 discloses lower eukaryote host cells capable of producing a glycoprotein comprising a Gal₂GlcNAc₂Man₃GlcNAc₂ glycoform. The glycoprotein produced in the above cells can be treated *in vitro* with a galactosidase to produce a recombinant glycoprotein comprising a GlcNAc₂Man₃GlcNAc₂ glycoform, for example a recombinant glycoprotein composition comprising predominantly a GlcNAc₂Man₃GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source and are capable of producing glycoproteins wherein the proportion of *N*-glycans containing galactose is greater than in host cells that have not been genetically engineered to include the above-mention Leloir pathway enzymes.

In a further embodiment, the immediately preceding host cell, which is capable of making complex *N*-glycans terminated with galactose and which is capable of assimilating galactose as a carbon source as disclosed herein, can further include a sialyltransferase catalytic domain fused to a cellular targeting signal peptide not normally associated with the catalytic domain and selected to target sialytransferase activity to the ER or Golgi apparatus of the host cell. Passage of the recombinant glycoprotein through the ER or Golgi apparatus of the host cell produces a recombinant glycoprotein comprising predominantly a NANA₂Gal₂GlcNAc₂Man₃GlcNAc₂ glycoform or NANAGal₂GlcNAc₂Man₃GlcNAc₂ glycoform or mixture thereof. For lower eukaryote host cells such as yeast and filamentous fungi, it is useful that the host cell further include a means for providing CMP-sialic acid for transfer to the N-glycan. U.S. Published Patent Application No. 2005/0260729 discloses a method for genetically engineering lower eukaryotes to have a CMP-sialic acid synthesis pathway and U.S. Published Patent Application No. 2006/0286637 discloses a method for genetically engineering lower eukaryotes to produce sialylated glycoproteins. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source and are capable of producing glycoproteins wherein the proportion of *N*-glycans containing galactose is greater than in host cells that have not been genetically engineered to include the above-mention Leloir pathway enzymes.

In another embodiment, the host cell that produces glycoproteins that have predominantly GlcNAcMansGlcNAc₂ *N*-glycans further includes a galactosyltransferase catalytic domain fused to a cellular targeting signal peptide not normally associated with the catalytic domain and selected to target Galactosyltransferase activity to the ER or Golgi apparatus of the host cell. Passage of the recombinant glycoprotein through the ER or Golgi apparatus of the host cell produces a recombinant glycoprotein comprising predominantly the GalGlcNAcMan₅GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source.

In a further embodiment, the immediately preceding host cell, which is capable of making hybrid *N*-glycans terminated with galactose and which is capable of assimilating galactose as a carbon source as disclosed herein, can further include a sialyltransferase catalytic domain fused to a cellular targeting signal peptide not normally associated with the catalytic domain and selected to target sialytransferase activity to the ER or Golgi apparatus of the host cell. Passage of the recombinant glycoprotein through the ER or Golgi apparatus of the host cell produces a recombinant glycoprotein comprising a NANAGalGlcNAcMan₅GlcNAc₂ glycoform. These host cells when further genetically engineered to express a galactokinase activity, a UDP-galactose-4-epimerase activity, a galactoctose-1-phosphate uridyl transferase activity and optionally a galactose permease activity as taught herein are capable of using galactose as a carbon source.

Various of the preceding host cells further include one or more sugar transporters such as UDP-GlcNAc transporters (for example, *Kluyveromyces lactis* and *Mus musculus* UDP-GlcNAc transporters), UDP-galactose transporters (for example, *Drosophila melanogaster* UDP-galactose transporter), and CMP-sialic acid transporter (for example, human sialic acid transporter). Because lower eukaryote host cells such as yeast and filamentous fungi lack the above transporters, it is preferable that lower eukaryote host cells such as yeast and filamentous fungi be genetically engineered to include the above transporters.

Host cells further include the cells that are genetically engineered to eliminate glycoproteins having α-mannosidase-resistant *N*-glycans by deleting or disrupting one or more of the β-mannosyltransferase genes (e.g., *BMT1, BMT2, BMT3,* and *BMT4*)(*See,* U.S. Published Patent Application No. 2006/0211085) and glycoproteins having phosphomannose residues by deleting or disrupting one or both of the phosphomannosyl transferase genes *PNO1* and *MNN4B* (*See* for example, U.S. Patent Nos. 7,198,921 and 7,259,007), which in further aspects can also include deleting or disrupting the *MNN4A* gene. Disruption includes disrupting the open reading frame encoding the particular enzymes or disrupting expression of the open reading frame or abrogating translation of RNAs encoding one or more of the β-mannosyltransferases and/or phosphomannosyltransferases using interfering RNA, antisense RNA, or the like. The host cells can further include any one of the aforementioned host cells modified to produce particular *N-*glycan structures.

Host cells further include lower eukaryote cells (e.g., yeast such as *Pichia pastoris*) that are genetically modified to control *O*-glycosylation of the glycoprotein by deleting or disrupting one or more of the protein *O*-mannosyltransferase (Dol-P-Man:Protein (Ser/Thr) Mannosyl Transferase genes) (*PMTs*) *(See* U.S. Patent No. 5,714,377) or grown in the presence of Pmtp inhibitors and/or an alpha-mannosidase as disclosed in Published International Application No. WO 2007061631, or both. Disruption includes disrupting the open reading frame encoding the Pmtp or disrupting expression of the open reading frame or abrogating translation of RNAs encoding one or more of the Pmtps using interfering RNA, antisense RNA, or the like. The host cells can further include any one of the aforementioned host cells modified to produce particular *N*-glycan structures.

Pmtp inhibitors include but are not limited to a benzylidene thiazolidinediones. Examples of benzylidene thiazolidinediones that can be used are 5-[[3,4-bis(phenylmethoxy) phenyl]methylene]-4-oxo-2-thioxo-3-thiazolidineacetic Acid; 5-[[3-(1-Phenylethoxy)-4-(2-phenylethoxy)]phenyl]methylene]-4-oxo-2-thioxo-3-thiazolidineacetic Acid; and 5-[[3-(1-Phenyl-2-hydroxy)ethoxy)-4-(2-phenylethoxy)]phenyl]methylene]-4-oxo-2-thioxo-3-thiazolidineacetic Acid.

In particular embodiments, the function or expression of at least one endogenous *PMT* gene is reduced, disrupted, or deleted. For example, in particular embodiments the function or expression of at least one endogenous *PMT* gene selected from the group consisting of the *PMT1, PMT2, PMT3,* and *PMT4* genes is reduced, disrupted, or deleted; or the host cells are cultivated in the presence of one or more PMT inhibitors. In further embodiments, the host cells include one or more *PMT* gene deletions or disruptions and the host cells are cultivated in the presence of one or more Pmtp inhibitors. In particular aspects of these embodiments, the host cells also express a secreted alpha-1,2-mannosidase.

*PMT* deletions or disruptions and/or Pmtp inhibitors control *O*-glycosylation by reducing *O*-glycosylation occupancy, that is by reducing the total number of *O*-glycosylation sites on the glycoprotein that are glycosylated. The further addition of an Alpha- ,2-mannosidase that is secreted by the cell controls *O*-glycosylation by reducing the mannose chain length of the *O-*glycans that are on the glycoprotein. Thus, combining *PMT* deletions or disruptions and/or Pmtp inhibitors with expression of a secreted alpha-1,2-mannosidase controls *O*-glycosylation by reducing occupancy and chain length. In particular circumstances, the particular combination of *PMT* deletions or disruptions, Pmtp inhibitors, and alpha-1,2-mannosidase is determined empirically as particular heterologous glycoproteins (Fabs and antibodies, for example) may be expressed and transported through the Golgi apparatus with different degrees of efficiency and thus may require a particular combination of *PMT* deletions or disruptions, Pmtp inhibitors, and alpha-1,2-mannosidase. In another aspect, genes encoding one or more endogenous mannosyltransferase enzymes are deleted. This deletion(s) can be in combination with providing the secreted alpha-1,2-mannosidase and/or *PMT* inhibitors or can be in lieu of providing the secreted alpha-1,2-mannosidase and/or *PMT* inhibitors.

Thus, the control of *O*-glycosylation can be useful for producing particular glycoproteins in the host cells disclosed herein in better total yield or in yield of properly assembled glycoprotein. The reduction or elimination of *O*-glycosylation appears to have a beneficial effect on the assembly and transport of whole antibodies and Fab fragments as they traverse the secretory pathway and are transported to the cell surface. Thus, in cells in which *O-*glycosylation is controlled, the yield of properly assembled antibodies or Fab fragments is increased over the yield obtained in host cells in which *O*-glycosylation is not controlled.

Thus, contemplated are host cells that have been genetically modified to produce glycoproteins wherein the predominant *N*-glycans thereon include but are not limited to MangGlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAc₂, Man₃GlcNAc₂, GlcNAc(₁₋₄)Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄)Man₃GlcNAc₂. Further included are host cells that produce glycoproteins that have particular mixtures of the aforementioned *N*-glycans thereon.

The host cells and methods herein are useful for producing a wide range of recombinant protein and glycoproteins. Examples of recombinant proteins and glycoproteins that can be produced in the host cells disclosed herein include but are not limited to erythropoietin (EPO); cytokines such as interferon α, interferon β, interferon γ, and interferon ω; and granulocyte-colony stimulating factor (GCSF); GM-CSF; coagulation factors such as factor VIII, factor IX, and human protein C; antithrombin III; thrombin,; soluble IgE receptor α-chain; immunoglobulins such as IgG, IgG fragments, IgG fusions, and IgM; immunoadhesins and other Fc fusion proteins such as soluble TNF receptor-Fc fusion proteins; RAGE-Fc fusion proteins; interleukins; urokinase; chymase; and urea trypsin inhibitor; IGF-binding protein; epidermal growth factor; growth hormone-releasing factor; annexin V fusion protein; angiostatin; vascular endothelial growth factor-2; myeloid progenitor inhibitory factor-1; osteoprotegerin; α-1-antitrypsin; α-feto proteins; DNase II; kringle 3 of human plasminogen; glucocerebrosidase; TNF binding protein 1; follicle stimulating hormone; cytotoxic T lymphocyte associated antigen 4 - Ig; transmembrane activator and calcium modulator and cyclophilin ligand; glucagon like protein 1; and IL-2 receptor agonist.

The recombinant host cells of the present invention disclosed herein are particularly useful for producing antibodies, Fc fusion proteins, and the like where it is desirable to provide antibody compositions wherein the percent galactose-containing *N*-glycans is increased compared to the percent galactose obtainable in the host cells prior to modification as taught herein. In general, the host cells enable antibody compositions to be produced wherein the ratio of G0:G1/G2 glycoforms is less than 2:1. Examples of antibodies that can be made in the host cells herein and have a ratio of G0:G1/G2 of less than 2:1 include but are not limited to human antibodies, humanized antibodies, chimeric antibodies, heavy chain antibodies (e.g., camel or llama). Specific antibodies include but are not limited to the following antibodies recited under their generic name (target): Muromonab-CD3 (anti-CD3 receptor antibody), Abciximab (anti-CD41 7E3 antibody), Rituximab (anti-CD20 antibody), Daclizumab (anti-CD25 antibody), Basiliximab (anti-CD25 antibody), Palivizumab (anti-RSV (respiratory syncytial virus) antibody), Infliximab (anti-TNFα antibody), Trastuzumab (anti-Her2 antibody), Gemtuzumab ozogamicin (anti-CD33 antibody), Alemtuzumab (anti-CD52 antibody), Ibritumomab tiuxeten (anti-CD20 antibody), Adalimumab (anti-TNFα antibody), Omalizumab (anti-IgE antibody), Tositumomab-¹³¹I (iodinated derivative of an anti-CD20 antibody), Efalizumab (anti-CD11a antibody), Cetuximab (anti-EGF receptor antibody), Golimumab (anti-TNFα antibody), Bevacizumab (anti VEGF-A antibody), and variants thereof. Examples of Fc-fusion proteins that can be made in the host cells disclosed herein include but are not limited to etancercept (TNFR-Fc fusion protein), FGF-21-Fc fusion proteins, GLP-I-Fc fusion proteins, RAGE-Fc fusion proteins, EPO-Fc fusion proteins, ActRIIA-Fc fusion proteins, ActRIIB-Fc fusion proteins, glucagon-Fc fusions, oxyntomodulin-Fc-fusions, and anlogs and variants thereof.

The recombinant cells disclosed herein can be used to produce antibodies and Fc fragments suitable for chemically conjugating to a heterologous peptide or drug molecule. For example, WO2005047334, WO2005047336, WO2005047337 and WO200610712 discloses chemically conjugating peptides or drug molecules to Fc fragments. EP1180121, EP1105409, and US6593295 disclose chemically conjugating peptides and the like to blood components, which includes whole antibodies.

The host cells and/or plasmid vectors encoding various combinations of the Leloir pathway enzymes as taught herein can be provided as kits that provide a selection system for making recombinant *Pichia pastoris* that express heterologous proteins. The *Pichia pastoris* host cell is genetically engineered to express one or two of the Leloir pathway enzymes selected from the group consisting of galactokinase, UDP-galactose-4-epimerase, and galactose-1-phosphate uridyl transferase. Optionally, the host cell can express a galactose permease as well. The cloning vector comprises a multiple cloning site and an expression cassette encoding the Leloir pathway enzyme or enzymes not in the provided host cell. The vector can further comprise a *Pichia pastoris* operable promoter and transcription termination sequence flanking the multiple cloning site and can further comprise a targeting sequence for targeting the vector to a particular location in the host cell genome. In some embodiments, the kit provides a vector that encodes all three Leloir pathway enzymes (galactokinase, UDP-galactose-4-epimerase, and galactose-1-phosphate uridyl transferase) and includes a multiple cloning site and a host cell that lacks the three Leloir pathway enzymes. The kit will further include instructions, vector maps, and the like.

The following examples are intended to promote a further understanding of the present invention.

### EXAMPLE 1

In this example, a *Pichia pastoris* host cell capable of producing galactose-containing *N*-glycans was constructed in general following the methods disclosed in Davidson et al. in U.S. Published Application No. 2006/0040353. The methods herein can be used to make recombinant host cells of other species that are normally incapable of using galactose as a carbon source into a recombinant host cell that is capable of using galactose as a sole carbon source.

*The Galactosyltransferase I chimeric enzyme.* The *Homo sapiens* β-1,4-galactosyltransferase I gene (*hGalTI,* GenBank AH003575) was PCR amplified from human kidney cDNA (Clontech) using PCR primers RCD192 (5'-GCCGCGACCTGAGCC GCCTGCCCCAAC-3' (SEQ ID NO:1)) and RCD186 (5'-CTAGCTCGGTGTCCCGATGTCCACTGT-3' (SEQ ID NO:2)). This PCR product was cloned into the pCR2.1 vector (Invitrogen) and sequenced. From this clone, a PCR overlap mutagenesis was performed for three purposes: 1) to remove a *Not*I site within the open reading frame while maintaining the wild-type protein sequence, 2) to truncate the protein immediately downstream of the endogenous transmembrane domain to provide only the catalytic domain, and 3) to introduce *Ascl* and *Pac*I sites at the 5' and 3' ends, respectively, for modular cloning. To do this, the 5' end of the gene up to the *Not*I site was PCR amplified using PCR primers RCD198 (5'-CTTAGGCGCGCCGGCCGCGACCTGAGCCGCCTGCCC-3' (SEQ ID NO:3)) and RCD201 (5'-GGGGCATATCTGCCGCCCATC-3' (SEQ ID NO:4)) and the 3' end was PCR amplified with PCR primers RCD200 (5'-GATGGGCGGCAGATATGCCCC-3' (SEQ ID NO:5)) and RCD199 (5'-CTTCTTAATTAACTAGCTCGGTGTCCCGATGTCCAC-3' (SEQ ID NO:6)). The products were overlapped together with primers RCD198 and RCD199 to re-synthesize the truncated open reading frame (ORF) encoding the galactosyltransferase with the wild-type amino acid sequence while eliminating the *Not*I site. The new *hGalTIβ* PCR catalytic domain product was cloned into the pCR2.1 vector (Invitrogen, Carlsbad, CA) and sequenced. The introduced *Asc*I and *Pac*I sites were cleaved with their cognizant restriction enzyme and the DNA fragment subcloned into plasmid pRCD259 downstream of the *PpGAPDH* promoter to create plasmid pRCD260. The nucleotide sequence encoding the *hGalT143* catalytic domain (lacking the first 43 amino acids; SEQ ID NO:50) is shown in SEQ ID NO:49.

A library of yeast leader sequences from *S. cerevisiae, P. pastoris,* and *K. lactis* that target proteins to various location in the Golgi was then ligated into this vector between the *Not*I and *Asc*I sites, thus fusing these leader encoding sequences in-frame with the open reading frame encoding the *hGalTIβ43* catalytic domain. The above described combinatorial library of GalT fusion proteins was expressed in YSH44 and the resulting transformants were analyzed by releasing the *N*-glycans from purified K3 from each transformant and determining their respective molecular mass by MALDI-TOF MS. The *P. pastoris* strain YSH44 expresses the kringle 3 domain of human plasminogen (K3) as a virtually uniform complex glycoform with biantennary terminal GlcNAc residues (GlcNAc₂Man₃GlcNAc₂, *See* Figure 1). One of the active constructs was *Mnn2-hGalTIβ43,* which encoded a fusion protein comprising the *N*-terminus of *S. cerevisiae Mnn2* targeting peptide (amino acids 1-36 (53) SEQ ID NO:20) fused to the N-terminus of the *hGalTIβ43* catalytic domain (amino acids 44-398; SEQ ID NO:50). The leader sequence contained the first 108 bp of the *S. cerevisiae MNN2* gene and it was this sequence that had been inserted between the *Not*I and *Asc*I sites of pRCD260 to create plasmid pXB53. Plasmid pXB53 was linearized with *Xba*I and transformed into yeast strain YSH44 to generate strain YSH71. Strain YSH44 has been described in U.S. Published Application Nos. 20070037248,20060040353,20050208617, and 20040230042 and Strain YSH71 has been described in U.S. Published Application No. 20060040353.

As shown in Figure 3C, a minor portion (about 10%) of the *N*-glycans produced by strain YSH71 was of a mass consistent with the addition of a single galactose sugar to the GlcNAc₂Man₃GlcNAc₂ (G0) *N*-glycan substrate on the K3 to make a GalGlcNAc₂Man₃GlcNAc₂ (G1) *N*-glycan, while the remainder of the *N*-glycans are identical to the *N*-glycans produced in the parent strain YSH44 (Fig. 3B). Figure 3A shows the *N*-glycans produced in wild-type yeast.

We considered several explanations for the incomplete galactose transfer. These explanations included poor UDP-galactose transport, low endogenous levels of UDP-galactose, or suboptimal GalT activity. It appears that the transfer galactose onto *N*-glycans might be low because the strains might require a transporter to translocate UDP-galactose from the cytosol to the Golgi apparatus (Ishida et al., J. Biochem. 120: 1074-1078 (1996); Miura et al., J. Biochem (Tokyo) 120: 236-241 (1996); Tabuchi et al., Biochem. Biophys. Res. Com. 232: 121-125 (1997); Segawa et al., FEBS Letts. 451: 295-298 (1999)). Transporters are complex proteins with multiple transmembrane domains that may not localize properly in a heterologous host. However, several sugar nucleotide transporters, including UDP-galactose transporters have been actively expressed in heterologous systems (Sun-Wada et al., J. Biochem. (Tokyo) 123: 912-917 (1998); Segawa et al. Eur. J. Biochem. 269: 128-138 (2002); Kainuma et al., Glycobiol. 9: 133-141 (1999); Choi et al., Proc Natl Acad Sci U S A 100(9): 5022-5027 (2003)). To ensure efficient transport of UDP-galactose into the Golgi, the *Drosophila melanogaster* gene encoding a UDP-galactose transporter, *DmUGT* (GenBank accession no. AB055493), was cloned and the clone transformed into strain YSH71 expressing the *MNN2-hGalTIβ43* construct as follows.

*Cloning of UDP-galactose Transporter.* The *D. melanogaster* gene encoding the UDP Galactose Transporter (GenBank AB055493) referred to as *DmUGT* was PCR amplified from a *D. melanogaster* cDNA library (UC Berkeley Drosophila Genome Project, ovary λ-ZAP library GM) using PCR primers DmUGT-5' (5'- GGCTCGAGCGGC CGCCACCATGAATAGCATACACATGAACGCCAATACG-3' (SEQ ID NO:7)) and DmUGT-3'(5'- CCCTCGAGTTAATTAACTAGACGCGCGGCAGCAGCTTCTCCTCATCG-3' (SEQ ID NO:8)) and the PCR amplified DNA fragment was cloned into pCR2.1 (Invitrogen, Carlsbad, CA) and sequenced. The *Not*I and *Pac*I sites were then used to subclone this open reading frame into plasmid pRCD393 downstream of the *PpOCH1* promoter between the *Not*I and *Pac*I sites to create plasmid pSH263. The nucleotide sequence encoding the *DmUGT* is shown in SEQ ID NO:37 and the amino acid sequence of the *DmUGT* is shown in SEQ ID NO:38. This plasmid was linearized with *Age*I and transformed into strain YSH71 to generate strain YSH80. However, no significant change in the *N*-glycan profile of K3 was found when the plasmid encoding the *DmUGT* was transformed into YSH71. Therefore, we decided to focus our efforts on enhancing the intracellular pool of UDP-galactose.

Because *P. pastoris* cannot assimilate galactose as a carbon source (Kurtzman Pichia. The Yeasts: A Taxonomic Study. C. P. a. F. Kurtzman, J. W. Amsterdam, Elsevier Science Publ.: 273-352 (1998)), we speculated that the pool of UDP-galactose in the strain might not be sufficient. The enzyme UDP-galactose 4-epimerase, which is conserved among galactose assimilating organisms, including bacteria and mammals, catalyzes the 3^{rd} step of the Leloir pathway. This enzyme is typically localized in the cytosol of eukaryotes and is responsible for the reversible conversion of UDP-glucose and UDP-galactose (Allard et al., Cell. Mol. Life Sci. 58: 1650-1665 (2001)). We reasoned that expression of a heterologous UDP-galactose 4-epimerase would generate a cytosolic UDP-galactose pool that upon transport into the Golgi would allow the galactose transferase to transfer galactose onto *N*-glycans.

*Cloning of UDP-galactose 4-epimerase.* A previously uncharacterized gene encoding a protein that has significant identity with known UDP-galactose 4-epimerases was cloned from the yeast *Schizosaccharomyces pombe,* designated *SpGALE* as follows. The 1.1 Kb *S. pombe* gene encoding a predicted UDP galactose-4-epimerase (GenBank NC_003423), referred to as *SpGALE,* was PCR amplified from *S. pombe* (ATCC24843) genomic DNA using primers PCR primers GALE2-L (5'-ATGACTGGTGTTCATGAAGGG-3' (SEQ ID NO:9)) and GALE2-R (5'-TTACTTATA TGTCTTGGTATG-3' ((SEQ ID NO:10)). The PCR amplified product was cloned into pCR2.1 (Invitrogen, Carlsbad, CA) and sequenced. Sequencing revealed the presence of an intron (175bp) at the +66 position. To eliminate the intron, upstream PCR primer GD1 (5'-GCGGCCGCATGA CTGGTGTTCA TGAAGGGACT GTGTTGGTTA CTGGCGGCGC TGGTTATATA GGTTCTCATA CGTGCGTTGT TTTGTTAGAA AA-3' ((SEQ ID NO:11)) was designed, which has a *Not*I site, 66 bases upstream of the intron, followed by 20 bases preceding the intron and downstream PCR primer GD2 (5'-TTAATTAATT ACTTATATGT CTTGGTATG-3' ((SEQ ID NO:12)), which has a *PacI* site. Primers GD1 and GD2 were used to amplify the *SpGALE* intronless gene from the pCR2.1 subclone and the product cloned again into pCR2.1 and sequenced. *SpGALE* was then subcloned between the *Not*I and *Pac*I sites into plasmids pRCD402 and pRCD403 to create plasmids pRCD406 (*P_{OCH1}-SpGALE-CYC1TT*) and pRCD407 (*P_{SEC4}-SpGALE-CYC1TT*)*,* respectively. These plasmids have been described previously in described in U.S. Published Application No. 20060040353. The nucleotide sequence encoding *SpGALE* without intron is shown in SEQ ID NO:35 and the amino acid sequence shown in SEQ ID NO:36.

The human UDP galactose-4-epimerase (hGalE) has the amino acid sequence shown in SEQ ID NO:48, which is encoded by the nucleotide sequence shown in SEQ ID NO:47. The hGalE can be used in place of the *SpGALE.*

*Construction of a double GalT*/*galactose-4-epimerase construct.* Plasmid pXB53, containing the *ScMNN2*-hGalTIβ43 fusion gene, was linearized with *Xho*I and made blunt with T4 DNA polymerase. The *P_{PpOCH1}SpGALE-CYC1TT* cassette was then removed from plasmid pRCD406 with *Xho*I and *Sph*I*,* the ends made blunt with T4 DNA polymerase, and the fragment inserted into the pXB53 plasmid above to create plasmid pRCD425. This plasmid was linearized with *Xba*I and transformed into strain YSH44 to generate strain RDP52, which has been previously described in described in U.S. Published Application No. 20060040353. N-glycans on purified K3 isolated from several of the transfonnants were analyzed by MALDI-TOF MS. As shown in Figure 3D, a significant proportion of the *N*-glycans were found to have acquired a mass consistent with the addition of either two (about 20% G2: Gal₂GlcNAc₂Man₃GlcNAc₂) or a single galactose moiety (about 40% G1: Gal₁GlcNAc₂Man₃GlcNAc₂) onto the G0 (GlcNAc₂Man₃GlcNAc₂) substrate while the remainder of the *N*-glycans remained unchanged from that found in the YSH44 parent (Figure 3B), that is G0.

*Construction of a triple GalT*/*galactose-4-epimerase*/*UDP galactose transporter construct.* The G418R plasmid containing *P_{OCH1}-DmUGT-CYC1TT,* pSH263, was linearized by digesting with *Sac*I and making the ends blunt with T4 DNA polymerase. The *P_{SEC4}-SpGALE-CYC1TT* cassette was removed from plasmid pRCD407 by digesting with *Xho*I and *Sph*I and making the ends blunt with T4 DNA polymerase. The blunt-ended *SpGALE* fragment was then inserted into the pSH263 above to create plasmid pRCD446. The *P_{GAPDH}ScMNN2-hGalTIβ43-CYC1TT* cassette was released from plasmid pXB53 by digesting with *Bgl*II/*Bam*HI and the ends made blunt with T4 DNA polymerase. The blunt-ended *hGalTI-53* was then inserted into the blunt *Eco*RI site of pRCD446 to create plasmid pRCD465, which is a triple G418^{R} plasmid containing *hGalTI-53, SpGALE,* and *DmUGT.* Plasmid pRCD465 was linearized with *Age*I and transformed into strain YSH44 to generate strain RDP80, which as been described in described in U.S. Published Application No. 20060040353. *N*-glycans released from secreted K3 produced by the strain were analyzed by MALDI-TOF MS. The *N*-glycans were found to be of a mass consistent with the quantitative addition of two galactose residues to the G0 substrate to yield the human galactosylated, biantennary complex *N*-glycan, G2 (Gal₂GlcNAc₂Man₃GlcNAc₂) (Figure 3E). *In vitro* β-galactosidase digestion of this *N*-glycan resulted in a mass decrease corresponding to the removal of two galactose residues yielding G0 (GlcNAc₂Man₃GlcNAc₂) (Figure 3F).

In addition, *in vitro* treatment of purified K3 from strain RDP80 with rat α-2,6-*N-*sialyltransferase in the presence of CMP-Sialic acid resulted in nearly uniform conversion to NANA₂Gal₂GlcNAc₂Man₃GlcNAc₂ (Figure 3G). These results indicate that highly efficient extension of complex *N*-glycans produced in *P. pastoris* is achievable through (1) the metabolic engineering of a sufficient intracellular UDP-galactose pool, (2) the expression of an active and properly localized GalT, and (3) the translocation of UDP-galactose into the Golgi apparatus by an active UDP-galactose transporter. However, the efficiency of galactose transfer was improved by further including the enzymes of the Leloir pathway into the host cell as shown in Example 2.

*Strains and Media. E. coli* strains TOP10 or DH5α were used for recombinant DNA work. *P. pastoris* strain YSH44 (Hamilton et al., Science 301: 1244-1246 (2003)), derived from strain JC308 (J. Cregg, Claremont, CA) was used for generation of various yeast strains. Transformation of yeast strains was performed by electroporation as previously reported (Cregg, et al., Mol. Biotechnol. 16: 23-52 (2000)). Protein expression was carried out at room temperature in a 96-well plate format (except for bioreactor experiments) with buffered glycerol-complex medium (BMGY) consisting of 1% yeast extract, 2% peptone, 100 mM potassium phosphate buffer, pH 6.0, 1.34% yeast nitrogen base, 4 X 10⁻⁵% biotin, and 1% glycerol as a growth medium; and buffered methanol-complex medium (BMMY) consisting of I % methanol instead of glycerol in BMGY as an induction medium. YPD is 1% yeast extract, 2% peptone, 2% dextrose and 2% agar.

Restriction and modification enzymes were from New England BioLabs (Beverly, MA). Oligonucleotides were obtained from Integrated DNA Technologies (Coralville, IA). The β-Galactosidase enzyme was obtained from QA bio (San Mateo, CA). Ninety-six-well lysate-clearing plates were from Promega (Madison, WI). Protein-binding 96-well plates were from Millipore (Bedford, MA). Salts and buffering agents were from Sigma (St. Louis, MO). MALDI matrices were from Aldrich (Milwaukee, WI).

*Protein purification and N*-glycan *analysis.* Purification of K3 was described previously *(*Choi et al., Proc. Natl. Acad. Sci. U.S.A. 100: 5022-5027 (2003)). *N*-glycans were released from K3 using the enzyme *N*-glycosidase F, obtained from New England Biolabs (Beverly, MA) as described previously (Choi *et al., ibid*.). Molecular weights of glycans were determined using a Voyager DE PRO linear MALDI-TOF Mass Spectrometer from Applied Biosystems (Foster City, CA) as described previously (Choi *et al, ibid*.).

*Bioreactor Cultivations.* A 500 mL baffled volumetric flask with 150 mL of BMGY media was inoculated with 1 mL of seed culture (see flask cultivations). The inoculum was grown to an OD₆₀₀ of 4-6 at 24°C (approx 18 hours). The cells from the inoculum culture were then centrifuged and resuspended into 50 mLof fermentation media (per liter of media: CaSO₄·2H₂O 0.30 g, K₂SO₄ 6.00 g, MgSO₄.7H₂O 5.00 g, Glycerol 40.0 g, PTM₁ salts 2.0 mL, Biotin 4x10⁻³ g, H₃PO₄ (85%) 30 mL, PTM₁ salts per liter: CuSO₄.H₂O 6.00 g, NaI 0.08 g, MnSO₄.7H₂ 3.00 g, NaMoO₄.2H₂O 0.20 g, H₃BO₃ 0.02 g, CoCl₂.6H₂O 0.50 g, ZnCl₂ 20.0 g, FeSO₄.7H₂O 65.0 g, Biotin 0.20 g, H₂SO₄ (98%) 5.00 mL).

Fermentations were conducted in three-liter dished bottom (1.5 liter initial charge volume) Applikon bioreactors. The fermenters were run in a fed-batch mode at a temperature of 24°C, and the pH was controlled at 4.5 ±0.1 using 30% ammonium hydroxide. The dissolved oxygen was maintained above 40% relative to saturation with air at 1 atm by adjusting agitation rate (450-900 rpm) and pure oxygen supply. The air flow rate was maintained at I vvm. When the initial glycerol (40g/L) in the batch phase is depleted, which is indicated by an increase of DO, a 50% glycerol solution containing 12 ml/L of PTM₁ salts was fed at a feed rate of 12 mL/L/h until the desired biomass concentration was reached. After a half an hour starvation phase, the methanol feed (100% methanol with 12 mL/L PTM₁) is initiated. The methanol feed rate is used to control the methanol concentration in the fermenter between 0.2 and 0.5%. The methanol concentration is measured online using a TGS gas sensor (TGS822 from Figaro Engineering Inc.) located in the offgas from the fermenter. The fermenters were sampled every eight hours and analyzed for biomass (OD₆₀₀, wet cell weight and cell counts), residual carbon source level (glycerol and methanol by HPLC using Aminex 87H) and extracellular protein content (by SDS page, and Bio-Rad protein assay).

In vitro *β-galactosidase digest. N*-glycans from RDP80 were incubated with β1,4-galactosidase (QA bio, San Mateo, CA) in 50 mM NH₄HCO₃, pH6.0 at 37°C for 16-20 hours.

*In vitro sialic acid transfer.* K3 purified from strain RDP80 was used as the substrate for sialic acid transfer. Of this protein, 200 µg was incubated with 50 µg CMP-sialic acid and 15 mU rat recombinant α-(2,6)-(N)-sialyltransferase from EMD Biosciences (San Diego, CA, formerly Calbiochem) in 50 mM NH₄HCO₃, pH6.0 at 37°C for 16-20 hours. *N-*glycan was then released by PNGaseF digest and detected by MALDI-TOF MS.

### EXAMPLE 2

The enzyme UDP-galactose 4-epimerase catalyzes the 3^{rd} step of the Leloir pathway (Figure 4). As shown in Example 1, heterologous expression of the gene encoding this enzyme in a glycoengineered strain of *P. pastoris* resulted in the generation of an intracellular pool of UDP-galactose as evidenced by the dramatic increase in galactose transfer in strains expressing this heterologous gene. However, as also shown, addition of this enzyme alone did not confer upon *P. pastoris* strains the ability to grow on galactose as a sole carbon source *(See* Figure 7, strain RDP578-1). Therefore, the remainder of the Leloir pathway in *S*. *cerevisiae* was introduced into various strains of Example 1. Thus, in this example, a *Pichia pastoris* host cell capable of using galactose as a sole carbon source was constructed. The methods herein can be used to make recombinant host cells of other species that are normally incapable of using galactose as a carbon source into a recombinant host cell that is capable of using galactose as a sole carbon source.

*Cloning of S. cerevisiae GAL1.* The *S*. *cerevisiae* gene encoding the galactokinase (GenBank NP_009576) referred to as *ScGAL1* was PCR amplified from S. *cerevisiae* genomic DNA (Strain W303, standard smash and grab genomic DNA preparation) using PCR primers PB158 (5'- TTAGCGGCCGCAGGAATGACTAAATCTCATTCA-3' (SEQ ID NO:13)) and PB159 (5'- AACTTAATTAAGCTTATAATTCATATAGACAGC-3' (SEQ ID NO:14)) and the PCR amplified DNA fragment was cloned into pCR2.1 (Invitrogen, Carlsbad, CA) and sequenced. The resulting plasmid was named pRCD917. The DNA fragment encoding the galactokinase was released from the plasmid with *Not*I and *Pac*I and the DNA fragment subcloned into plasmid pGLY894 downstream of the *P. pastoris HHT1* strong constitutive promoter between the *Not*I and *Pac*I sites to create plasmid pGLY939. The galactokinase has the amino acid sequence shown in SEQ ID NO:40 and is encoded by the nucleotide sequence shown in SEQ ID NO:39.

*Cloning of S. cerevisiae GAL2.* The *S. cerevisiae* gene encoding the galactose permease (GenBank NP_013182) referred to as *ScGAL2* was PCR amplified from *S. cerevisiae* genomic DNA (Strain W303, standard "smash and grab" genomic DNA preparation) using PCR primers PB156 (5'- TTAGCGGCCGC -3' (SEQ ID NO:15)) and PB157 (5'- AACTTAATTAA - 3' (SEQ ID NO:16)) and the PCR amplified DNA fragment was subcloned into pCR2.1 (Invitrogen, Carlsbad, CA) and sequenced. The resulting plasmid was named pPB290. The DNA fragment encoding the galactose permease was released from the plasmid with *Not*I and *Pac*I and the DNA fragment subcloned into plasmid pJN664 downstream of the *PpPMA1* promoter between the *Not*I and *Pac*I sites to create plasmid pPB292. The galactose permease has the amino acid sequence shown in SEQ ID NO:44 and is encoded by the nucleotide sequence shown in SEQ ID NO:43.

*Cloning of S. cerevisiae GAL7.* The *S. cerevisiae* gene encoding the galactose-1-phosphate uridyl transferase (GenBank NP_009574) referred to as *ScGAL7* was PCR amplified from *S. cerevisiae* genomic DNA (Strain W303, standard smash and grab genomic DNA preparation) using PCR primers PB160 (5'- TTAGCGGCCG CAGGAATGAC TGCTGAAGAA TT-3' (SEQ ID NO:17)) and PB161 (5'- AACTTAATTA AGCTTACAGT CTTTGTAGAT AATC-3' (SEQ ID NO:18) and the PCR amplified DNA fragment was cloned into pCR2.1 (Invitrogen, Carlsbad, CA) and sequenced. The resulting plasmid was named pRCD918. The DNA fragment encoding the galactose-1-phosphate uridyl transferase was released from the plasmid with *Not*I and *Pac*I and the DNA fragment subcloned into plasmid pGLY143 downstream of the *PpPMA1* strong constitutive promoter at *Not*I/*Pac*I to create plasmid pGLY940. Separately, the *Not*I and *Pac*I sites were also used to subclone this ORF into plasmid pRCD830 downstream of the *P. pastoris TEF1* strong constitutive promoter at *Not*I*lPac*I to create plasmid pRCD929. The galactose-1-phosphate uridyl transferase has the amino acid sequence shown in SEQ ID NO:42 and is encoded by the nucleotide sequence shown in SEQ ID NO:41.

*Construction of a triple SeGAL1*/*ScGAL7*/*ScGAL2 construct.* The *ScGAL1* open reading frame from pGLY917 was subcloned into pJN702, a *P. pastoris his1* knock-out vector with the *P. pastoris ARG1* selectable marker *(his1::ARG1,* see U.S. Patent No. 7,479,389), and also containing a *P_{GAPDH}-* promoter cassette and this new vector containing the *P_{GAPDH}-ScGAL1* fusion was named pRCD928. The *P_{TEF1}-ScGAL7* cassette from pGLY929 was subcloned into pGLY928 and the new vector was named pGLY946a. Next, the *P_{OCH1}-DmUGT* (Golgi UDP-galactose transporter) cassette from pRCD634 was subcloned into pGLY946a to create pGLY956b. Finally, the *P_{GAPDH}-ScGAL2* cassette from pPB292 was subcloned into pRCD956b to produce plasmid pRCD977b *(See* Figure 6). Plasmid pRCD977b contains *DmUGT, ScGAL1, ScGAL7,* and *ScGAL2* expression cassettes along with the *ARG1* dominant selectable marker cassette.

*Construction of a double ScGAL1*/*ScGAL7 construct.* The *P_{TEF1}-ScGAL1* cassette from pGLY939 was subcloned into pGLY941, a knock-in vector with the *P. pastoris ARG1* selectable marker, *TRP1* locus knock-in region, and also containing a *P_{GAPDH}-*hGalTIβ cassette and this new vector was named pGLY952. The *P_{PMA1}-ScGAL7* cassette from pGLY940 was subcloned into pGLY952 and the new vector was named pGLY955. Finally, the Nourseothricin resistance cassette (NATR) was subcloned from pGLY597 (originally from pAG25 from EROSCARF, Scientific Research and Development GmbH, Daimlerstrasse 13a, D-61352 Bad Homburg, Germany, *See* Goldstein et al., 1999, Yeast 15: 1541) into pGLY952 to produce plasmid pGLY1418 *(See* Figure 12), which contains hGalTIβ, *ScGAL1,* and *ScGAL7* expression cassettes along with the NATR dominant selectable marker cassette.

The single integration plasmid harboring all three genes, pRCD977b (Figure 6), was transformed into the *P. pastoris* strain RDP578-1 to produce strains RDP635-1, -2, and -3. Strain RDP578-1 already contained the heterologous genes and gene knockouts for producing human *N*-glycan containing terminal β-1,4-galactose residues (*See* Figure 5 and Example 3 for construction. Strain RDP578-1 also includes an expression cassette encoding the *Saccharomyces cerevisiae* UDP-Galactose 4-epimerase encoding gene, *ScGAL10,* and expresses the test protein human kringle 3. The resulting strains RDP635-1, -2, and -3 have two copies of the *DmUGT* galactose transporter.

The parental strain, RDP578-1, and the transformants with the *ScGAL1, ScGAL2, ScGAL7,* and *ScGAL10* genes (RDP635-1, -2, and -3) were grown on minimal medium containing glucose, galactose, or no carbon source for five days and then photographed. Interestingly, despite having the ability to secrete proteins with galactose-terminated N-glycans, RDP578-1 displayed no ability to assimilate galactose, while growing nonnally on glucose, as would be expected for wild-type *P. pastoris.* However, the transformants expressing the *ScGAL1, ScGAL2, and ScGAL7* genes were capable of assimilating galactose as shown in Figure 7. As expected, minimal growth was observed on the plates lacking a carbon source. These results indicate that a recombinant *P. pastoris* can be constructed that can assimilate galactose as a carbon source when reconstituted with the basic structural (but not regulatory) elements of the Leloir galactose assimilation pathway.

*Determination of N-glycans at Asn residue 297 of Fc expressed in glycoengineered P. pastoris.* The Fc portion of human IgGs contains a single N-glycan site per heavy chain dimer (Asn₂₉₇, Kabat numbering) that typically contains an *N*-glycan profile distinct from that of other secreted human proteins. Generally, naturally occurring human antibodies contain *N*-glycans with terminal GlcNAc and an amount of terminal galactose that can differ based on various factors and rarely contain a significant amount of terminal sialic acid. After demonstrating a high level of terminal β-1,4-galactose to *N*-glycans, we sought to determine the profile of *N*-glycans that are observed on antibodies produced in such a glycoengineered yeast strain. Therefore, a *P. pastoris* strain that had been genetically engineered to produce *N*-glycans with terminal galactose (YGB02; *See* Figure 8 and Example 4) was transformed with a plasmid (pBK138) encoding the Fc domain or C-terminal half of the human Immunoglobulin G1 (IgG1) heavy chain under control of the *AOX1* promoter. A selected positive clone identified by PCR was named PBP317-36 (Figure 8 and Example 4). This strain was grown in a shake flask and induced with methanol as a sole carbon source. The supernatant was harvested by centrifugation and was subjected to purification by protein A affinity chromatography. Purified protein was separated on SDS-PAGE and coomassie stained. A labeled band of the expected size was observed. The purified protein was then subjected to PNGase digestion and the released N-glycans analyzed by MALDI-TOF MS. The resulting N-glycans (Figure 10A) revealed a predominant mass consistent with a complex human core structure with terminal GlcNAc (G0: GlcNAc₂Man₃GlcNAc₂), a lesser species with a single terminal galactose (G1: GalGlcNAc₂Man₃GlcNAc₂), and a minor species where both arms of a complex species are capped with galactose (G2: Gal₂GlcNAc₂Man₃GlcNAc₂). The masses of these species differed predictably from the canonical values reported in the literature due to the lack of a single fucose residue. Glycoengineered yeast strains do not contain an endogenous fucosyltransferase and therefore lack the inherent ability to add a fucose to the core human N-glycan structure. Another minor species consistent with Man₅GlcNAc₂ was also observed.

Strain PBP317-36 above which expressed the glycosylation activities required to assemble human-like *N*-glycans with terminal galactose and which also expressed *SpGALE* (UDP-galactose 4-epimerase) was then genetically engineered to be able to use exogenous galactose as a carbon source and to control *N*-glycosylation in a metabolically engineered manner. An integration plasmid expressing both *ScGAL1* and *ScGAL7* under the control of a constitutive promoter, pGLY954, was constructed (Figure 9) and transformed into the *P. pastoris* strain PBP317-36. Plasmid pGLY954 conferred upon strain PBP317-36 (which already contained the *SpGALE* UDP-galactose epimerase, Figure 8) the ability to grow on galactose as a sole carbon source. This gal⁺ strain was named RDP783. Because the cells could use galactose as a carbon source even though we had not introduced the galactose permease into the cell, we concluded that general hexose transporters endogenous to *P. pastoris* are able to transport galactose sufficiently across the cell membrane.

*P. pastoris* strain PBP317-36 and RDP783 both harbor an integrated plasmid construct encoding the human Fc domain as a secreted reporter protein under control of the methanol-inducible AOX1 promoter. Strains PBP317-36 and RDP783 were grown in shake flasks in standard media containing glycerol and induced in the presence of either methanol as a sole carbon source or with methanol combined with glucose or galactose at different concentrations. Harvested supernatant protein was affinity purified by protein A, subjected to PNGase digestion, and analyzed by MALDI-TOF MS. *N*-glycans released from the human Fc from strain RDP783 yielded a similar *N*-glycan to the profile observed with PBP317-36 upon methanol induction alone or in the presence of glucose or mannose, with the predominant glycoform G0 (GlcNAc₂Man₃GlcNAc₂) (Figure 10). However upon exogenous galactose feed, strain RDP783 (but not the parent strain PBP317-36) yielded a dose-dependent increase in galactose-containing *N*-glycans on the human Fc, with a shift in the predominant glycoform now to G1 (GalGlcNAc₂Man₃GlcNAc₂) and a concomitant increase in the fully β-1,4-galactose capped glycoform G2 (Gal₂GlcNAc₂Man₃GlcNAc₂) (Figure 10).

Finally, to demonstrate that the ability to control glycosylation using exogenous galactose observed with the human Fc could be applied to a full-length monoclonal antibody, a glycoengineered yeast strain was generated, YDX477 (Figure 11, Example 5), that expresses an anti-Her2 monoclonal antibody. This strain was also engineered to transfer human *N*-glycans of the form G2 (Gal₂GlcNAc₂Man₃GlcNAc₂) on secreted glycoproteins. Release of *N*-glycans after expression of mAb-A revealed an *N*-glycan pattern (Figure 13A) consisting of a predominant peak consistent with G0 (GlcNAc₂Man₃GlcNAc₂), with a less predominant peak consistent with G1 (GalGlcNAc₂Man₃GlcNAc₂), as well as minor peaks of G2 (Gal₂GlcNA.c₂Man₃GlcNAc₂) and M5 (Man₅GlcNAc₂). This data is similar with what was observed for the truncated Fc portion of human IgG₁ and was expected because both are N-glycosylated at the same residue (Asn-297). An integration plasmid harboring *ScGAL1* and *ScGAL7,* pGLY1418, was constructed (Figure 12) and transformed into the *P. pastoris* strain YDX477 to make strain RDP968-1. This plasmid conferred upon strain YDX477 (which already contains the *SpGALE* UDP-galactose epimerase, Figure 11) the ability to grow on galactose as a sole carbon source.

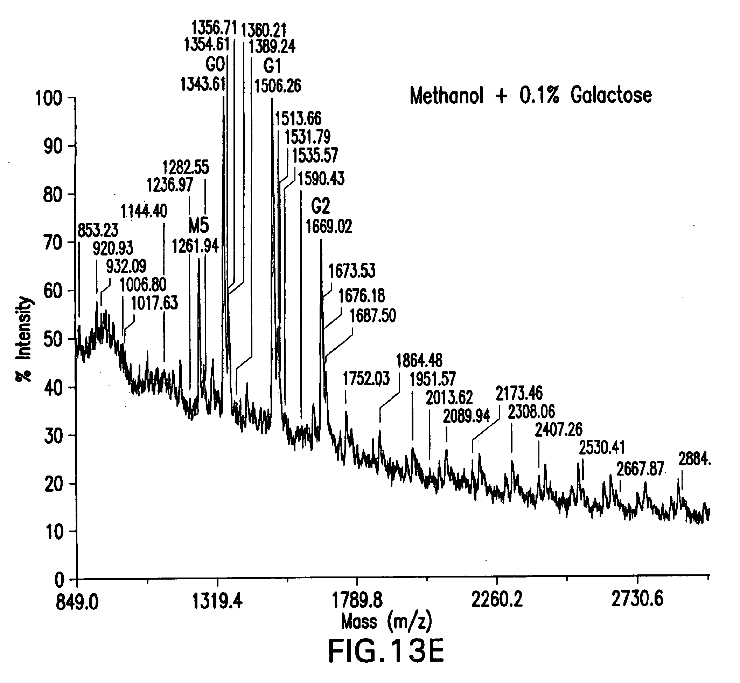

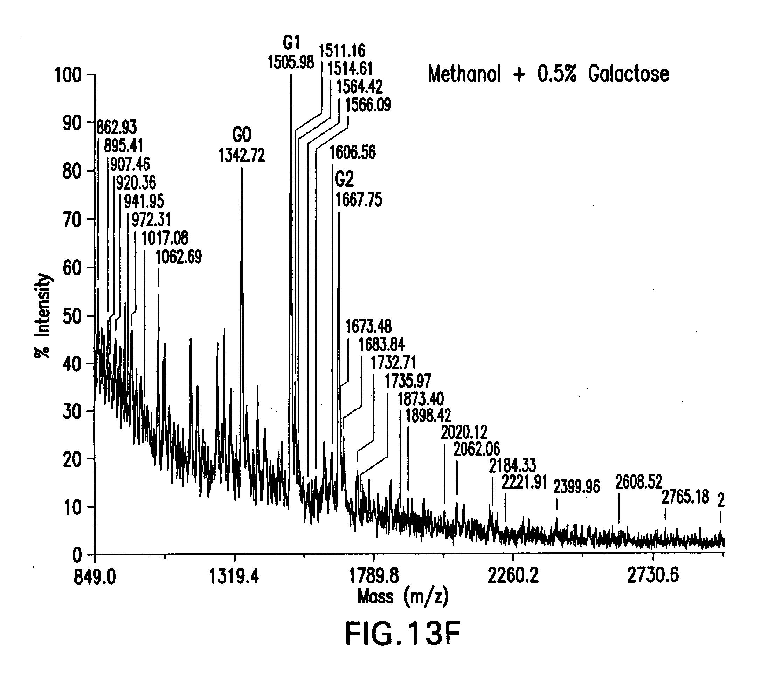

Strains YDX477 and RDP968-1 were grown in shake flasks in standard media containing glycerol and induced in the presence of either methanol as a sole carbon source or with methanol combined with galactose at different concentrations. Harvested supernatant protein was affinity purified by protein A, subjected to PNGase digestion, and analyzed by MALDI-TOF MS. Both strains yielded *N*-glycans similar to the profile observed previously with PBP317-36 upon methanol induction alone or in the presence of glucose or mannose, with the predominant glycoform G0 (GlcNAc₂Man₃GlcNAc₂) (Figure 10A vs. Figures 13A and 13D). However upon exogenous galactose feed, strain RDP968-1 (but not the parent strain YDX477) yielded a dose-dependent increase in galactose-containing *N*-glycans on the antibody, with a shift in the predominant glycoform now to G 1 (GalGlcNAc₂Man₃GlcNAc₂) and a concomitant increase in the fully β-1,4-galactose capped glycoform G2 (Gal₂GlcNAc₂Man₃GlcNAc₂) (Figure 13E and F).

### EXAMPLE 3

Construction of strain RDP578-1 is shown in Figure 5 and involved the following steps. Strain JC308 was the starting strain. This strain has been described in Choi et al., Proc. Natl. Acad. Sci. USA 100: 5022-5027 (2003) but briefly, the strain is *ura3, ade1, arg4, his4.* This strain was rendered deficient in alpha-1,6 mannosyltransferase activity by disrupting the *OCH1* gene using plasmid pJN329 and following the procedure described in Choi *et al. (ibid.)* and in U.S. Patent No. 7,449,308 to produce strain YJN153. Plasmid pJN329 carries the *PpURA3* dominant selection marker, after counterselecting for *ura-* activity, resulting strain YJN156 was rendered deficient in phosphomannosyltransferase activity by disrupting the *PNO1, MMN4A,* and *MNN4B* genes using plasmid vectors pJN503b and pAS19 following the procedure described in U.S. Patent No. 7,259,007 to produce strain YAS180-2. The secretory pathway targeting leader peptides comprising the fusion proteins herein localize the catalytic domain it is fused to the ER, Golgi, or the trans Golgi network.

After counterselecting for *ura-* activity, resulting strain YAS 187-2 was rendered deficient in beta-mannosyltransferase activity generally as described in U.S. Patent No. 7,465,577 using plasmid pAS24 *(See* Figure 14) to make strain YAS218-2. Plasmid pAS24 is a *P. pastoris BMT2* knock-out plasmid that contains the *PpURA3* selectable marker and contains an expression cassette encoding the full length mouse Golgi UDP-GlcNAc Transporter (MmSLC35A3) downstream of the *PpOCH1* promoter. MmSLC35A3 has the amino acid sequence shown in SEQ ID NO:34 which is encoded by the nucleotide sequence shown in SEQ ID NO:33. 5' and 3' *BMT2* flanking sequences for removing beta-mannosyltransferase activity attributed to bmt2p can be obtained as shown in U.S. Patent No. 7,465,577. After counterselecting strain YAS218-2 for *ura-* activity, resulting strain YAS269-2 is *ura-* and has the mouse Golgi UDP-GlcNAc Transporter inserted into the *BMT2* gene.

Strain YAS269-2 was then transformed with plasmid pRCD742b (*See* Figure 15), which comprises expression cassettes encoding a chimeric mouse alpha-1,2-mannosyltransferase I (FB8 MannI), a chimeric human GlcNAc Transferase I (CONA10), and the full-length gene encoding the Mouse Golgi UDP-GlcNAc transporter (MmSLC35A3) and targets the plasmid to the *ADE1* locus *(See* PCT/US2008/13719). Plasmid pRCD742b is a Knock-In Knock-Out (KINKO) plasmid, which has been described in WO2007/136865 and W02007136752. The plasmid integrates into the *P. pastoris ADE1* gene without deleting the open reading frame encoding the Ade1p. The plasmid also contains the *PpURA5* selectable marker. The expression cassette encoding a secretory pathway targeted fusion protein (FB8 MannI) comprises a *ScSec12* leader peptide (the first 103 amino acids of *ScSec12* (8): SEQ ID NO:32) fused to the *N*-terminus of the mouse alpha-1,2-mannosyltransferase I catalytic domain (FB MannI: SEQ ID NO:54) under the control of the *PpGAPDH* promoter. The expression cassette encoding the secretory pathway targeted fusion protein CONA10 comprises a *PpSec12* leader peptide (the first 29 amino acids of *PpSec12* (10): SEQ ID NO:28) fused to the *N*-terminus of the human GlcNAc Transferase I (GnT 1) catalytic domain (SEQ ID NO:52) under the control of the *PpPMA1* promoter. The plasmid further included an expression cassette encoding the full-length mouse Golgi UDP-GlcNAc transporter (MmSLC35A3) under the control of the *PpSEC4* promoter. Transfection of plasmid pRCD742b into strain YAS269-2 resulted in strain RDP307. This strain is capable of making glycoproteins that have GlcNAcMan₅GlcNAc₂ *N*-glycans. SEQ ID NOs:53 and 51 are the nucleotide sequences encoding the mouse alpha-1,2-mannosyltransferase I and human GlcNAc Transferase I (GnT I) catalytic domains, respectively. The nucleotide sequence encoding the human GnT I was codon-optimized for expression in *Pichia pastoris.* SEQ ID NOs:27 and 31 are the nucleotide sequences encoding the *PpSEC12* (10) and the *ScSEC12* (8), respectively.

Strain RDP361 was constructed by transforming strain RDP307 with plasmid pDMG47 to produce strain RDP361. Plasmid pDMG47 *(See* Figure 16) is a KINKO plasmid that integrates into the *P. pastoris TRP1* locus without deleting the open reading frame encoding the Trplp. The plasmid also contains the *PpURA3* selection marker and comprises an expression cassette encoding a secretory pathway targeted fusion protein (KD53) comprising an *ScMnn2* leader targeting peptide (the first 36 amino acids of *ScMnn2* (53): SEQ ID NO:19) fused to the *N*-terminus of the catalytic domain of the *Drosophila melanogaster* Mannosidase II (KD: SEQ ID NO:63) under the control of the *PpGAPDH* promoter. The plasmid also contains an expression cassette encoding a secretory pathway targeted fusion protein (TC54) comprising an *ScMnn2* leader targeting peptide (the first 97 amino acids of *ScMnn2* (54): SEQ ID NO:22) fused to the *N*-terminus of the catalytic domain of the rat GlcNAc Transferase II (TC: SEQ ID NO:58) under the control of the *PpPMA1* promoter. The nucleic acid sequence of the *ScMnn2* leaders 53 and 54 are shown in SEQ ID NOs:19 and 21, respectively. The nucleic acid sequences encoding the catalytic domains of the *Drosophila melanogaster* mannosidase II and rat GIcNAc transferase II (GnT II) are shown in SEQ ID NOs:62 and 57, respectively.

Strain RDP361 above was transformed with plasmid pRCD823b to produce strain RDP415-1. Plasmid pRCD823b (*See* Figure 17) is a KINKO plasmid that integrates into the *P. pastoris HIS4* locus *(See* U.S. Patent No. 7,479,389) without deleting the open reading frame encoding the His4p and contains the *PpURA5* selectable marker *(See* U.S. Pub. Application No. 20040229306) as well as an expression cassette encoding a secretory pathway targeted fusion protein (TA54) comprising the rat GIcNAc Transferase II catalytic domain (TA: SEQ ID NO:61) fused at its *N*-terminus to the first 97 amino acids of *ScMnn2* (54) as above but under the control of the *PpGAPDH* promoter. The plasmid also contains expression cassettes encoding the full-length *D. melanogaster* Golgi UDP-galactose transporter (*DmUGT*) under the control of the *PpOCH1* promoter and the full-length *S. cerevisiae* UDP-galactose 4-epimerase (*ScGAL10*) under the control of the *PpPMA1* promoter. The *SeGAL10* has the amino acid sequence shown in SEQ ID NO:46, which is encoded by the nucleotide sequence shown in SEQ ID NO:45. The nucleotide sequence of rat GlcNAc Transferase II (TA) is shown in SEQ ID NO:60.

Strain RDP415-1 above was transformed with plasmid pRCD893a to produce strain RDP523-1. Plasmid pGLY893a *(See* Figure 18) is a *P. pastoris his1* knock-out plasmid that contains the *PpARG4* selectable marker *(See* U.S. Patent No. 7,479,389). The plasmid comprises an expression cassette encoding a secretory pathway targeted fusion protein (KD10) comprising a *PpSEC12* leader targeting peptide (the first 29 amino acids of *PpSEC12* (10): SEQ ID NO:28) fused to the *N*-terminus of the catalytic domain of the *Drosophila melanogaster* Mannosidase II (KD: SEQ ID NO:63) under the control of the *PpPMA1* promoter. The plasmid also contains an expression cassette encoding a secretory pathway targeted fusion protein (TA33) comprising an ScMntIp (ScKre2p) leader targeting peptide (the first 53 amino acids of *Sc*MntIp (ScKre2p) (33): SEQ ID NO:30) fused to the *N*-terminus of the catalytic domain of the rat GIcNAc Transferase II (TA: SEQ ID NO:61) under the control of the *PpTEF1* promoter. The plasmid also contains an expression cassette encoding a secretory pathway targeted fusion protein (XB53) comprising the first 36 amino acids of *Sc*Mnn2p leader peptide (53) fused to the N-terminus of the catalytic domain of the human Galactosyl Transferase I (*hGalTIβ43;* SEQ ID NO:50). The nucleic acid sequence of the *PpSECI2* and *ScMNTI* (*ScKRE2*) leaders are shown in SEQ ID NOs:27 and 29, respectively. The nucleic acid sequences encoding the catalytic domains of the *Drosophila melanogaster* mannosidase II, rat GlcNAc transferase II (GnT II), and human GalTI are shown in SEQ ID NOs:62, 60, and 49, respectively. This strain can make glycoproteins that have *N*-glycans that have terminal galactose residues. The strain encodes two copies of the *Drosophila melanogaster* mannosidase II catalytic domain and three copies of the rat GnT II catalytic domain.

Finally, strain RDP523-1 above was transformed with plasmid pBK64 to produce strain RDP578-1. Plasmid pBK64 encodes the human kringle3 test protein and has been described in Choi et al., Proc. Natl. Acad. Sci. USA 100: 5022-5027 (2003).

### EXAMPLE 4

Construction of strain PBP317-36 is shown in Figure 8. The starting strain was YGLY16-3. This is a *ura-* strain with deletions of the *OCH1, PNO1, MNN4A, Mnn4B,* and the *BMT2* genes and can be made following the process that was used in Example 3. Strain YGLY16-3 has also been disclosed in WO2007136752.

Strain YGLY16-3 was transformed with plasmid pRCD742a *(See* Figure 19) to make strain RDP616-2. Plasmid pRCD742a (*See* Figure 19) is a KINKO plasmid that integrates into the *P. pastoris ADE1* gene without deleting the open reading frame encoding the Adelp. The plasmid also contains the *PpURA5* selectable marker and includes expression cassettes encoding the chimeric mouse alpha-1,2-mannosyltransferase (FB8 MannI), the chimeric human GlcNAc Transferase I (CONA10), and the full-length mouse Golgi UDP-GlcNAc transporter (MmSLC35A3). The plasmid is the same as plasmid pRCD742b except that the orientation of the expression cassette encoding the chimeric human GlcNAc Transferase I is in the opposite orientation. Transfection of plasmid pRCD742a into strain YGLY16-3 resulted in strain RDP616-2. This strain is capable of making glycoproteins that have GlcNAcMansGlcNAc₂ N-glycans.

After counterselecting strain RDP616-2 to produce *ura-* strain RDP641-3, plasmid pRCD1006 was then transformed into the strain to make strain RDP666. Plasmid pRCD1006 (*See* Figure 20) is a *P. pastoris his1* knock-out plasmid that contains the *PpURAS* gene as a selectable marker. The plasmid contains an expression cassette encoding a secretory pathway targeted fusion protein (XB33) comprising the first 58 amino acids of ScMntlp (ScKre2p) (33) fused to the *N*-terminus of the human Galactosyl Transferase I catalytic domain (*hGalTIβ43*) under control of the *PpGAPDH* promoter; an expression cassette encoding the full length D. *melanogaster* Golgi UDP-galactose transporter (*DmUGT*) under control of the *PpOCH1* promoter; and an expression cassette encoding the *S. pombe* UDP-galactose 4-epimerase (*SpGALE*) under control of the *PpPMA1* promoter.

Strain RDP666 was transformed with plasmid pGLY167b to make strain RDP696-2. Plasmid pGLY167b *(See* Figure 21) is a *P. pastoris arg1* knock-out plasmid that contains the *PpURA3* selectable marker. The plasmid contains an expression cassette encoding a secretory pathway targeted fusion protein (CO-KD53) comprising the first 36 amino acids of *Sc*Mnn2p (53) fused to *N*-terminus of the *Drosophila melanogaster* Mannosidase II catalytic domain (KD) under the control of *PpGAPDH* promoter and an expression cassette expressing a secretory pathway targeted fusion protein (CO-TC54) comprising the first 97 amino acids of ScMnn2p (54) fused to the *N*-terminus of the rat GlcNAc Transferase II catalytic domain (TC) under the control of the *PpPMA1* promoter. Resulting strain RDP696-2 was subjected to chemostat selection *(See* Dykhuizen and Hartl, Microbiol. Revs. 47: 150-168 (1983) for a review of chemostat selection). Chemostat selection produced strain YGB02. Strain YGB02 can make glycoproteins that have *N*-glycans that have terminal galactose residues. In this strain, the mannosidase II catalytic domain (KD) and the GnT II (TC) were encoded by nucleic acid molecules that were codon-optimized for expression in *Pichia pastoris* (SEQ ID NO:64 and 59, respectively).

Strain YGB02 was transfected with plasmid pBK138 to produce strain PBP317-36. Plasmid pBK138 (*See* Figure 22) is plasmid is a roll-in plasmid that integrates into the *P. pastoris AOX1* promoter while duplicating the promoter. The plasmid contains an expression cassette encoding a fusion protein comprising the S. *cerevisiae* Alpha Mating Factor pre-signal sequence (SEQ ID NO:24) fused to the *N*-terminus of the human Fc antibody fragment (C-terminal 233-aa of a human IgG1 heavy chain; SEQ ID NO:66). The nucleic acid sequence encoding the *S*. *cerevisiae* Alpha Mating Factor pre-signal sequence is shown in SEQ ID NO:23 and the nucleic acid sequence encoding the C-terminal 233-aa of the human IgG1 Heavy chain is shown in SEQ ID NO:65).

### EXAMPLE 5

Construction of strain YDX477 is shown in Figure 11. The starting strain was YGLY16-3. Strain YGLY16-3 was transformed with plasmid pRCD742a (See Figure 19) to make strain RDP616-2. Plasmid pRCD742a (*See* Figure 19) is a KINKO plasmid that integrates into the *P. pastoris ADE1* gene without deleting the open reading frame encoding the adelp. The plasmid also contains the *PpURA5* selectable marker and includes expression cassettes encoding the chimeric mouse alpha-1,2-mannosyltransferase (FB8 MannI), the chimeric human GlcNAc Transferase I (CONA10), and the full length mouse Golgi UDP-GlcNAc transporter (MmSLC35A3). The plasmid is the same as plasmid pRCD742b except that the orientation of the expression cassette encoding the chimeric human GlcNAc Transferase I is in the opposite orientation. Transfection of plasmid pRCD742a into strain YGLY16-3 resulted in strain RDP616-2. This strain is capable of making glycoproteins that have GleNACMan₅GIcNAC₂ *N-*glycans.

After counterselecting strain RDP616-2 to produce *ura-* strain RDP641-4, plasmid pRCD1006 was then transformed into the strain to make strain RDP667-1. Plasmid pRCD1006 (*See* Figure 20) is a *P. pastoris his1* knock-out plasmid that contains the *PpURA5* gene as a selectable marker. The plasmid contains an expression cassette encoding a secretory pathway targeted fusion protein (XB33) comprising the first 58 amino acids of ScMntlp (ScKre2p) (33) fused to the *N*-terminus of the human Galactosyl Transferase I catalytic domain *(hGalTIβ43)* under control of the *PpGAPDH* promoter; an expression cassette encoding the full-length D. *melanogaster* Golgi UDP-galactose transporter (*DmUGT*) under control of the *PpOCH1* promoter; and an expression cassette encoding the full-length *S*. *pombe* UDP-galactose 4-epimerase (*SpGALE*) under control of the *PpPMA1* promoter.

Strain RDP667-1 was transformed with plasmid pGLY167b to make strain RDP697-1. Plasmid pGLY 167b (*See* Figure 21) is a *P. pastoris arg1* knock-out plasmid that contains the *PpURA3* selectable marker. The plasmid contains an expression cassette encoding a secretory pathway targeted fusion protein (CO-KD53) comprising the first 36 amino acids of *Sc*Mnn2p (53) fused to *N*-terminus of the *Drosophila melanogaster* Mannosidase II catalytic domain (KD) under the control of *PpGAPDH* promoter and an expression cassette expressing a secretory pathway targeted fusion protein (CO-TC54) comprising the first 97 amino acids of ScMnn2p (54) fused to the *N*-terminus of the rat GIcNAc Transferase II catalytic domain under the control of the *PpPMA1* promoter. The nucleic acid molecules encoding the mannosidase II and GnT II catalytic domains were codon-optimized for expression in *Pichia pastoris* (SEQ ID NO:64 and 59, respectively). This strain can make glycoproteins that have *N*-glycans that have terminal galactose residues.

Strain RDP697-1 was transformed with plasmid pGLY510 to make strain YDX414. Plasmid pGLY510 (*See* Figure 23) is a roll-in plasmid that integrates into the P. *pastoris TRP2* locus while duplicating the gene and contains an *AOX1* promoter-*ScCYC1* terminator expression cassette as well as the *PpARG1* selectable marker.

Strain YDX414 was transformed with plasmid pDX459-1 (mAb-A) to make strain YDX458. Plasmid pDX459-1 *(See* Figure 24) is a roll-in plasmid that targets and integrates into the *P. pastoris AOX2* promoter and contains the ZeoR while duplicating the promoter. The plasmid contains separate expression cassettes encoding an anti-HER2 antibody heavy chain and an anti-HER2 antibody light chain (SEQ ID NOs:68 and 70, respectively), each fused at the *N*-terminus to the *Aspergillus niger* alpha-amylase signal sequence (SEQ ID NO:26) and controlled by the *P. pastoris AOX1* promoter. The nucleic acid sequences encoding the heavy and light chains are shown in SEQ ID NOs:67 and 69, respectively, and the nucleic acid sequence encoding the *Aspergillus niger* alpha-amylase signal sequence is shown in SEQ ID NO:25.

Strain YDX458 was transformed with plasmid pGLY1138 to make strain YDX477. Plasmid pGLY1138 *(See* Figure 25) is a roll-in plasmid that integrates into the *P. pastoris ADE1* locus while duplicating the gene. The plasmid contains a *ScARR3* selectable marker gene cassette. The *ARR3* gene from S. *cerevisiae* confers arsenite resistance to cells that are grown in the presence of arsenite (Bobrowicz et al., Yeast, 13:819-828 (1997); Wysocki et al., J. Biol. Chem. 272:30061-30066 (1997)). The plasmid contains an expression cassette encoding a secreted fusion protein comprising the *S. cerevisiae* alpha factor pre signal sequence (SEQ ID NO:24) fused to the *N*-terminus of the *Trichoderma reesei* (MNS1) catalytic domain (SEQ ID NO:56 encoded by the nucleotide sequence in SEQ ID NO:55) under the control of the *PpAOX1* promoter. The fusion protein is secreted into the culture medium.

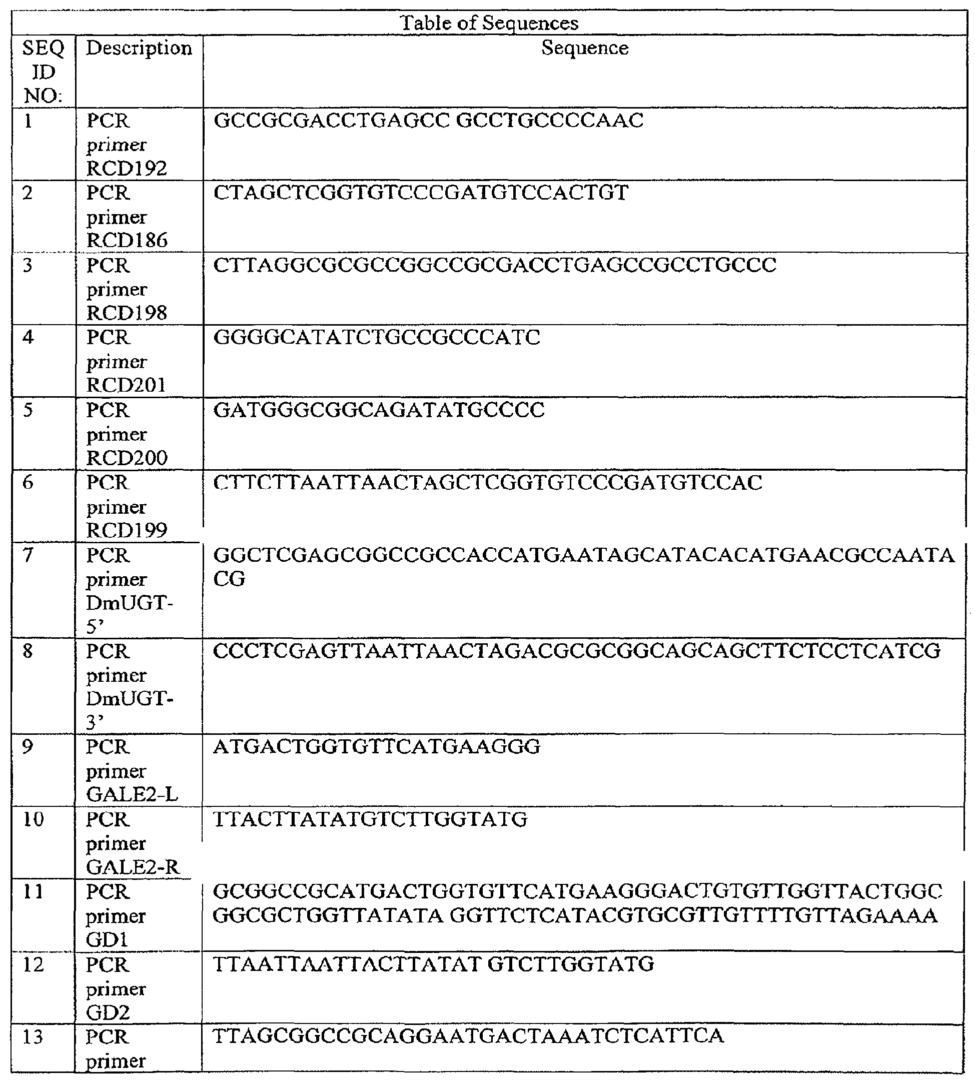

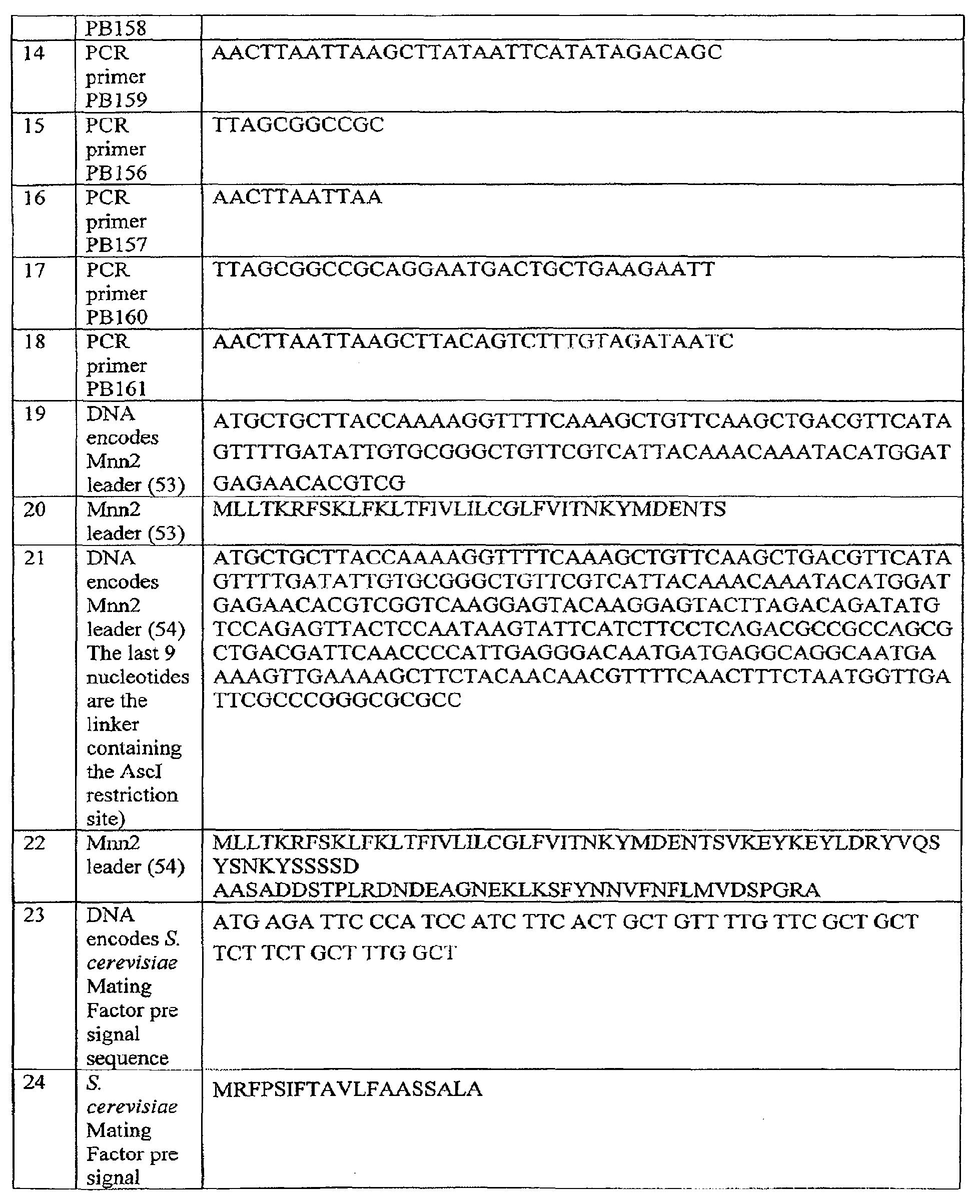

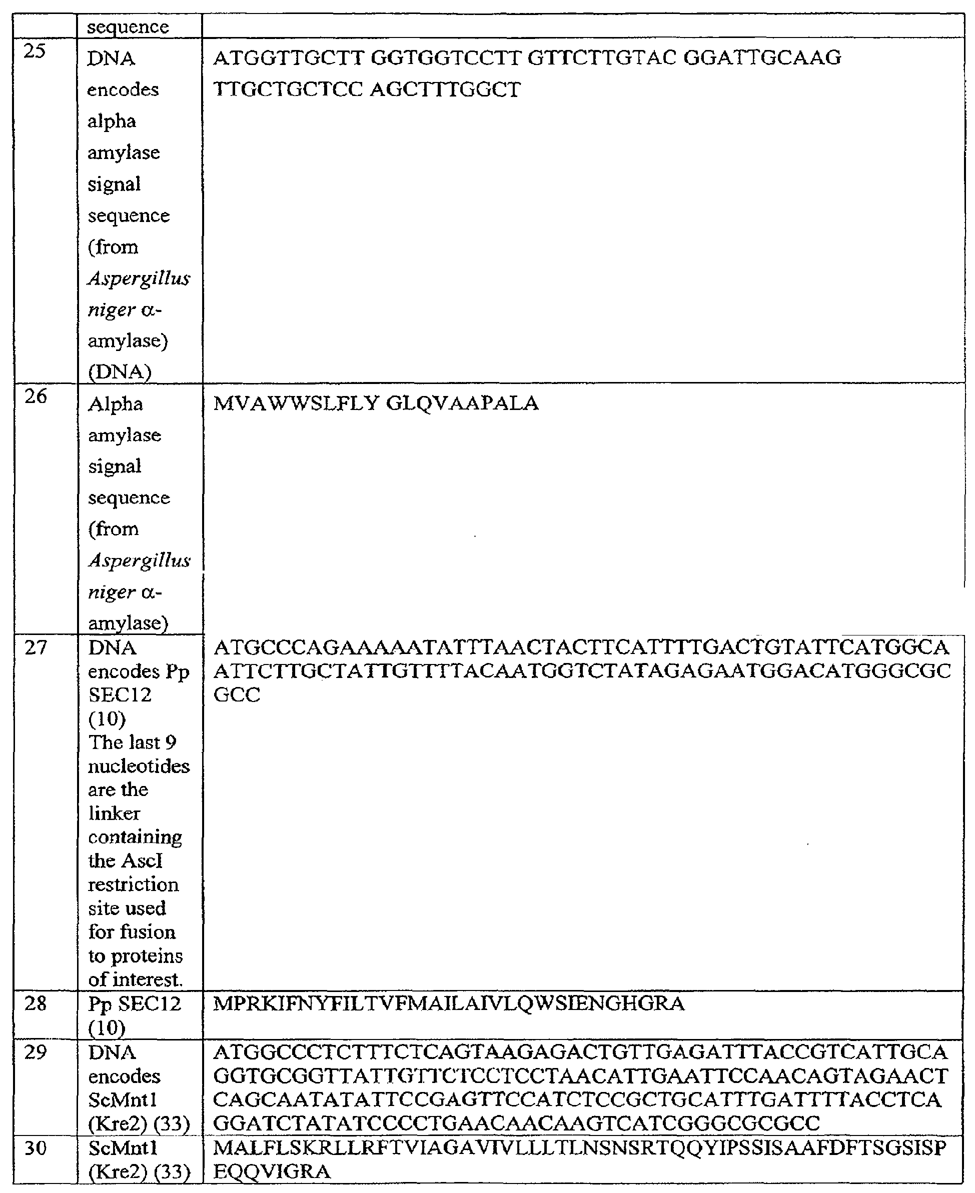

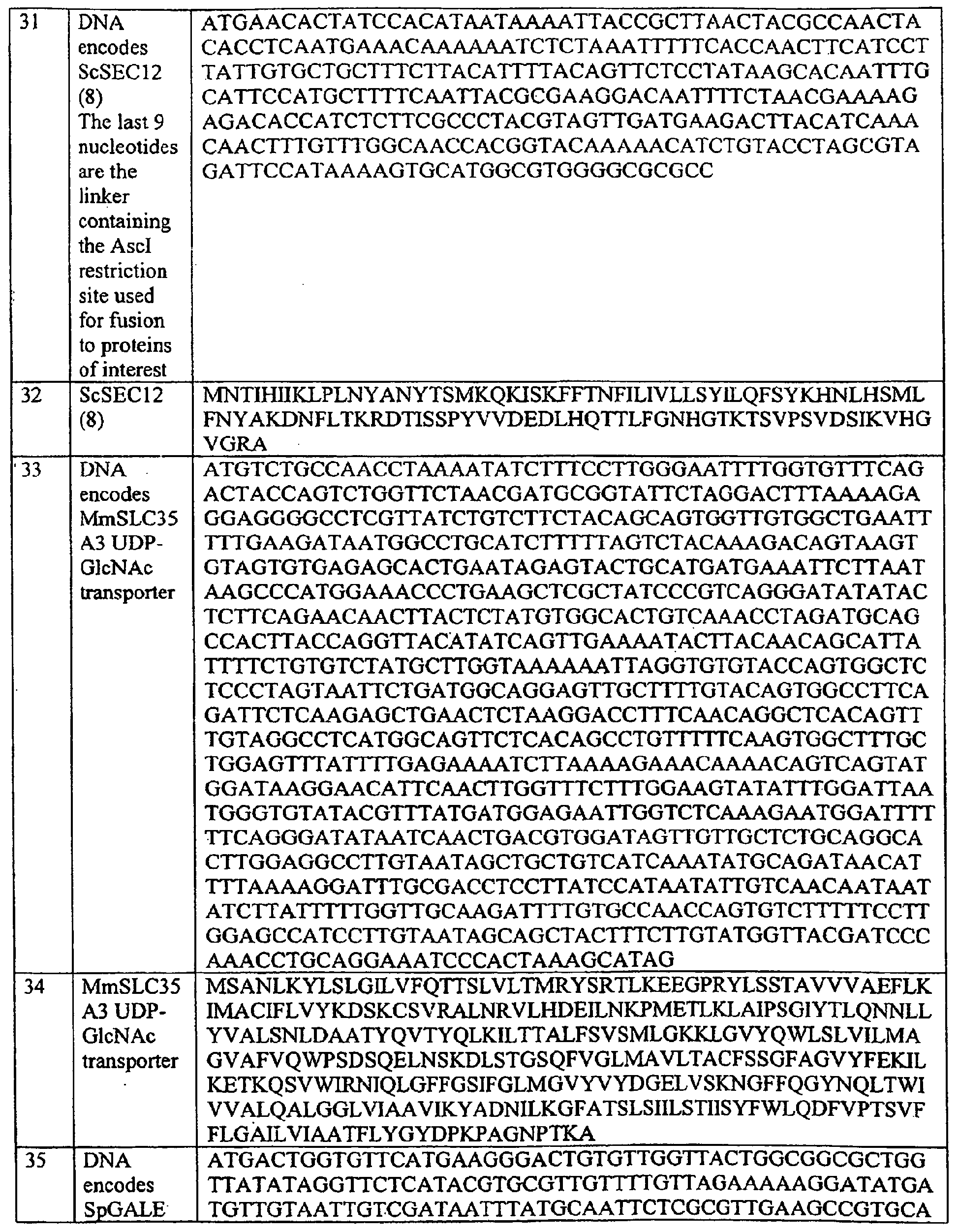

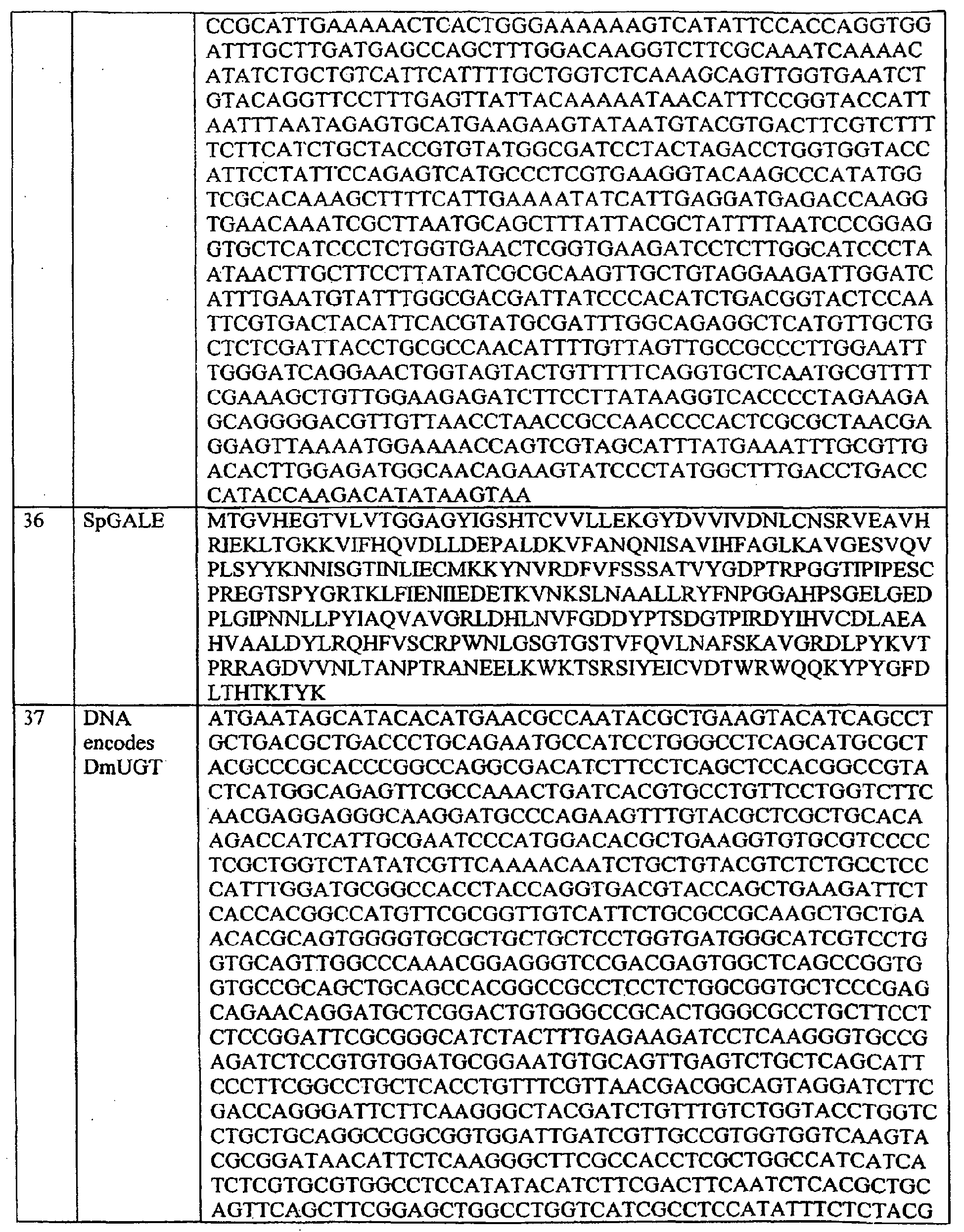

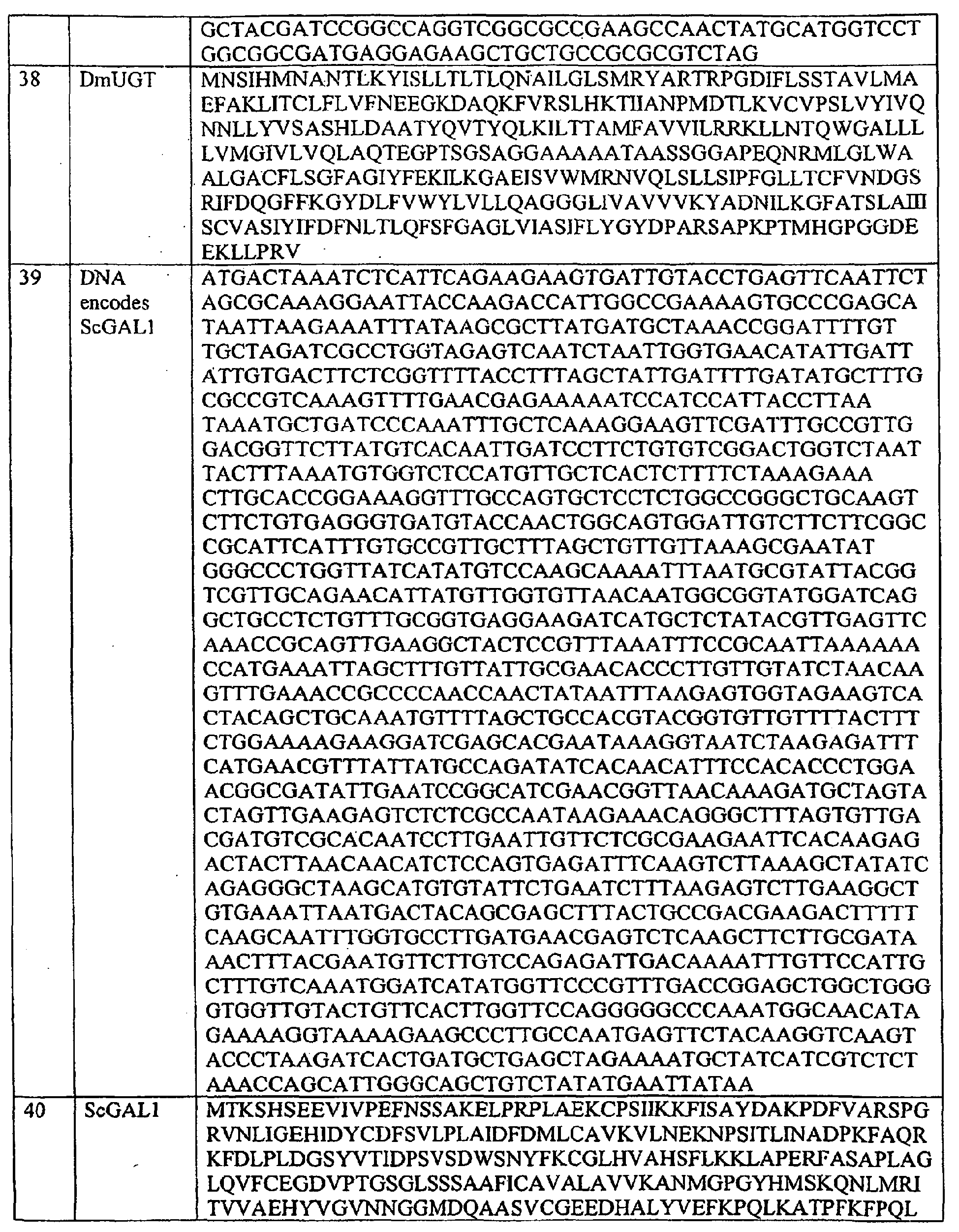

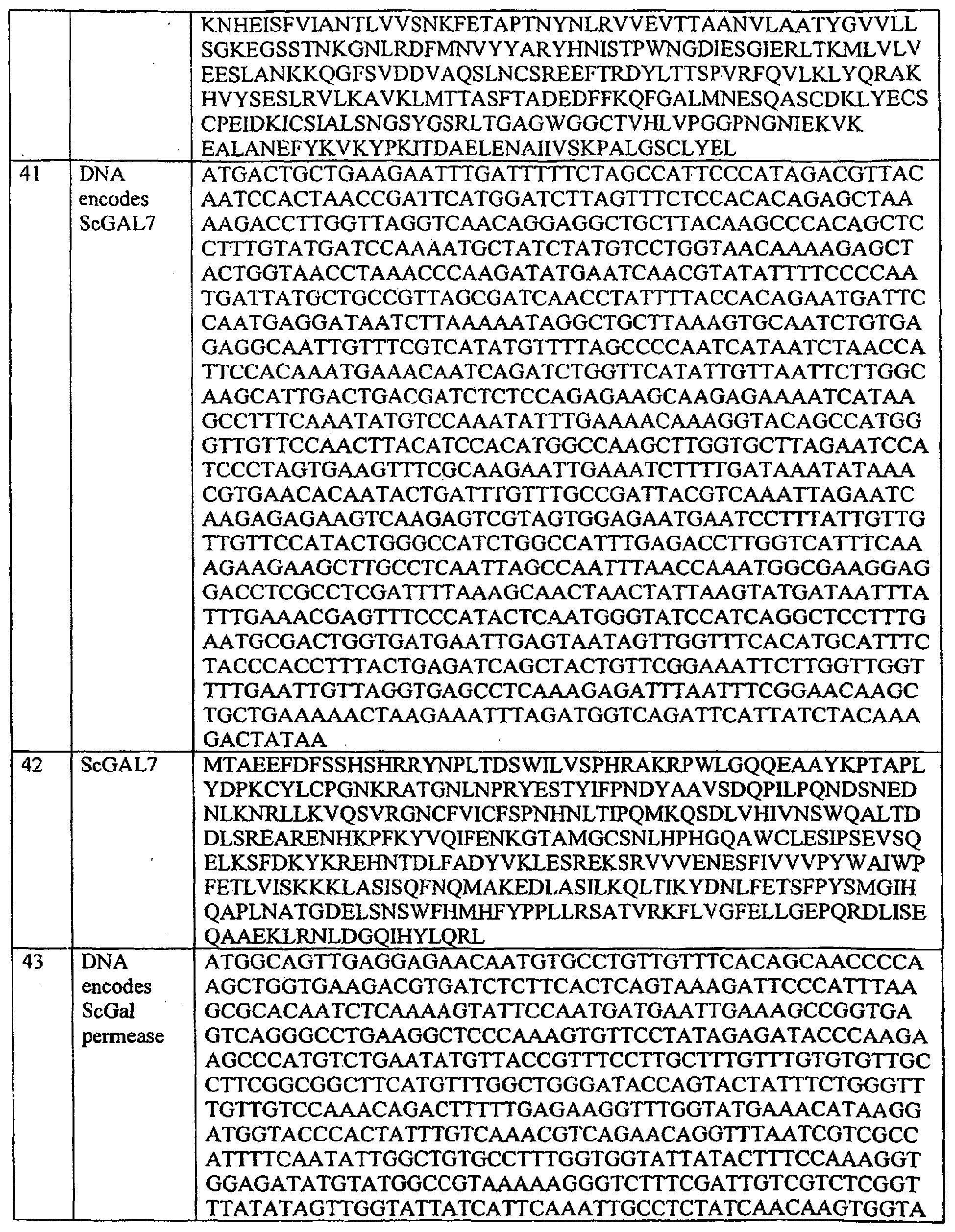

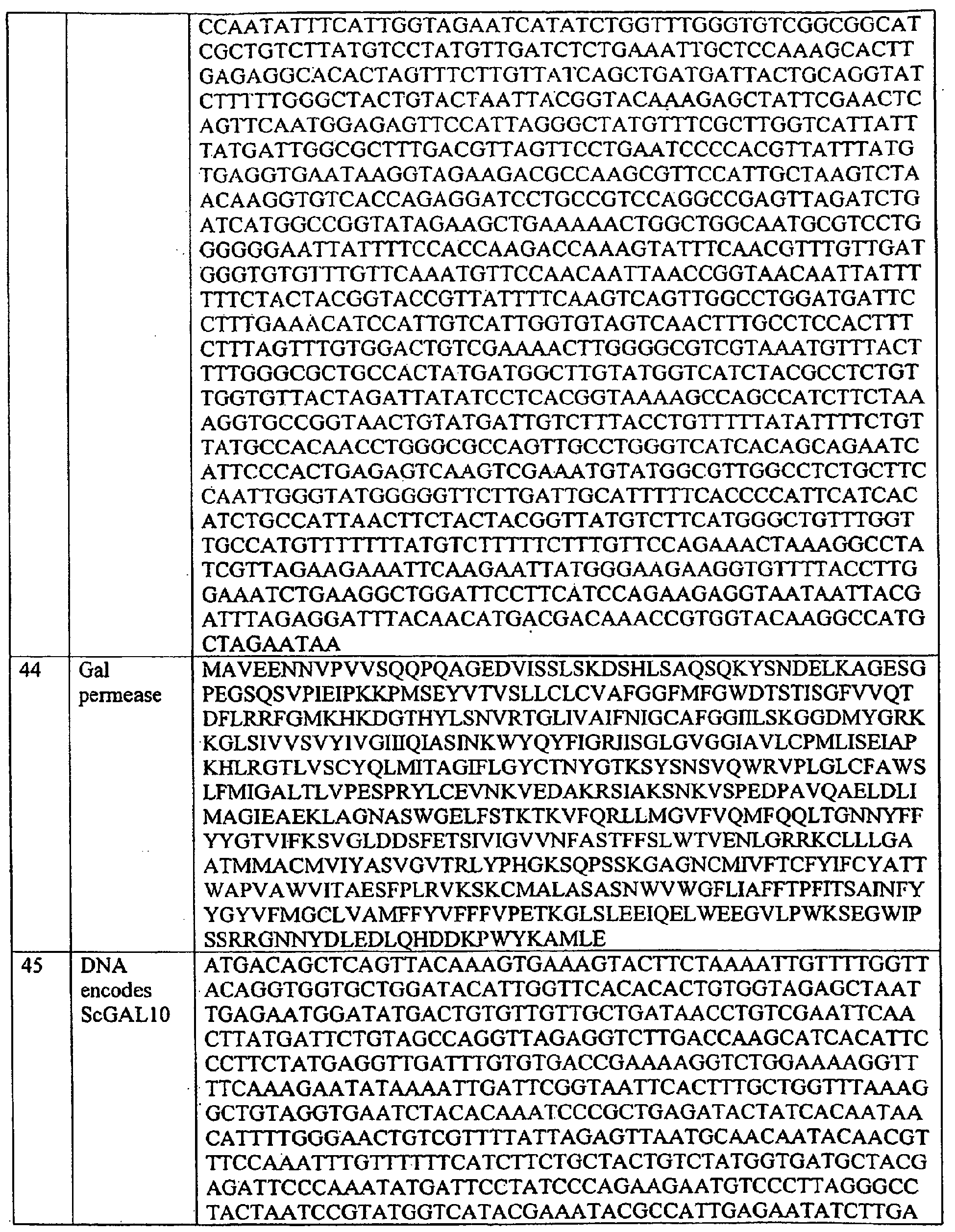

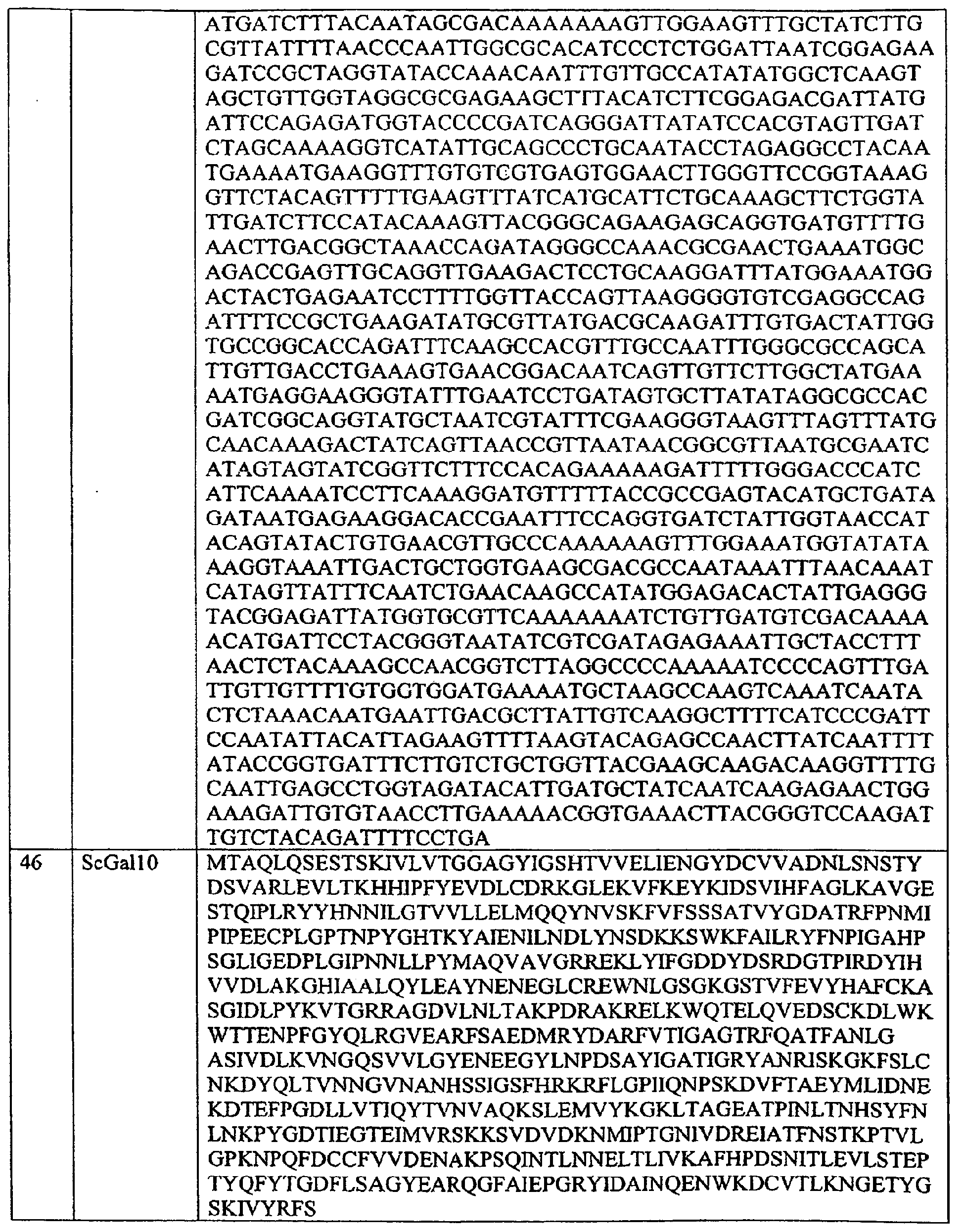

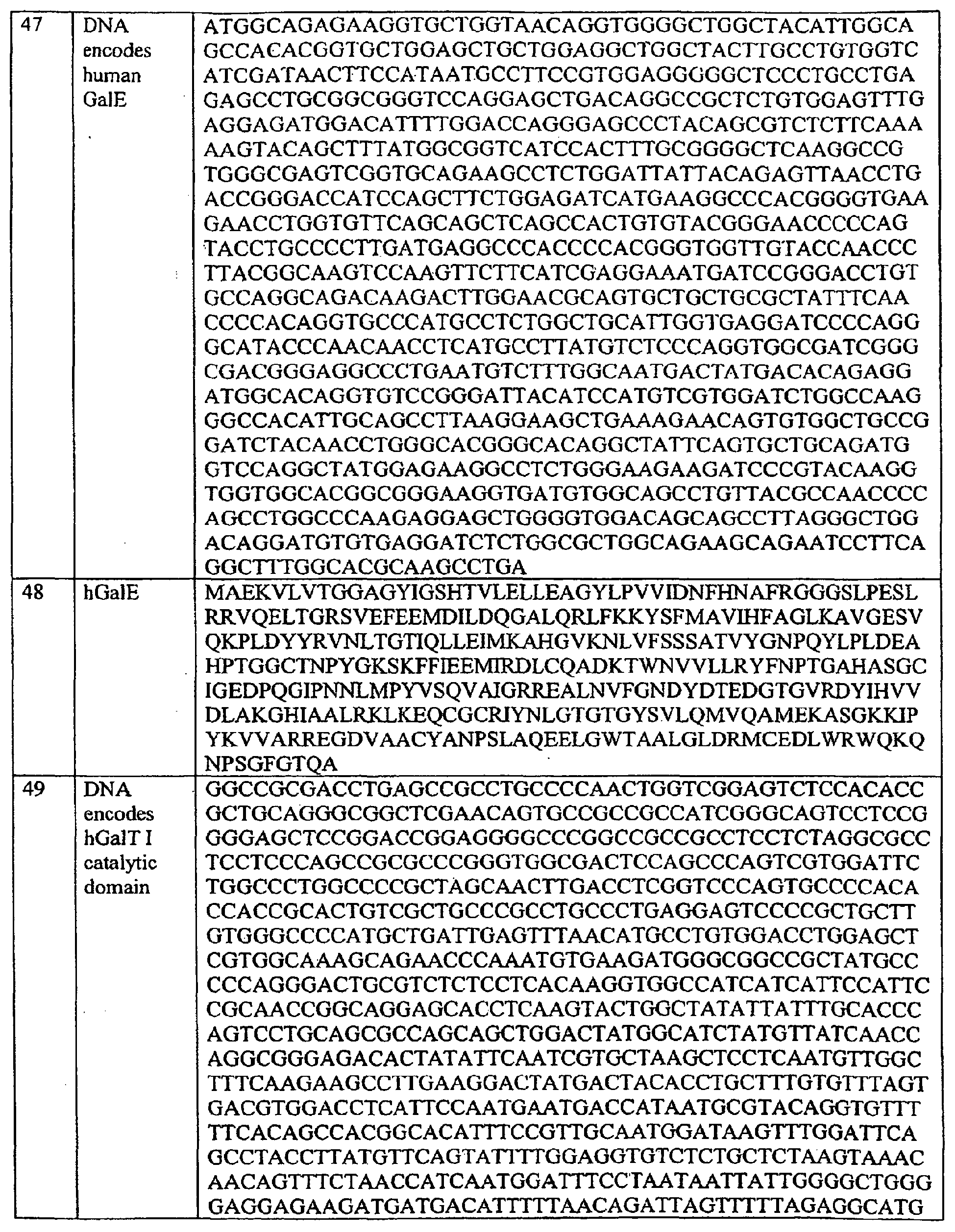

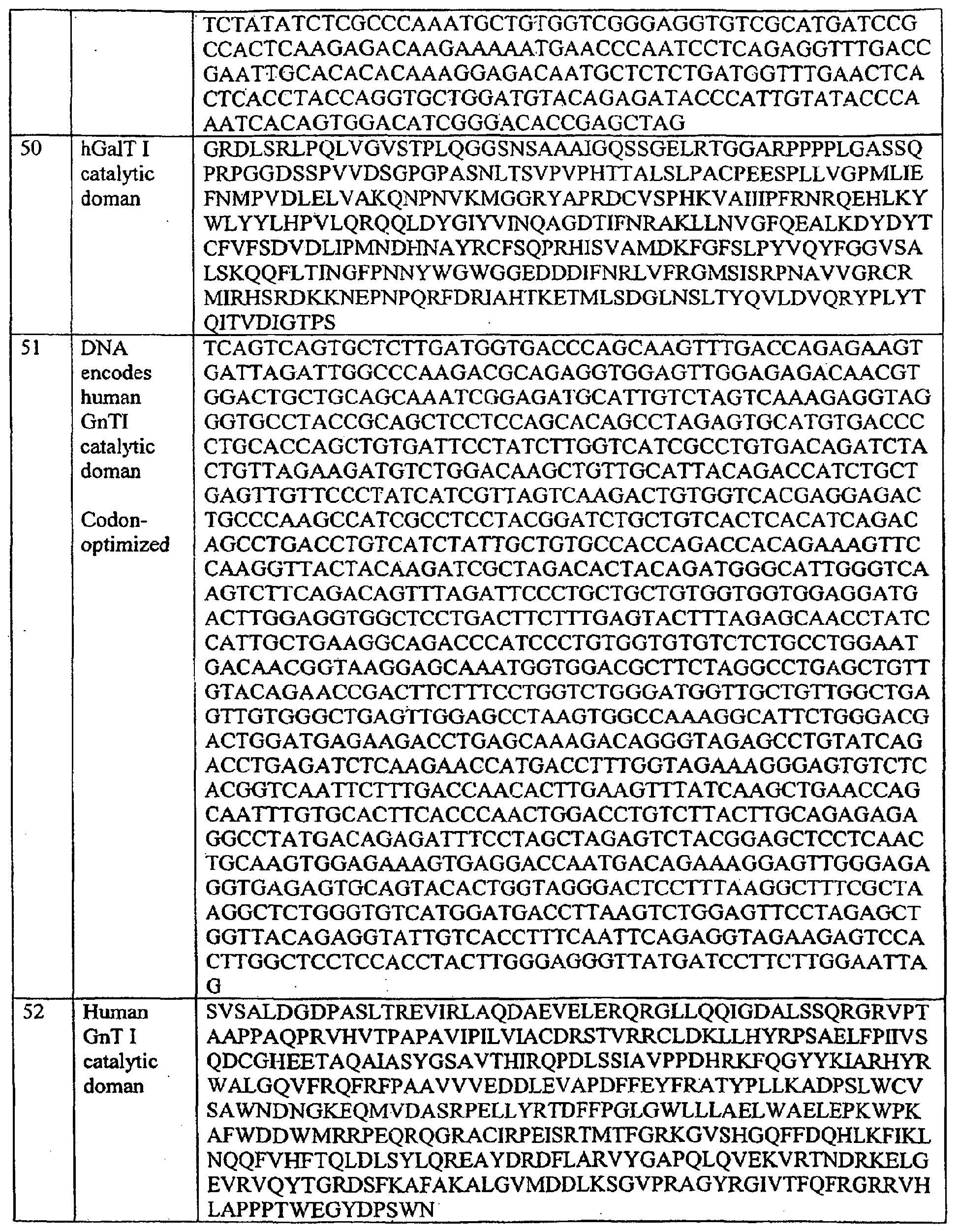

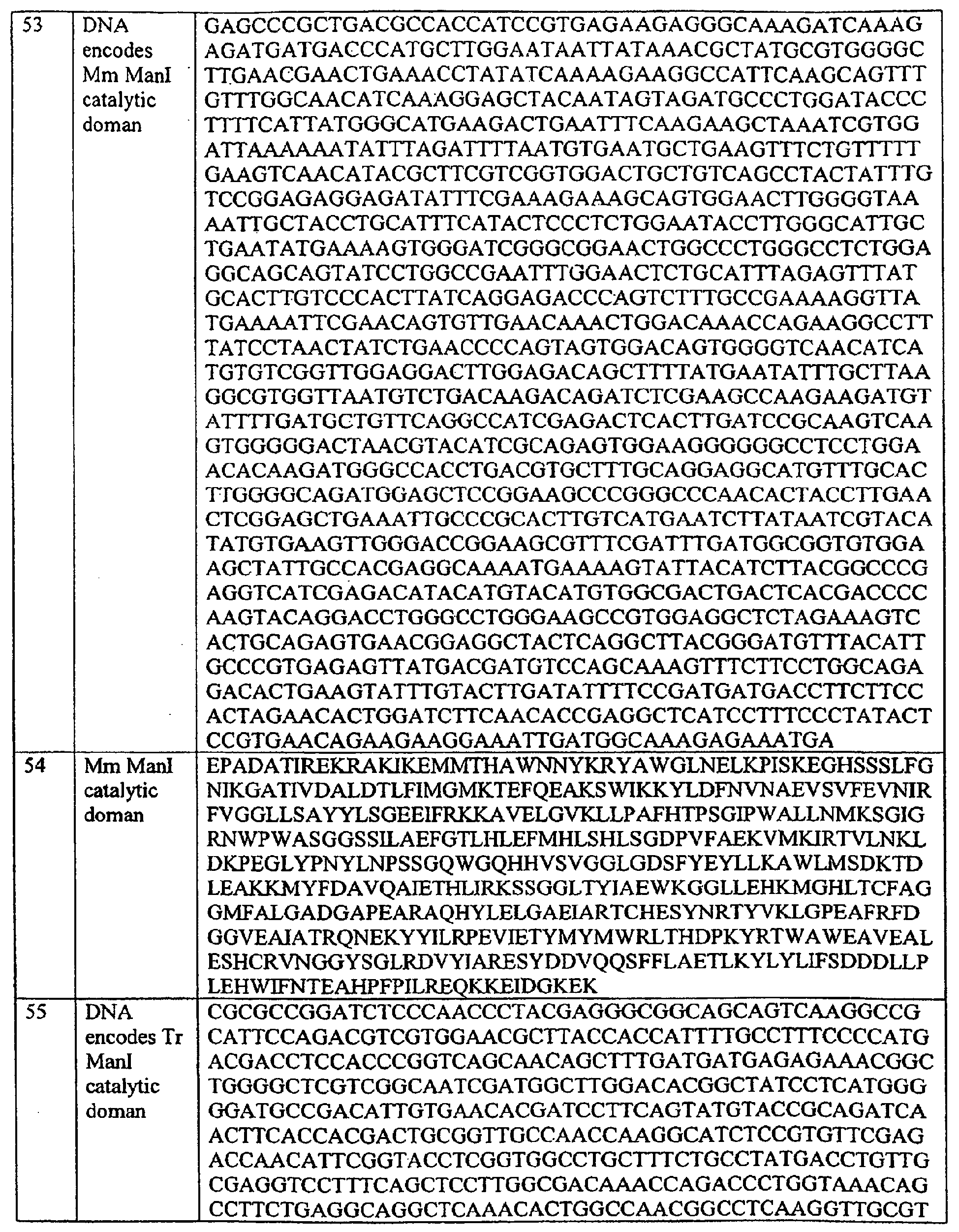

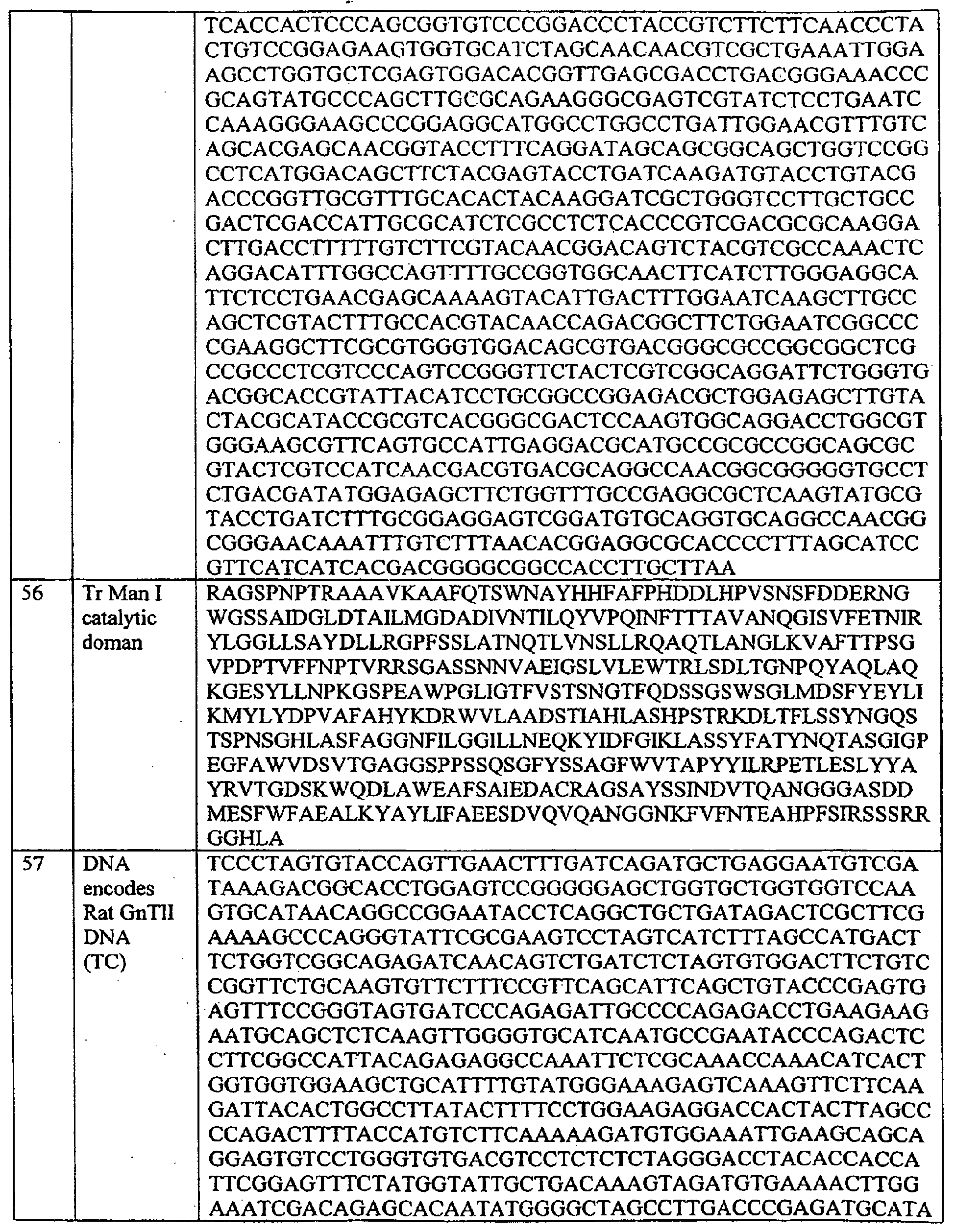

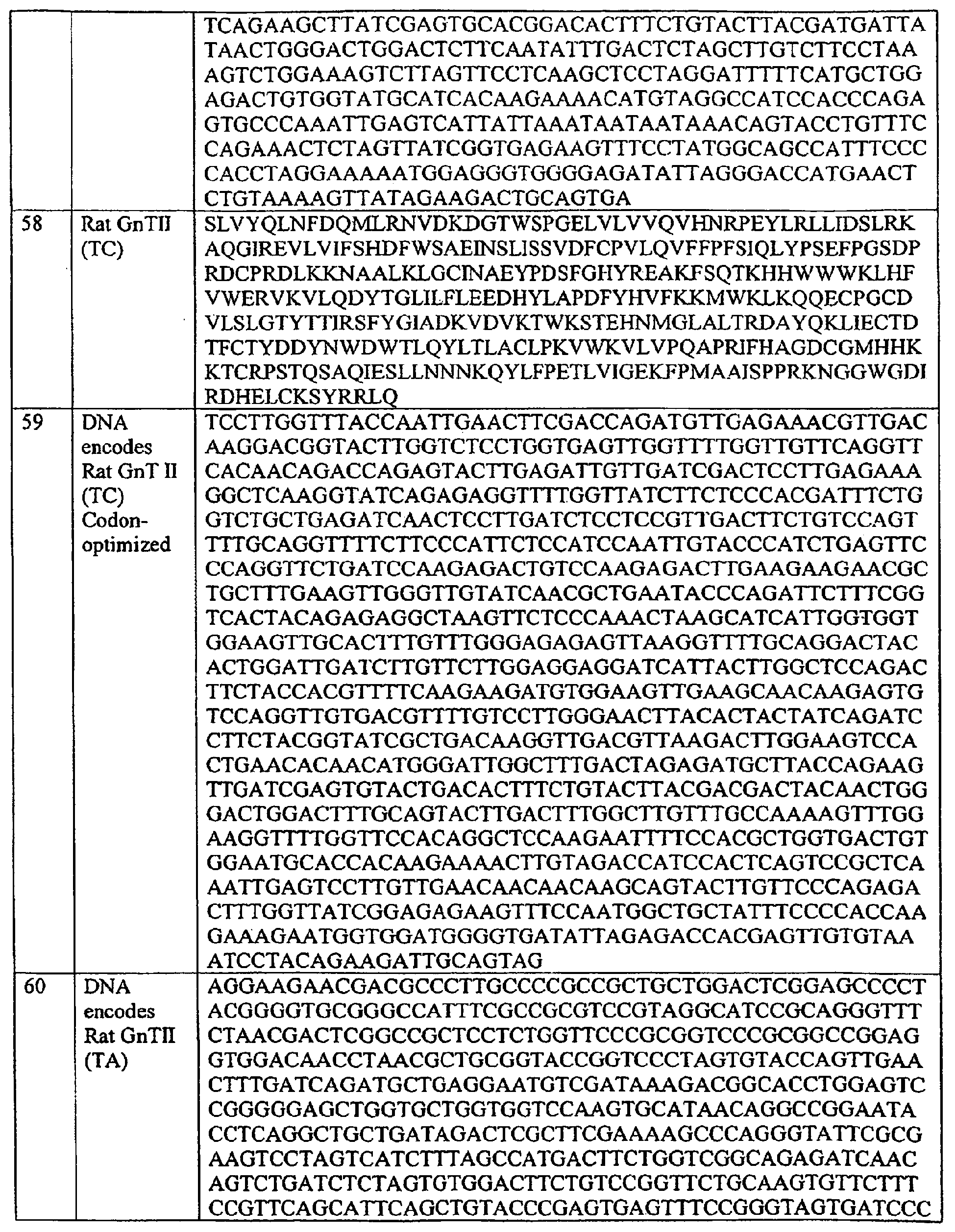

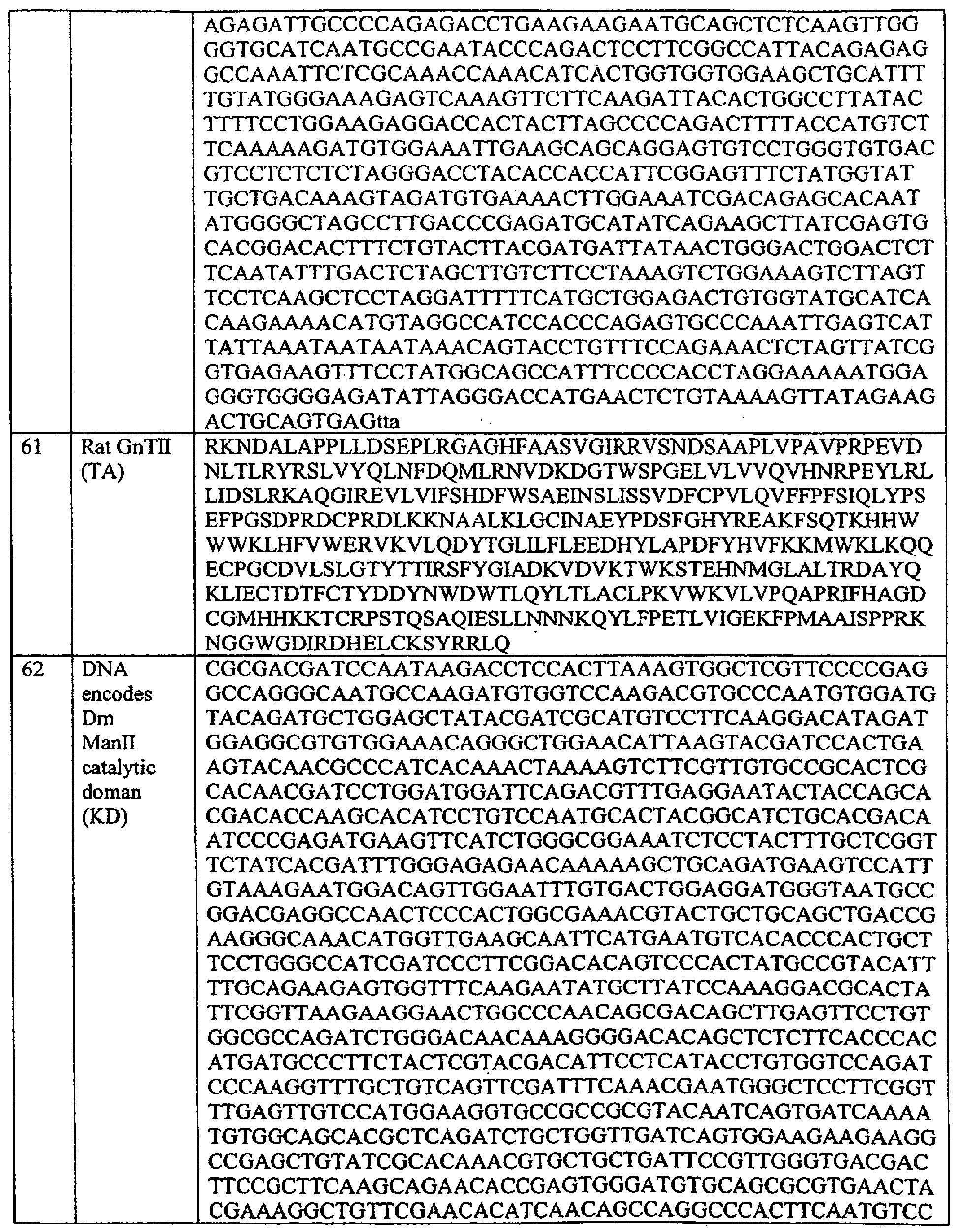

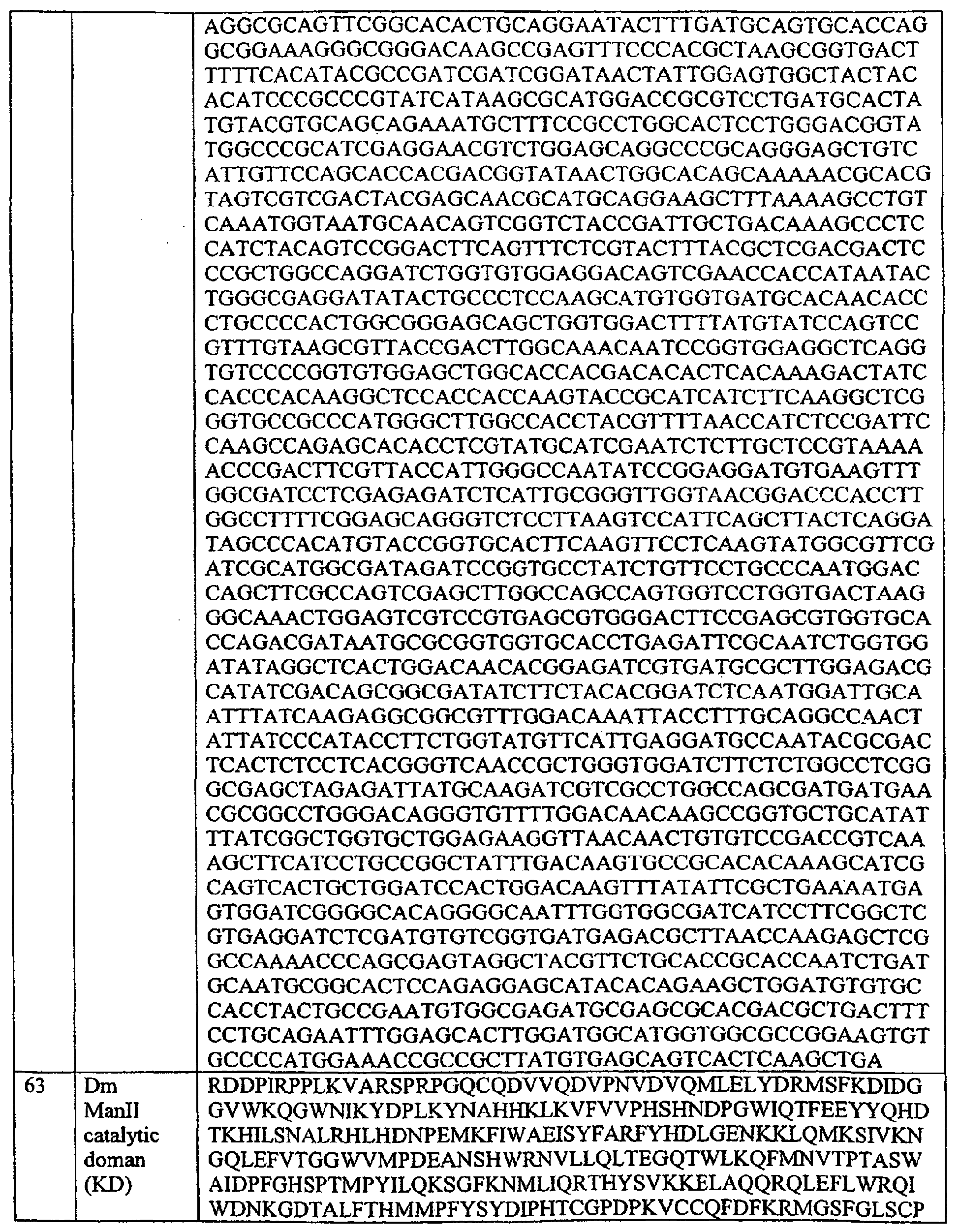

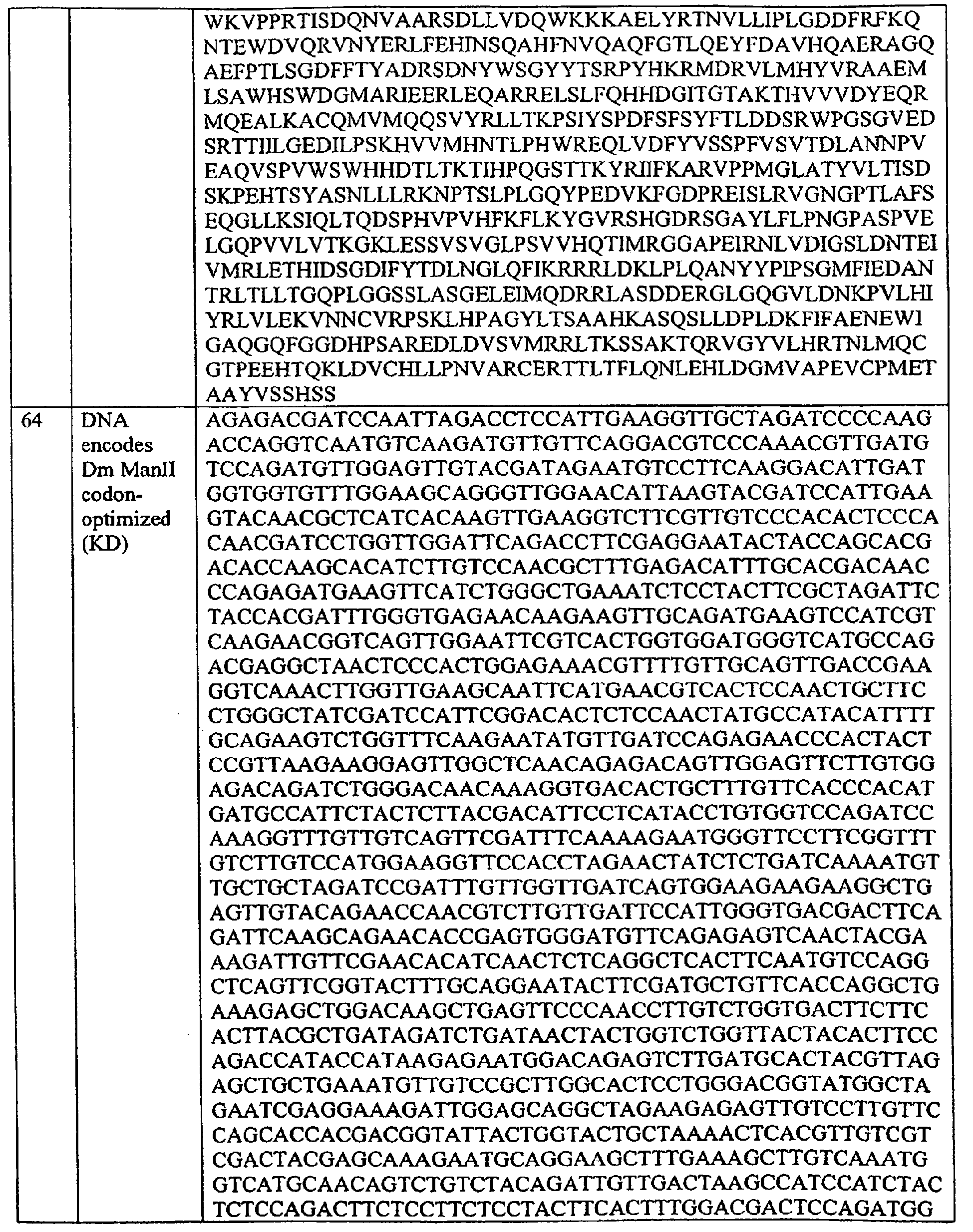

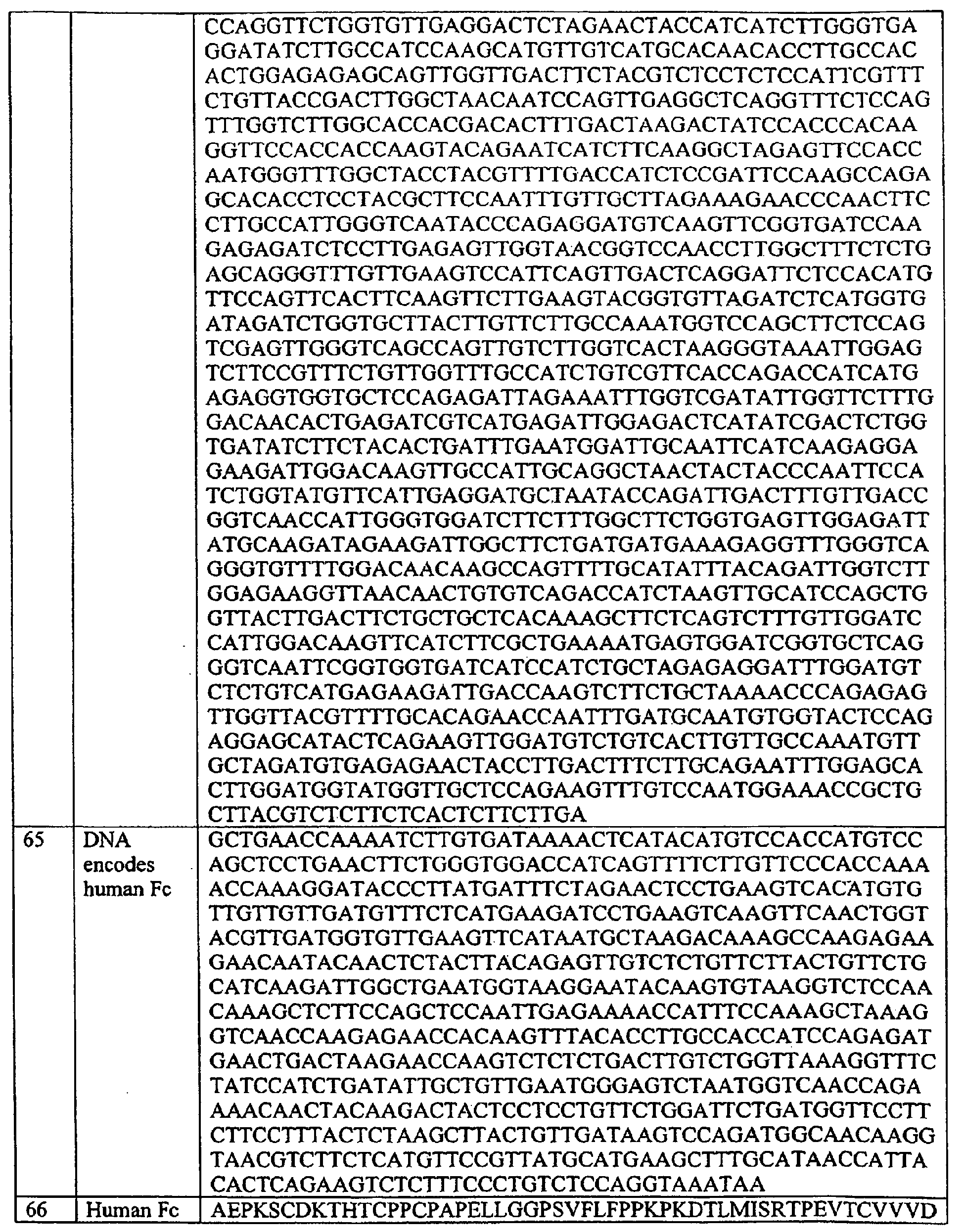

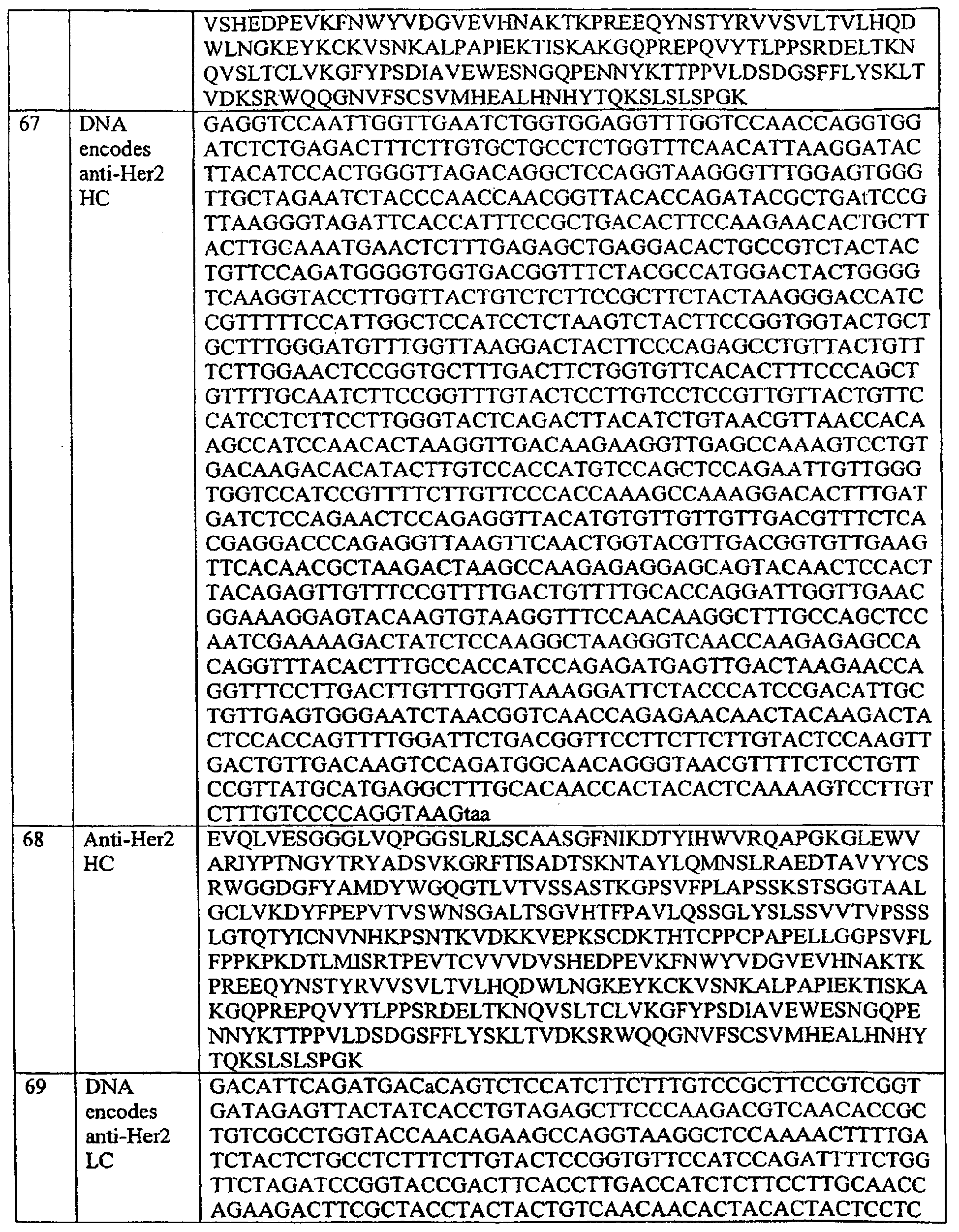

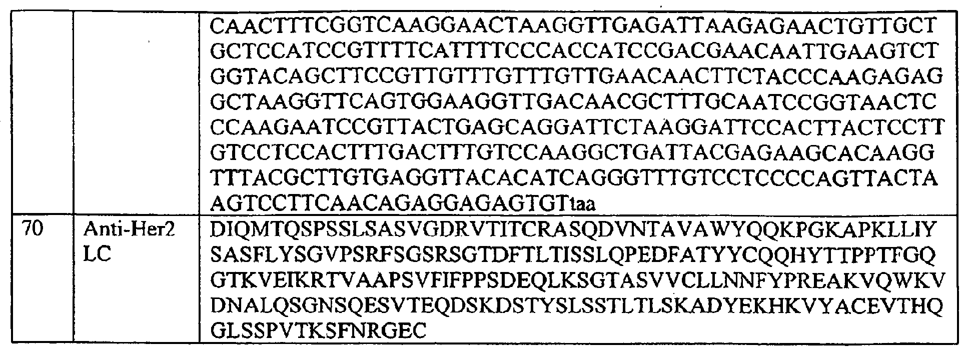

While the present invention is described herein with reference to illustrated embodiments, it should be understood that the invention is not limited hereto. Those having ordinary skill in the art and access to the teachings herein will recognize additional modifications and embodiments within the scope thereof. Therefore, the present invention is limited only by the claims attached herein.

## Claims

1. A *Pichia pastoris* host cell which has been genetically engineered to express a galactokinase activity, a UDP-galactose-4-epimerase activity, a galactose-1-phosphate uridyl transferase activity and optionally a galactose permease activity wherein the host cell is capable of using galactose as a sole carbon energy source.

2. The host cell of claim 1, wherein the host cell has been further engineered to be capable of producing recombinant glycoproteins that have hybrid or complex *N*-glycans that comprise galactose residues.

3. The host cell of claim 2 wherein the UDP-galactose-4-epimerase activity is provided in a fusion protein comprising the catalytic domain of a galactosyltransferase and the catalytic domain of an UDP-galactose-4-epimerase.

4. The host cell of claim 2, wherein the host cell produces glycoproteins that have complex *N*-glycans in which the G0:G1/G2 ratio is less than 2:1.

5. The host cell of claim 2, wherein the host cell produces glycoproteins having predominantly an *N*-glycan selected from the group consisting of GalGlcNAcMan₅GlcNAc₂; NANAGalGlcNAcMan₅GlcNAc₂; GalGlcNAcMan₃GlcNAc₂; NANAGalGlcNAcMan₃GlcNAc₂; GalGlcNAc₂Man₃GlcNAC₂; Gal₂GlcNAc₂Man₃GlcNAc₂; NANAGal₂GlcNAc₂Man₃GlcNAc₂; and NANA₂Gal₂GlcNAc₂Man₃GlcNAc₂.

6. The host cell of claim 2, wherein the *N*-glycan is a galactose-terminated *N-*glycan selected from the group consisting of GalGlcNAcMansGlcNAc_{2;} Gal₂GlcNAc₂Man₃GlcNAc₂; and Gal₂GlcNAc₂Man₃GlcNAc₂.

7. The host cell of claim 2, wherein the *N*-glycan is a galactose-terminated hybrid *N*-glycan.

8. The host cell of claim 2, wherein the *N*-glycan is a sialylated *N*-glycans selected from the group consisting of NANAGalGlcNAcMan₅GlcNAc₂₁ NANAGal₂GlcNAc₂Man₃GlcNAc₂; and NANA₂Gal₂GlcNAc₂Man₃GlcNAc₂.

9. The host cell of claim 2, wherein the recombinant glycoprotein is selected from the group consisting erythropoietin (EPO); cytokines such as interferon α, interferon β, interferon γ, and interferon ω; and granulocyte-colony stimulating factor (GCSF); GM-CSF; coagulation factors such as factor VIII, factor IX, and human protein C; antithrombin III; thrombin,; soluble IgE receptor α-chain; immunoglobulins such as IgG, IgG fragments, IgG fusions, and IgM; immunoadhesins and other Fc fusion proteins such as soluble TNF receptor-Fc fusion proteins; RAGE-Fc fusion proteins; interleukins; urokinase; chymase; and urea trypsin inhibitor; IGF-binding protein; epidermal growth factor; growth hormone-releasing factor; annexin V fusion protein; angiostatin; vascular endothelial growth factor-2; myeloid progenitor inhibitory factor-1; osteoprotegerin; α-1-antitrypsin; α-feto proteins; DNase II; kringle 3 of human plasminogen; glucocerebrosidase; TNF binding protein 1; follicle stimulating hormone; cytotoxic T lymphocyte associated antigen 4 - Ig; transmembrane activator and calcium modulator and cyclophilin ligand; glucagon like protein 1; and IL-2 receptor agonist.

10. A method of producing a recombinant glycoprotein in a *Pichia pastoris* host with *N*-glycans that have galactose residues, said method comprising;
a) providing a recombinant host cell that has been genetically engineered to express
(i) a glycosylation pathway that renders the host cell capable of producing recombinant glycoproteins that have hybrid or complex *N*-glycans that comprise galactose residues;
(ii) a galactokinase activity, a UDP-galactose-4-epimerase activity, a galactose-1-phosphate uridyl transferase activity, and optionally a galactose permease activity; and
(iii) a recombinant glycoprotein; and
b) culturing the host cells in a medium containing galactose to produce the recombinant glycoprotein that has one or more *N*-glycans that have galactose residues.

11. The method of claim 10 wherein the UDP-galactose-4-epimerase activity is provided in a fusion protein comprising the catalytic domain of a galactosyltransferase and the catalytic domain of an UDP-galactose-4-epimerase.

12. The method of claim 10, wherein the G0:G1/G2 ratio of the *N*-glycans is less than 2:1.

13. The method of claim 10, wherein the recombinant glycoprotein has predominantly an *N*-glycan selected from the group consisting of GalGlcNAcMan₅GlcNAc₂; NANAGalGlcNAcMansGlcNAc₂; GalGlcNAcMan₃GlcNAc₂; NANAGalGlcNAcMan₃GlcNAc₂; GalGlcNAc₂Man₃GlcNAc₂; Gal₂GlcNAc₂Man₃GlcNAc₂; NANAGal₂GlcNAc₂Man₃GlcNAc₂; and NANA₂Gal₂GlcNAc₂Man₃GlcNAc₂.

14. The method of claim 10, wherein the *N*-glycan is a galactose-terminated N-glycan selected from the group consisting of GalGlcNAcMan₅GlcNAc_{2;} Gal₂GlcNAc₂Man₃GlcNAc₂; and Gal₂GlcNAc₂Man₃GlcNAc₂.

15. The method of claim 10, wherein the *N*-glycan is a galactose-terminated hybrid *N*-glycan.

16. The method of claim 10, wherein the *N*-glycan is a sialylated *N*-glycans selected from the group consisting of NANAGalGlcNAcMan₅GlcNAc₂; NANAGal₂GlcNAc₂Man₃GlcNAc₂; and NANA₂Gal₂GlcNAc₂Man₃GlcNAc₂.

17. The method of claim 10, wherein the recombinant glycoprotein is selected from the group consisting erythropoietin (EPO); cytokines such as interferon α, interferon β, interferon γ, and interferon ω; and granulocyte-colony stimulating factor (GCSF); GM-CSF; coagulation factors such as factor VIII, factor IX, and human protein C; antithrombin III; thrombin,; soluble IgE receptor α-chain; immunoglobulins such as IgG, IgG fragments, IgG fusions, and IgM; immunoadhesins and other Fc fusion proteins such as soluble TNF receptor-Fc fusion proteins; RAGE-Fc fusion proteins; interleukins; urokinase; chymase; and urea trypsin inhibitor; IGF-binding protein; epidermal growth factor; growth hormone-releasing factor; annexin V fusion protein; angiostatin; vascular endothelial growth factor-2; myeloid progenitor inhibitory factor-1; osteoprotegerin; α-1-antitrypsin; α-feto proteins; DNase II; kringle 3 of human plasminogen; glucocerebrosidase; TNF binding protein 1; follicle stimulating hormone; cytotoxic T lymphocyte associated antigen 4 - Ig; transmembrane activator and calcium modulator and cyclophilin ligand; glucagon like protein 1; and IL-2 receptor agonist.

18. A method for producing a recombinant *Pichia pastoris* host cell that expresses a heterologous protein, comprising:
(a) providing a host cell that has been genetically engineered to express one or two enzyme activities selected from the group consisting of galactokinase activity, UDP-galactose-4-epimerase activity, and galactose-1-phosphate uridyl transferase activity;
(b) transforming the host cell with one or more nucleic acid molecules encoding the heterologous protein; and with one or more nucleic acid molecules encoding the enzyme or enzymes from the group in step (a) that are not expressed in the host cell of step (a) so that all three enzymes from the group in step (a) are present in the host cell; and
(c) culturing the host cells in a medium containing galactose as the sole carbon source to provide the recombinant *Pichia pastoris* host cell that expresses a heterologous protein.

19. The method of claim 18, wherein the host cell is further genetically engineered to express a galactose permease.

20. The method of claim 18, wherein the host cell is genetically modified to produce glycoproteins that have one or more *N*-glycans that comprise galactose.

21. A method of producing and selecting *Pichia pastoris* host cells capable of using galactose as a sole carbon source, the method comprising;
a) providing a *Pichia pastoris* host cell;
b) transforming the host cell with one or more nucleic acid molecules encoding a galactokinase activity, a UDP-galactose-4-epimerase activity, a galactose-1-phosphate uridyl transferase activity, and optionally a galactose permease;
c) culturing the transformed host cells of on a medium containing galactose as the sole carbon source; and
d) selecting the host cells that can grow on the medium containing galactose as the sole carbon source.

## Patentansprüche

1. Eine *Pichia-pastoris*-Wirtszelle, die gentechnisch derart verändert wurde, dass sie eine Galactokinase-Aktivität, eine UDP-Galactose-4-Epimerase-Aktivität, eine Galactose-1-phosphat-Uridyltransferase-Aktivität und gegebenenfalls eine Galactose-Permease-Aktivität exprimiert, wobei die Wirtszelle in der Lage ist, Galactose als einzige Kohlenstoffenergiequelle zu nutzen.

2. Die Wirtszelle nach Anspruch 1, wobei die Wirtszelle ferner derart verändert wurde, dass sie rekombinante Glycoproteine erzeugen kann, die Hybrid- oder komplexe *N-*Glykane besitzen, die Galactosereste umfassen.

3. Die Wirtszelle nach Anspruch 2, wobei die UDP-Galactose-4-Epimerase-Aktivität in einem Fusionsprotein zur Verfügung gestellt wird, das die katalytische Domäne einer Galactosyltransferase und die katalytische Domäne einer UDP-Galactose-4-Epimerase umfasst.

4. Die Wirtszelle nach Anspruch 2, wobei die Wirtszelle Glycoproteine erzeugt, die komplexe *N*-Glykane besitzen, wobei das G0:G1/G2-Verhältnis weniger als 2:1 beträgt.

5. Die Wirtszelle nach Anspruch 2, wobei die Wirtszelle Glycoproteine mit überwiegend einem *N*-Glykan erzeugt, ausgewählt aus der Gruppe, bestehend aus GalGlcNAcMan₅GlcNAc₂, NANAGalGlcNAcMan₅GlcNAc₂, GalGlcNAcMan₃GlcNAC₂, NANAGalGlcNAcMan₃GlcNAc₂, GalGlcNAc₂Man₃GlcNAc₂, Gal₂GlcNAC₂Man₃GlcNAc₂, NANAGal₂GlcNAc₂Man₃GlcNAc₂ und NANA₂Gal₂GlcNAc₂Man₃GlcNAc₂.

6. Die Wirtszelle nach Anspruch 2, wobei das *N*-Glykan ein galactoseterminiertes *N-*Glykan ist, ausgewählt aus der Gruppe, bestehend aus GalGlcNAcMan₅GlcNAc₂, Gal₂GlcNAc₂Man₃GlcNAc₂ und Gal₂GlcNAc₂Man₃GlcNAc₂.

7. Die Wirtszelle nach Anspruch 2, wobei das *N*-Glykan ein galactoseterminiertes Hybrid-*N*-Glykan ist.

8. Die Wirtszelle nach Anspruch 2, wobei das *N*-Glykan ein sialyliertes *N*-Glykan ist, ausgewählt aus der Gruppe, bestehend aus NANAGalGlcNAcMan₅GlcNAc₂, NANAGal₂GlcNAc₂Man₃GlcNAc₂ und NANA₂Gal₂GlcNAc₂Man₃GlcNAc₂.

9. Die Wirtszelle nach Anspruch 2, wobei das rekombinante Glycoprotein ausgewählt ist aus der Gruppe, bestehend aus Erythropoietin (EPO), Zytokinen, wie z.B. Interferon α, Interferon β, Interferon γ und Interferon ω; und Granulozyten-Kolonie-stimulierendem Faktor (GCSF); GM-CSF; Koagulationsfaktoren, wie z.B. Faktor VIII, Faktor IX und menschlichem Protein C; Antithrombin III; Thrombin; löslicher IgE-Rezeptor-α-Kette; Immunglobulinen, wie z.B. IgG, IgG-Fragmente, IgG-Fusionen und IgM; Immunadhäsinen und anderen Fc-Fusionsproteinen, wie z.B. lösliche TNF-Rezeptor-Fc-Fusionsproteine; RAGE-Fc-Fusionsproteinen; Interleukinen; Urokinase; Chymase und Harnstofftrypsin-Inhibitor; IgF-Bindungsprotein; epidermalem Wachstumsfaktor; Wachstumshormon-freisetzendem Faktor; Annexin-V-Fusionsprotein; Angiostatin; vaskulärem endothelialem Wachstumsfaktor 2; Myeloid-Progenitor-Inhibitor-Faktor 1; Osteoprotegerin; α-1-Antitrypsin, α-Feto-Proteine; DNase II; Kringle 3 von humanem Plasminogen; Glucocerebrosidase; TNF-Bindungsprotein 1; Follikelstimulationshormon; zytotoxischem T-Lymphozyten-assoziiertem Antigen 4 - Ig; Transmembranaktivator und Calciummodulator und Cyclophilin-Ligand; Glucagon-ähnlichem Protein 1 und IL-2-Rezeptor-Agonist.

10. Ein Verfahren zur Erzeugung eines rekombinanten Glycoproteins in einem *Pichia-pastoris-*Wirt mit *N*-Glykanen, die Galactosereste besitzen, wobei das Verfahren umfasst:
a) Bereitstellen einer rekombinanten Wirtszelle, die gentechnisch derart verändert wurde, dass sie
(i) einen Glycosylierungsweg, der die Wirtszelle in die Lage versetzt, rekombinante Glycoproteine zu erzeugen, die Hybrid- oder komplexe *N*-Glykane besitzen, die Galactosereste umfassen,
(ii) eine Galactokinase-Aktivität, eine UDP-Galactose-4-Epimerase-Aktivität, eine Galactose-1-phosphat-Uridyltransferase-Aktivität und gegebenenfalls eine Galactose-Permease-Aktivität, und
(iii) ein rekombinantes Glycoprotein exprimieren, und
b) Kultivieren der Wirtszellen in einem Medium, das Galactose enthält, um das rekombinante Glycoprotein zu erzeugen, das ein oder mehrere *N*-Glykane besitzt, welche Galactosereste besitzen.

11. Das Verfahren nach Anspruch 10, wobei die UDP-Galactose-4-Epimerase-Aktivität in einem Fusionsprotein zur Verfügung gestellt wird, das die katalytische Domäne einer Galactosyltransferase und die katalytische Domäne einer UDP-Galactose-4-Epimerase umfasst.

12. Das Verfahren nach Anspruch 10, wobei das G0:G1/G2-Verhältnis der *N-*Glykane weniger als 2:1 beträgt.

13. Das Verfahren nach Anspruch 10, wobei das rekombinante Glycoprotein überwiegend ein *N*-Glykan besitzt, ausgewählt aus der Gruppe, bestehend aus GalGlcNAcMan₅GlcNAc₂, NANAGalGlcNAcMan₅GlcNAc₂, GalGlcNAcMan₃GlcNAc₂, NANAGalGlcNAcMan₃GlcNAc₂, GalGlcNAc₂Man₃GlcNAc₂, Gal₂GlcNAc₂Man₃GlcNAc₂, NANAGal₂GlcNAc₂Man₃GlcNAc₂ und NANA₂Gal₂GlcNAc₂Man₃GlcNAc₂.

14. Das Verfahren nach Anspruch 10, wobei das *N*-Glykan ein galactoseterminiertes *N*-Glykan ist, ausgewählt aus der Gruppe, bestehend aus GalGlcNAcMan₅GlcNAc₂, Gal₂GlcNAc₂Man₃GlcNAc₂ und Gal₂GlcNAc₂Man₃GlcNAc₂.

15. Das Verfahren nach Anspruch 10, wobei das *N*-Glykan ein galactoseterminiertes Hybrid-*N*-Glykan ist.

16. Das Verfahren nach Anspruch 10, wobei das *N*-Glykan ein sialyliertes *N*-Glykan ist, ausgewählt aus der Gruppe, bestehend aus NANAGalGlcNAcMan₅GlcNAc₂, NANAGal₂GlcNAc₂Man₃GlcNAc₂ und NANA₂Gal₂GlcNAc₂Man₃GlcNAc₂.

17. Das Verfahren nach Anspruch 10, wobei das rekombinante Glycoprotein ausgewählt ist aus der Gruppe, bestehend aus Erythropoietin (EPO), Zytokinen, wie z.B. Interferon α, Interferon β, Interferon γ und Interferon ω; und Granulozyten-Kolonie-stimulierendem Faktor (GCSF); GM-CSF; Koagulationsfaktoren, wie z.B. Faktor VIII, Faktor IX und menschlichem Protein C; Antithrombin III; Thrombin; löslicher IgE-Rezeptor-α-Kette; Immunglobulinen, wie z.B. IgG, IgG-Fragmente, IgG-Fusionen und IgM; Immunadhäsinen und anderen Fc-Fusionsproteinen, wie z.B. lösliche TNF-Rezeptor-Fc-Fusionsproteine; RAGE-Fc-Fusionsproteinen; Interleukinen; Urokinase; Chymase und Harnstofftrypsin-Inhibitor; IgF-Bindungsprotein; epidermalem Wachstumsfaktor; Wachstumshormon-freisetzendem Faktor; Annexin-V-Fusionsprotein; Angiostatin; vaskulärem endothelialem Wachstumsfaktor 2; Myeloid-Progenitor-Inhibitor-Faktor 1; Osteoprotegerin; α-1-Antitrypsin, α-Feto-Proteinen; DNase II; Kringle 3 von humanem Plasminogen; Glucocerebrosidase; TNF-Bindungsprotein 1; Follikelstimulationshormon; zytotoxischem T-Lymphozyten-assoziiertem Antigen 4 - Ig; Transmembranaktivator und Calciummodulator und Cyclophilin-Ligand; Glucagon-ähnlichem Protein 1 und IL-2-Rezeptor-Agonist.

18. Ein Verfahren zur Erzeugung einer rekombinanten *Pichia-pastoris*-Wirtszelle, die ein heterologes Protein exprimiert, umfassend:
(a) Bereitstellen einer Wirtszelle, die gentechnisch derart verändert wurde, dass sie eine oder zwei Enzym-Aktivitäten, ausgewählt aus der Gruppe, bestehend aus Galactokinase-Aktivität, UDP-Galactose-4-Epimerase-Aktivität und Galactose-1-phosphat-Uridyltransferase-Aktivität, exprimiert,
(b) Transformieren der Wirtszelle mit einem oder mehreren Nukleinsäuremolekülen, die das heterologe Protein kodieren, und mit einem oder mehreren Nukleinsäuremolekülen, die das Enzym oder die Enzyme aus der Gruppe in Schritt (a) kodieren, die nicht in der Wirtszelle von Schritt (a) exprimiert werden, so dass alle drei Enzyme aus der Gruppe in Schritt (a) in der Wirtszelle vorliegen, und
(c) Kultivieren der Wirtszellen in einem Medium, das Galactose als die einzige Kohlenstoffquelle enthält, um die rekombinante Pichia-pastoris-Wirtszelle zu erzeugen, die ein heterologes Protein exprimiert.

19. Das Verfahren nach Anspruch 18, wobei die Wirtszelle gentechnisch derart verändert ist, dass eine Galactose-Permease exprimiert wird.

20. Das Verfahren nach Anspruch 18, wobei die Wirtszelle gentechnisch derart verändert ist, dass sie Glycoproteine erzeugt, die ein oder mehrere *N*-Glykane besitzt, die Galactose umfassen.

21. Ein Verfahren zur Erzeugung und Auswahl von *Pichia-pastoris*-Wirtszellen, die in der Lage sind, Galactose als einzige Kohlenstoffquelle zu nutzen, wobei das Verfahren umfasst:
a) Bereitstellen einer *Pichia-pastoris*-Wirtszelle,
b) Transformieren der Wirtszelle mit einem oder mehreren Nukleinsäuremolekülen, die eine Galactokinase-Aktivität, eine UDP-Galactose-4-Epimerase-Aktivität, eine Galactose-1-phosphat-Uridyltransferase-Aktivität und gegebenenfalls eine Galactose-Permease kodieren,
c) Kultivieren der transformierten Wirtszellen auf einem Medium, das Galactose als die einzige Kohlenstoffquelle enthält, und
d) Auswählen der Wirtszellen, die auf einem Medium wachsen können, das Galactose als die einzige Kohlenstoffquelle enthält.

## Revendications

1. Cellule hôte *Pichia pastoris* qui a été synthétisée par génie génétique pour exprimer une activité de galactokinase, une activité de UDP-galactose-4-épimérase, une activité de galactose-1-phosphate uridyltransférase et optionnellement une activité de galactose perméase, où la cellule hôte est capable d'utiliser du galactose comme seule source d'énergie de carbone.

2. Cellule hôte selon la revendication 1, où la cellule hôte a été en outre synthétisée pour être capable de produire des glycoprotéines recombinantes qui possèdent des N-glycanes hybrides ou complexes qui comprennent des résidus de galactose.

3. Cellule hôte selon la revendication 2, dans laquelle l'activité de UDP-galactose-4-épimérase est apportée dans une protéine de fusion comprenant le domaine catalytique d'une galactosyltransférase et le domaine catalytique d'une UDP-galactose-4-épimérase.

4. Cellule hôte selon la revendication 2, où la cellule hôte produit des glycoprotéines qui possèdent des N-glycanes complexes dans lesquels le rapport G0:G1/G2 est inférieur à 2:1.

5. Cellule hôte selon la revendication 2, où la cellule hôte produit des glycoprotéines possédant d'une manière prédominante un N-glycane choisi dans le groupe constitué de GalGlcNAcMan₅GlcNAc₂; NANAGalGlcNAcMan₅GlcNAc₂, GalGlcNAcMan₃GlcNAc₂; NANAGalGlcNAcMan₃GlcNAc₂; GalGlcNAc₂Man₃GlcNAc₂; Gal₂GlcNAc₂Man₃GlcNAc₂; NANAGal₂GlcNAc₂Man₃GlcNAc₂; et NANA₂Gal₂GlcNAc₂Man₃GlcNAc₂.

6. Cellule hôte selon la revendication 2, dans laquelle le N-glycane est un N-glycane terminé par un galactose choisi dans le groupe constitué de GalGlcNAcMan₅GlcNAc₂; Gal₂GlcNAc₂Man₃GlcNAc₂; et Gal₂GlcNAc₂Man₃GlcNAc₂.

7. Cellule hôte selon la revendication 2, dans laquelle le N-glycane est un N-glycane hybride terminé par un galactose.

8. Cellule hôte selon la revendication 2, dans laquelle le N-glycane est un N-glycane sialylé choisi dans le groupe constitué de NANAGalGlcNAcMan₅GlcNAc₂, NANAGal₂GlcNAc₂Man₃GlcNAc₂; et NANA₂Gal₂GlcNAc₂Man₃GlcAc₂.

9. Cellule hôte selon la revendication 2, dans laquelle la glycoprotéine recombinante est choisie dans le groupe constitué de: érythropoïétine (EPO); cytokines telles que l'interféron α, l'interféron β, l'interféron γ et l'interféron ω; et facteur de stimulation de colonies de granulocytes (GCSF); GM-CSF; facteurs de coagulation tels que le facteur VIII, le facteur IX et la protéine C humaine; anti-thrombine III; thrombine; chaîne α de récepteur de IgE soluble; immunoglobulines telles que IgG, des fragments de IgG, des fusions de IgG et IgM; immunoadhésines et autres protéines de fusion Fc telles que des protéines de fusion récepteur de TNF soluble-Fc; protéines de fusion RAGE-Fc; interleukines; urokinase; chymase; et inhibiteur de trypsine d'urée; protéine de liaison de IGF; facteur de croissance épidermique; facteur de libération d'hormone de croissance; protéine de fusion d'annexine V; angiostatine; facteur-2 de croissance endothéliale vasculaire; facteur-1 inhibiteur de progéniteur myéloïde; ostéoprotégérine; α-1-antitrypsine; protéines α-féto; ADN-ase II; kringle 3 de plasminogène humain; glucocérébrosidase; protéine de liaison de TNF; hormone folliculostimulante; antigène 4-Ig associé aux lymphocytes T cytotoxiques; activateur transmembranaire et modulateur de calcium et ligand de cyclophiline; protéine 1 de type glucagon; et agoniste de récepteur IL-2.

10. Méthode pour la production d'une glycoprotéine recombinante dans un hôte *Pichia pastoris* avec des N-glycanes qui possèdent des résidus de galactose, ladite méthode comprenant:
a) la fourniture d'une cellule hôte recombinante qui a été synthétisée par génie génétique pour exprimer:
(i) une voie de glycosylation qui rend la cellule hôte capable de produire des glycoprotéines recombinantes qui possèdent des N-glycanes hybrides ou complexes qui comprennent des résidus de galactose;
(ii) une activité de galactokinase, une activité de UDP-galactose-4-épimérase, une activité de galactose-1-phosphate uridyltransférase et optionnellement une activité de galactose perméase; et
(iii) une glycoprotéine recombinante; et
b) la culture des cellules hôtes dans un milieu contenant du galactose pour produire la glycoprotéine recombinante qui possède un ou plusieurs N-glycanes qui comprennent des résidus de galactose.

11. Méthode selon la revendication 10, dans laquelle l'activité de UDP-galactose-4-épimérase est apportée dans une protéine de fusion comprenant le domaine catalytique d'une galactosyltransférase et le domaine catalytique d'une UDP-galactose-4-épimérase.

12. Méthode selon la revendication 10, dans laquelle le rapport G0:G1/G2des N-glycanes est inférieur à 2:1.

13. Méthode selon la revendication 10, dans laquelle la glycoprotéine recombinante possède d'une manière prédominante un N-glycane choisi dans le groupe constitué de GalGlcNAcMan₅GlcNAc₂; NANAGalGlcNAcMan₅GlcNAc₂, GalGlcNAcMan₃GlcNAc₂; NANAGalGlcNAcMan₃GlcNAc₂; GalGlcNAc₂Man₃GlcNAc₂; Gal₂GlcNAc₂Man₃GlcNAc₂; NANAGal₂GlcNAc₂Man₃GlcNAc₂; et NANA₂Gal₂GlcNAc₂Man₃GlcNAc₂.

14. Méthode selon la revendication 10, dans laquelle le N-glycane est un N-glycane terminé par un galactose choisi dans le groupe constitué de GalGlcNAcMan₅GlcNAc₂; Gal₂GlcNAc₂Man₃GlcNAc₂; et Gal₂GlcNAc₂Man₃GlcNAc₂.

15. Méthode selon la revendication 10, dans laquelle le N-glycane est un N-glycane hybride terminé par un galactose.

16. Méthode selon la revendication 10, dans laquelle le N-glycane est un N-glycane sialylé choisi dans le groupe constitué de NANAGalGlcNAcMan₅GlcNAc₂, NANAGal₂GlcNAc₂Man₃GlcNAc₂; et NANA₂Gal₂GlcNAc₂Man₃GlcAc₂.

17. Méthode selon la revendication 10, dans laquelle la glycoprotéine recombinante est choisie dans le groupe constitué de: érythropoïétine (EPO); cytokines telles que l'interféron α, l'interféron β, l'interféron γ et l'interféron ω; et facteur de stimulation de colonies de granulocytes (GCSF); GM-CSF; facteurs de coagulation tels que le facteur VIII, le facteur IX et la protéine C humaine; anti-thrombine III; thrombine; chaîne α de récepteur de IgE soluble; immunoglobulines telles que IgG, des fragments de IgG, des fusions de IgG et IgM; immunoadhésines et autres protéines de fusion Fc telles que des protéines de fusion récepteur de TNF soluble-Fc; protéines de fusion RAGE-Fc; interleukines; urokinase; chymase; et inhibiteur de trypsine d'urée; protéine de liaison de IGF; facteur de croissance épidermique; facteur de libération d'hormone de croissance; protéine de fusion d'annexine V; angiostatine; facteur-2 de croissance endothéliale vasculaire; facteur-1 inhibiteur de progéniteur myéloïde; ostéoprotégérine; α-1-antitrypsine; protéines α-féto; ADN-ase II; kringle 3 de plasminogène humain; glucocérébrosidase; protéine de liaison de TNF; hormone folliculostimulante; antigène 4-Ig associé aux lymphocytes T cytotoxiques; activateur transmembranaire et modulateur de calcium et ligand de cyclophiline; protéine 1 de type glucagon; et agoniste de récepteur IL-2.

18. Méthode pour la production d'une cellule hôte *Pichia pastoris* recombinante qui exprime une protéine hétérologue, comprenant:
(a) la fourniture d'une cellule hôte qui a été synthétisée par génie génétique pour exprimer une ou deux activités enzymatiques choisies dans le groupe constitué d'une activité de galactokinase, d'une activité de UDP-galactose-4-épimérase et d'une activité de galactose-1-phosphate uridyltransférase;
(b) la transformation de la cellule hôte avec une ou plusieurs molécules d'acide nucléique codant pour la protéine hétérologue; et avec une ou plusieurs molécules d'acide nucléique codant pour l'enzyme ou les enzymes issues du groupe dans l'étape (a) qui ne sont pas exprimées dans la cellule hôte de l'étape (a) de sorte que la totalité des trois enzymes issues du groupe dans l'étape (a) sont présentes dans la cellule hôte; et
(c) la culture des cellules hôtes dans un milieu contenant du galactose comme seule source de carbone pour donner la cellule hôte *Pichia pastoris* recombinante qui exprime une protéine hétérologue.

19. Méthode selon la revendication 18, dans laquelle la cellule hôte est en outre synthétisée par génie génétique pour exprimer une galactose perméase.

20. Méthode selon la revendication 18, dans laquelle la cellule hôte est modifiée génétiquement pour produire des glycoprotéines qui possèdent un ou plusieurs N-glycanes qui comprennent du galactose.

21. Méthode pour la production et la sélection de cellules hôtes *Pichia pastoris* capables d'utiliser le galactose comme seule source de carbone, la méthode comprenant:
a) la fourniture d'une cellule hôte *Pichia pastoris;*
b) la transformation de la cellule hôte avec une ou plusieurs molécules d'acide nucléique codant pour une activité de galactokinase, une activité de UDP-galactose-4-épimérase, une activité de galactose-1-phosphate uridyltransférase et optionnellement une galactose perméase;
c) la culture des cellules hôtes transformées dans un milieu contenant du galactose comme seule source de carbone, et
d) la sélection des cellules hôtes qui peuvent croître dans le milieu contenant du galactose comme seule source de carbone.
